(19) Europäisches Patentamt
European Patent Office
Office européen des brevets

(11) EP 1 593 673 A1

(12) **EUROPEAN PATENT APPLICATION**
published in accordance with Art. 158(3) EPC

(43) Date of publication:
**09.11.2005 Bulletin 2005/45**

(21) Application number: **04708471.0**

(22) Date of filing: **05.02.2004**

(51) Int Cl.[7]: **C07D 233/64**, C07D 401/12, C07D 401/14, C07D 403/12, C07D 491/20, A61K 31/4164, A61K 31/4178, A61K 31/4439

(86) International application number:
**PCT/JP2004/001181**

(87) International publication number:
**WO 2004/069808 (19.08.2004 Gazette 2004/34)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **07.02.2003 JP 2003031068**

(71) Applicant: **Takeda Pharmaceutical Company Limited**
**Osaka-shi, Osaka 541-0045 (JP)**

(72) Inventors:
- **SHIRAISHI, M.**
  **c/o Takeda Pharmaceutical Comp. Ltd.**
  **Osaka-shi, Osaka 532-8686 (JP)**
- **SETO, M. c/o Takeda pharmaceutical Comp. Ltd.**
  **Osaka-shi, Osaka 532-8686 (JP)**
- **AIKAWA, K.**
  **c/o Takeda Pharmaceutical Comp. Ltd.**
  **Osaka-shi, Osaka 532-8686 (JP)**
- **KANZAKI, N.**
  **c/o Takeda Pharmaceutical Comp. Ltd.**
  **Osaka-shi, Osaka 532-8686 (JP)**
- **BABA, Masanori**
  **Kagoshima-shi, Kagoshima 8910103 (JP)**

(74) Representative: **Lewin, John Harvey**
**Takeda Euro IP Department,**
**11-12 Charles II Street**
**London SW1Y 4QU (GB)**

(54) **ACRYLAMIDE DERIVATIVE, PROCESS FOR PRODUCING THE SAME, AND USE**

(57) A compound represented by the formula:

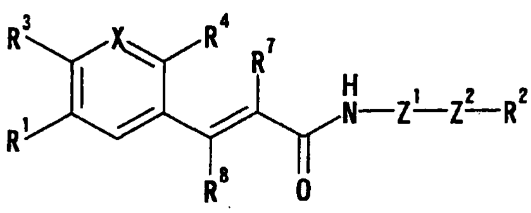

wherein $R^1$ is a 5- or 6-membered ring; $R^3$ is a hydrogen atom, a lower alkyl group or a lower alkoxy group; $R^7$ and $R^8$ are each a hydrogen atom or a lower alkyl group; $Z^1$ is another 5- or 6-membered aromatic ring; $Z^2$ is a group represented by $-Z^{2a}-W^1-Z^{2b}-$ [wherein $Z^{2a}$ and $Z^{2b}$ are each O, $S(O)_m$ (wherein m is 0, 1 or 2), an imino group or a bond, and $W^1$ is an alkylene chain]; X is CR (wherein R is a hydrogen atom, a lower alkyl group, a lower alkoxy group, an acyl group, or R and adjacent $R^4$ may form a 5- or 6-membered alicyclic heterocyclic group) or N; $R^4$ is $NR^5R^6$ (wherein $R^5$ and $R^6$ are each a hydrogen atom, a hydrocarbon group, a heterocyclic group or an acyl group), or $R^5$ and $R^6$ are bonded to each other to form a heterocyclic group of $NR^5R^6$; and $R^2$ is (1) an amino group which may be a quaternary ammonium or oxide, (2) a nitrogen-containing heterocyclic group which may contain a sulfur atom or an oxygen atom as the ring-constituting atom, in which the nitrogen atom may be converted to a quaternary ammonium or an oxide, or the like; or a salt thereof.

The compound has excellent CCR5 antagonistic activity and thus is useful as a prophylactic and/or therapeutic medicine for HIV infection into human peripheral blood monocyte, especially for AIDS.

EP 1 593 673 A1

## Description

Technical Field

The present invention relates to a new cyclic compound having CCR antagonist activity, especially CCR5 antagonist activity, and to use thereof.

Background Art

Recently, HIV (human immunodeficiency virus) protease inhibitors have been developed for treatment of AIDS (acquired immune deficiency syndrome). With combined use of the protease inhibitors with two HIV reverse transcriptase inhibitors which have been commonly used, treatment of AIDS has made remarkable progress. However, the treatment is still not efficient enough for the eradication of AIDS, and development of a new anti-AIDS medicine based on a different mechanism of action is desired.

As a receptor upon invasion of HIV into a target cell, CD4 has already been known. Recently, CCR5 as a second receptor of macrophage directed HIV, and CXCR4 as a second receptor of T cell directed HIV, which are G-protein coupled chemokine receptors having a seven-transmembrane protein structure, have been found, and these chemokine receptors are considered to play an essential role for infection and transmission of HIV. As a matter of fact, it has been reported that a man having resistance to HIV infection even after repeated exposures to the virus had a mutation in which CCR5 gene was deleted homozygously. Thus, the CCR5 antagonists have been expected to become a new anti-HIV medicine, and examples of synthesis of new anilide derivatives having CCR5 antagonist activity have been reported in the below-mentioned patent applications such as Patent Document 1, Patent Document 2 and Patent Document 3, while there has been no report of a CCR5 antagonist which has been commercialized as a therapeutic medicine for AIDS. Further, a compound having CCR5 antagonist activity is described to be useful as a prophylactic and/or therapeutic medicine for AIDS in the below-mentioned Patent Document 4, but said compound has a different structure from the compound of the present invention.

Patent Document 1: WO99/32100
Patent Document 2: Japanese Patent Application No. 10-234388
Patent Document 3: Japanese Patent Application No. 10-363404
Patent Document 4: JP-A No. 2001-026586

Disclosure of the Invention

The present invention is to provide a new bicyclic compound that is useful for preventing and treating HIV infection, especially AIDS, due to its CCR antagonist activity, especially CCR5 antagonist activity.

The present inventors have intensively studied compounds having CCR5 antagonist activity and found that a compound of the following formula (I) or a salt thereof (hereinafter, sometimes referred to as Compound (I)) has a clinically favorable pharmacological effect including CCR antagonist activity, especially excellent CCR5 antagonist activity, thereby completing the invention.

Thus, the invention provides:

[1] a compound represented by the formula:

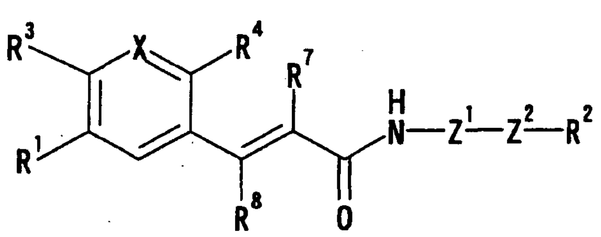

(I)

wherein $R^1$ is a 5- or 6-membered ring which may be substituted;

$R^3$ is a hydrogen atom, a lower alkyl group which may be substituted or a lower alkoxy group which may be substituted;

$R^7$ and $R^8$ are each a hydrogen atom or a lower alkyl group which may be substituted;

$Z^1$ is a 5- or 6-membered aromatic ring which may be further substituted;

$Z^2$ is a group represented by $-Z^{2a}-W^1-Z^{2b}-$, wherein $Z^{2a}$ and $Z^{2b}$ are each O, $S(O)_m$ (wherein m is 0, 1 or 2), an imino group which may be substituted, or a bond, and $W^1$ is an alkylene chain which may be substituted, an alkenylene chain which may be substituted, or a bond;

X is N or CR, wherein R represents a hydrogen atom, a lower alkyl group which may be substituted, a lower alkoxy group which may be substituted or an acyl group which may be substituted, or R and the adjacent $R^4$ may form a 5- or 6-membered alicyclic heterocyclic group;

$R^4$ is $NR^5R^6$, wherein $R^5$ and $R^6$ each represent a hydrogen atom, a hydrocarbon group which may be substituted, a heterocyclic group which may be substituted or an acyl group which may be substituted, or $R^5$ and $R^6$ are bonded to each other to form a heterocyclic group which may be substituted represented by $NR^5R^6$; and

$R^2$ is (1) an amino group which may be substituted, in which the nitrogen atom may be converted to a quaternary ammonium or an oxide, (2) a nitrogen-containing heterocyclic group which may be substituted and may contain a sulfur atom or an oxygen atom as the ring-constituting atom, in which the nitrogen atom may be converted to a quaternary ammonium or an oxide, (3) a group represented by the formula:

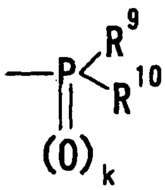

(a)

wherein k represents 0 or 1, and when k is 0, the phosphorus atom may form a phosphonium salt; $R^9$ and $R^{10}$ are each a hydrocarbon group which may be substituted, a hydroxy group which may be substituted or an amino group which may be substituted; or $R^9$ and $R^{10}$ may be bonded to each other to form a cyclic group with the adjacent phosphorus atom, (4) an amidino group which may be substituted, or (5) a guanidino group which may be substituted; or a salt thereof;

[2] a prodrug of the compound according to the above [1] ;

[3] the compound according to the above [1], wherein $R^1$ is a benzene, a furan, a thiophene, a pyridine, a cyclopentane, a cyclohexane, a pyrrolidine, a piperidine, a piperazine, a morpholine, a thiomorpholine or a tetrahydropyran, each of which may be substituted;

[4] the compound according to the above [1], wherein $R^1$ is a benzene which may be substituted;

[5] the compound according to the above [1], wherein $NR^5R^6$ is a heterocyclic group which may be substituted;

[6] the compound according to the above [1], wherein $Z^1$ is a benzene which may be substituted with a substituent selected from (1) a halogen atom, (2) a $C_{1-4}$ alkyl group which may be substituted with halogen atom(s), and (3) a $C_{1-4}$ alkoxy group which may be substituted with a halogen atom;

[7] the compound according to the above [1], wherein $Z^1$ is a benzene which may be substituted with a methyl group or a trifluoromethyl group;

[8] the compound according to the above [1], wherein $Z^2$ is a group represented by $-Z^{2a}-W^2-Z^{2b}-$, wherein $Z^{2a}$ and $Z^{2b}$ are each O, $S(O)_m$ (wherein m is 0, 1 or 2), an imino group which may be substituted, or a bond, and $W^2$ is an alkylene chain which may be substituted;

[9] the compound according to the above [1], wherein $Z^2$ is a group represented by $-CH_2-$, $-CH(OH)-$ or $-S(O)_m-CH_2-$ (wherein m is 0, 1 or 2) ;

[10] the compound according to the above [1], wherein $Z^2$ is a group represented by $-S(O)_m-CH_2-$ (wherein m is 0, 1 or 2) ;

[11] the compound according to the above [1], wherein $R^2$ is (1) an amino group which may be substituted, in which the nitrogen atom may be converted to a quaternary ammonium or an oxide, (2) a nitrogen-containing heterocyclic group which may be substituted and may contain a sulfur atom or an oxygen atom as the ring-constituting atom, in which the nitrogen atom may be converted to a quaternary ammonium or an oxide, (3) an amidino group which may be substituted, or (4) a guanidino group which may be substituted;

[12] the compound according to the above [1], wherein $R^2$ is an amino group which may be substituted, or a nitrogen-containing heterocyclic group which may be substituted and may contain a sulfur atom or an oxygen atom as the ring-constituting atom;

[13] the compound according to the above [1], wherein $R^2$ is -NRR', wherein R and R' are each an aliphatic hydrocarbon group which may be substituted or an alicyclic heterocyclic group which may be substituted;

[14] the compound according to the above [1], wherein $R^2$ is a nitrogen-containing aromatic heterocyclic group

which may be substituted;

[15] the compound according to the above [1], wherein $R^2$ is an imidazolyl group which may be substituted or a triazolyl group which may be substituted;

[16] the compound according to the above [1], wherein $R^1$ is a benzene, a furan, a thiophene, a pyridine, a cyclopentane, a cyclohexane, a pyrrolidine, a piperidine, a piperazine, a morpholine, a thiomorpholine or a tetrahydropyran, each of which may be substituted with a halogen, a nitro, a cyano, a $C_{1-6}$ alkyl, a $C_{1-6}$ alkoxy, a $C_{1-6}$ an alkoxy-$C_{1-6}$ alkyl or a $C_{1-6}$ alkoxy-$C_{1-6}$ alkoxy;

$Z^1$ is benzene which may be substituted with a substituent selected from (1) a halogen atom, (2) a $C_{1-4}$ alkyl group which may be substituted with a halogen atom, and (3) a $C_{1-4}$ alkoxy group which may be substituted with a halogen atom;

$Z^2$ is $-Z^{2a}-W^1-Z^{2b}-$, wherein $Z^{2a}$ and $Z^{2b}$ are each O, $S(O)_m$ (wherein m is 0, 1 or 2), an imino group which may be substituted with a $C_{1-4}$ alkyl group, or a bond, and $W^1$ is a bond, or a $C_{1-4}$ alkylene chain or a $C_{2-4}$ alkenylene chain, each of which may be substituted with a $C_{1-6}$ alkyl, a hydroxy group, a hydroxyimino or a $C_{1-6}$ alkoxyimino; and

$R^2$ is an amino group which may be substituted with a $C_{1-4}$ alkyl group, or a nitrogen-containing heterocyclic group which may contain a sulfur atom or an oxygen atom as the ring-constituting atom and may be substituted with a $C_{1-4}$ alkyl group;

[17] a compound represented by the formula:

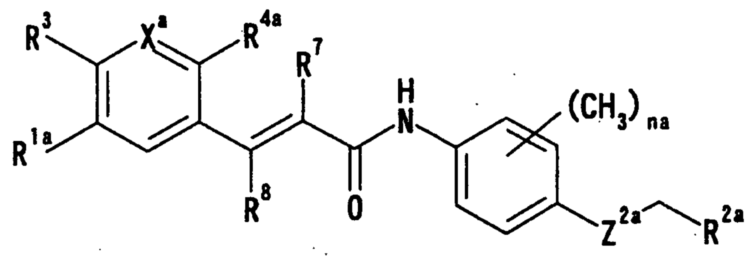
(1a)

wherein $R^{1a}$ is a ($C_{1-6}$ alkoxy-$C_{1-6}$ alkoxy) phenyl;

$R^{2a}$ is (1) an N-$C_{1-6}$ alkyl-N-tetrahydropyranylamino, (2) an imidazolyl which may be substituted with a $C_{1-6}$ alkyl which may be substituted, or (3) a triazolyl which may be substituted with a $C_{1-6}$ alkyl which may be substituted;

$R^{4a}$ is $NR^{5a}R^{6a}$, wherein $R^{5a}$ and $R^{6a}$ are bonded to each other to form a heterocyclic group which may be substituted represented by $NR^{5a}R^{6a}$;

$X^a$ is CH or N;

na is 0 or 1;

$Z^{2a}$ is a bond, S, SO or $SO_2$; and

the other symbols have the same meanings as defined above;

or a salt thereof;

[18] the compound according to the above [17], wherein $Z^{2a}$ is SO;

[19] the compound according to the above [18], wherein $Z^{2a}$ is SO having a configuration of (S);

[20] the compound according to the above [17], wherein $R^{4a}$ is a 1-pyrrolidinyl group which may be substituted;

[21] (Ss)-(2E)-3-[4'-(2-butoxyethoxy)-4-(3-methylpyrrolidin-1-yl)-1,1'-biphenyl-3-yl]-2-methyl-N-[4-[[(1-propyl-1H-imidazol-5-yl)methyl]sulfinyl]phenyl]acrylamide, (Ss)-(2E)-3-[4'-(2-butoxyethoxy)-4-[3-(hydroxymethyl)pyrrolidin-1-yl]-1,1'-biphenyl-3-yl]-2-methyl-N-[4-[[(1-propyl-1H-imidazol-5-yl)methyl] sulfinyl]phenyl] acrylamide, (Ss)-(2E)-3-[4'-(2-butoxyethoxy)-4-(3-carboxypyrrolidin-1-yl)-1,1'-biphenyl-3-yl]-2-methyl-N-[4-[[(1-propyl-1H-imidazol)-5-yl]methyl]sulfinyl]phenyl]acrylamide and diastereomers thereof;

[22] (Ss)-(2E)-3-[5-[4-(2-butoxyethoxy)phenyl]-2-[3-(hydroxymethyl)pyrrolidin-1-yl]pyridin-3-yl]-2-methyl-N-[4-[[(1-propyl-1H-imidazol-5-yl)methyl]sulfinyl]phenyl]acrylamide and a diastereomer thereof, and (S)-(2E)-3-[5-[4-(2-butoxyethoxy)phenyl]-2-pyrrolidin-1-ylpyridin-3-yl]-2-methyl-N-[4-[[(1-propyl-1H-imidzol-5-yl)methyl] sulfinyl]phenyl]acrylamide;

[23] a process for producing a compound represented by the formula:

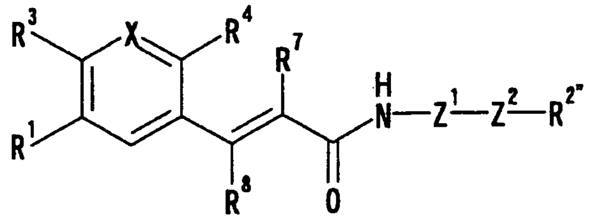

(Ib)

wherein $R^{2''}$ is (1) an amino group which may be substituted, in which the nitrogen atom may be converted to a quaternary ammonium, (2) a nitrogen-containing heterocyclic group which may be substituted and may contain a sulfur atom or an oxygen atom as the ring-constituting atom, in which the nitrogen atom may be converted to a quaternary ammonium, or (3) a group represented by formula (Ia), and the other symbols have the same meanings as defined above,
or a salt thereof, which comprises subjecting a compound represented by the formula:

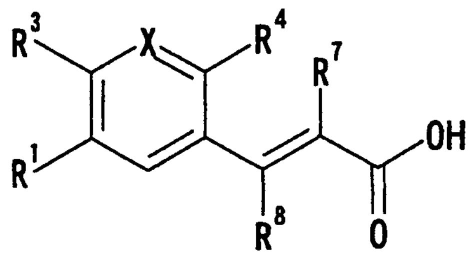

(II)

wherein each symbol has the same meaning as defined above, a salt or a reactive derivative thereof, and a compound represented by the formula:

$$H_2N—Z^1—Z^2—R^{2''} \qquad (III)$$

wherein each symbol has the same meaning as defined above, or a salt thereof to a condensation reaction, and then optionally to deprotection, oxidation-reduction and/or quaternization reaction;
[24] a pharmaceutical composition comprising the compound represented by formula (I), a salt or prodrug thereof;
[25] the pharmaceutical composition according to the above [24], which is a CCR antagonist;
[26] the pharmaceutical composition according to the above [25], wherein CCR is CCR5 and/or CCR2;
[27] the pharmaceutical composition according to the above [25], wherein CCR is CCR5;
[28] the pharmaceutical composition according to the above [24], which is a prophylactic and/or therapeutic agent for HIV infection, chronic rheumatoid arthritis, autoimmune diseases, allergic diseases, ischemic brain cell disorder, cardiac infarction, nephritis/nephropathy or arteriosclerosis;
[29] the pharmaceutical composition according to the above [24], which is a prophylactic and/or therapeutic agent for HIV infection;
[30] the pharmaceutical composition according to the above [24], which is a prophylactic and/or therapeutic agent for AIDS;
[31] the pharmaceutical composition according to the above [24], which is a suppressive agent for disease progression of AIDS;
[32] a method for preventing or treating HIV infection, chronic rheumatoid arthritis, autoimmune diseases, allergic diseases, ischemic brain cell disorder, cardiac infarction, nephritis/nephropathy, arteriosclerosis or graft-versus-host diseases, which comprises administering an effective amount of the compound according to the above [1], a salt or prodrug thereof to a subject in need thereof; and
[33] use of the compound according to the above [1], a salt or prodrug thereof, for the manufacture of a prophylactic and/or therapeutic agent for HIV infection, chronic rheumatoid arthritis, autoimmune diseases, allergic diseases, ischemic brain cell disorder, cardiac infarction, nephritis/nephropathy, arteriosclerosis or graft-versus-host diseases.

Best Mode for Carrying Out the Invention

In the above-described formula (I), the "5- or 6-membered ring" in the "5- or 6-membered ring group which may be substituted" represented by $R^1$ may be exemplified by a group which is formed by eliminating a hydrogen atom from 6-membered aromatic hydrocarbon such as benzene, etc.; 5- or 6-membered aliphatic hydrocarbon such as cyclopentane, cyclohexane, cyclopentene, cyclohexene, cyclopentanediene, cyclohexanediene, etc.; 5- or 6-membered aromatic heterocycle containing 1 to 4 heteroatoms of one or two kinds selected from nitrogen, sulfur and oxygen atoms, such as furan, thiophene, pyrrole, imidazole, pyrazole, thiazole, oxazole, isothiazole, isoxazole, tetrazole, pyridine, pyrazine, pyrimidine, pyridazine, triazole, etc.; 5- or 6-membered non-aromatic heterocycle containing 1 to 4 heteroatoms of one or two kinds selected from nitrogen, sulfur and oxygen atoms, such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran, etc.; or the like. Among them, the "5- or 6-membered ring" is preferably benzene, furan, thiophene, pyridine, cyclopentane, cyclohexane, pyrrolidine, piperidine, piperazine, morpholine, thiomorpholine, tetrahydropyran (preferably, 6-membered ring), or the like, it being particularly preferably benzene.

The substituent which may be carried by the "5- or 6-membered ring" of the "5- or 6-membered ring group which may be substituted" represented by $R^1$ may be exemplified by halogen atom, nitro, cyano, alkyl which may be substituted, cycloalkyl which may be substituted, hydroxy group which may be substituted, thiol group which may be substituted (wherein the sulfur atom may be oxidized, and may form sulfinyl which may be substituted or sulfonyl which may be substituted), amino group which may be substituted, acyl which may be substituted, carboxyl group which may be esterified, an aromatic group which may be substituted, or the like.

Examples of the halogen as the substituent of $R^1$ include fluorine, chlorine, bromine, iodine and the like, it being preferably fluorine and chlorine.

The alkyl of the "alkyl which may be substituted" as the substituent of $R^1$ may be exemplified by linear or branched alkyl having 1 to 10 carbon atoms, for example, $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower ($C_{1-6}$) alkyl. Examples of the substituent of the "alkyl which may be substituted" include halogen (for example, fluorine, chlorine, bromine, iodine, etc.), nitro, cyano, hydroxy group, thiol group which may be substituted (for example, thiol group, $C_{1-4}$ alkylthio, etc.), amino group which may be substituted (for example, amino, mono-$C_{1-4}$ alkylamino, di-$C_{1-4}$ alkylamino, 5- or 6-membered cycloamino such as tetrahydropyrrole, piperazine, piperidine, morpholine, thiomorpholine, pyrrole, imidazole, etc.), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), $C_{1-4}$ alkoxy which may be halogenated (for example, methoxy, ethoxy, propoxy, butoxy, trifluoromethoxy, trifluoroethoxy, etc.), $C_{1-4}$ alkoxy-$C_{1-4}$ alkoxy which may be halogenated (for example, methoxymethoxy, methoxyethoxy, ethoxyethoxy, trifluoromethoxyethoxy, trifluoroethoxyethoxy, etc.), formyl, $C_{2-4}$ alkanoyl (for example, acetyl, propionyl, etc.), $C_{1-4}$ alkylsulfonyl (for example, methanesulfonyl, ethanesulfonyl, etc.), and the like, and the number of the substituents is preferably 1 to 3.

Examples of the cycloalkyl of the "cycloalkyl which may be substituted" as the substituent of $R^1$ include $C_{3-7}$ cycloalkyl such as cyclopropyl, cyclobutyl, cyclopentyl, cyclohexyl, cycloheptyl, etc. Examples of the substituent in the "cycloalkyl which may be substituted" include halogen (for example, fluorine, chlorine, bromine, iodine, etc.), nitro, cyano, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- or 6-membered cycloamino such as tetrahydropyrrole, piperazine, piperidine, morpholine, thiomorpholine, pyrrole, imidazole, etc.), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), $C_{1-4}$ alkoxy which may be halogenated (for example, methoxy, ethoxy, propoxy, butoxy, trifluoromethoxy, trifluoroethoxy, etc.), $C_{1-4}$ alkoxy-$C_{1-4}$ alkoxy which may be halogenated (for example, methoxymethoxy, methoxyethoxy, ethoxyethoxy, trifluoromethoxyethoxy, trifluoroethoxyethoxy, etc.), formyl, $C_{2-4}$ alkanoyl (for example, acetyl, propionyl, etc.), $C_{1-4}$ alkylsulfonyl (for example, methanesulfonyl, ethanesulfonyl, etc.), and the like, and the number of the substituents is preferably 1 to 3.

The substituent of the "hydroxy group which may be substituted" as the substituent of $R^1$ may be exemplified by

(1) alkyl which may be substituted (for example, $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower ($C_{1-6}$) alkyl; or the like);
(2) cycloalkyl which may be substituted and may contain heteroatom(s) (for example, $C_{3-7}$ cycloalkyl such as cyclopropyl, cyclobutyl, cyclopentyl, cyclohexyl, cycloheptyl, etc.; 5- or 6-membered saturated heterocyclic group containing 1 or 2 heteroatoms, such as tetrahydrofuranyl, tetrahydrothienyl, pyrrolidinyl, pyrazolidinyl, piperidinyl,

piperazinyl, morpholinyl, thiomorpholinyl, tetrahydropyranyl, tetrahydrothiopyranyl, etc., and preferably tetrahydropyranyl, etc.; or the like);

(3) alkenyl which may be substituted (for example, alkenyl having 2 to 10 carbon atoms, such as allyl, crotyl, 2-pentenyl, 3-hexenyl, etc., and preferably lower ($C_{2-6}$) alkenyl; or the like);

(4) cycloalkenyl which may be substituted (for example, cycloalkenyl having 3 to 7 carbon atoms, such as 2-cyclopentenyl, 2-cyclohexenyl, 2-cyclopentenylmethyl, 2-cyclohexenylmethyl, etc.; or the like);

(5) aralkyl which may be substituted (for example, phenyl-$C_{1-4}$ alkyl such as benzyl, phenethyl, etc.; or the like) ;

(6) formyl, or acyl which may be substituted (for example, alkanoyl having 2 to 4 carbon atoms, such as acetyl, propionyl, butyryl, isobutyryl, etc.), alkylsulfonyl having 1 to 4 carbon atoms (for example, methanesulfonyl, ethanesulfonyl, etc.), or the like);

(7) aryl which may be substituted (for example, phenyl, naphthyl, etc.); or the like.

The substituent of the above-described (1) alkyl which may be substituted, (2) cycloalkyl which may be substituted, (3) alkenyl which may be substituted, (4) cycloalkenyl which may be substituted, (5) aralkyl which may be substituted, (6) acyl which may be substituted, and (7) aryl which may be substituted, may be exemplified by halogen (for example, fluorine, chlorine, bromine, iodine, etc.), nitro, cyano, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- or 6-membered cycloamino such as tetrahydropyrrole, piperazine, piperidine, morpholine, thiomorpholine, pyrrole, imidazole, etc.), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), $C_{1-4}$ alkyl which may be halogenated (for example, trifluoromethyl, methyl, ethyl, etc.), $C_{1-6}$ alkoxy which may be halogenated (for example, methoxy, ethoxy, propoxy, butoxy, trifluoromethoxy, trifluoroethoxy, etc.; preferably $C_{1-4}$ alkoxy which may be halogenated), formyl, $C_{2-4}$ alkanoyl (for example, acetyl, propionyl, etc.), $C_{1-4}$ alkylsulfonyl (for example, methanesulfonyl, ethanesulfonyl, etc.), 5- or 6-membered aromatic heterocycle which may be substituted [for example, 5- or 6-membered aromatic heterocycle containing 1 to 4 heteroatoms of one or two kinds selected from nitrogen, sulfur and oxygen atoms, such as furan, thiophene, pyrrole, imidazole, pyrazole, thiazole, oxazole, isothiazole, isoxazole, tetrazole, pyridine, pyrazine, pyrimidine, pyridazine, triazole, etc.; examples of the substituent which may be carried by said heterocycle include halogen (for example, fluorine, chlorine, bromine, iodine, etc.), nitro, cyano, hydroxy group, thiol group, amino group, carboxyl group, $C_{1-4}$ alkyl which may be halogenated (for example, trifluoromethyl, methyl, ethyl, etc.), $C_{1-4}$ alkoxy which may be halogenated (for example, methoxy, ethoxy, propoxy, butoxy, trifluoromethoxy, trifluoroethoxy, etc.), formyl, $C_{2-4}$ alkanoyl (for example, acetyl, propionyl, etc.), $C_{1-4}$ alkylsulfonyl (for example, methanesulfonyl, ethanesulfonyl, etc.), and the like; and the number of the substituents is preferably 1 to 3], or the like; and the number of the substituents is preferably 1 to 3.

The substituent of the "thiol group which may be substituted" as the substituent of $R^1$ may be exemplified by the same one as the "substituent of hydroxy group which may be substituted as the substituent of $R^1$," and preferred among them are:

(1) alkyl which may be substituted (for example, $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower ($C_{1-6}$) alkyl; or the like);

(2) cycloalkyl which may be substituted (for example, $C_{3-7}$ cycloalkyl such as cyclopropyl, cyclobutyl, cyclopentyl, cyclohexyl, cycloheptyl, etc., or the like);

(3) aralkyl which may be substituted (for example, phenyl-$C_{1-4}$ alkyl such as benzyl, phenethyl, etc.);

(4) aryl which may be substituted (for example, phenyl, naphthyl, etc.); and the like.

The substituent which may be carried by the above-described (1) alkyl which may be substituted, (2) cycloalkyl which may be substituted, (3) aralkyl which may be substituted, and (4) aryl which may be substituted, may be exemplified by halogen (for example, fluorine, chlorine, bromine, iodine, etc.), nitro, cyano, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- or 6-membered cycloamino such as tetrahydropyrrole, piperazine, piperidine, morpholine, thiomorpholine, pyrrole, imidazole, etc.), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), $C_{1-4}$ alkoxy which may be halogenated (for example, methoxy, ethoxy, propoxy, butoxy, trifluoromethoxy, trifluoroethoxy, etc.), $C_{1-4}$ alkoxy-$C_{1-4}$ alkoxy which may be halogenated (for example, methoxymethoxy, methoxyethoxy, ethoxyethoxy, trifluoromethoxyethoxy, trifluoroethoxyethoxy, etc.), formyl, $C_{2-4}$ alkanoyl (for example, acetyl, propionyl, etc.), $C_{1-4}$ alkylsulfonyl (for example, methanesulfonyl, ethanesulfonyl, etc.), or the like, and the number of the substituents is preferably 1 to 3.

The substituent of the "amino group which may be substituted" as the substituent of $R^1$ may be exemplified

by the same one as the "substituent of hydroxy group which may be substituted as the substituent of $R^1$," and the number of substituents on the amino group may be 1 or 2. Among them, the substituent is preferably:

(1) alkyl which may be substituted (for example, $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower ($C_{1-10}$) alkyl, or the like) ;
(2) cycloalkyl which may be substituted (for example, $C_{3-7}$ cycloalkyl group such as cyclopropyl, cyclobutyl, cyclopentyl, cyclohexyl, cycloheptyl, etc., or the like);
(3) alkenyl which may be substituted (for example, alkenyl having 2 to 10 carbon atoms, such as allyl, crotyl, 2-pentenyl, 3-hexenyl, etc., and preferably lower ($C_{2-6}$) alkenyl, or the like);
(4) cycloalkenyl which may be substituted (for example, cycloalkenyl having 3 to 7 carbon atoms, such as 2-cyclopentenyl, 2-cyclohexenyl, 2-cyclopentenylmethyl, 2-cyclohexenylmethyl, etc.; or the like);
(5) formyl, or acyl which may be substituted (for example, alkanoyl having 2 to 4 carbon atoms (for example, acetyl, propionyl, butyryl, isobutyryl, etc.), alkylsulfonyl having 1 to 4 carbon atoms (for example, methanesulfonyl, ethanesulfonyl, etc.) or the like);
(6) aryl which may be substituted (for example, phenyl, naphthyl, etc.); and the like.

Examples of the substituent of the above-described (1) alkyl which may be substituted, (2) cycloalkyl which may be substituted, (3) alkenyl which may be substituted, (4) cycloalkenyl which may be substituted, (5) acyl which may be substituted, (6) aryl which may be substituted, include halogen (for example, fluorine, chlorine, bromine, iodine, etc.), nitro, cyano, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- or 6-membered cycloamino such as tetrahydropyrrole, piperazine, piperidine, morpholine, thiomorpholine, pyrrole, imidazole, etc.), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), $C_{1-4}$ alkoxy which may be halogenated (for example, methoxy, ethoxy, propoxy, butoxy, trifluoromethoxy, trifluoroethoxy, etc.), $C_{1-4}$ alkoxy-$C_{1-4}$ alkoxy which may be halogenated (for example, methoxymethoxy, methoxyethoxy, ethoxyethoxy, trifluoromethoxyethoxy, trifluoroethoxyethoxy, etc.), formyl, $C_{2-4}$ alkanoyl (for example, acetyl, propionyl, etc.), $C_{1-4}$ alkylsulfonyl (for example, methanesulfonyl, ethanesulfonyl, etc.), and the like, and the number of the substituents is preferably 1 to 3.

Further, the substituents of the "amino group which may be substituted" as the substituent of $R^1$ may be bonded to each other to form a cycloamino group (for example, a group which is formed by eliminating a hydrogen atom from the ring-constituting nitrogen atom of a 5- or 6-membered ring such as tetrahydropyrrole, piperazine, piperidine, morpholine, thiomorpholine, pyrrole, imidazole, etc. so that a bond is made available on the nitrogen atom, or the like). This cycloamino group may be substituted, and examples of the substituent include halogen (for example, fluorine, chlorine, bromine, iodine, etc.), nitro, cyano, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- or 6-membered cycloamino such as tetrahydropyrrole, piperazine, piperidine, morpholine, thiomorpholine, pyrrole, imidazole, etc.), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), $C_{1-4}$ alkoxy which may be halogenated (for example, methoxy, ethoxy, propoxy, butoxy, trifluoromethoxy, trifluoroethoxy, etc.), $C_{1-4}$ alkoxy-$C_{1-4}$ alkoxy which may halogenated (for example, methoxymethoxy, methoxyethoxy, ethoxyethoxy, trifluoromethoxyethoxy, trifluoroethoxyethoxy, etc.), formyl, $C_{2-4}$ alkanoyl (for example, acetyl, propionyl, etc.), $C_{1-4}$ alkylsulfonyl (for example, methanesulfonyl, ethanesulfonyl, etc.), and the like, while the number of the substituents is preferably 1 to 3.

The "acyl which may be substituted" as the substituent of $R^1$ may be exemplified by a group in which

(1) hydrogen;
(2) alkyl which may be substituted (for example, $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower ($C_{1-6}$) alkyl, or the like);
(3) cycloalkyl which may be substituted (for example, $C_{3-7}$ cycloalkyl such as cyclopropyl, cyclobutyl, cyclopentyl, cyclohexyl, cycloheptyl, etc., or the like);
(4) alkenyl which may be substituted (for example, alkenyl having 2 to 10 carbon atoms, such as allyl, crotyl, 2-pentenyl, 3-hexenyl, etc., and preferably lower ($C_{2-6}$) alkenyl, or the like);
(5) cycloalkenyl which may be substituted (for example, cycloalkenyl of 3 to 7 carbon atoms, such as 2-cyclopentenyl, 2-cyclohexenyl, 2-cyclopentenylmethyl, 2-cyclohexenylmethyl, etc., or the like);
(6) 5- or 6-membered monocyclic aromatic group which may be substituted (for example, phenyl, pyridyl, etc.) or the like;

is bonded to a carbonyl group or a sulfonyl group (for example, formyl, acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, methanesulfonyl, ethanesulfonyl, etc.). Examples of the substituent of the above-described (2) alkyl which may be substituted, (3) cycloalkyl which may be substituted, (4) alkenyl which may be substituted, (5) cycloalkenyl which may be substituted, and (6) 5- or 6-membered monocyclic aromatic group which may be substituted, include halogen (for example, fluorine, chlorine, bromine, iodine, etc.), nitro, cyano, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- or 6-membered cycloamino such as tetrahydropyrrole, piperazine, piperidine, morpholine, thiomorpholine, pyrrole, imidazole, etc.), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), $C_{1-4}$ alkoxy which may be halogenated (for example, methoxy, ethoxy, propoxy, butoxy, trifluoromethoxy, trifluoroethoxy, etc.), $C_{1-4}$ alkoxy-$C_{1-4}$ alkoxy which may be halogenated (for example, methoxymethoxy, methoxyethoxy, ethoxyethoxy, trifluoromethoxyethoxy, trifluoroethoxyethoxy, etc.), formyl, $C_{2-4}$ alkanoyl (for example, acetyl, propionyl, etc.), $C_{1-4}$ alkylsulfonyl (for example, methanesulfonyl, ethanesulfonyl, etc.), and the like, and the number of the substituents is preferably 1 to 3.

The "carboxyl which may be esterified" as the substituent of $R^1$ may be exemplified by a group in which (1) hydrogen;

(2) alkyl which may be substituted (for example, $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower ($C_{1-6}$) alkyl, or the like) ;

(3) cycloalkyl which may be substituted (for example, $C_{3-7}$ cycloalkyl such as cyclopropyl, cyclobutyl, cyclopentyl, cyclohexyl, cycloheptyl, etc., or the like);

(4) alkenyl which may be substituted (for example, alkenyl having 2 to 10 carbon atoms, such as allyl, crotyl, 2-pentenyl, 3-hexenyl, etc., and preferably lower ($C_{2-6}$) alkenyl, or the like);

(5) cycloalkenyl which may be substituted (for example, cycloalkenyl having 3 to 7 carbon atoms, such as 2-cyclopentenyl, 2-cyclohexenyl, 2-cyclopentenylmethyl, 2-cyclohexenylmethyl, etc.);

(6) aryl which may be substituted (for example, phenyl, naphthyl, etc.); or the like

is bonded to a carbonyloxy group, preferably carboxyl, lower ($C_{1-6}$) alkoxycarbonyl, aryloxycarbonyl (for example, methoxycarbonyl, ethoxycarbonyl, propoxycarbonyl, phenoxycarbonyl, naphthoxycarbonyl, etc.), or the like.

Examples of the substituent of the above-described (2) alkyl which may be substituted, (3) cycloalkyl which may be substituted, (4) alkenyl which may be substituted, (5) cycloalkenyl which may be substituted, and (6) aryl which may be substituted, include halogen (for example, fluorine, chlorine, bromine, iodine, etc.), nitro, cyano, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- or 6-membered cycloamino such as tetrahydropyrrole, piperazine, piperidine, morpholine, thiomorpholine, pyrrole, imidazole, etc.), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), $C_{1-4}$ alkoxy which may be halogenated (for example, methoxy, ethoxy, propoxy, butoxy, trifluoromethoxy, trifluoroethoxy, etc.), $C_{1-4}$ alkoxy-$C_{1-4}$ alkoxy which may halogenated (for example, methoxymethoxy, methoxyethoxy, ethoxyethoxy, trifluoromethoxyethoxy, trifluoroethoxyethoxy, etc.), formyl, $C_{2-4}$ alkanoyl (for example, acetyl, propionyl, etc.), $C_{1-4}$ alkylsulfonyl (for example, methanesulfonyl, ethanesulfonyl, etc.), and the like, and the number of the substituents is preferably 1 to 3.

The "aromatic group" of the "aromatic group which may be substituted" as the substituent of $R^1$ may be exemplified by 5- or 6-membered homocyclic or heterocyclic aromatic group such as phenyl, pyridyl, furyl, thienyl, pyrrolyl, imidazolyl, pyrazolyl, thiazolyl, oxazolyl, isothiazolyl, isoxazolyl, tetrazolyl, pyrazinyl, pyrimidinyl, pyridazinyl, triazolyl, etc.; fused heterocyclic aromatic group such as benzofuran, indole, benzothiophene, benzoxazole, benzothiazole, indazole, benzimidazole, quinoline, isoquinoline, quinoxaline, phthalazine, quinazoline, cinnoline, imidazopyridine, etc.; or the like. Examples of the substituent of the aromatic group include halogen (for example, fluorine, chlorine, bromine, iodine, etc.), nitro, cyano, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- or 6-membered cycloamino such as tetrahydropyrrole, piperazine, piperidine, morpholine, thiomorpholine, pyrrole, imidazole, etc.), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), $C_{1-4}$ alkyl which may be halogenated (for example, trifluoromethyl, methyl, ethyl, etc.), $C_{1-4}$ alkoxy which may be halogenated (for example, methoxy, ethoxy, propoxy, butoxy, trifluoromethoxy, trifluoroethoxy, etc.), formyl, $C_{2-4}$ alkanoyl (for example, acetyl, propionyl, etc.), $C_{1-4}$ alkylsulfonyl (for example, methanesulfonyl, ethanesulfonyl, etc.), and the like, and the number of the substituents is preferably 1 to 3.

The number of the above substituents of $R^1$ may be 1 to 4, preferably 1 to 2, and the substituents which may be identical with or different from each other may be present at any possible positions of the ring. When the "5- or 6-membered ring" of the "5- to 6-membered ring which may be substituted" represented by $R^1$ has two or more substituents, two of the substituents may be bonded to each other to form, for example, lower ($C_{1-6}$) alkylene (for example, trimethylene, tetramethylene, etc.), lower ($C_{1-6}$) alkyleneoxy (for example, $-CH_2-O-CH_2-$, $-O-CH_2-CH_2-$, $-O-CH_2-CH_2-CH_2-$, $-O-CH_2-CH_2-CH_2-CH_2-$, $-O-C(CH_3)(CH_3)-CH_2-CH_2-$, etc.), lower ($C_{1-6}$) alkylenethio (for example, $-CH_2-S-CH_2-$, $-S-CH_2-CH_2-$, $-S-CH_2-CH_2-CH_2-$, $-S-CH_2-CH_2-CH_2-CH_2-$, $-S-C(CH_3)(CH_3)-CH_2-CH_2-$, etc.), lower ($C_{1-6}$) alkylenedioxy (for example, $-O-CH_2-O-$, $-O-CH_2-CH_2-O-$, $-O-CH_2-CH_2-CH_2-O-$, etc.), lower ($C_{1-6}$) alkylenedithio (for example, $-S-CH_2-S-$, $-S-CH_2-CH_2-S-$, $-S-CH_2-CH_2-CH_2-S-$, etc.), oxy-lower ($C_{1-6}$) alkyleneamino (for example, $-O-CH_2-NH-$, $-O-CH_2-CH_2-NH-$, etc.), oxy-lower ($C_{1-6}$) alkylenethio (for example, $-O-CH_2-S-$, $-O-CH_2-CH_2-S-$, etc.), lower ($C_{1-6}$) alkyleneamino (for example, $-NH-CH_2-CH_2-$, $-NH-CH_2-CH_2-CH_2-$, etc.), lower ($C_{1-6}$) alkylenediamino (for example, $-NH-CH_2-NH-$, $-NH-CH_2-CH_2-NH-$, etc.), thialower ($C_{1-6}$) alkyleneamino (for example, $-S-CH_2-NH-$, $-S-CH_2-CH_2-NH-$, etc.), lower ($C_{2-6}$) alkenylene (for example, $-CH_2-CH=CH-$, $-CH_2-CH_2-CH=CH-$, $-CH_2-CH=CH-CH_2-$, etc.), lower ($C_{4-6}$) alkadienylene (for example, $-CH=CH-CH=CH-$, etc.), and the like.

Further, the divalent group formed by bonding of two substituents of $R^1$ may contain 1 to 3 substituents which are the same as the "substituents" of the "5- or 6-membered ring" of the "5- or 6-membered ring which may be substituted" represented by $R^1$ (halogen atom, nitro, cyano, alkyl which may be substituted, cycloalkyl which may be substituted, hydroxy group which may be substituted, thiol group which may be substituted (wherein the sulfur atom may be oxidized, and may form sulfinyl group which may be substituted or sulfonyl group which may be substituted), amino group which may be substituted, acyl which may be substituted, carboxyl group which may be esterified or amidated, an aromatic group which may be substituted, and the like).

The "substituent" of the "5- or 6-membered ring" of the "5- or 6-membered ring group which may be substituted" represented by $R^1$ may be exemplified by, in particular, lower ($C_{1-4}$) alkyl which may be halogenated or lower ($C_{1-4}$) alkoxylated (for example, methyl, ethyl, t-butyl, trifluoromethyl, methoxymethyl, ethoxymethyl, propoxymethyl, butoxymethyl, methoxyethyl, ethoxylethyl, propoxyethyl, butoxyethyl, etc.), lower ($C_{1-4}$) alkoxy which may be halogenated or lower ($C_{1-4}$) alkoxylated (for example, methoxy, ethoxy, propoxy, butoxy, t-butoxy, trifluoromethoxy, methoxymethoxy, ethoxymethoxy, propoxymethoxy, butoxymethoxy, methoxyethoxy, ethoxyethoxy, propoxyethoxy, butoxyethoxy, methoxypropoxy, ethoxypropoxy, propoxypropoxy, butoxypropoxy, etc.), halogen (for example, fluorine, chlorine, etc.), nitro, cyano, amino which may be substituted with one or two of lower ($C_{1-4}$) alkyl, formyl or lower ($C_{2-4}$) alkanoyl (for example, amino, methylamino, dimethylamino, formylamino, acetylamino, etc.), 5- or 6-membered cycloamino (for example, 1-pyrrolidinyl, 1-piperazinyl, 1-piperidinyl, 4-morpholino, 4-thiomorpholino, 1-imidazolyl, 4-tetrahydropyranyl, etc.), or the like.

Examples of the lower alkyl group of the "lower alkyl group which may be substituted" represented by $R^3$ above include $C_{1-6}$ alkyl such as methyl, ethyl, propyl, isopropyl, butyl, isobutyl, sec-butyl, tert-butyl, pentyl, isopentyl, neopentyl, hexyl, etc., and the like.

Examples of the lower alkoxy group of the "lower alkoxy group which may be substituted" represented by $R^3$ above include $C_{1-6}$ alkoxy such as methoxy, ethoxy, propoxy, butoxy, etc., and the like.

Examples of the substituent which may be carried by the "lower alkyl group which may be substituted" and "lower alkoxy group which may be substituted" include halogen (for example, fluorine, chlorine, bromine, iodine), hydroxy group, amino, mono(lower alkyl)amino, di(lower alkyl)amino, lower alkanoyl and the like.

The lower alkyl carried by said mono(lower alkyl)amino and di(lower alkyl)amino may be exemplified by the same one as the lower alkyl group of the "lower alkyl group which may be substituted" represented by $R^3$ above.

The lower alkanoyl may be exemplified by $C_{2-6}$ alkanoyl such as acetyl, propionyl, butyryl, isobutyryl or the like.

Among them, for $R^3$, the lower $C_{1-6}$ alkyl group which may be substituted is preferred, and particularly a methyl group which may be substituted is preferred.

With respect to the above-described $NR^5R^6$ represented by $R^4$, the "hydrocarbon group" of the "hydrocarbon group which may be substituted" represented by $R^5$ and $R^6$ may be exemplified by:

(1) alkyl (for example, $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower ($C_{1-6}$) alkyl, and more preferably lower ($C_{1-4}$) alkyl, or the like) ;

(2) cycloalkyl (for example, $C_{3-7}$ cycloalkyl such as cyclopropyl, cyclobutyl, cyclopentyl, cyclohexyl, cycloheptyl, etc., or the like);

(3) alkenyl (for example, alkenyl having 2 to 10 carbon atoms, such as allyl, crotyl, 2-pentenyl, 3-hexenyl, etc., and preferably lower ($C_{2-6}$) alkenyl, or the like);

(4) cycloalkenyl (for example, cycloalkenyl having 3 to 7 carbon atoms such as 2-cyclopentenyl, 2-cyclohexenyl, 2-cyclopentenylmethyl, 2-cyclohexenylmethyl, etc., or the like);

(5) alkynyl (for example, alkynyl having 2 to 10 carbon atoms, such as ethynyl, 1-propynyl, 2-propynyl, 1-butynyl,

2-pentynyl, 3-hexynyl, etc., and preferably lower ($C_{2-6}$) alkynyl, or the like);
(6) aralkyl (for example, phenyl-$C_{1-4}$ alkyl (for example, benzyl, phenethyl, etc.) or the like);
(7) aryl (for example, phenyl, naphthyl, etc.);
(8) cycloalkyl-alkyl (for example, $C_{3-7}$ cycloalkyl-$C_{1-4}$ alkyl such as cyclopropylmethyl, cyclobutylmethyl, cyclopentylmethyl, cyclohexylmethyl, cycloheptylmethyl, etc.); or the like.

Examples of the substituent which may be carried by the above-described (1) alkyl, (2) cycloalkyl, (3) alkenyl, (4) cycloalkenyl, (5) alkynyl, (6) aralkyl, (7) aryl and (8) cycloalkyl-alkyl, include halogen (for example, fluorine, chlorine, bromine, iodine, etc.), nitro, cyano, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- or 6-membered cycloamino such as tetrahydropyrrole, piperazine, piperidine, morpholine, thiomorpholine, pyrrole, imidazole, etc.), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), $C_{1-4}$ alkyl which may be halogenated (for example, trifluoromethyl, methyl, ethyl, etc.), $C_{1-4}$ alkoxy which may be halogenated (for example, methoxy, ethoxy, propoxy, butoxy, trifluoromethoxy, trifluoroethoxy, etc.), $C_{1-4}$ alkylenedioxy (for example, $-O-CH_2-O-$, $-O-CH_2-CH_2-O-$, etc.), sulfonamide which may be substituted [for example, a group formed by bonding of an amino group which may be substituted (for example, amino, mono-$C_{1-4}$ alkylamino, di-$C_{1-4}$ alkylamino, 5- or 6-membered cycloamino such as tetrahydropyrrole, piperazine, piperidine, morpholine, thiomorpholine, pyrrole, imidazole, etc.) with $-SO_2-$, etc.], formyl, $C_{2-4}$ alkanoyl (for example, acetyl, propionyl, etc.), $C_{1-4}$ alkylsulfonyl (for example, methanesulfonyl, ethanesulfonyl, etc.), heterocyclic group which may be substituted, and the like, and the number of the substituents is preferably 1 to 3.

The "heterocyclic group" of said "heterocyclic group which may be substituted" and the "heterocyclic group which may be substituted" represented by $R^4$, may be exemplified by a group formed by eliminating one hydrogen atom from an aromatic heterocycle or a non-aromatic heterocycle, or the like. Examples of such aromatic heterocycle include 5- or 6-membered aromatic heterocycle containing 1 to 4 heteroatoms of one or two kinds selected from nitrogen, sulfur and oxygen atoms, such as furan, thiophene, pyrrole, imidazole, pyrazole, thiazole, oxazole, isothiazole, isoxazole, tetrazole, pyridine, pyrazine, pyrimidine, pyridazine, triazole, oxadiazole, thiadiazole and the like, while examples of such non-aromatic heterocycle include 5- or 6-membered non-aromatic heterocycle containing 1 to 4 heteroatoms of one or two kinds selected from nitrogen, sulfur and oxygen atoms, such as tetrahydrofuran, tetrahydrothiophene, dioxolane, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, etc., and non-aromatic heterocycle in which all or part of the bonds in the above-mentioned aromatic heterocycles are saturated bonds (preferably, aromatic heterocycle such as pyrazole, thiazole, oxazole, tetrazole, etc.).

With respect to the above-described $NR^5R^6$ represented by $R^4$, the substituent of the "heterocyclic group" of the "heterocyclic group which may be substituted" represented by $R^5$ and $R^6$, may be exemplified by the same substituent of the "hydrocarbon group" of the "hydrocarbon group which may be substituted" represented by $R^4$.

The hydrocarbon group which may be substituted is preferably $C_{1-6}$ alkyl which may be halogenated or hydroxylated, carboxyl which may be esterified or amidated, or $C_{2-6}$ alkenyl which may be halogenated or hydroxylated. With respect to the above-described $NR^5R^6$ represented by $R^4$, the "acyl group which may be substituted" represented by $R^5$ and $R^6$ may be exemplified by the same one as the "acyl group which may be substituted" as the substituent which may be carried by the "5- or 6-membered ring" of the "5- or 6-membered ring which may be substituted" represented by $R^1$, and among these, $C_{1-4}$ alkylsulfonyl which may be halogenated or hydroxylated, formyl, $C_{2-5}$ alkanoyl which may be halogenated or hydroxylated, and the like are preferred.

For $R^5$ and $R^6$, $C_{1-4}$ alkyl which may be halogenated or hydroxylated, formyl, $C_{2-5}$ alkanoyl which may be halogenated or hydroxylated, and the like are more preferred, and propyl, isobutyl, isobutenyl or 3-hydroxy-2-methylpropyl are particularly preferred. Another preferred embodiment of $R^5$ and $R^6$ may be exemplified by a group represented by the formula $-(CH_2)_s-R^x$, wherein s is 0 or 1, and $R^x$ is a 5- or 6-membered ring which may be substituted (for example, those such as the "5- or 6-membered ring which may be substituted" represented by $R^1$, etc.; preferably phenyl, pyridyl, pyrazolyl, thiazolyl, oxazolyl, tetrazolyl, etc., each of which may be substituted with halogen, $C_{1-4}$ alkyl which may be halogenated or hydroxylated, $C_{1-4}$ alkoxy which may be halogenated or hydroxylated, etc.), or the like.

Among these, $R^5$ and $R^6$ are preferably 1) $C_{1-6}$ alkyl, 2) $C_{2-6}$ alkenyl, 3) $C_{6-10}$ aryl, 4) $C_{6-10}$ aryl-methyl, 5) heterocyclic group and 6) heterocyclic methyl (wherein the above 1) and 2) may be substituted with halogen, hydroxy group, or carboxyl group which may be esterified or amidated; and the above 3), 4), 5) and 6) may be substituted with $C_{1-6}$ alkyl which may be substituted with halogen, hydroxy group, or carboxyl group which may be esterified or amidated, or $C_{1-6}$ alkoxy which may be substituted with halogen, hydroxy group, or carboxyl group which may be esterified or amidated).

With respect to the above-described $NR^5R^6$ represented by $R^4$, the heterocyclic group which may be substituted, which is formed by $NR^5R^6$ as the result of bonding of $R^5$ and $R^6$ , may be exemplified by 4- to 10-membered alicyclic cycloamino such as azetidinyl, pyrrolidinyl, oxazolidinyl, thiazolidinyl, imidazolidinyl, oxazolinyl, thiazolinyl,

imidazolinyl, piperidinyl, morpholinyl, thiomorpholinyl, dihydropyridinyl, piperadinyl, azepinyl, oxazepinyl, thiazepinyl, diazepinyl, azocinyl, oxazocinyl, thiazocinyl, diazocinyl, etc.; 5- to 10-membered aromatic cycloamino such as pyrrolyl, imidazolyl, triazolyl, tetrazolyl, etc. (preferably 5- to 8-membered alicyclic cycloamino, more preferably 5-membered alicyclic cycloamino such as pyrrolidinyl, etc.), or the like. These cycloamino groups may be substituted, and examples of such substituent include halogen (for example, fluorine, chlorine, bromine, iodine, etc.), nitro, cyano, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- or 6-membered cycloamino such as pyrrolidine, piperidine, piperazine, morpholine, thiomorpholine, pyrrole, imidazole, etc., or the like), carboxyl group which may be esterified or amidated [for example, carboxyl, $C_{1-4}$ alkoxycarbonyl (for example, methoxycarbonyl, ethoxycarbonyl, etc.), carbamoyl, mono-$C_{1-4}$ alkylcarbamoyl (for example, methylcarbamoyl, ethylcarbamoyl, etc.), di-$C_{1-4}$ alkylcarbamoyl (for example, dimethylcarbamoyl, diethylcarbamoyl, ethylmethylcarbamoyl, etc.), etc.], $C_{1-4}$ alkyl which may be substituted (for example, in addition to methyl, ethyl, propyl, etc., halogenated alkyl such as trifluoromethyl, for example, $C_{2-3}$ alkanoyloxy-$C_{1-3}$ alkyl such as acetyloxymethyl, propionyloxymethyl, acetyloxyethyl, propionyloxyethyl, etc., for example, $C_{1-4}$ hydroxyalkyl such as hydroxymethyl, hydroxyethyl, etc.), $C_{1-4}$ alkoxy which may be substituted (for example, methoxy, ethoxy, propoxy, butoxy, trifluoromethoxy, trifluoroethoxy, carboxy-$C_{1-4}$ alkoxy, carbamoyl-$C_{1-4}$ alkoxy, tetrahydrofuranyloxy, tetrahydropyranyloxy, etc.), formyl, $C_{2-4}$ alkanoyl (for example, acetyl, propionyl, etc.), $C_{1-4}$ alkylsulfonyl (for example, methanesulfonyl, ethanesulfonyl, etc.), $C_{1-3}$ alkylenedioxy (for example, methylenedioxy, ethylenedioxy, etc.), oxo group which may be acetalized ($C_{1-4}$ dialkoxy, 1,3-diodisolane, 1,3-dioxane, etc.), or the like. The number of the substituents is preferably 1 to 3.

The lower alkyl group of the "lower alkyl group which may be substituted" represented by each of the above-described $R^7$ and $R^8$, may be exemplified by $C_{1-6}$ alkyl such as methyl, ethyl, propyl, isopropyl, butyl, isobutyl, sec-butyl, tert-butyl, pentyl, isopentyl, neopentyl, hexyl, etc., or the like.

Examples of the substituent which may be carried by said "lower alkyl group which may be substituted" and "lower alkoxy group which may be substituted" include halogen (for example, fluorine, chlorine, bromine, iodine), hydroxy group, amino, mono(lower alkyl)amino, di(lower alkyl)amino, lower alkanoyl or the like.

The lower alkyl carried by said mono(lower alkyl)amino and di(lower alkyl)amino may be exemplified by the same lower alkyl group of the "lower alkyl group which may be substituted" represented by each of the above-described $R^7$ and $R^8$.

Examples of the lower alkanoyl include $C_{2-6}$ alkanoyl such as acetyl, propionyl, butyryl, isobutyryl and the like.

Among them, each of $R^7$ and $R^8$ is preferably lower $C_{1-6}$ alkyl group which may be substituted, and particularly preferably methyl group which may be substituted.

With respect to CR represented by the above-described X, the lower alkyl group of the "lower alkyl group which may be substituted" represented by R may be exemplified by $C_{1-6}$ alkyl such as methyl, ethyl, propyl, isopropyl, butyl, isobutyl, sec-butyl, tert-butyl, pentyl, isopentyl, neopentyl, hexyl or the like.

For CR represented by the above-described X, the lower alkoxy group of the "lower alkoxy group which may be substituted" represented by R may be exemplified by $C_{1-6}$ alkoxy such as methoxy, ethoxy, propoxy, butoxy or the like.

Examples of the substituent which may be carried by said "lower alkyl group which may be substituted" and "lower alkoxy group which may be substituted" include halogen (for example, fluorine, chlorine, bromine, iodine), hydroxy group, amino, mono(lower alkyl)amino, di(lower alkyl)amino, lower alkanoyl and the like.

The lower alkyl of said mono(lower alkyl)amino and di(lower alkyl)amino may be exemplified by the same lower alkyl group as the "lower alkyl group which may be substituted" represented by the above-described $R^3$.

Examples of said lower alkanoyl include $C_{2-6}$ alkanoyl such as acetyl, propionyl, butyryl, isobutyryl and the like.

With respect to CR represented by the above-described X, the "acyl group which may be substituted" represented by R may be exemplified by the same one as the "acyl group which may be substituted" as the substituent which may be carried by the "5- or 6-membered ring" of the "5- or 6-membered ring which may be substituted" represented by $R^1$, and among them, preferred are $C_{1-4}$ alkylsulfonyl which may be halogenated or hydroxylated, formyl, $C_{2-5}$ alkanoyl which may be halogenated or hydroxylated, and the like.

Among them, for R, lower $C_{1-6}$ alkyl group which may be substituted is preferred, and in particular, methyl group which may be substituted is preferred.

The 5- or 6-membered alicyclic heterocycle which is formed by bonding of X and $R^4$, may be exemplified by pyrrolidine, oxazolidine, thiazolidine, imidazolidine, piperidine, morpholine, thiomorpholine, piperazine or the like. These may be substituted at any arbitrary positions on the ring, and examples of the substituent include those described as the substituents of the "5- or 6-membered ring" with respect to the "5- or 6-membered ring which may be substituted" represented by $R^1$.

In the above formula (I), the "5- or 6-membered aromatic ring which may be substituted" represented by $Z^1$ may be exemplified by 6-membered aromatic hydrocarbon such as benzene; 5- to 6-membered aromatic heterocycle containing 1 to 4 heteroatoms of one or two kinds selected from nitrogen, sulfur and oxygen atoms, such as furan,

thiophene, pyrrole, imidazole, pyrazole, thiazole, oxazole, isothiazole, isoxazole, tetrazole, pyridine, pyrazine, pyrimidine, pyridazine, triazole, etc.; fused aromatic heterocycle such as benzofuran, indole, benzothiophene, benzoxazole, benzothiazole, indazole, benzimidazole, quinoline, isoquinoline, quinoxaline, phthalazine, quinazoline, cinnoline, imidazopyridine, etc.; or the like. Among them, preferred are benzene, furan, thiophene, pyridine, pyridazine, pyrimidine, benzimidazole and the like, and particularly preferably used are benzene, pyridine, pyridazine and benzimidazole (preferably benzene).

The "5- or 6-membered aromatic ring which may be substituted" represented by $Z^1$ may have the same substituent as the "substituent" which may be carried by the "5- or 6-membered ring" of the "5- or 6-membered ring which may be substituted" represented by $R^1$, and among the substituents, a halogen atom (for example, fluorine, chlorine, bromine, etc.), a $C_{1-4}$ alkyl group which may be substituted with halogen atom(s) (for example, methyl, ethyl, trifluoromethyl, trifluoroethyl, etc.), a $C_{1-4}$ alkoxy group which may be substituted with halogen atom(s) (for example, methoxy, ethoxy, propoxy, trifluoromethoxy, trifluoroethoxy, etc.) and the like are preferred. However, it is preferred that there is no other substituent than $X^2$ and $Z^2$, and it is preferred that when $Z^1$ is a 6-membered ring (preferably benzene), the position of substitution of $Z^2$ is para to $X^2$. Further, for the substituent of $Z^1$, benzene which may be substituted with 1) a halogen atom, 2) a $C_{1-4}$ alkyl group which may be substituted with halogen atom(s), or 3) a $C_{1-4}$ alkoxy group which may be substituted with halogen atom(s) is preferred, and in particular, benzene which may be substituted with methyl or trifluoromethyl is preferred.

In the above formula (I), with respect to the formula $-Z^{2a}-W^1-Z^{2b}-$ and $-Z^{2a}-W^2-Z^{2b}-$ represented by $Z^2$, the substituent ($R^a$) of the "imino group which may be substituted" represented by each of $Z^{2a}$ and $Z^{2b}$ may be exemplified by hydrogen atom, lower ($C_{1-6}$) alkyl which may be substituted [for example, methyl, ethyl, propyl, isopropyl, butyl, isobutyl, sec-butyl, tert-butyl, pentyl, isopentyl, neopentyl, hexyl, hydroxy-$C_{1-6}$ alkyl (for example, hydroxyethyl, hydroxypropyl, hydroxybutyl, etc.), halogenated $C_{1-6}$ alkyl (for example, trifluoromethyl, trifluoroethyl, etc.), cyanated $C_{1-6}$ alkyl (for example, cyanoethyl, cyanopropyl, etc.), carboxyl-$C_{1-6}$ alkyl which may be esterified or amidated, etc.], formyl, lower ($C_{2-5}$) alkanoyl (for example, acetyl, propionyl, butyryl, etc.), lower ($C_{1-5}$) alkylsulfonyl (methylsulfonyl, ethylsulfonyl, etc.), or the like.

The alkylene chain of the "alkylene group which may be substituted" represented by $W^1$ and $W^2$ may be exemplified by the alkylene chain represented by $-(CH_2)_{k1}-$ (wherein k1 is an integer of 1 to 4) or the like. The alkenylene group of the "alkenylene group which may be substituted" represented by $W^1$ may be exemplified by the alkenylene chain represented by $-(CH_2)_{k2}-(CH=CH)-(CH_2)_{k3}-$ (wherein k2 and k3 are identical or different, and represent 0, 1 or 2, respectively, provided that the sum of k2 and k3 is 2 or less) or the like. The alkylene group and alkenylene group represented by said $W^1$ and $W^2$ may be substituted at any arbitrary position (preferably on a carbon atom), and such substituent may be any substituent capable of bonding to the alkylene chain or alkenylene chain which constitutes the straight chain moiety. Examples thereof include lower ($C_{1-6}$) alkyl (for example, methyl, ethyl, propyl, isopropyl, butyl, isobutyl, sec-butyl, tert-butyl, pentyl, isopentyl, neopentyl, hexyl, etc.), lower ($C_{3-7}$)cycloalkyl (for example, cyclopropyl, cyclobutyl, cyclopentyl, cyclohexyl, cycloheptyl, etc.), formyl, lower ($C_{2-7}$) alkanoyl, (for example, acetyl, propionyl, butyryl, etc.), phosphono which may be esterified, carboxyl which may be esterified or amidated, hydroxy group, oxo, hydroxyimino group, lower ($C_{1-6}$) alkoxyimino group which may be substituted, and the like, and preferably lower alkyl having 1 to 6 carbon atoms (preferably, $C_{1-3}$ alkyl), hydroxy group, oxo, hydroxyimino group, lower ($C_{1-6}$) alkoxyimino group (which may be substituted with a polar group such as hydroxy group, cyano group, carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), etc.) or the like.

The phosphono group which may be esterified may be exemplified by a group represented by $P(O)(OR^{12})(OR^{13})$, wherein $R^{12}$ and $R^{13}$ are each hydrogen, an alkyl group having 1 to 6 carbon atoms, or a cycloalkyl group having 3 to 7 carbon atoms, or $R^{12}$ and $R^{10}$ may be bonded to each other to form a 5- to 7-membered ring.

In the above-described formula, the alkyl group having 1 to 6 carbon atoms represented by $R^{12}$ and $R^{13}$ may be exemplified by methyl, ethyl, propyl, isopropyl, butyl, isobutyl, sec-butyl, tert-butyl, pentyl, isopentyl, neopentyl, hexyl or the like, and the cycloalkyl having 3 to 7 carbon atoms may be exemplified by cyclopropyl, cyclobutyl, cyclopentyl, cyclohexyl, cycloheptyl or the like. Preferred is linear lower alkyl having 1 to 6 carbon atoms, and more preferred is lower alkyl having 1 to 3 carbon atoms. $R^{12}$ and $R^{13}$ may be identical with or different from each other, and preferably identical. When $R^{12}$ and $R^{13}$ are bonded to each other to form a 5- to 7-membered ring, $R^{12}$ and $R^{13}$ are bonded to each other to form a linear $C_{2-4}$ alkylene side chain represented by $-(CH_2)_2-$, $-(CH_2)_3-$ or $-(CH_2)_4-$. This side chain may be substituted, and examples of such substituent include hydroxy group, halogen and the like.

The ester product of the above-described carboxyl group which may be esterified may be exemplified by a product resulting from bonding between a carboxyl group and an alkyl group having 1 to 6 carbon atoms or a cycloalkyl group having 3 to 7 carbon atoms, for example, methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, cyclopentyloxycarbonyl, cyclohexyloxycarbonyl or the like.

The amide product of the above-described carboxyl group which may be amidated may be exemplified by a

product resulting from bonding between a carboxyl group and an alkylamino group having 1 to 6 carbon atoms, a cycloalkylamino group having 3 to 7 carbon atoms or a 5- to 8-membered cyclic amine (for example, pyrrolidine, piperidine, morpholine, etc.), for example, carbamoyl, mono-$C_{1-6}$ alkylcarbamoyl, di-$C_{1-6}$ alkylcarbamoyl, cyclopentylaminocarbonyl, cyclohexylaminocarbonyl, pyrrolidinocarbonyl, piperidinocarbonyl, morpholinocarbonyl, thiomorpholinocarbonyl or the like.

For $Z^2$, preferably, one of $Z^{2a}$ and $Z^{2b}$ is O, $S(O)_m$ (wherein m is an integer of 0, 1 or 2), or $-N(R^a)-$ (wherein $R^a$ is a hydrogen atom or a lower $C_{1-4}$ alkyl group which may be substituted), the other being a bond, and W is $-(CH_2)_p-$(wherein p is an integer of 1 to 3), or $Z^2$ is a divalent group of the formula -CH(OH)-. More preferably, one of $Z^{2a}$ and $Z^{2b}$ is O or $S(O)_m$ (wherein m is an integer of 0, 1 or 2), the other being a bond, and W is $-(CH_2)_p-$ (wherein p is an integer of 1 to 3), or $Z^2$ is a divalent group of the formula -CH(OH)-. Even more preferably, $Z^2$ is $-CH_2-$, -CH(OH)-, $-S(O)_m-CH_2-$ (wherein m is 0, 1 or 2), with $-S(O)_m-CH_2-$(wherein m is 0, 1 or 2) being particularly preferred. In particular, when $Z^{2a}$ is bonded to $Z^1$, $Z^2$ is preferably $-SOCH_2-$.

$Z^{2a}$ represents a bond, S, SO or $SO_2$, and among them, SO is preferred. In this case, the configuration of SO is preferably (S).

The bonding position of $Z^2$ with respect to $Z^1$ is such that when $Z^1$ is a benzene ring for example, any position may be selected, but the para position is preferred.

In the above formula (I), the "amino group which may be substituted, in which the nitrogen atom may be converted to a quaternary ammonium or an oxide" represented by $R^2$ may be exemplified by an amino group which may have 1 or 2 substituents, an amino group which has three substituents, in which the nitrogen atom is converted to a quaternary ammonium, or the like. When the amino group has two or more substituents on its nitrogen atom, the substituents may be identical or different; and when the nitrogen atom has 3 substituents, the amino group may be of any type among the following formulas, $-N^+R^pR^pR^p$, $-N^+R^pR^pR^q$, and $-N^+R^pR^qR^r$, wherein $R^p$, $R^q$, and $R^r$ are different from each other, each being hydrogen or a substituent. Examples of the counter anion of the amino group, in which the nitrogen atom is converted to a quaternary ammonium include, in addition to anions of halogen (for example, $Cl^-$, $Br^-$, $I^-$, etc.), anions derived from inorganic acids such as hydrochloric acid, hydrobromic acid, nitric acid, sulfuric acid, phosphoric acid, etc.; anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; and anions derived from acidic amino acids such as aspartic acid, glutamic acid, etc., and among them, $Cl^-$, $Br^-$, $I^-$ and the like are preferred.

Examples of the substituent of said amino group include:

(1) alkyl which may be substituted (for example, $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower ($C_{1-6}$) alkyl, or the like); and

(2) cycloalkyl which may be substituted (for example, $C_{3-8}$ cycloalkyl such as cyclopropyl, cyclobutyl, cyclopentyl, cyclohexyl, cycloheptyl, cyanooctyl, etc., or the like);

(2-1) the cycloalkyl may contain one heteroatom selected from sulfur, oxygen and nitrogen atoms, forming oxirane, thiolane, aziridine, tetrahydrofuran, tetrahydrothiophene, pyrrolidine, tetrahydropyran, tetrahydrothiopyran, tetrahydrothiopyran 1-oxide, piperidine, etc. (preferably, a 6-membered ring such as tetrahydropyran, tetrahydrothiopyran, piperidine, etc.), and the bond with the amino group is preferably present at the 3- or 4-position (preferably, at the 4-position);

(2-2) also, the cycloalkyl may be fused to a benzene ring, forming indane (for example, indan-1-yl, indan-2-yl, etc.), tetrahydronaphthalene (for example, tetrahydronaphthalen-5-yl, tetrahydronaphthalen-6-yl, etc.), or the like (preferably, indane, etc.);

(2-3) further, the cycloalkyl may be bridged via a straight atomic chain having 1 or 2 carbon atoms, forming a bridged cyclic hydrocarbon residue such as bicyclo[2.2.1]heptyl, bicyclo[2.2.2]octyl, bicyclo[3.2.1]octyl, bicyclo[3.2.2]nonyl, etc. (preferably, cyclohexyl bridged via a straight atomic chain having 1 to 2 carbon atoms, and more preferably, bicyclo[2.2.1]heptyl, etc.);

(3) alkenyl which may be substituted (for example, alkenyl having 2 to 10 carbon atoms, such as allyl, crotyl, 2-pentenyl, 3-hexenyl, etc., and preferably lower ($C_{2-6}$) alkenyl, or the like);

(4) cycloalkenyl which may be substituted (for example, cycloalkenyl having 3 to 7 carbon atoms, such as 2-cyclopentenyl, 2-cyclohexenyl, 2-cyclopentenylmethyl, 2-cylcohexenylmethyl, etc., or the like);

(5) aralkyl which may be substituted (for example, phenyl-$C_{1-4}$ alkyl (for example, benzyl, phenethyl, etc.), or the like);

(6) formyl, or acyl which may be substituted (for example, alkanoyl having 2 to 4 carbon atoms (for example, acetyl, propionyl, butyryl, isobutyryl, etc.), alkylsulfonyl having 1 to 4 carbon atoms (for example, methanesulfonyl,

ethanesulfonyl, etc.), alkoxycarbonyl having 1 to 4 carbon atoms (for example, methoxycarbonyl, ethoxycarbonyl, tert-butoxycarbonyl, etc.), aralkyloxycarbonyl having 7 to 10 carbon atoms (for example, benzyloxycarbonyl, etc.), or the like) ;

(7) aryl which may be substituted (for example, phenyl, naphthyl, etc.);

(8) heterocyclic group which may be substituted (for example, a group formed by eliminating a hydrogen atom from a 5- or 6-membered aromatic heterocycle containing 1 to 4 heteroatoms of one or two kinds selected from nitrogen, sulfur and oxygen atoms, such as furan, thiophene, pyrrole, imidazole, pyrazole, thiazole, oxazole, isothiazole, isoxazole, tetrazole, pyridine, pyrazine, pyrimidine, pyridazine, triazole, oxadiazole, thiadiazole, etc., or from a fused heterocyclic aromatic group such as benzofuran, indole, benzothiophene, benzoxazole, benzothiazole, indazole, benzimidazole, quinoline, isoquinoline, quinoxaline, phthalazine, quinazoline, cinnoline, imidazopyridine, etc.; a group formed by eliminating a hydrogen atom from a 5- or 6-membered non-aromatic heterocycle containing 1 to 4 heteroatoms of one or two kinds selected from nitrogen, sulfur and oxygen atoms, such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, etc.; or the like; and preferably, a group formed by eliminating a hydrogen atom from a 5- or 6-membered non-aromatic heterocycle; more preferably, a group formed by eliminating a hydrogen atom from a 5- or 6-membered non-aromatic heterocycle containing one heteroatom, such as tetrahydrofuran, piperidine, tetrahydropyran, tetrahydrothiopyran, etc.); and the like. The substituents on the amino group may be bonded to each other to form 5- to 7-membered cycloamino such as piperidine, piperazine, morpholine, thiomorpholine, etc.

Examples of the substituent which may be carried by the above-described (1) alkyl which may be substituted, (2) cycloalkyl which may be substituted, (3) alkenyl which may be substituted, (4) cycloalkenyl which may be substituted, (5) aralkyl which may be substituted, (6) acyl which may be substituted, (7) aryl which may be substituted, and (8) heterocyclic group which may be substituted, include halogen (for example, fluorine, chlorine, bromine, iodine, etc.); lower ($C_{1-4}$) alkyl which may be halogenated; lower ($C_{1-4}$) alkyl which may be substituted with a polar group such as a hydroxy group, a cyano group, a carboxyl group which may be esterified or amidated, etc. (for example, hydroxy-$C_{1-4}$ alkyl, cyano-$C_{1-4}$ alkyl, carboxy-$C_{1-4}$ alkyl, $C_{1-4}$ alkoxycarbonyl-$C_{1-4}$ alkyl, carbamoyl-$C_{1-4}$ alkyl, mono-$C_{1-4}$ alkylcarbamoyl-$C_{1-4}$ alkyl, di-$C_{1-4}$ alkylcarbamoyl, di-$C_{1-4}$ alkylcarbamoyl-$C_{1-4}$ alkyl, pyrrolidinocarbonyl-$C_{1-4}$ alkyl, piperidinocarbonyl-$C_{1-4}$ alkyl, morpholinocarbonyl-$C_{1-4}$ alkyl, thiomorpholinocarbonyl-$C_{1-4}$ alkyl, etc.); $C_{1-4}$ alkoxy which may be halogenated (for example, methoxy, ethoxy, propoxy, butoxy, trifluoromethoxy, trifluoroethoxy, etc.); $C_{1-4}$ alkylenedioxy (for example, $-O-CH_2-O-$, $-O-CH_2-CH_2-O-$, etc.); formyl; $C_{2-4}$ alkanoyl (for example, acetyl, propionyl, etc.); $C_{1-4}$ alkylsulfonyl (for example, methanesulfonyl, ethanesulfonyl, etc.); phenyl-lower ($C_{1-4}$) alkyl; $C_{3-7}$ cycloalkyl; cyano; nitro; hydroxy group; thiol group which may be substituted (for example, thiol, $C_{1-4}$ alkylthio, etc.); amino group which may be substituted (for example, amino, mono-$C_{1-4}$ alkylamino, di-$C_{1-4}$ alkylamino, 5- or 6-membered cycloamino such as tetrahydropyrrole, piperazine, piperidine, morpholine, thiomorpholine, pyrrole, imidazole, etc.); carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.); lower ($C_{1-4}$) alkoxycarbonyl; lower ($C_{7-10}$) aralkyloxy-carbonyl; oxo group (preferably, halogen, lower ($C_{1-4}$) alkyl which may be halogenated, lower ($C_{1-4}$) alkoxy which may be halogenated, phenyl-lower ($C_{1-4}$) alkyl, $C_{3-7}$ cycloalkyl, cyano, hydroxy group, etc); and the like. The number of the substituents is preferably 1 to 3.

In the above formula (I), the "amino group which may be substituted, in which the nitrogen atom may be converted to a quaternary ammonium or an oxide" represented by $R^2$ is preferably an amino group which may have 1 to 3 substituents selected from:

(1) linear or branched lower ($C_{1-6}$) alkyl which may be substituted with one to three of halogen, cyano, hydroxy group or $C_{3-7}$ cycloalkyl;

(2) $C_{5-8}$ cycloalkyl which may be substituted with one to three of halogen, lower ($C_{1-4}$) alkyl which may be halogenated, or phenyl-lower ($C_{1-4}$) alkyl, which may contain one heteroatom selected from sulfur, oxygen and nitrogen atoms, which may be fused to a benzene ring, and which may be bridged via a straight atomic chain having 1 or 2 carbon atoms (for example, cyclopentyl, cyclohexyl, cycloheptyl, cyclooctyl, tetrahydropyranyl, tetrahydrothiapyranyl, piperidinyl, indanyl, tetrahydronaphthalenyl, bicyclo[2.2.1]heptyl, etc., each of which may be substituted);

(3) phenyl-lower ($C_{1-4}$) alkyl which may have one to three of halogen, lower ($C_{1-4}$) alkyl which may be halogenated, or lower ($C_{1-4}$) alkoxy which may be halogenated;

(4) phenyl which may have one to three of halogen, lower ($C_{1-4}$) alkyl which may be halogenated, or lower ($C_{1-4}$) alkoxy which may be halogenated; and

(5) 5- to 6-membered aromatic heterocyclic group which may have one to three of halogen, lower ($C_{1-4}$) alkyl which may be halogenated, lower ($C_{1-4}$) alkoxy groups which may be halogenated, lower ($C_{1-4}$) alkoxy-lower ($C_{1-4}$) alkoxy which may be halogenated, phenyl-lower ($C_{1-4}$) alkyl, cyano or hydroxy group (for example, a group formed by eliminating one hydrogen atom from furan, thiophene, pyrrole, pyridine, etc.).

In the above formula (I), the "nitrogen-containing heterocyclic group" of the "nitrogen-containing heterocyclic group which may be substituted, which may contain a sulfur atom or an oxygen atom as the ring-constituting atom, in which the nitrogen atom may be converted to a quaternary ammonium or an oxide" represented by $R^2$, may be exemplified by 5- to 6-membered aromatic heterocycle containing 1 to 4 heteroatoms of one or two kinds selected from nitrogen, sulfur and oxygen atoms, such as pyrrole, imidazole, pyrazole, thiazole, oxazole, isothiazole, isoxazole, tetrazole, pyridine, pyrazine, pyrimidine, pyridazine, triazole, oxadiazole, thiadiazole, etc.; fused aromatic heterocycle such as benzofuran, indole, benzothiophene, benzoxazole, benzothiazole, indazole, benzimidazole, quinoline, isoquinoline, quinoxaline, phthalazine, quinazoline, cinnoline, imidazopyridine, etc.; 5- to 8-membered non-aromatic heterocycle containing a nitrogen atom and additionally 1 to 3 heteroatoms of one or two kinds selected from nitrogen, sulfur and oxygen atoms, such as pyrrolidine, pyrroline, imidazolidine, imidazoline, pyrazolidine, pyrazoline, piperidine, piperazine, oxazine, oxadiazine, thiazine, thiadiazine, morpholine, thiomorpholine, azacycloheptane, azacyclooctane (azocane), etc.; or the like, and these nitrogen-containing heterocycles may be bridged via a straight atomic chain having 1 or 2 carbon atoms, forming a bridged-ring nitrogen-containing heterocycle such as azabicyclo[2.2.1]heptane, azabicyclo[2.2.2]octane (quinuclidine), etc. (preferably, piperidine bridged via a straight atomic chain having 1 or 2 carbon atoms, etc.).

Among specific examples of the above-described nitrogen-containing heterocycle, preferred are pyridine, pyridazine, pyrazole, imidazole, triazole, tetrazole, imidazopyridine, pyrrolidine, piperidine, piperazine, morpholine, thiomorpholine and azabicyclo[2.2.2]octane (preferably, pyridine, imidazole, triazole, imidazopyridine, pyrrolidine, piperidine and morpholine).

The nitrogen atom of the "nitrogen-containing heterocycle" may be converted to a quaternary ammonium or may be oxidized. When the nitrogen atom of the "nitrogen-containing heterocycle" is conveted to a quaternary ammonium, the counter anion of the "nitrogen-containing heterocyclic group in which the nitrogen atom is converte to a quaternary ammonium" may be exemplified by, in addition to anions of halogen (for example, $Cl^-$, $Br^-$, $I^-$, etc.), anions derived from inorganic acids such as hydrochloric acid, hydrobromic acid, nitric acid, sulfuric acid, phosphoric acid, etc.; anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; anions derived from acidic amino acids such as aspartic acid, glutamic acid, etc.; or the like, and among them, $Cl^-$, $Br^-$, $I^-$ and the like are preferred.

The "nitrogen-containing heterocyclic group" may be bonded to a divalent group represented by $Z^2$ via a carbon atom or a nitrogen atom, and may be bonded to a ring-constituting carbon atom as in 2-pyridyl, 3-pyridyl, 2-piperidinyl, etc., or to a ring-constituting nitrogen atom as in the following:

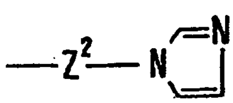, 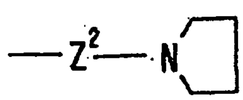, 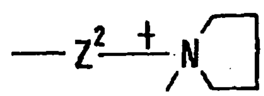,

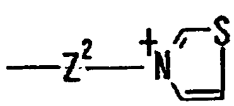, 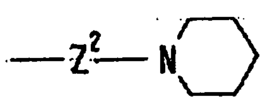, 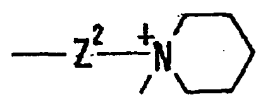,

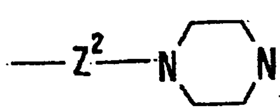, 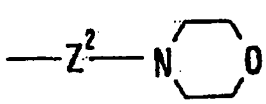, 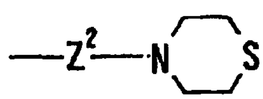,

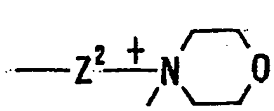, 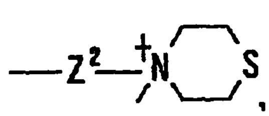, 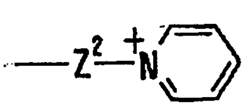,

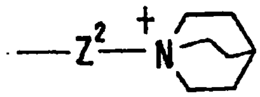, 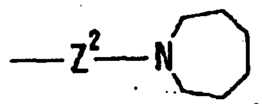, 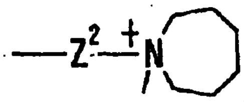,

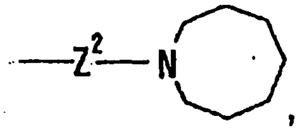, 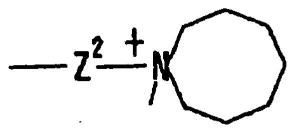

The substituent which may be carried by the "nitrogen-containing heterocycle" may be exemplified by halogen (for example, fluorine, chlorine, bromine, iodine, etc.), lower ($C_{1-4}$) alkyl which may be substituted, lower ($C_{1-4}$) alkoxy which may be substituted, phenyl which may be substituted, mono- or diphenyl-lower ($C_{1-4}$) alkyl which may be substituted, $C_{3-7}$ cycloalkyl which may be substituted, cyano, nitro, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- to 6-membered cycloamino such as tetrahydropyrrole, piperazine, piperidine, morpholine, thiomorpholine, pyrrole, imidazole, etc.), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), lower ($C_{1-4}$) alkoxy-carbonyl, formyl, lower ($C_{2-4}$) alkanoyl, lower ($C_{1-4}$) alkylsulfonyl, heterocyclic group which may be substituted (for example, a group formed by eliminating a hydrogen atom from a 5- to 6-membered aromatic heterocycle containing 1 to 4 heteroatoms of one or two kinds selected from nitrogen, sulfur and oxygen atoms, such as furan, thiophene, pyrrole, imidazole, pyrazole, thiazole, oxazole, isothiazole, isoxazole, tetrazole, pyridine, pyrazine, pyrimidine, pyridazine, triazole, oxadiazole, thiadiazole, etc., or from a fused aromatic heterocyclic group containing 1 to 4 heteroatoms of one or two kinds selected from nitrogen, sulfur and oxygen atoms, such as benzofuran, indole, benzothiophene, benzoxazole, benzothiazole, indazole, benzimidazole, quinoline, isoquinoline, quinoxaline, phthalazine, quinazoline, cinnoline, imidazopyridine, etc.; a group formed by eliminating a hydrogen atom from a 5- to 6-membered non-aromatic heterocycle containing 1 to 4 heteroatoms of one or two kinds selected from nitrogen, sulfur and oxygen atoms, such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran, etc.), or the like, and the number of the substituents is preferably 1 to 3. The nitrogen atom in the nitrogen-containing heterocyclic ring may be oxidized.

Examples of the substituent which may be carried respectively by the "lower ($C_{1-4}$) alkyl which may be substituted", the "lower ($C_{1-4}$) alkoxy which may be substituted", the "phenyl which may be substituted", the "mono- or diphenyl-lower ($C_{1-4}$) alkyl which may be substituted", the "$C_{3-7}$ cycloalkyl which may be substituted", and the "heterocyclic group which may be substituted", all of which are the substituents that may be carried by the "nitrogen-containing heterocycle", include halogen atom (for example, fluorine, chlorine, bromine, iodine, etc.); lower ($C_{1-4}$) alkyl which may be halogenated; lower ($C_{1-4}$) alkyl which may be substituted by a polar group such as a hydroxy group, a cyano group, a carboxyl group which may be esterified or amidated, etc. (for example, hydroxy-$C_{1-4}$ alkyl, cyano-$C_{1-4}$ alkyl, carboxy-$C_{1-4}$ alkyl, $C_{1-4}$ alkoxycarbonyl-$C_{1-4}$ alkyl, carbamoyl-$C_{1-4}$ alkyl, mono-$C_{1-4}$ alkylcarbamoyl-$C_{1-4}$ alkyl, di-$C_{1-4}$ alkylcarbamoyl-$C_{1-4}$ alkyl, pyrrolidinocarbonyl-$C_{1-4}$ alkyl, piperidinocarbonyl-$C_{1-4}$ alkyl, morpholinocarbonyl-$C_{1-4}$ alkyl, thiomorpholinocarbonyl-$C_{1-4}$ alkyl, etc.); lower ($C_{3-10}$) cycloalkyl; lower ($C_{3-10}$) cycloalkenyl; $C_{1-4}$ alkoxy which may be halogenated (for example, methoxy, ethoxy, trifluoromethoxy, trifluoroethoxy, etc.); formyl; $C_{2-4}$ alkanoyl (for example, acetyl, propionyl, etc.); $C_{1-4}$ alkylsulfonyl (for example, methanesulfonyl, ethanesulfonyl, etc.); $C_{1-3}$ alkylenedioxy (for example, methylenedioxy, ethylenedioxy, etc.); cyano; nitro; hydroxy group; thiol group which may be substituted (for example, thiol, $C_{1-4}$ alkylthio, etc.); amino group which may be substituted (for example, amino, mono-$C_{1-4}$ alkylamino, di-$C_{1-4}$ alkylamino, 5-to 6-membered cycloamino such as tetrahydropyrrole, piperazine, piperidine, morpholine, thiomorpholine, pyrrole, imidazole, etc.); carboxyl group which may be esterified or amidated (for example, carboxyl, $C_{1-4}$ alkoxy-carbonyl, carbamoyl, mono-$C_{1-4}$ alkylcarbamoyl, di-$C_{1-4}$ alkylcarbamoyl, etc.); lower ($C_{1-4}$) alkoxycarbonyl; and the like, and the number of the substituents is preferably 1 to 3.

In the above formula (I), the substituent which may be carried by the "nitrogen-containing heterocyclic group" of the "nitrogen-containing heterocyclic group which may be substituted and may contain a sulfur atom or an oxygen atom as the ring-constituting atom, in which the nitrogen atom may be converted to a quaternary ammonium or an oxide" is preferably (1) halogen, (2) cyano, (3) hydroxy group, (4) carboxyl group, (5) carbamoyl, (6) lower ($C_{1-4}$) alkoxycarbonyl, (7) lower ($C_{1-4}$) alkylcarbamoyl, or 5- or 6-membered cycloamino (piperidino, morpholino, etc.)-carbonyl,

(8) lower ($C_{1-4}$) alkyl which may be substituted with halogen, hydroxy group, cyano group, lower ($C_{1-4}$) alkoxy, or carboxyl which may be esterified or amidated, (9) lower ($C_{1-4}$) alkoxy which may be substituted with halogen, hydroxy group or lower ($C_{1-4}$) alkoxy, (10) phenyl which may be substituted with halogen, lower ($C_{1-4}$) alkyl, hydroxy group, lower ($C_{1-4}$) alkoxy or $C_{1-3}$ alkylenedioxy, (11) mono- or diphenyl-lower ($C_{1-4}$) alkyl which may be substituted with halogen, lower ($C_{1-4}$) alkyl, hydroxy group, lower ($C_{1-4}$) alkoxy or $C_{1-3}$ alkylenedioxy, or (12) a group formed by eliminating a hydrogen atom from a 5- or 6-membered aromatic heterocycle such as furan, thiophene, pyrrole, pyridine, etc., or the like.

In the above formula (I), with respect to the group represented by formula (a) represented by $R^2$, the "hydrocarbon group which may be substituted" represented by $R^9$ and $R^{10}$ may be exemplified by:

(1) alkyl which may be substituted (for example, $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower ($C_{1-6}$) alkyl, or the like) ;
(2) cycloalkyl which may be substituted (for example, $C_{3-7}$ cycloalkyl such as cyclopropyl, cyclobutyl, cyclopentyl, cyclohexyl, cycloheptyl, etc., or the like);
(3) alkenyl which may be substituted (for example, alkenyl having 2 to 10 carbon atoms, such as allyl, crotyl, 2-pentenyl, 3-hexenyl, etc., and preferably lower ($C_{2-6}$) alkenyl, or the like);
(4) cycloalkenyl which may be substituted (for example, cycloalkenyl having 3 to 7 carbon atoms, such as 2-cyclopentenyl, 2-cyclohexenyl, 2-cyclopentenylmethyl, 2-cylcohexenylmethyl, etc., or the like);
(5) alkynyl which may be substituted (for example, alkynyl having 2 to 10 carbon atoms, such as ethynyl, 1-propynyl, 2-propynyl, 1-butynyl, 2-pentynyl, 3-hexynyl, etc., and preferably lower ($C_{2-6}$) alkynyl, or the like);
(6) aralkyl which may be substituted (for example, phenyl-$C_{1-4}$ alkyl (for example, benzyl, phenethyl, etc.) or the like) ;
(7) aryl which may be substituted (for example, phenyl, naphthyl, etc.); or the like. Examples of the substituents which may be carried by the above-described (1) alkyl which may be substituted, (2) cycloalkyl which may be substituted, (3) alkenyl which may be substituted, (4) cycloalkenyl which may be substituted, (5) alkynyl which may be substituted, (6) aralkyl which may be substituted, and (7) aryl which may be substituted, include halogen (for example, fluorine, chlorine, bromine, iodine, etc.), nitro, cyano, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- to 6-membered cycloamino such as tetrahydropyrrole, piperazine, piperidine, morpholine, thiomorpholine, pyrrole, imidazole, etc.), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), $C_{1-4}$ alkyl which may be halogenated (for example, trifluoromethyl, methyl, ethyl, etc.), $C_{1-4}$ alkoxy which may be halogenated (for example, methoxy, ethoxy, propoxy, butoxy, trifluoromethoxy, trifluoroethoxy, etc.), formyl, $C_{2-4}$ alkanoyl (for example, acetyl, propionyl, etc.), $C_{1-4}$ alkylsulfonyl (for example, methanesulfonyl, ethanesulfonyl, etc.), and the like, and the number of the substituents is preferably 1 to 3.

The "hydroxy group which may be substituted" represented by $R^9$ and $R^{10}$ may be exemplified by a hydroxy group which may have:

(1) alkyl which may be substituted (for example, $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower ($C_{1-6}$) alkyl, or the like) ;
(2) cycloalkyl which may be substituted (for example, $C_{3-7}$ cycloalkyl such as cyclopropyl, cyclobutyl, cyclopentyl, cyclohexyl, cycloheptyl, etc., or the like);
(3) alkenyl which may be substituted (for example, alkenyl having 2 to 10 carbon atoms, such as allyl, crotyl, 2-pentenyl, 3-hexenyl, etc., and preferably lower ($C_{2-6}$) alkenyl, or the like);
(4) cycloalkenyl which may be substituted (for example, cycloalkenyl having 3 to 7 carbon atoms, such as 2-cyclopentenyl, 2-cyclohexenyl, 2-cyclopentenylmethyl, 2-cyclohexenylmethyl, etc., or the like);
(5) aralkyl which may be substituted (for example, phenyl-$C_{1-4}$ alkyl (for example, benzyl, phenethyl, etc.), or the like) ;
(6) formyl, or acyl which may be substituted (for example, alkanoyl having 2 to 4 carbon atoms (for example, acetyl, propionyl, butyryl, isobutyryl, etc.), alkylsulfonyl having 1 to 4 carbon atoms (for example, methanesulfonyl, ethanesulfonyl, etc.), or the like);
(7) aryl which may be substituted (for example, phenyl, naphthyl, etc.); or the like.

Examples of the substituent which may be carried by the above-described (1) alkyl which may be substituted, (2) cycloalkyl which may be substituted, (3) alkenyl which may be substituted, (4) cycloalkenyl which may be substituted,

(5) aralkyl which may be substituted, (6) acyl which may be substituted, and (7) aryl which may be substituted, include halogen (for example, fluorine, chlorine, bromine, iodine, etc.), nitro, cyano, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- to 6-membered cycloamino such as tetrahydropyrrole, piperazine, piperidine, morpholine, thiomorpholine, pyrrole, imidazole, etc., or the like), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), $C_{1-4}$ alkyl which may be halogenated (for example, trifluoromethyl, methyl, ethyl, etc.), $C_{1-4}$ alkoxy which may be halogenated (for example, methoxy, ethoxy, trifluoromethoxy, trifluoroethoxy, etc.), formyl, $C_{2-4}$ alkanoyl (for example, acetyl, propionyl, etc.), $C_{1-4}$ alkylsulfonyl (for example, methanesulfonyl, ethanesulfonyl, etc.), and the like; and the number of the substituents is preferably 1 to 3.

Further, in the above-described formula, $R^9$ and $R^{10}$ may be bonded to each other to form a cyclic group (preferably, 5- to 7-membered ring) together with the adjacent phosphorus atom. Such cyclic group may be substituted, and examples of the substituents include halogen (for example, fluorine, chlorine, bromine, iodine, etc.), nitro, cyano, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- to 6-membered cycloamino such as tetrahydropyrrole, piperazine, piperidine, morpholine, thiomorpholine, pyrrole, imidazole, etc., or the like), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc.), $C_{1-4}$ alkyl which may be halogenated (for example, trifluoromethyl, methyl, ethyl, etc.), $C_{1-4}$ alkoxy which may be halogenated (for example, methoxy, ethoxy, trifluoromethoxy, trifluoroethoxy, etc.), formyl, $C_{2-4}$ alkanoyl (for example, acetyl, propionyl, etc.), $C_{1-4}$ alkylsulfonyl (for example, methanesulfonyl, ethanesulfonyl, etc.), and the like; and the number of the substituents is preferably 1 to 3.

In the above formula (I), the counter anion for the case where the phosphorus atom forms a phosphonium salt, may be exemplified by, in addition to anions of halogen (for example, $Cl^-$, $Br^-$, $I^-$, etc.), anions derived from inorganic acids such as hydrochloric acid, hydrobromic acid, nitric acid, sulfuric acid, phosphoric acid, etc.; anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; and anions derived from acidic amino acids such as aspartic acid, glutamic acid, etc., and among them, $Cl^-$, $Br^-$, $I^-$ and the like are preferred.

The amino group which may be substituted represented by $R^9$ and $R^{10}$ may be exemplified by an amino group which may have one or two of:

(1) alkyl which may be substituted (for example, $C_{1-10}$ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower ($C_{1-6}$) alkyl, or the like) ;
(2) cycloalkyl which may be substituted (for example, $C_{3-7}$ cycloalkyl such as cyclopropyl, cyclobutyl, cyclopentyl, cyclohexyl, cycloheptyl, etc., or the like);
(3) alkenyl which may be substituted (for example, alkenyl having 2 to 10 carbon atoms, such as allyl, crotyl, 2-pentenyl, 3-hexenyl, etc., and preferably lower ($C_{2-6}$) alkenyl, or the like);
(4) cycloalkenyl which may be substituted (for example, cycloalkenyl having 3 to 7 carbon atoms, such as 2-cyclopentenyl, 2-cyclohexenyl, 2-cyclopentenylmethyl, 2-cyclohexenylmethyl, etc., or the like);
(5) formyl, or acyl which may be substituted (for example, alkanoyl having 2 to 4 carbon atoms (for example, acetyl, propionyl, butyryl, isobutyryl, etc.), alkylsulfonyl having 1 to 4 carbon atoms (for example, methanesulfonyl, ethanesulfonyl, etc.), or the like);
(6) aryl which may be substituted (for example, phenyl, naphthyl, etc.); or the like.

Examples of the substituent of the above-described (1) alkyl which may be substituted, (2) cycloalkyl which may be substituted, (3) alkenyl which may be substituted, (4) cycloalkenyl which may be substituted, (5) acyl which may be substituted, and (6) aryl which may be substituted, include halogen (for example, fluorine, chlorine, bromine, iodine, etc.), nitro, cyano, hydroxy group, thiol group which may be substituted (for example, thiol, $C_{1-4}$ alkylthio, etc.), amino group which may be substituted (for example, amino, mono-$C_{1-4}$ alkylamino, di-$C_{1-4}$ alkylamino, 5- to 6-membered cycloamino such as tetrahydropyrrole, piperazine, piperidine, morpholine, thiomorpholine, pyrrole, imidazole, etc.), carboxyl group which may be esterified or amidated (for example, carboxyl, $C_{1-4}$ alkoxycarbonyl, carbamoyl, mono-$C_{1-4}$ alkylcarbamoyl, di-$C_{1-4}$ alkylcarbamoyl, etc,), $C_{1-4}$ alkyl which may be halogenated (for example, trifluoromethyl, methyl, ethyl, etc.); $C_{1-4}$ alkoxy which may be halogenated (for example, methoxy, ethoxy, trifluoromethoxy, trifluoroethoxy, etc.), formyl, $C_{2-4}$ alkanoyl (for example, acetyl, propionyl, etc.), $C_{1-4}$ alkylsulfonyl (for example, methanesulfonyl, ethanesulfonyl, etc.), and the like, and the number of the substituents is preferably 1 to 3.

The substituent of the "amidino group which may be substituted" and the "guanidino group which may be substituted" represented by $R^2$, may be exemplified by the same one as the substituent of the above-described "amino

which may be substituted, in which the nitrogen atom may be converted to a quaternary ammonium or an oxide" represented by $R^2$.

$R^2$ is preferably (1) an amino group which may be substituted, in which the nitrogen atom may be converted to a quaternary ammonium or an oxide, (2) a nitrogen-containing heterocyclic group which may be substituted and may contain a sulfur atom or an oxygen atom as the ring-constituting atom, in which the nitrogen atom may be converted to a quaternary ammonium or an oxide, (3) an amidino group which may be substituted, or (4) a guanidino group which may be substituted. $R^2$ is more preferably an amino group which may be substituted, in which the nitrogen atom may be converted to a quaternary ammonium; a nitrogen-containing heterocyclic group which may be substituted and may contain a sulfur atom or an oxygen atom as the ring-constituting atom, which may be converted to an oxide; or the like. In particular, it is preferably an amino group which may be substituted; a nitrogen-containing heterocyclic group which may be substituted and may contain a sulfur atom or an oxygen atom as the ring-constituting atom; or the like.

$R^2$ is more preferably a group represented by the formula -NRR" or $-N^+RR'R''$, wherein R, R' and R" are each an aliphatic hydrocarbon group (linear aliphatic hydrocarbon group and cyclic aliphatic hydrocarbon group) which may be substituted, or an alicyclic (non-aromatic) heterocyclic group which may be substituted, or a nitrogen-containing aromatic heterocyclic group which may be substituted, in which the nitrogen atom may be converted to an oxide.

In the above formulas, the "aliphatic hydrocarbon group which may be substituted" and the "alicyclic heterocyclic group which may be substituted" represented by R, R' and R" may be exemplified by the same one as the "aliphatic hydrocarbon group which may be substituted (for example, alkyl, cycloalkyl, alkenyl, cycloalkenyl, etc., each of which may be substituted)" and the "alicyclic heterocyclic group which may be substituted (for example, 5- or 6-membered non-aromatic heterocycle which may be substituted, etc.)" that are exemplified as the substituent which may be carried by the "amino which may be substituted" represented by substituent $R^2$.

Among them, R and R' are each preferably a linear hydrocarbon group which may be substituted (for example, alkyl, alkenyl, etc., each of which may be substituted), more preferably a $C_{1-6}$ alkyl group which may be substituted, and particularly preferably a methyl group which may be substituted.

R" is preferably an alicyclic hydrocarbon group which may be substituted (preferably, a $C_{3-8}$ cycloalkyl group which may be substituted; more preferably, cyclohexyl which may be substituted) or an alicyclic heterocyclic group which may be substituted (preferably, a saturated alicyclic heterocyclic group which may be substituted (preferably, a 6-membered cyclic group); more preferably, tetrahydropyranyl which may be substituted, tetrahydrothiopyranyl which may be substituted, or piperidyl which may be substituted; particularly preferably, tetrahydropyranyl which may be substituted).

Furthermore, as the "nitrogen-containing aromatic heterocyclic group" of the "nitrogen-containing aromatic heterocyclic group which may be substituted, in which the nitrogen atom may be converted to an oxide" represented by $R^2$ , pyridine, imidazole, triazole and imidazopyridine are exemplified as preferred, and among these, imidazole and triazole are particularly preferred.

The "amino group which may be substituted, in which the nitrogen atom may be converted to a quaternary ammonium or an oxide" represented by $R^{2'}$ and $R^{2''}$ or the like may be exemplified respectively by the same one as the group corresponding to the above-described $R^2$.

The compound represented by formula (I) is preferably the compound of the following:

(Ss)-(2E)-3-[4'-(2-butoxyethoxy)-4-(2-methylpyrrolidin-1-yl)-1,1'-biphenyl-3-yl]-2-methyl-N-[4-[[(1-propyl-1H-imidazol-5-yl)methyl]sulfinyl]phenyl]acrylamide, (Ss)-(2E)-3-[4'-(2-butoxyethoxy)-4-(3-hydroxypyrrolidin-1-yl)-1,1'-biphenyl-3-yl]-2-methyl-N-[4-[[(1-propyl-1H-imidazol-5-yl)methyl]sulfinyl]phenyl]acrylamide, (Ss)-(2E)-3-[4-(3-(acetoxymethyl)pyrrolidin-1-yl)-4'-(2-butoxyethoxy)-1,1'-biphenyl-3-yl]-2-methyl-N-[4-[[(1-propyl-1H-imidazol-5-yl)methyl]sulfinyl]phenyl]acrylamide, (Ss)-(2E)-3-[4'-(2-butoxyethoxy)-4-(3-(methoxycarbonyl)pyrrolidin-1-yl]-1,1'-biphenyl-3-yl]-2-methyl-N-[4-[[(1-propyl-1H-imidazol-5-yl)methyl]sulfinyl]phenyl]acrylamide, (Ss)-(2E)-3-[4'-(2-butoxyethoxy)-4-(3-carbamoylpyrrolidin-1-yl)-1,1'-biphenyl-3-yl]-2-methyl-N-[4-[[(1-propyl-1H-imidazol-5-yl)methyl]sulfinyl]phenyl]acrylamide, (Ss)-(2E)-3-[4'-(2-butoxyethoxy)-4-(3-methylpyrrolidin-1-yl)-1,1'-biphenyl-3-yl]-2-methyl-N-[4-[[(1-propyl-1H-imidazol-5-yl)methyl]sulfinyl]phenyl]acrylamide, (Ss)-(2E)-3-[4'-(2-butoxyethoxy)-4-(3-(hydroxymethyl)pyrrolidin-1-yl)-1,1'-biphenyl-3-yl]-2-methyl-N-[4-[[(1-propyl-1H-imidazol-5-yl)methyl]sulfinyl]phenyl]acrylamide, (Ss)-(2E)-3-[4'-(2-butoxyethoxy)-4-(3-carboxypyrrolidin-1-yl)-1,1'-biphenyl-3-yl]-2-methyl-N-[4-[[(1-propyl-1H-imidazol-5-yl)methyl]sulfinyl]phenyl]acrylamide and diastereomers thereof,

(Ss)-(2E)-3-[5-[4-(2-butoxyethoxy)phenyl]-2-(3-methylpyrrolidin-1-yl)pyridin-3-yl]-2-methyl-N-[4-[[(1-propyl-1H-imidazol-5-yl)methyl]sulfinyl]phenyl]acrylamide, (Ss)-(2E)-3-[2-[3-(acetoxymethyl)pyrrolidin-1-yl]-5-[4-(2-butoxyethoxy)phenyl]pyridin-3-yl]-2-methyl-N-[4-[[(1-propyl-1H-imidazol-5-yl)methyl]sulfinyl]phenyl]acrylamide, (Ss)-(2E)-3-[5-[4-(2-butoxyethoxy)phenyl]-2-[3-(hydroxymethyl)pyrrolidin-1-yl)pyridin-3-yl]-2-methyl-N-[4-[[(1-propyl-1H-imidazol-5-yl)methyl]sulfinyl]phenyl]acrylamide and diastereomers thereof,

(S)-(2E)-3-[4'-(2-butoxyethoxy)-4-pyrrolidin-1-yl-1,1'-biphenyl-3-yl]-N-[4-[[(1-propyl-1H-imidazol-5-yl)methyl]sulfinyl]phenyl]but-2-enamide, (S)-(2E)-3-[4'-(2-butoxyethoxy)-4-(3,4-dimethylpyrrolidin-1-yl)-1,1'-biphenyl-3-yl]-2-methyl-N-[4-[[(1-propyl-1H-imidazol-5-yl)methyl]sulfinyl]phenyl]acrylamide, (S)-(2E)-3-[5-[4-(2-butoxyethoxy) phenyl]-2-pyrrolidin-1-ylpyridin-3-yl]-2-methyl-N-[4-[[(1-propyl-1H-imidazol-5-yl) methyl] sulfinyl]phenyl] acrylamide, (S)-(2E)-3-[6-[4-(2-butoxyethoxy)phenyl]-1-methyl-1,2,3,4-tetrahydroquinolin-8-yl]-2-methyl-N-[4-[[(1-propyl-1H-imidazol-5-yl)methyl]sulfinyl]phenyl]acrylamide and the like.

The salt of the compound represented by formula (I) of the present invention is preferably a pharmaceutically acceptable salt and may be exemplified by salts with inorganic bases, salts with organic bases, salts with inorganic acids, salts with organic acids, salts with basic or acidic amino acids, or the like. Preferred examples of the salt with inorganic base include alkali metal salts such as sodium salt, potassium salt, etc.; alkaline earth metal salts such as calcium salt, magnesium salt, etc.; aluminum salt, ammonium salt and the like. Preferred examples of the salt with organic base include salts with trimethylamine, triethylamine, pyridine, picoline, ethanolamine, diethanolamine, triethanolamine, dicyclohexylamine, N,N'-dibenzylethylenediamine and the like. Preferred examples of the salt with inorganic acid include salts with hydrochloric acid, hydrobromic acid, nitric acid, sulfuric acid, phosphoric acid and the like. Preferred examples of the salt with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like. Preferred examples of the salt with basic amino acid include salts with arginine, lysine, ornithine and the like. Preferred examples of the salt with acidic amino acid include salts with aspartic acid, glutamic acid and the like. The compound represented by formula (I) of the present invention may be either hydrate or anhydrate. When the compound represented by formula (I) of the present invention exists as configurational isomers, diastereomers, conformers or the like, each form can be isolated by the separation and purification means that are known per se in the art, if desired. Further, when the compound represented by formula (I) is a racemate, the (S) and (R) isomers may be separated by general means for optical resolution, and each of the optical isomers as well as the racemates is included in the scope of the present invention.

The prodrug of the compound represented by formula (I) used in the invention or a salt thereof [hereinafter, may be sometimes referred to as Compound (I)] refers to a compound which is converted to Compound (I) by an in vivo reaction caused by an enzyme, gastric acid or the like under physiological conditions, that is, a compound which is converted to Compound (I) upon occurrence of enzymatic oxidation, reduction, hydrolysis or the like, or a compound which is converted to Compound (I) upon occurrence of hydrolysis or the like by gastric acid or the like. The prodrug of Compound (I) may be exemplified by compounds resulting from acylation, alkylation or phosphorylation of the amino group of Compound (I) (for example, the compounds in which the amino group of Compound (I) is in the form of eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, tert-butyl or the like); compounds resulting from acylation, alkylation, phosphorylation or boration of the hydroxy group of Compound (I) (for example, the compounds in which the hydroxy group of Compound (I) is in the form of acetyl, palmitoyl, propanoyl, pivaloyl, succinyl, fumaryl, alanyl, dimethylaminomethylcarbonyl or the like); compounds resulting from esterification or amidation of the carboxyl group of Compound (I) (for example, the compounds in which the carboxyl group of Compound (I) is in the form of ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methylamide or the like); or the like. These compounds can be prepared from Compound (I) by methods known per se in the art.

Furthermore, the prodrug of Compound (I) may be also a compound which is converted to Compound (I) under physiological conditions, as described in "Development of Pharmaceutical Products", Vol.7, Design of Molecules, Hirokawa Publisher, pp.163-198 (1990).

Also, Compound (I) may be labeled with isotopes (for example, $^{3}H$, $^{14}C$, $^{35}S$, $^{125}I$, etc.).

Hereinafter, a process for producing the compound represented by formula (I) or a salt thereof will be explained.

The compound represented by formula (I) or a salt thereof can be produced by those processes known per se. For example, it can be produced by the following processes. In addition, the compound of formula (I) or a salt thereof can be produced by the process described in JP-A No. 8-73476 or a similar method thereto.

The compounds which will be used in each of the following processes may form salts similar to the salt of Compound (I), as far as the salts do not interfere with reactions.

Further, in the following reactions, when the starting compounds have amino, carboxyl or hydroxy groups as substituents, these groups may be protected by protective groups which are commonly used in peptide chemistry, and if necessary, the protective groups may be removed after the reactions to obtain desired compounds.

Examples of the protective group to be used for an amino group include $C_{1-6}$ alkylcarbonyl which may be substituted (for example, acetyl, propionyl, etc), formyl, phenylcarbonyl, $C_{1-6}$ alkyloxycarbonyl (for example, methoxycarbonyl, ethoxycarbonyl, t-butoxycarbonyl, etc.), phenyloxycarbonyl (for example, benzoxycarbonyl, etc.), $C_{7-10}$ aralkyloxycarbonyl (for example, benzyloxycarbonyl, etc.), trityl, phthaloyl and the like. Examples of the substituent of the above protective groups include halogen atom (for example, fluorine, chlorine, bromine, iodine, etc.), $C_{1-6}$ alkyl-

carbonyl (for example, acetyl, propionyl, butyryl, etc.), nitro group and the like, and the number of the substituents is about 1 to 3.

Examples of the protective group to be used for a carboxyl group include $C_{1-6}$ alkyl which may be substituted (for example, methyl, ethyl, propyl, isopropyl, butyl, tert-butyl, etc.), phenyl, trityl, silyl and the like. Examples of these substituents include halogen atom (for example fluorine, chlorine, bromine, iodine, etc.), $C_{1-6}$ alkylcarbonyl (for example, acetyl, propionyl, butyryl, etc.), formyl, nitro and the like, and the number of the substituents is about 1 to 3.

Examples of the protective group to be used for a hydroxy group include $C_{1-6}$ alkyl which may be substituted (for example, methyl, ethyl, propyl, isopropyl, butyl, tert-butyl, etc.), phenyl, $C_{7-10}$ aralkyl (for example, benzyl, etc.), $C_{1-6}$ alkylcarbonyl (for example, acetyl, propionyl, etc.), formyl, phenyloxycarbonyl, $C_{7-10}$ aralkyloxycarbonyl (for example, benzyloxycarbonyl, etc.), pyranyl, furanyl, silyl and the like. The substituents of these protective groups include halogen atom (for example, fluorine, chlorine, bromine, iodine, etc.), $C_{1-6}$ alkyl, phenyl, $C_{7-10}$ aralkyl, nitro and the like, and the number of the substituents is about 1 to 4.

Introduction and removal of protective groups are carried out according to those methods known per se or similar methods thereto [for example, the method described in "Protective Groups in Organic Chemistry", (J.F.W. McOmie et al., Plenum Press)], and removal is carried out by, for example, the methods of treating with an acid, a base, a reducing agent, ultraviolet light, hydrazone, phenylhydrazine, sodium N-methyldithiocarbamate, tetrabutylammonium fluoride, palladium acetate or the like.

In the following description, the compounds represented by formulas (I), (Ia), (Ib), (II) and (III) including their salts may be also simply referred to as Compound (I), Compound (Ia), Compound (Ib), Compound (II) and Compound (III).

[Process A]

Compound (I) can be prepared by reacting Compound (II) with Compound (III) according to the following reaction:

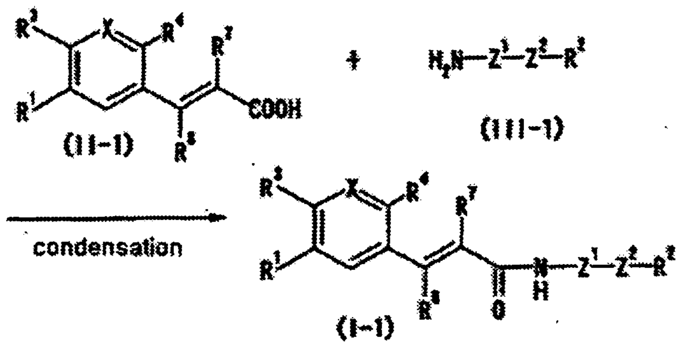


wherein each symbol has the same meaning as defined above.

In this reaction, a carboxylic acid derivative (II) is reacted with an amine derivative (III) to prepare Compound (I).

The condensation reaction of Compound (II) and Compound (III) may be conducted by a conventional means for peptide synthesis. The means for peptide synthesis may be carried out according to any method known in the art, for example, the methods described in M. Bodansky and M.A. Ondetti, Peptide Synthesis, Interscience, New York (1996); F.M. Finn and K. Hofmann, The Proteins, Vol.2; H. Nenrath and R.L. Hill, Ed., Academic Press Inc., New York (1976); and Nobuo Izumiya et al., Foundation and Experiments in Peptide Synthesis, Maruzen (1985), which include, for example, azide method, chloride method, acid anhydride method, mixed acid anhydride method, DCC method, activated ester method, method using Woodward's Reagent K, carbonyldiimidazole method, oxidation/reduction method, DCC/HONB method, as well as WSC method, diethyl cyanophosphate (DEPC) method and the like. In other words, examples of the reactive derivatives that may be used include acid halides (for example, acid chloride, acid bromide, etc.), acid azides, acid anhydrides, mixed acid anhydrides [for example, mixed acid anhydrides of mono-$C_{1-6}$ alkylcarbonic acid (for example, mixed acid anhydrides of a free acid with monomethylcarbonic acid, monoethylcarbonic acid, monoisopropylcarbonic acid, monoisobutylcarbonic acid, mono-tert-butylcarbonic acid, monobenzylcarbonic acid, mono(p-nitrobenzyl)carbonic acid, or monoallylcarbonic acid, etc.), mixed acid anhydrides of $C_{1-6}$ aliphatic carboxylic acid (for example, mixed acid anhydrides of a free acid with acetic acid, trichloroacetic acid, cyanoacetic acid, propionic acid, butyric acid, isobutyric acid, valeric acid, isovaleric acid, pivalic acid, trifluoroacetic acid, trichloroacetic acid, acetoacetic acid, etc.), mixed acid anhydrides of $C_{7-12}$ aromatic carboxylic acid (for example, mixed acid anhydrides

of a free acid with benzoic acid, p-toluic acid, p-chlorobenzoic acid, etc.), mixed acid anhydrides of organic sulfonic acid (for example, mixed acid anhydrides of a free acid with methanesulfonic acid, ethanesulfonic acid, benzenesulfonic acid, p-toluenesulfonic acid, etc.) etc.], activated amides, activated esters (for example, diethoxyphosphoric acid ester, diphenoxyphosphoric acid ester, p-nitrophenyl ester, 2,4-dinitrophenyl ester, etc.), activated thioesters (for example, 2-pyridylthiol ester, 2-benzothiazolylthiol ester, etc.) and the like. This condensation reaction can be carried out in a solvent. Examples of the solvent include N,N-dimethylformamide, dimethylsulfoxide, pyridine, chloroform, dichloromethane, tetrahydrofuran, dioxane, acetonitrile, each of which is dehydrated or hydrated, or appropriate mixtures thereof. The reaction temperature is usually about -20°C to about 50°C, and preferably about -10°C to about 30°C. The reaction time is about 1 to about 100 hours, and preferably about 2 to about 40 hours. The thus-obtained Compound (I-1) can be isolated and purified by known separation and purification means such as concentration, vacuum concentration, solvent extraction, crystallization, recrystallization, resolubilization, chromatography or the like.

[Process B]

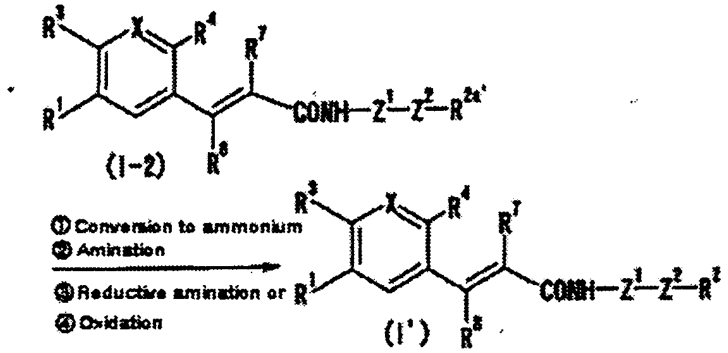


(I-2) (I')

(1) When $R^{2a'}$ as represented in Compound (I-2) is, for example, a tertiary amine residue, Compound (I-2) can be reacted with an alkyl halide or an aralkyl halide to prepare a quaternized Compound (I'). Herein, examples of the halogen atom include chlorine, bromine, iodine, etc, and the alkyl halide (for example, a lower ($C_{1-6}$) alkyl halide, etc.), or the aralkyl halide (for example, a lower ($C_{1-4}$) alkylphenyl halide, etc.) is typically used in an amount of about 1 to 5 moles with respect to 1 mole of Compound (I-2). The reaction may be carried out in an inert solvent, for example, toluene, benzene, xylene, dichloromethane, chloroform, 1,2-dichloroethane, dimethylformamide (DMF), dimethylacetamide, etc., or a mixture of the solvents above. The reaction temperature is in a range of about 10°C to about 160°C, and preferably about 20°C to about 120°C. The reaction time is about 1 to about 100 hours, and preferably about 2 to about 40 hours. The reaction is preferably carried out under an inert gas atmosphere (for example, nitrogen, argon, etc.).

(2) When $R^{2a'}$ as represented in Compound (I-2) is, for example, a secondary amine residue, Compound (I-2) can be reacted with an alkyl halide or an aralkyl halide to prepare a tertiarized Compound (I'). Herein, examples of the halogen atom include chlorine, bromine, iodine, etc., and the alkyl halide or aralkyl halide is typically used in an amount of about 1 to 2 moles with respect to 1 mole of Compound (I-2). The reaction can be facilitated, if necessary, by addition of about 1 to about 3 moles of a base such as triethylamine, diisopropylethylamine, pyridine, lithium hydride, sodium hydride, sodium methoxide, sodium ethoxide, sodium carbonate, potassium carbonate, sodium hydrogen carbonate or the like, and by further adding sodium iodide, potassium iodide, or the like.

This reaction of tertiary amination can be carried out in an inert solvent such as methanol, ethanol, propanol, isopropanol, n-butanol, tetrahydrofuran, diethyl ether, dimethoxyethane, 1,4-dioxane, toluene, benzene, xylene, dichloromethane, chloroform, 1,2-dichloroethane, dimethylformamide (DMF), dimethylsulfoxide (DMSO), pyridine, etc., or a mixture of the solvents above. The reaction is carried out at a temperature ranging from about 0°C to about 180°C for about 1 to about 40 hours. Also, the reaction is preferably carried out under an inert gas atmosphere (for example, nitrogen, argon, etc.).

(3) When $R^{2a'}$ as represented in Compound (I-2) is, for example, a secondary amine residue, Compound (I-2) can be reacted with an aldehyde compound in the presence of a reductive amino reagent such as sodium triacetoxyborohydride, sodium cyanoborohydride, sodium borohydride or the like to prepare a tertiarized Compound (I'). The reaction conditions for this reductive amination reaction are preferably changed depending on the reagent used.

For example, when sodium triacetoxyborohydride is used, the reaction is preferably conducted in an inert solvent, for example, dichloromethane, chloroform, 1,2-dichloroethane, tetrahydrofuran (THF), diethyl ether, dioxane, acetonitrile, dimethylformamide (DMF), etc., or a mixture of the solvents above. The reagent is used in an amount of about 1 to 2 molar equivalents with respect to 1 mole of Compound (I-2). The reaction is usually carried out at a temperature ranging from about 0°C to about 80°C for about 1 to about 40 hours. The reaction is preferably carried out under an inert gas atmosphere (for example, nitrogen, argon, etc.).

(4) When $R^{2a'}$ as represented in Compound (I-2) is, for example, a sulfide residue or a tertiary amine residue, or when $Z^2$ is, for example, a sulfide residue, Compound (I-2) can be reacted with an oxidizing agent, for example, m-chloroperbenzoic acid, perbenzoic acid, p-nitroperbenzoic acid, magnesium monoperoxyphthalate, peracetic acid, hydrogen peroxide, sodium periodate, potassium periodate, etc., to prepare a Compound (I') having a sulfinyl group, a sulfonyl group or an amine oxide group. The reaction conditions for this oxidation reaction are preferably changed in accordance with the oxidizing agent used. For example, when m-chloroperbenzoic acid is used, the reaction may be carried out in an inert solvent, for example, dichloromethane, chloroform, 1,2-dichloroethane, diethyl ether, tetrahydrofuran, acetone, ethyl acetate, etc., or a mixture of the solvents above. The oxidizing agent is used in an amount of about 1 to 3 molar equivalents with respect to 1 mole of Compound (I-2). The reaction is usually carried out at a temperature ranging from about -78°C to about 80°C (preferably from -50 to 25°C), for about 1 to about 40 hours.

Alternatively, when $Z^2$ as represented in Compound (I-2) is, for example, a sulfide residue, a Compound (I') having an optically active sulfinyl group can be prepared according to methods that are known per se in the art, for example, the method described in Ojima, I., Ed., Catalytic Asymmetric Synthesis, 2000, Wiley-VCH (New York), or a similar method thereto.

[Process C]

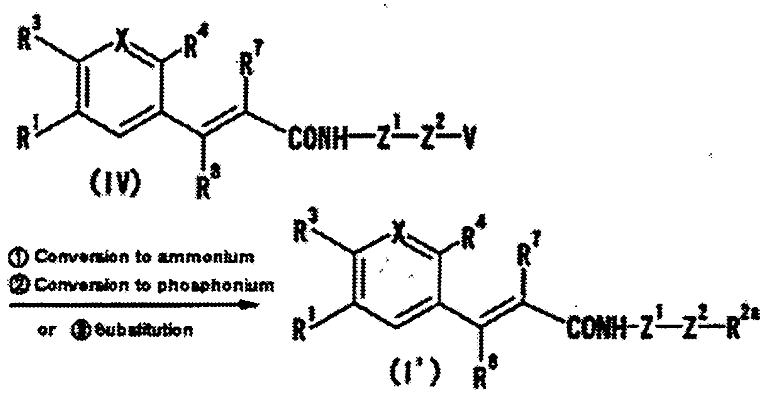


V of Compound (IV) represents a halogen atom (chlorine, bromine, iodine, etc.) or a sulfonyloxy group (methanesulfonyloxy group, trifluoromethanesulfonyloxy group, benzenesulfonyloxy group, toluenesulfonyloxy group, etc.), and the other symbols have the same meanings as defined above.

(1) Compound (IV) can be reacted with a tertiary amine to prepare a quaternized Compound (I'). This reaction can be carried out in an inert solvent, for example, toluene, benzene, xylene, dichloromethane, chloroform, 1,2-dichloroethane, dimethylformamide (DMF), dimethylacetamide, etc., or a mixture of the solvents above. The tertiary amine is used in an amount of about 1 to 3 moles with respect to 1 mole of Compound (IV). The reaction is carried out at a temperature ranging from about 10°C to about 120°C for about 1 to about 40 hours. The reaction is preferably carried out under an inert gas atmosphere (for example, nitrogen, argon, etc.).

(2) Compound (IV) can be reacted with a tertiary phosphine to prepare a quaternized Compound (I'). This reaction can be carried out in an inert solvent, for example, toluene, benzene, xylene, dichloromethane, chloroform, 1,2-dichloroethane, acetonitrile, dimethylformamide (DMF), etc., or a mixture of the solvents above. The tertiary phosphine is used in an amount of about 1 to 2 moles with respect to 1 mole of Compound (IV). The reaction is carried out at a temperature ranging from about 20°C to about 150°C for about 1 to about 50 hours. The reaction

is preferably carried out under an inert gas atmosphere (for example, nitrogen, argon, etc.).

(3) Compound (IV) can be reacted with a primary or a secondary amine compound or a thiol compound to prepare a Compound (I') having a secondary or tertiary amino group or a thio group. The primary or secondary amine compound or the thiol compound is usually used in an amount of about 1 to 3 moles with respect to 1 mole of Compound (IV). This reaction can be facilitated, if necessary, by adding about an equivalent to three-fold moles of a base such as triethylamine, diisopropylethylamine, pyridine, lithium hydride, sodium hydride, sodium methoxide, sodium ethoxide, sodium carbonate, potassium carbonate, sodium hydrogen carbonate or the like, and by further adding sodium iodide, potassium iodide or the like. The substitution reaction can be carried out in an inert solvent, for example, methanol, ethanol, propanol, isopropanol, n-butanol, tetrahydrofuran, diethyl ether, dimethoxyethane, 1,4-dioxane, toluene, benzene, xylene, dichloromethane, chloroform, 1,2-dichloroethane, dimethylformamide (DMF), dimethylsulfoxide (DMSO), pyridine, etc., or a mixture of the solvents above. The reaction is carried out at a temperature ranging from about -10°C to about 180°C for about 1 to about 40 hours. The reaction is preferably carried out under an inert gas atmosphere (for example, nitrogen, argon, etc.).

[Process D]

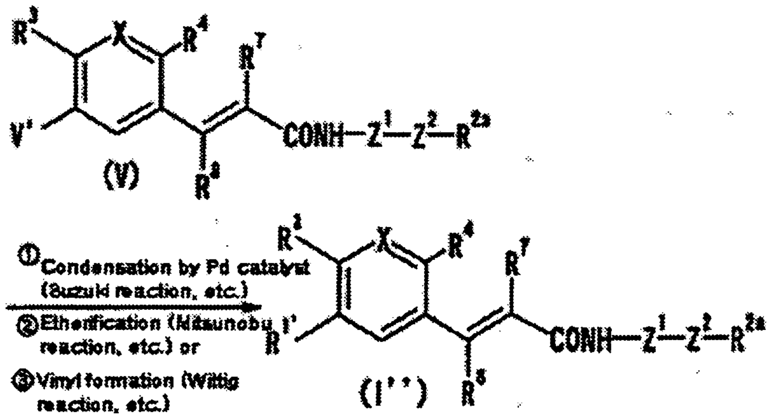


(1) Compound (V), wherein V' represents a halogen atom (bromine, iodine, etc.) or a sulfonyloxy group (trifluoromethanesulfonyloxy group, etc), and the other symbols have the same meanings as described above, can besubjected to, for example, the Suzuki reaction [a cross-condensation reaction of an arylboric acid and, for example, an aryl halide or aryloxytrifluoromethanesulfonate, catalyzed by a palladium catalyst; A. Suzuki et al., Synth. Commun., 11, 513 (1981)], to prepare a Compound (I") in which $X^1$ is a bond, and $R^{1'}$ is a 5- or 6-membered aromatic group. The aryl borate can be used in an amount of about an equivalent to 1.5-fold moles with respect to 1 mole of Compound (V) to give Compound (I").

Further, Compound (V) can be subjected to, for example, a cross-condensation reaction with an arylacetylene compound in the presence of a palladium catalyst [dichlorobis(triphenylphosphine)palladium, etc.] [K.S.Y. Lau et al., J. Org. Chem., 46, 2280 (1981); J.W. Tilley, S. Zawoisky et al., J, Org. Chem., 53, 386 (1988)] to give a Compound (I") having an acetylene bond, in which $X^1$ represents -C≡C-. The arylacetylene compound can be used typically in an amount of about an equivalent to two-fold moles with respect to 1 mole of Compound (V) to prepare Compound (I").

(2) Compound (V), wherein V' represents a hydroxy group, and the other symbols have the same meanings as described above, can be subjected to, for example, the Mitsunobu reaction [an etherification reaction using, for example, triphenylphosphine and diethyl azodicarboxylate as the condensing agents; O. Mitsunobu et al., Synthesis, 1 (1981)] to prepare Compound (I") having an ether bond. The corresponding alcohol compound or phenol compound can be used in an amount of about an equivalent to three-fold moles with respect to 1 mole of Compound (V) to prepare Compound (I").

The Compound (I") having an ether bond can also be prepared by an etherification reaction of Compound (V) with a reactive compound such as a halide (chloride, bromide, iodide, etc.) compound, a tosylate compound, a mesylate compound, etc. The reactive compound is used typically in an amount of about an equivalent to two-fold moles with respect to 1 mole of Compound (V). This reaction can be facilitated, if necessary, by adding about an equivalent to three-fold moles of a base such as triethylamine, diisopropylethylamine, pyridine, lithium hydride,

sodium hydride, sodium hydroxide, potassium hydroxide, sodium methoxide, sodium ethoxide, sodium carbonate, potassium carbonate, sodium hydrogen carbonate, etc., and by further adding sodium iodide, potassium iodide, etc. The reaction may be carried out in an inert solvent such as tetrahydrofuran, diethyl ether, dimethoxyethane, 1,4-dioxane, toluene, benzene, xylene, dichloromethane, chloroform, 1,2-dichloroethane, dimethylformamide (DMF), dimethylsulfoxide (DMSO), pyridine, etc., or a mixture of the solvents above. The reaction is carried out at a temperature ranging from about -10°C to 180°C for about 1 to about 40 hours. The reaction is preferably carried out under an inert gas atmosphere (for example, nitrogen, argon, etc.).

(3) Compound (V), wherein V' represents a carbonyl group which may be substituted, a phosphonium salt or a phosphonic acid ester residue, and the other symbols have the same meanings as defined above, can be subjected to, for example, the Wittig reaction [A. Maercker, Org. React., 14, 270 (1965)] or the Wittig-Horner-Emmons reaction [J. Boutagy and R. Thomas, Chem. Rev., 74, 87 (1974)] to prepare a Compound (I") having a vinyl bond. The corresponding carbonyl compound, phosphonium salt or phosphonic acid ester compound is used in an amount of about an equivalent to 1.5-fold moles with respect to 1 mole of Compound (V).

[Process E]

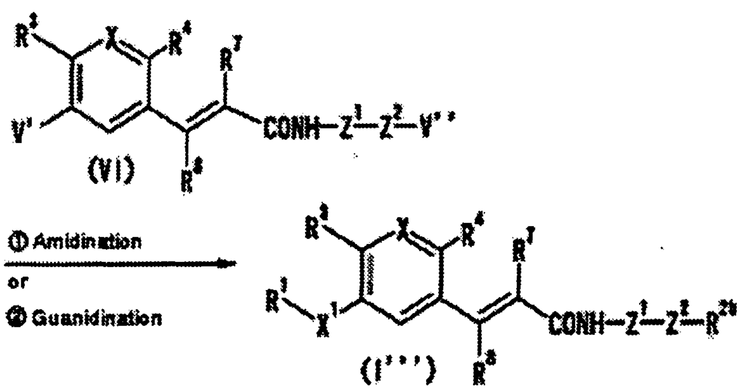


(1) First, Compound (VI), wherein V" represents a cyano group, and the other symbols have the same meanings as defined above, is reacted with a lower alcohol such as methanol, ethanol, propanol, etc. in the presence of an acid such as hydrochloric acid to give an imidate compound. This reaction is typically carried out using an excess of said alcohol, at a temperature ranging from about -10°C to 50°C for about 1 hour to about 40 hours. The reaction can be conducted in an inert solvent such as diethyl ether, 1,4-dioxane, toluene, benzene, xylene, dichloromethane, chloroform, 1,2-dichloroethane, etc., or a mixture of the solvents above.

Subsequently, the resulting imidate compound can be subjected to a substitution reaction with a primary or secondary amine compound to prepare an amidine compound [I''']. The primary or secondary amine compound is used typically in an amount of about 1 to 5 moles with respect to 1 mole of the imidate compound. The reaction can be facilitated, if necessary, by adding about an equivalent to three-fold moles of a demineralizing agent such as triethylamine, pyridine, sodium hydroxide, potassium hydroxide, sodium methoxide, sodium ethoxide, sodium carbonate, potassium carbonate, etc. This substitution reaction may be conducted in an inert solvent, for example, methanol, ethanol, propanol, isopropanol, n-butanol, tetrahydrofuran, diethyl ether, dimethoxyethane, 1,4-dioxane, toluene, benzene, xylene, dichloromethane, chloroform, 1,2-dichloroethane, dimethylformamide (DMF), dimethylsulfoxide (DMSO), pyridine, etc., or a mixture of the solvents above. The reaction is carried out at a temperature ranging from about 0°C to 150°C for about 1 to about 50 hours. The reaction is also preferably conducted under an inert gas (for example, nitrogen, argon, etc.) atmosphere.

(2) Compound (VI), wherein V" is an amino group, and the other symbols have the same meanings as defined above), can be subjected to a substitution reaction with an S-alkyl (for example, methyl, ethyl, etc.)-isothiourea compound to give a guanidine Compound (I'''). The S-alkyl-isothiourea compound is typically used in an amount of about an equivalent to two-fold moles with respect to 1 mole of Compound (VI). This reaction can be facilitated, if necessary, by adding about an equivalent to three-fold moles of a demineralizing agent such as triethylamine, pyridine, sodium hydroxide, potassium hydroxide, sodium methoxide, sodium ethoxide, sodium carbonate, potassium carbonate, etc. The substitution reaction may be carried out in an inert solvent, for example, methanol, ethanol, propanol, isopropanol, n-butanol, tetrahydrofuran, diethyl ether, dimethoxyethane, 1,4-dioxane, toluene, benzene,

xylene, dichloromethane, chloroform, 1,2-dichloroethane, dimethylformamide (DMF), dimethylsulfoxide (DMSO), pyridine, etc., or a mixture of the solvents above. The reaction is conducted at a temperature ranging from about 0°C to 150°C for about 1 to about 50 hours. The reaction is also preferably carried out under an inert gas atmosphere (for example, nitrogen, argon, etc.).

The thus-obtained Compound (I) can be isolated and purified by known separation and purification means, for example, concentration, vacuum concentration, solvent extraction, crystallization, recrystallization, resolubilization, chromatography and the like.

Compound (II-1) which is used as the starting material may be prepared by any known methods (for example, the methods described in JP-A No. 11-263764; and JP-A No. 2001-026586, etc.) or similar methods thereto, for example, the method of reaction scheme I, methods in Reference Examples described below and modifications thereof.

Reaction Scheme I

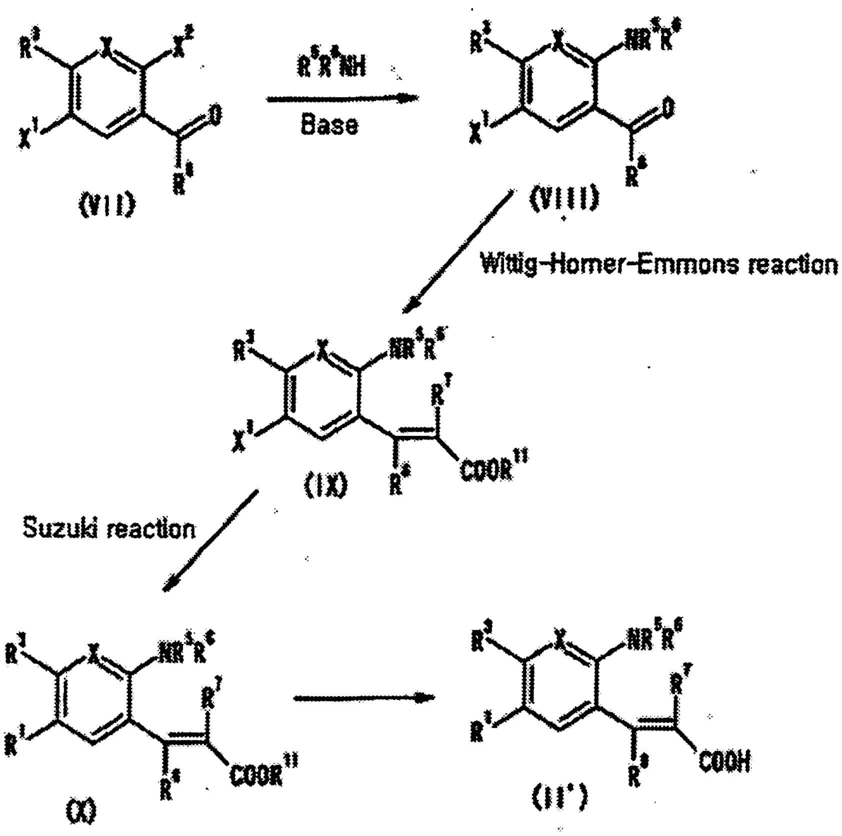


wherein $R^{11}$ represents a $C_{1-4}$ alkyl group, $X^1$ and $X^2$ each represent a leaving group [halogen atom (chlorine, bromine, iodine, etc.), methanesulfonyloxy, trifluoromethanesulfonyl, benzenesulfonyloxy, toluenesulfonyloxy, etc.], and the other symbols have the same meanings as defined above.

Compound (VII) can be subjected to a condensation reaction with an amine compound in the presence of a base, to prepare Compound (VIII). An unsaturated carboxylic acid ester (IX) can be prepared by subjecting Compound (VIII) to, for example, the Wittig reaction [A. Maercker, Org. React., 14, 270 (1965)] or the Wittig-Horner-Emmons reaction [J. Boutagy and R. Thomas, Chem. Rev., 74, 87 (1974)]. Compound (IX) can be subjected to, for example, the Suzuki reaction and subsequently to an ester hydrolysis reaction, to prepare an unsaturated carboxylic acid Compound (II').

Compound (II-1) which is used as the starting material can be prepared by any known methods (for example, the methods described in JP-A No. 8-73476; and JP-A No. 2001-058988, etc.) or similar methods thereto, for example,

the method of reaction scheme I, methods in Reference Examples described below and modifications thereof.

Compound (III-1) also can be prepared by any known methods (for example, the method described in JP-A No. 8-73476, etc.) or similar methods thereto, for example, the method of reaction scheme III, methods in Reference Examples described below and modifications thereof.

Reaction Scheme III

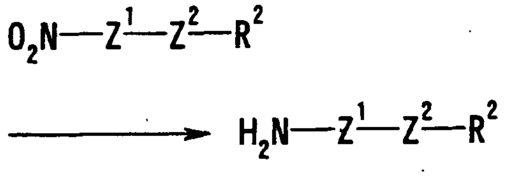

(XI)

(III-1)

wherein each symbol has the same meaning as defined above.

Reduction of Compound (XI) can be carried out by methods that are known per se in the art. For example, reduction by metal, metal hydride or metal hydrogen complex compound, reduction by diborane and substituted borane, catalytic hydrogenation, or the like is used. That is, this reaction is carried out by treating Compound (XI) with a reducing agent. Examples of the reducing agent include metals such as reduced iron, zinc powder, etc.; metal hydrogen complex compounds such as alkali metal borohydrides (for example, sodium borohydride, lithium borohydride, etc.), aluminum lithium hydride, etc.; metal hydrides such as sodium hydride, etc.; organic tin compounds (triphenyltin hydride, etc.); metals and metal salts such as nickel compounds, zinc compounds, etc.; catalytic reducing agents using hydrogen and transition metal catalysts such as palladium, platinum, rhodium, etc.; diborane; and the like. The catalytic reduction using hydrogen and a transition metal such as palladium, platinum, rhodium, etc., and the reduction by a metal such as reduced iron are advantageously employed. The reaction is carried out in an organic solvent which does not interfere with the reaction. The solvent is appropriately selected for use from, for example, benzene, toluene, xylene, chloroform, carbon tetrachloride, dichloromethane, 1,2-dichloroethane, 1,1,2,2-tetrachloroethane, diethyl ether, tetrahydrofuran, dioxane, methanol, ethanol, propanol, isopropanol, 2-methoxyethanol, N,N-dimethylformamide, acetic acid or a mixture of the solvents above, depending on the type of reducing agent. The reaction temperature is about -20°C to about 150°C, and particularly preferably about 0°C to about 100°C, while the reaction time is about 1 to about 24 hours.

The thus-obtained Compound (III-1) can be isolated and purified by known separation and purification means such as concentration, vacuum concentration, solvent extraction, crystallization, recrystallization, resolubilization, chromatography and the like.

The compound represented by formula (I) of the present invention or a salt thereof including the above-mentioned Compound (I-1), Compound (I-2), Compound (I'), Compound (I") and Compound (I'") (hereinafter, when it is said "the compound represented by formula (I)" in brief, it means to include a salt thereof and the compound represented by formula (I) and a salt thereof) can be administered orally or parenterally alone or by as a pharmaceutical composition comprising the compound mixed with a pharmaceutically acceptable carrier in the form of a solid preparation such as tablet, capsule, granule, powder, etc., or a liquid preparation such as syrup, injectable solution, etc.

Examples of the dosage form for parenteral administration include injectable solution, infusion, suppository, vaginal suppository, etc., and in particular, a vaginal suppository is useful for prevention of HIV infection.

As the pharmaceutically acceptable carrier, a variety of organic or inorganic carriers that are commonly used as materials for pharmaceutical preparation may be used, and they are added as excipient, lubricant, binder and disintegrant in solid preparations, and as solvent, solubilizing agent, suspending agent, isotonic agent, buffer, soothing agent or the like in liquid preparations. Other additives for preparation such as antiseptic agent, antioxidant, colorant, sweetener or the like may also be used, if necessary. Preferred examples of the excipient include lactose, sucrose, D-mannitol, starch, crystalline cellulose, light anhydrous silica and the like. Preferred examples of the lubricant include magnesium stearate, calcium stearate, talc, colloidal silica and the like. Preferred examples of the binder include crystalline cellulose, sucrose, D-mannitol, dextrin, hydroxypropylcellulose, hydroxypropylmethylcellulose, polyvinylpyrrolidone and the like. Preferred examples of the disintegrant include starch, carboxymethylcellulose, calcium carboxymethylcellulose, sodium croscarmellose, sodium carboxymethylstarch and the like. Preferred examples of the solvent include water for injection, alcohol, propylene glycol, macrogol, sesame oil, corn oil and the like. Preferred examples of the solubilizing agent include polyethylene glycol, propylene glycol, D-mannitol, benzyl benzoate, ethanol, trisaminomethane, cholesterol, triethanolamine, sodium carbonate, sodium citrate and the like. Preferred examples of the suspending agent include surfactants such as stearyltriethanolamine, sodium laurylsulfate, laurylaminopropionic acid, lecithin, benzalkonium chloride, benzethonium chloride, glycerin monostearate, etc.; and hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose, etc.; and the like. Preferred examples of the isotonic agent include sodium chloride, glycerin, D-mannose and the like. Preferred examples of the buffer include buffer solutions of salts such as phosphate, acetate, carbonate, citrate and the like. Preferred examples of the soothing agent include benzyl alcohol and the like. Preferred examples of the antiseptic agent include para-oxybenzoic acid esters, chlorobutanol, benzyl alcohol, phenethyl alcohol, dehydroacetic acid, sorbic acid and the like. Preferred examples of the antioxidant include sulfite salts, ascorbic acid and the like.

The compound represented by formula (I) of the present invention or a salt thereof has excellent CCR antagonistic action, in particular, CCR5 and/or CCR2 antagonistic action, and especially, a strong CCR5 antagonistic action, and therefore may be used in prevention and treatment of human HIV infection, for example, AIDS, and also in prevention and treatment of other various diseases. Further, the compound represented by formula (I) of the present invention or a salt thereof has low toxicity and can be used safely.

For example, the pharmaceutical composition containing the compound represented by formula (I) of the present invention or a salt thereof can be used as a CCR5 antagonist, for example, a prophylactic and/or therapeutic agent for AIDS and a suppressive agent for disease progression of AIDS. Furthermore, the pharmaceutical composition containing the compound represented by formula (I) of the present invention or a salt thereof may be used as a prophylactic and/or therapeutic agent for a variety of diseases, such as a prophylactic and/or therapeutic agent for graft-versus-host diseases (GVHD) and/or rejection reaction, a prophylactic and/or therapeutic agent for chronic rheumatoid arthritis, autoimmune diseases, allergic diseases, ischemic brain cell disorder, cardiac infarction, chronic nephritis and arteriosclerosis, and the like.

Examples of the diseases for which the prophylactic and/or therapeutic agent of the present invention is used, include graft rejection (posttransplantational rejection, posttransplantational polycythemia, hypertension, organ disorder, vascular hypertrophy, graft-versus-host diseases, etc.); arthritic osteopathic diseases such as periostitis, meningitis, etc. (chronic rheumatoid arthritis, osteoarthritis deformans, rheumatoid myelitis, osteoporosis, abnormal growth of cell, fracture, refracture, osteomalacia, osseous Behcet's disease, rigorous myelitis, articular tissue destruction by gonarthritis deformans and diseases similar thereto, etc.); autoimmune diseases (collagen disease , SLE(systemic lupus erythematosus), pachyderma, polyarteritis, myasthenia gravis, multiple sclerosis, etc.); allergic diseases (allergic nasal catarrh, conjunctivitis, gastrointestinal allergy, pollinosis, anaphylaxis, atopic dermatitis, bronchial asthma, etc.); inflammatory enteropathic diseases (ulcerative colitis, Crohn's disease, gastritis, gastric ulcer, gastric cancer, postgastrotomic disorder, dyspepsia, esophageal ulcer, pancreatitis, polyp of the colon, cholelithiasis, hemorrhoids, peptic ulcer, situational ileitis, etc.); inflammatory diseases (retinopathy, postoperative and posttraumatic inflammation, remission of puffiness, pharyngitis, cystitis, meningitis, inflammatory ophthalmic diseases, etc.); respiratory diseases (cold syndrome, pneumonia, asthma, pulmonary hypertension, pulmonary thrombi/pulmonary obliteration, pulmonary sarcoidosis, pulmonary tuberculosis, interstitial pneumonia, silicosis, adult tachypnea syndrome, chronic obliterative pulmonary diseases, etc.); infectious diseases (viral infection caused by cytomegalovirus, influenzavirus, herpesvirus and the like, Rickettsia infection, bacterial infection, sexually transmitted diseases, carinii pneumonia, Helicobacter pylori infection, systemic fungal infection, tuberculosis, invasive Staphylococcal infection, acute viral encephalitis, acute bacterial meningitis, AIDS encephalopathy, septicemia, sepsis, sepsis gravis, septic shock, endotoxin shock, toxic shock syndrome, etc.); cancers and accompanying cachexia, cancer metastases (bladder cancer, breast cancer, cervical cancer, ovarian cancer, chronic lymphoblastic leukemia, chronic myeloid leukemia, colon cancer, rectal cancer, colic cancer, multiple myeloma, malignant myeloma, prostatic cancer, lung cancer, gastric cancer, Hodgkin's disease, malignant melanoma, malignant lymphoma, etc.); non-Hodgkin's lymphoma; non-small cell lung cancer; malignant melanoma, neurodegenerative diseases (Alzheimer's disease, Parkinson's disease, amyotrophic lateral sclerosis (ALS), Huntington's chorea, diabetic neural disorder, Creutzfeldt-Jakob disease, etc.); mental diseases (depression, epilepsy, alcoholism etc.); schizophrenia; venous dysfunction; central nerve disorder (disorder and aftereffect/complication from intracerebral bleeding, brain infarction and the like, cephalic trauma, spinal damage, brain edema, sensory malfunction, sensory dysfunction, autonomic nervous malfunction, autonomic nervous dysfunction, etc.); central damage (cephalic trauma, spinal damage, whiplash injury, etc.); vascular dementia (multi-infarct dementia, Binswanger's disease, etc.); cerebrovascular accident (asymptomatic cerebrovascular accident, transient cerebral ischemic attack, stroke, cerebrovascular dementia, hypertensive encephalopathy, etc.); recurrence and aftereffect of cerebrovascular accident (neural symptoms, mental symptoms, subjective symptoms, operational disorder in daily life, etc.); cerebral vascular dementia; post-cerebrovascular obliteration central hypofunction; disorder or abnormality in autoregulation of cerebral circulation and renal circulation; blood brain barrier disorder; anxiety symptom; acute coronary artery syndromes including unstable angina, etc.; anxious mental state; amnesia; prosopalgia; otolaryngological diseases (Meniere's syndrome, tinnitus, gustation disorder, dizziness, dysequilibrium, dysphagia, etc.); migraine; chronic pain; dermatoses (keloid, angioma, psoriasis, etc.); arteriosclerosis obliterans; thromboangiitis obliterans; peripheral obstruction; postischemic reperfusion injury; Raynaud's disease; Buerger's disease; myocarditis; cardiac ischemia; cardiac infarction; progress of cardiac failure after cardiac infarction; cardiomyopathy; cardiac hypertrophy; acute cardiac failure and chronic (including estatic) cardiac failure; angina pectoris; arrhythmia; tachycardia; circadian rhythm disorder

of blood pressure; abnormality in characteristic of blood haemocyte components (enhancement in platelet aggregation, abnormality of erythrocyte deformability, enhancement in leucocyte adhesiveness, increase in blood viscosity, polycythemia , vascular peliosis, autoimmune hemolytic anemia, disseminated intravascular coagulation syndrome, multiple myelopathy, etc.); arteriosclerosis including atherosclerosis (aneurysm, coronary arteriosclerosis, cerebral arteriosclerosis, peripheral arteriosclerosis, etc.); vascular reocclusion and restenosis after bypass operation; vascular hyperplasia or occlusion and organ malfunction after intervention (transdermal coronary arterioplasty, stent detention, coronary autoscope, vascular ultrasound therapy, coronary injection thrombolytic therapy, etc.); production and enhancement of vasoactive materials and thrombi inducing materials (endothelin, thromboxane A2, etc.); arterialization (including abnormal vasculogenesis in abnormal capillary vasoganglion formation in atherosclerotic outer membrane); thrombosis; fat storage disease acceleration; ophthalmic diseases (glaucoma, ocular hypertension, etc.); hypertension; hypertensive tinnitus; dialysis hypotension; endothelial cell and organ disorders; endocrinopathy (Addison's disease, Cushing's syndrome, melanocytoma, primary aldosteronism, etc.); nephritis; renal diseases (nephritis, glomerulonephritis, glomerulosclerosis, renal failure, thrombotic microangiopathy, dialysis complications, organ disorders including nephropathy by radiation, diabetic nephropathy, etc.); diabetic diseases (insulin-dependent diabetes, diabetic complications, diabetic retinopathy, diabetic microangiopathy, diabetic neuropathy, etc.); glucose tolerance abnormality; hepatic diseases (hepatitis (including chronic hepatitis), hepatic cirrhosis, etc.); interstitial hepatic diseases; chronic pancreatitis; portal pressure enhancement; obesity; male sterility; gynecologic diseases (climacteric disorder, gestational toxicosis, endometriosis, hysteromyoma, ovarian diseases, mammary diseases, etc.); edema; chronic fatigue syndromes; prostatomegaly; Behcet's disease; Hodgkin's disease; lacunar infarction; consciousness disorder; psoriasis; diseases due to environmental or occupational factors (disorder caused by radiation, disorders caused by ultraviolet ray/infrared ray/laser ray, altitude sickness, etc.); intermittent claudication; and the like.

The pharmaceutical composition containing the compound represented by formula (I) or a salt thereof may vary depending on the kind of disease to be treated and may be used in combination with other drugs. Examples of the other drugs include HDL-increasing drugs [squalene synthase inhibitor, CETP inhibitor, LPL activator, etc.]; prophylactic and/or therapeutic agents for HIV infection [nucleic acid reverse transcriptase inhibitors such as zidovudine, didanosine, zalcitabine, lamivudine, stavudine, abacavir, adefovir, adefovir dipivoxil, fozivudine tidoxil, etc., non-nucleic acid reverse transcriptase inhibitors such as nevirapine, delavirdine, efavirenz, loviride, immunocal, oltipraz, etc., protease inhibitors such as saquinavir, ritonavir, indinavir, nelfinavir, amprenavir, palinavir, lasinavir, lopinavir, etc.]; NMG-CoA reductase inhibitors [cerivastatin, atorvastatin, pravastatin, simvastatin, itavastatin, lovastatin, fluvastatin, (+)-3R,5S-7-[4-[4-fluorophenyl]-6-isopropyl-2-(N-methyl-N-methanesulfonylamino]pyrimidin-5-yl]-3,5-dihydroxy-6(E)-heptenoic acid, etc.]; atopic dermatitis drugs [sodium cromoglycate, etc.]; allergic nasal catarrh drugs [sodium cromoglycate, chlorpheniramine maleate, alimemazine tartrate, clemastine fumarate, homochlorcyclizine hydrochloride, terfenadine, mequitazine, etc.]; imipenem·cilastatin sodium; endotoxin antagonists or antibodies; oxidosqualene-lanosterol cyclases [e.g., decalin derivatives, azadecalin derivatives and indane derivatives]; calcium antagonists (diltiazem, etc.); glycerol; cholinesterase inhibitors (e.g., Aricept (donepezil), etc.); compounds suppressing cholesterol uptake [e.g., sitosterol, neomycin, etc.]; compounds inhibiting cholesterol biosyntheses [e.g., HMG-CoA reductase inhibitors such as lovastatin, simvastatin, pravastatin, etc.];

cyclooxygenase inhibitors [Cox-I, Cox-II inhibitors such as celecoxib, rofecoxib, salicylic acid derivatives such as aspirin and the like, diclofenac, indometacin, loxoprofen, etc.]; signal transduction inhibitors, squalene epoxidase inhibitors [e.g., NB-598 and the analogous compounds, etc.]; steroidal drugs [dexamethasone, hexestrol, methimazole, betamethasone, triamcinolone, triamcinolone acetonide, fluocinonide, fluocinolone acetonide, prednisolone, methylprednisolone, cortisone acetate, hydrocortisone, fluorometholone, beclomethasone propionate, estriol, etc.]; diacerin; nicotinic acid and derivatives and analogues thereof [e.g., acipimox and probucol]; nicergoline, nephrotic syndrome drugs: prednisolone (Predonine), prednisolone sodium succinate (Predonine), methylprednisolone sodium succinate (Solumedrol), betamethasone (Rinderon), dipyridamole (Persantine), dilazep hydrochloride (Comelian), ticlopidine, clopidogrel, antiplatelet drugs and anticoagulants such as FXa inhibitors, etc.; barpital-based anticonvulsants or anaesthetic drugs (phenobarbital, mephobarbital, metharbital, etc.); Parkinson's disease drugs (e.g., L-DOPA, etc.); histamine receptor blockers (cimetidine, famotidine, etc.); hydantoin-based anticonvulsant drugs (phenytoin, mephenytoin, ethotoin, etc.); piroxicam, fibrates [e.g., clofibrate, benzafibrate, gemfibrozil, etc.]; prostaglandins; megestrol acetate; gastric and intraduodenal ulcer drugs: antacids [e.g., histamine H2 antagonists (cimetidine, etc.), proton pump inhibitors (lansoprazole, etc.), etc.]; inflammatory mediator inhibitors; coronary vasodilators: nifedipine, diltiazem, nicoradil, nitrite drugs, etc.; infectious disease drugs: [e.g., antibiotic formulations (cefotiam hydrochloride, cefozopran hydrochloride, ampicillin, etc.), chemotherapeutic agents (sulfa drugs, synthetic antibacterial agents, antiviral agents, etc.), biological formulations (vaccines, blood preparations including immunoglobulins) etc.] etc.; hepatic disease drugs: glycyrrhizin formulations [e.g., Stronger Minophagen, etc.]; liver hydrolysate; SH compounds [e.g., glutathione, etc.]; special amino acid formulations [e.g., aminoleban, etc.]; phospholipids [e.g., polyene-phosphatidylcholine, etc.]; vitamins [e.g., vitamin $B_1$, $B_2$, $B_6$, $B_{12}$, C, etc.]; adrenocortical hormones [e.g., dexamethasone, betamethasone, etc.]; interferons [e.g., interferon $\alpha$, $\beta$, etc.]; hepatic encephalopathy drugs [e.g., lactulose, etc.];

hemostatic agents used in cases of rupture of esophageal and gastric varices [e.g., vasopressin, somatostatin, etc.] etc.; arthritis drugs; muscle relaxants [pridinol, tubocurarine, pancuronium, tolperisone hydrochloride, chlorphenesin carbamate, baclofen, chlormezanone, mephenesin, chlorzoxazone, eperisone, tizanidine, etc.]; vasodilators [oxyfedrine, diltiazem, tolazoline, hexobendine, bamethan, clonidine, methyldopa, guanabenz, etc.]; vasoconstrictors [dopamine, dobutamine, denopamine, etc.]; platelet coagulation inhibitors (ozagrel, etc.); thrombogenesis prophylactic and/or therapeutic drugs: anticoagulant drugs [e.g., heparin sodium, heparin calcium, warfarin calcium (Warfarin), Xa inhibitor]; thrombolytic drugs [e.g., tPA, urokinase]; antiplatelet drugs [e.g., aspirin, sulfinpyrazone (Anturan), dipyridamole (Persantine), ticlopidine (Panaldine), cilostazol (Pletal), GPIIb/IIIa antagonists (ReoPro)]; antidepressants [imipramine, clomipramine, noxiptiline, fenelzin, amitriptyline hydrochloride, nortriptyline hydrochloride, amoxapine, mianserin hydrochloride, maprotiline hydrochloride, sulpiride, fluvoxamine maleate, trazodone hydrochloride, etc.]; antiepileptic drugs [gabapentin, phenytoin, ethosuximide, acetazolamide, chlordiazepoxide, trimethadione, carbamazepine, phenobarbital, primidone, sultiame, sodium valproate, clonazepam, diazepam, nitrazepam, etc.]; antiallergic drugs [diphenhydramine, chlorpheniramine, tripelennamine, methodilamine, clemizole, diphenylpyraline, methoxyphenamine, sodium cromoglycate, tranilast, repirinast, amlexanox, ibudilast, ketotifen, terfenadine, mequitazine, azalastine, epinastine, ozagrel hydrochloride, pranlukast hydrate, seratrodast, fexofenadine, ebastine, bucillamine, oxatomide, Stronger Neo-Minophagen C, tranexamic acid, ketotifen fumarate, etc.]; anticholinergic drugs (e.g., ipratropium bromide, flutropium bromide, oxitropium bromide, etc.); anti-Parkinson drugs (dopamine, levodopa, etc.); antirheumatic drugs; anti-inflammatory drugs (e.g., aspirin, acetaminophen, diclofenac sodium, ibuprofen, indometacin, loxoprofen sodium, dexamethasone, etc.); anticoagulant and antiplatelet drugs [sodium citrate, activated protein C, tissue factor pathway inhibitors, antithrombin III, dalteparin sodium, argatroban, gabexate, ozagrel sodium, ethyl icosapentate, beraprost sodium, alprostadil, pentoxifylline, tisokinase, streptokinase, hebarin, etc.]; anticoagulant therapeutic drugs [dipyridamole (Bersantine), dilazep hydrochloride (Comelian), ticlopidine, clopidogrel, Xa inhibitors]; antibacterial drugs [(1) sulfa drugs [sulfamethizole, sulfisoxazole, sulfamonomethoxine, sulfamethizole, salazosulfapyridine, sulfadiazine silver, etc.], (2) quinoline-based antibacterial drugs [nalidixic acid, pipemidic acid trihydrate, enoxacin, norfloxacin, ofloxacin, tosufloxacin tosilate, ciprofloxacin hydrochloride, lomefloxacin hydrochloride, sparfloxacin, fleroxacin, etc.], (3) antituberculous drugs [isoniazid, ethambutol (ethambutol hydrochloride), p-aminosalicylic acid (calcium p-aminosalicylate), pyrazinamide, ethionamide, prothionamide, rifampicin, streptomycin sulfate, kanamycin sulfate, cycloserine, etc.], (4) anti-acid fast bacterial drugs [diaphenylsulfone, rifampicilin, etc.], (5) antiviral drugs [idoxuridine, acyclovir, vidarabine, ganciclovir, etc.], (6) anti-HIV drugs [zidovudine, didanosine, zalcitabine, indinavir sulfate ethanolate, ritonavir, etc.], (7) spirocheticide, (8) antibiotics [tetracycline hydrochloride, ampicillin, piperacillin, gentamicin, dibekacin, kanendomycin, lividomycin, tobramycin, amikacin, fradiomycin, sisomicin, tetracycline, oxytetracycline, rolitetracycline, doxycycline, ampicillin, piperacillin, ticarcillin, cephalothin, cephapirin, cephaloridine, cefaclor, cephalexin, cefroxadine, cefadroxil, cefamandole, cefotiam, cefuroxime, cefotiam, cefotiam hexetil, cefuroxime axetil, cefdinir, cefditoren pivoxil, ceftazidime, cefpiramide, cefsulodin, cefmenoxime, cefpodoxime proxetil, cefpirome, cefozopran, cefepime, cefsulodin, cefmetazole, cefminox, cefoxitin, cefbuperazone, latamoxef, flomoxef, cefazolin, cefotaxime, cefoperazone, ceftizoxime, moxalactam, thienamycin, sulfazecin, aztreonam or salts thereof, griseofulvin, lankacidins [J. Antibiotics, 38, 877-885 (1985)], etc.],

cefixime, levofloxacin]; antithrombotic drugs (argatroban, etc.); antiprotozoal drugs [metronidazole, tinidazole, diethylcarbamazine citrate, quinine hydrochloride, quinine sulfate, etc.]; antitumor drugs [6-O-(N-chloroacetylcarbamoyl]fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, peplomycin sulfate, vincristine sulfate, vinblastine sulfate, irinotecan hydrochloride, cyclophosphamide, melphalan, busulfan, thiotepa, procarbazine hydrochloride, cisplatin, azathioprine, mercaptopurine, tegafur, carmofur, cytarabine, methyltestosterone, testosterone propionate, testosterone enanthate, mepitiostane, fosfestrol, chlormadinone acetate, leuproline acetate, buserelin acetate, etc.]; antifungal drugs [(1) polyethylene-based antibiotics (e.g., amphotericin B, nystatin, trichomycin), (2) griseofulvin, pyrrolnitrin, etc., (3) cytosine metabolism antagonists (e.g., flucytosine), (4) imidazole derivatives (e.g., econazole, clotrimazole, miconazole nitrate, bifonazole, croconazole), (5) triazole derivatives (e.g., fluconazole, itoraconazole, azole compounds [2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl-3-(2H,4H)-1,2,4-triazolone], (6) thiocarbamate derivatives [e.g., trinaphthol], (7) echinocandin-based derivatives (e.g., caspofungin, FK-463, V-echinocandin), etc.]; antipsychotic drugs [chlorpromazine hydrochloride, prochlorperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, reserpine, clocapramine hydrochloride, sulpiride, zotepine, etc.];

antiulcer drugs [metoclopramide, histidine hydrochloride, lansoprazole, metoclopramide, pirenzepine, cimetidine, ranitidine, famotidine, urogastron, oxethazaine, proglumide, omeprazole, sucralfate, sulpiride, cetraxate, gefarnate, aldioxa, teprenone, prostaglandins, etc.]; antidiabetic drugs [e.g., pioglitazone, nateglinide, voglibose, acarbose, etc.]; antiobesity drugs (mazindol, etc.); antirheumatic drugs, etc.; antianxiety drugs [diazepam, lorazepam, oxazepam, chlordiazepoxide, medazepam, oxazolam, cloxazolam, clotiazepam, bromazepam, etizolam, fludiazepam, hydroxyzine, etc.]; antiarrhythmic drugs : disopyramide, lidocaine, quinidine sulfate, flecainide acetate, mexiletine hydrochloride, amiodarone hydrochloride, and β blockers, Ca antagonists, etc.; antiasthmatic drugs [isoprenaline hydrochloride, salbutamol sulfate, procaterol hydrochloride, terbutaline sulfate, trimetoxynol hydrochloride, tulobuterol hydrochloride, orciprenaline sulfate, fenoterol hydrobromide, ephedrine hydrochloride, ipratropium bromide, oxitropium bromide, flutropium bromide, theophylline, aminophylline, sodium cromoglycate, tranilast, repirinast, amlexanox, ibudilast, ketotifen, terfenadine, mequitazine, azelastine, epinastine, ozagrel hydrochloride, pranlukast hydrate, seratrodast, dexamethasone, prednisolone, hydrocortisone, beclomethasone propionate, fluticasone propionate, beclomethasone propionate, procaterol, etc.]; anti-hypothyroidism drugs [dried thyroid (Thyreoid), levothyroxine sodium (Thyradin S), liothyronine sodium (thyronine, tyronamine)]; nephrotic syndrome drugs [prednisolone (Predonine), prednisolone sodium succinate (Predonine), methylprednisolone sodium succinate (Solumedrol), betamethasone (Rinderon)]; antihypertensive drugs [(1) sympathetic nerve inhibitors [α2 stimulants (e.g., clonidine, guanabenz, guanfacine, methyldopa, etc.), ganglionic blockers (e.g., hexamethonium, trimethaphan, etc.), presynaptic blockers (e.g., ArsA-Oxylone, dimethylaminoreserpinate, rescinnamine, reserpine, syrosingopine, etc.), neuronal blockers (e.g., betanidine , guanethidine, etc.), α1 blockers (e.g., bunazosin, doxazosin, prazosin, terazosin, urapidil, etc.),

β blockers (e.g., propranolol, nadolol, timolol, nipradilol, bunitrolol, indenolol, penbutolol, carteolol, carvedilol, pindolol, acebutolol, atenolol, pisoprolol, metoprolol, labetalol, amosulalol, arotinolol, etc.), etc.], (2) vasodilators [calcium channel antagonists (e.g., manidipine, nicardipine, nilvadipine, nisoldipine, nitrendipine, benidipine, amlodipine, aranidipine, etc.), phthalazine derivatives (e.g., budralazine, cadralazine, ecarazine, hydralazine, todralazine, etc.), etc.], (3) ACE inhibitors [alacepril, captopril, cilazapril, delapril, enalapril, lisinopril, temocapril, trandolapril, quinapril, imidapril, benazepril, perindopril, etc.)], (4) AII antagonists [losartan, candesartan, valsartan, telmisartan, irbesartan, forasartan, etc.], (5) diuretic drugs [e.g., diuretic drugs described above, etc.]; antihypertensive drugs: diuretic drugs [e.g., furosemide (Lasix), bumetanide (Lunetoron), azosemide (Diart)], antihypertensive drugs [e.g., ACE inhibitors, (enalapril maleate (Renivace), etc.) and Ca antagonists (manidipine, amlodipine, etc.), α or β receptor blockers, etc.], antihyperlipemia drugs [HMG-CoA reductase inhibitors (e.g., fluvastatin, cerivastatin, atorvastatin, etc.), fibrates [e.g., simfibrate, aluminum clofibrate, clinofibrate, fenofibrate, etc.], anion exchange resins (e.g., cholestyramine, etc.), nicotinic acid drugs (e.g., nicomol, niceritrol, tocopherol nicotinate etc.), polyvalent unsaturated fatty acid derivatives (e. g., ethyl icosapentate, polyene phosphatidylcholine, melinamide, etc.), phytosterols (e.g., gamma-oryzanol, soy sterol, etc.), elastase, sodium dextran sulfate, squalene synthase inhibitors, CETP inhibitors, ethyl 2-chloro-3[4-(2-methyl-2-phenylpropoxy)phenyl]propionate [Chem. Pharm. Bull., 38, 2792-2796 (1990)], etc.]; osteopathic disease drugs: calcium formulations (e.g., calcium carbonate, etc.), calcitonin formulations, activated vitamin $D_3$ formulations (e.g., alfacalcidol (Alfarol, etc.), calcitriol (Rocaltrol), etc.),

sex hormones (e.g., estrogen, estrandiol, etc.), hormone formulations [e.g., conjugated estrogen (Premarin), etc.], ibriflavone formulations [Osten, etc.], vitamin $K_2$, vitamin $K_2$ formulations [e.g., menatetrenone (Glakay), etc.], bisphosphonate-based formulations (etidronate, etc.), prostaglandin E2, fluorine compounds (e.g., sodium fluoride, etc.), bone morphogenetic protein (BMP), fibroblast growth factor (FGF), platelet derived growth factor (PDGF), transforming growth factor (TGF-β), insulin-like growth factor-1 and -2 (IGF-1,-2), parathyroid adrenal hormones (PTH), and compounds described in EP-A1-376197, EP-A1-460488, and EP-A1-719782 (e.g., (2R,4S)-(-)-N-[4-(diethoxyphosphorylmethyl)phenyl]-1,2,4,5-tetrahydro-4-methyl-7,8-methylenedioxy-5-oxo-3-benzothiepin-2-carboxamide, etc.), etc., fat-soluble vitamin drugs [(1) vitamin A family: vitamin $A_1$, vitamin $A_2$, and retinol palmitate, (2) vitamin D family: vitamin $D_1$, $D_2$, $D_3$, $D_4$ and $D_5$, (3) vitamin E family: α-tocopherol, β-tocopherol, γ-tocopherol, 8-tocopherol, dl-α-tocopherol nicotinate, (4) vitamin K family: vitamin $K_1$, $K_2$, $K_3$ and $K_4$, (5) folic acids (vitamin M, etc.); vitamin derivatives [various vitamin derivatives, e.g., vitamin $D_3$ derivatives such as 5,6-trans-cholecalciferol, 2,5-hydroxycholecalciferol, 1-α-hydroxycholecalciferol, vitamin $D_2$ derivatives such as 5,6-trans-ergocalciferol, and the like]; disease-modifying antirheumatic and immunosuppressive drugs [e.g., methotrexate, leflunomide, prograf, sulfasalazine, D-penicillamine, oral gold drugs]; hypertensors [dopamine, dobutamine, denopamine, digitoxin, digoxin, methyldigoxin, lanatoside C, G-strophanthin, etc.]; myocardial protective drugs: heart ATP-K opener, Na-H exchange inhibitors, endothelin antagonists, urotensin antagonist, etc., cardiac failure drugs [cardiac stimulants (e.g., digitoxin, digoxin, methyldigoxin, lanatoside C, proscillaridin, etc.), α, β stimulants (e.g., epinephrine, norepinephrine, isoproterenol, dopamine, docarpamine, dobutamine, denopamine, etc.), phosphodiesterase inhibitors (e.g., amrinone, milrinone, olprinone hydrochloride, etc.),

calcium channel sensitivity enhancers (e.g., pimobentan, etc.), nitrate drugs (e.g., nitroglycerin, isosorbide nitrate, etc.), ACE inhibitors (e.g., the ACE inhibitor described above, etc.), diuretic drugs (e.g., diuretic drugs described above, etc.), calperitide, ubidecarenone, vesnarinone, aminophylline, etc.]; neurotrophic factors; renal failure and nephropathy drugs; biological formulations [e.g., monoclonal antibodies (e.g., anti-TNF-α antibodies, anti-IL-12 antibodies, anti-IL-6 antibodies, anti-ICAM-I antibodies, anti-CD4 antibodies, etc.), soluble receptors (e.g., soluble TNF-α receptors, etc.), protein ligands (IL-I receptor antagonist, etc.)]; bile acid binding resins [e.g., cholestyramine, cholestipol, etc.]; biliary tract disease drugs: cholepoietic drugs [e.g., dehydrocholic acid, etc.], cholekinetic drugs [e.g., magnesium sulfate, etc.], etc.; central nervous system agonists: antianxiety drugs, hypnotic and sedative drugs, anesthetic drugs, spasmolytic drugs, autonomic drugs, anti-Parkinson drugs and other psychoneuro drugs, etc.; antitussive and expectorants [ephedrine hydrochloride, noscapine hydrochloride, codeine phosphate, dihydrocodeine phosphate, isoproterenol hydrochloride, ephedrine hydrochloride, methylephedrine hydrochloride, alloclamide, clofedanol, picoperidamine, cloperastine, protokylol, isoproterenol, salbutamol, terbutaline, oxymetebanol, morphine hydrochloride, dextromethorphan hydrobromide, oxycodone hydrochloride, dimemorfan phosphate, tipepidine hibenzate, pentoxyverine citrate, clofedanol hydrochloride, benzonatate, guaifenesin, bromhexine hydrochloride, ambroxol hydrochloride, acetylcysteine, ethylcysteine hydrochloride, carbocisteine, etc.], sedative drugs [chlorpromazine hydrochloride, atropine sulfate, phenobarbital, barbital, amobarbital, pentobarbital, thiopental sodium, thiamylal sodium, nitrazepam, estazolam, flurazepam, haloxazolam, triazolam, flunitrazepam, bromovalerylurea, chloral hydrate, triclofos sodium, etc.], analgesic and antiphlogistic drugs [e.g., central analgesic drugs (e.g., morphine, codeine, pentazocine etc.), steroidal drugs (e.g., prednisolone, dexamethasone, betamethasone, etc.), antiphlogistic enzymic drugs (e.g., bromelain, lysozymes, proctase, etc.)], diabetic drugs [sulfonylurea drugs (e.g., tolbutamide, chlorpropamide, glyclopyramide, acetohexamide, tolazamide, glibenclamide, glibuzole, etc.), biguanide drugs (e.g., metformin hydrochloride, buformin hydrochloride, etc.),

α-glucosidase inhibitors (e.g., voglibose, acarbose, etc.), insulin resistance improvers (e.g., pioglitazone, troglitazone, etc.), insulin, glucagon, diabetic complication drugs (e.g., epalrestat, thioctic acid, etc.), Actos, rosiglitazone, Kinedak, penfill, humulin, euglucon, glimicron, daonil, novolin, monotard, insulin family, glucobay, dimelin, rastinone, bacilcon, deamelin S, Iszilin acid, etc.]; brain function activating agents (e.g., idebenone, vinpocetine, etc.); urinary and male genital disease drugs [e.g., prostatomegaly drugs (tamsulosin hydrochloride, prazosin hydrochloride, chlormadinone acetate, etc.), prostate cancer drugs (leuprorelin acetate, goserelin acetate, chlormadinone acetate, etc.)], etc; nonsteroidal antiinflammatory drugs [acetaminophen, phenacetin, ethenzamide, sulpyrine, antipyrine, migrenin, aspirin, mefenamic acid, fulfenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, epirizole, tiaramide hydrochloride, zaltoprofen, gabexate mesilate, camostat mesilate, urinastatin, colchicine, probenecid, sulfinpyrazone, benzbromarone, allopurinol, sodium aurothiomalate, sodium hyaluronate, sodium salicylate, morphine hydrochloride, salicylic acid, atropine, scopolamine, morphine, pethidine, levorphanol, ketoprofen, naproxen, oxymorphone or salts thereof, etc.]; frequent urination and incontinence drugs [flavoxate hydrochloride, etc.]; unstable plaque stabilizers [MMP inhibitors, chymase inhibitors, etc.]; arrhythmic drugs [sodium channel blockers (e.g., quinidine, procainamide, disopyramide, ajmaline, cibenzoline, lidocaine, diphenylhydantoin, mexiletine, propafenone, flecainide, pilsicainide, phenytoin, etc.), β blockers (e.g., propranolol, alprenolol, bufetolol, oxprenolol, atenolol, acebutolol, metoprolol, pisoprolol, pindolol, carteolol, arotinolol, etc.), potassium channel blockers (e.g., amiodarone, etc.), calcium channel blockers (e.g., verapamil, diltiazem, etc.), etc.];

gynecologic disease drugs [e.g., climacteric disorder drugs (conjugated estrogen, estradiol, testosterone enanthate, estradiol valerate, etc.), breast cancer drugs (tamoxifen citrate, etc.), endometriosis and hysteromyoma drugs (leuprorelin acetate, danazol, etc.)], etc.; anesthetic drugs [a. local anaesthetic drugs [cocaine hydrochloride, procaine hydrochloride, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine], etc.]; b. systemic anesthetic drugs [(1) inhalation anesthetic drugs (e.g., ether, halothane, nitrous oxide, influrane, enflurane), (2) intravenous anesthetic drugs (e.g., ketamine hydrochloride, droperidol, thiopental sodium, thiamylal sodium, pentobarbital), etc.]]; anesthetic antagonists [levallorphan, nalorphine, naloxone, or salts thereof, etc.]; chronic cardiac failure drugs: cardiac stimulants [e.g., cardiac glycoside (digoxin), etc., β receptor stimulants (catecholamine preparations such as denopamine, dobutamine), PDE inhibitors, etc.]; diuretic drugs [e.g., furosemide (Lasix), spironolactone (Aldactone), bumetanide (Lunetoron), azosemide (Diart), etc.]; ACE inhibitors [e.g., enalapril maleate (Renivace), etc.]; Ca antagonists [e.g., amlodipine, manidipine, etc.] and β receptor blockers, etc.; immunomodulators [cyclosporin, tacrolimus, gusperimus, azathioprine, antilymphocyte sera, dried sulfonated immunoglobulins, erythropoietins, growth promoting glycoproteins, interleukins, interferons, etc.]; diuretic drugs [thiazide-based diuretic drugs (benzylhydrochlorothiazide, cyclopenthiazide, ethiazide, hydrochlorothiazide, hydroflumethiazide, methyclothiazide, penfluthiazide, polythiazide, trichlormethiazide, etc.), loop diuretic drugs (chlortalidone, clofenamide, indapamide, mefruside, meticrane, sotrazone, tribamide, quinethazone, metolazone, furosemide, mefruside, etc.), potassium-sparing diuretic drugs (spironolactone, triamterene, etc.)]; erectile dysfunction drugs (Viagra, apomorphine, etc.); and the like.

These drugs may be formulated, separately or simultaneously, by mixing with pharmaceutically acceptable carriers, excipients, binders, diluents or the like, and can be administered either orally or parenterally. When the drugs are formulated separately, the separately prepared formulations may be mixed using a diluent or the like at the time of use and then administered, or each of the separately prepared formulations may be administered, simultaneously or separately with a time interval, to the same subject. Kit products that are to be used for mixing the separately prepared formulations using a diluent or the like at the time of use and administering (for example, an injection kit including ampoules for containing individual powdery drug, and a diluent for mixing and dissolving two or more drugs at the time of use, and the like), kit products that are to be used for administering each of the separately prepared formulations, simultaneously or separately with a time interval, to the same subject (for example, a tablet kit for administering two

or more tablets, simultaneously or separately with a time interval, each tablet containing each of the drugs and placed in the same or separate bags, with space for memorandum provided, if necessary, on the bags for indication of the drug administration time, or the like), and the like are also included to the pharmaceutical composition of the present invention.

Dosage of the pharmaceutical composition of the present invention can be appropriately selected by taking into consideration of the subject to be administered, age and body weight of the subject, symptoms, administration time, method of administration, dosage form and the like.

The dosage of a particular subject can be determined according to the subject's age, body weight, general health condition, gender, meal, administration time, method of administration, excretion rate and the extent of disease condition of the patient at the time of treatment, by taking into consideration of these and other factors.

When the pharmaceutical composition described above is used as a prophylactic and therapeutic agent for AIDS and as a suppressive agent for disease progression of AIDS, the dosage may vary depending on the patient's condition, body weight or the method of administration. However, in the case of oral administration, the daily dosage is in a range of about 5 to 1000 mg, preferably about 10 to 600 mg, more preferably about 10 to 300 mg, and particularly preferably about 15 to 150 mg, as the active ingredient [i.e. as the compound of formula (I)], for an adult having a body weight of 50 kg, and the composition is administered once, or in 2 or 3 divided doses a day.

When the pharmaceutical composition described above is used as a prophylactic and therapeutic agent for graft-versus-host diseases and/or rejection associated with transplantation of organ such as heart, kidney, liver, bone marrow or the like, administration of the composition starts three days before the transplantation and is continued even after the transplantation. The daily dosage of the pharmaceutical composition may vary depending on the patient's condition, body weight or method of administration, but in the case of oral administration, it is about 5 to 1000 mg, preferably about 10 to 600 mg, more preferably about 10 to 300 mg, and particularly preferably about 15 to 150 mg, as the active ingredient [i.e., as the compound represented by formula (I)], for an adult having a body weight of 50 kg, and the composition is administered once, or in 2 or 3 divided doses a day. In this case, the composition may also be used in combination with other suppressive agents for graft-versus-host diseases and/or rejection associated with organ transplantation. Specific examples of the suppressive agent for graft-versus-host diseases and/or rejection associated with organ transplantation, which are used in combination with the compound represented by the above formula (I) or a salt thereof, include cyclosporin, tacrolimus, rapamycin, steroids, azathioprine, mycophenolate mofetil, mizoribine, etc. In the case of using these drugs in combination, if one of the drugs interferes with metabolism of other drugs, the dosage of each drug is to be appropriately adjusted, but in general, the dosage for administration of a single drug is employed for each of the drugs.

When the compound represented by formula (I) described above or a salt thereof is used for diseases other than the suppressive agents for graft-versus-host diseases and/or rejection associated with organ transplantation, the daily dosage thereof may vary depending on the kind of the disease to be treated, the patient's condition, body weight, or method of administration. But, in the case of oral administration, the dosage is about 5 to 1000 mg, preferably about 10 to 600 mg, more preferably about 10 to 300 mg, and particularly preferably about 15 to 150 mg, as the active ingredient [i.e., as the compound represented by formula (I)], for an adult having a body weight of 50 kg, and the composition is administered once, or in 2 or 3 divided doses a day. When the compound is used in combination with other drugs, the dosage of the other drugs is appropriately selected in a range of, for example, about 1/200 to 1/2 or more and about 2 to 3 times or less of a general dosage. Further, in the case of using the compound in combination with two or more drugs, if one of the drugs interferes with metabolism of the other drugs, the dosage of each drug is to be appropriately adjusted, but in general, the dosage for administration of a single drug is employed for each of the drugs.

Furthermore, the compound represented by formula (I) or a salt thereof can be also included in or used in combination with blood for transfusion or blood derivatives. Blood for transfusion or blood derivatives are usually produced by mixing blood obtained from a plurality of persons, and in some cases, cells infected by HIV virus may be co-present with HIV-uninfected cells. In such a case, there is fear for infection of the uninfected cells. Thus, when the compound represented by formula (I) of the present invention is added to blood for transfusion or a blood derivative, it is possible to prevent or control infection and proliferation of the virus. Especially, upon storage of blood derivatives, addition of the compound represented by formula (I) of the present invention is effective for prevention or control of infection and proliferation of the virus. In addition, when blood for transfusion or a blood derivative contaminated with HIV virus is administered to a person, infection and proliferation of the HIV virus in the body of the person administered with blood for transfusion or a blood derivative can be prevented by the compound represented by formula (I) that has been added to the blood or blood derivative. For example, in the case of orally administering the compound to an adult (body weight of about 60 kg) for preventing HIV infection upon blood transfusion and use of blood derivatives, the dosage for a single administration is usually in a range of about 0.02 to 50 mg/kg, preferably about 0.05 to 30 mg/kg, and more preferably about 0.1 to 10 mg/kg, as the CCR antagonist, and the compound is preferably administered in about 1 to about 3 doses a day. As a matter of fact, the range of dosage can be adjusted on the basis of the unit dosage

necessary for dividing the daily dosage; however, as described above, the dosage can be determined by taking into consideration of the nature and severity of the disease, the patient's age, body weight, general health condition, gender, meal, administration time, method of administration, excretion rate and other factors. In this case, the method of administration can be also appropriately selected, and the above-described prophylactic agent for HIV infection of the present invention may be added directly to the blood or blood derivative to be transfused, prior to blood transfusion or use of blood derivative. In such a case, the addition of the compound is preferably carried out immediately before to 24 hours before, preferably immediately before to 12 hours before, and more preferably immediately before to 6 hours before, the transfusion or use of blood derivative.

When the prophylactic agent for HIV infection of the present invention is further administered in addition to the blood or blood derivative to be transfused, at the time of blood transfusion or use of blood derivative, the agent is preferably administered from 1 hour before to simultaneously with transfusion or use of blood derivative, and more preferably, the agent is administered 1 to 3 times per day, continuously for 4 weeks.

Moreover, when the compound represented by formula (I) or a salt thereof is used in combination with a reverse transcriptase inhibitor and/or a protease inhibitor, the dosage of the reverse transcriptase inhibitor or the protease inhibitor is appropriately selected in a range of, for example, about 1/200 to 1/2 or more and about 2 to 3 times or less of the general dosage.

The general dosages for representative reverse transcriptase inhibitors and protease inhibitors are as follows:

zidovudine: 100 mg
didanosine: 125-200 mg
zalcitabine: 0.75 mg
lamivudine: 150 mg
stavudine: 30-40 mg
saquinavir: 600 mg
ritonavir: 600 mg
indinavir: 800 mg
nelfinavir: 750 mg

Also, a specific embodiment of the case where the compound represented by formula (I) or a salt thereof is used in combination with a reverse transcriptase inhibitor and/or a protease inhibitor will be described below.

(1) About 10 to 300 mg of the compound represented by formula (I) or a salt thereof and about 50 to 200 mg of zidovudine for an adult (body weight of 50 kg), are administered in combination to the same subject. Each drug may be administered simultaneously or separately with a time interval of 12 hours or less.

(2) About 10 to 300 mg of the compound represented by formula (I) or a salt thereof and about 300 to 1200 mg of saquinavir for an adult (body weight of 50 kg), are administered in combination to the same subject. Each drug may be administered simultaneously or separately with a time interval of 12 hours or less.

The following Examples, Reference Examples, Experimental Example and Formulation Examples further illustrate the present invention in detail but are not to be construed to limit the scope thereof.

Example 1 (Preparation of Compound 1)

To (S)-4-[[(1-propyl-1H-imidazol-5-yl)methyl]sulfinyl]aniline di-p-toluoyl-D-tartrate monohydrate (0.89 g) was added 1 N hydrochloric acid (5 ml), and the mixture was extracted with ethyl acetate. To the aqueous layer was added an aqueous 25% potassium carbonate solution (5 ml), followed by extraction with ethyl acetate-2-propanol (4 : 1) three times. The organic layer was washed with saturated brine and dried over magnesium sulfate, which was concentrated under reduced pressure to give (S)-4-[[(1-propyl-1H-imidazol-5-yl)methyl]sulfinyl]aniline as a colorless amorphous material.

To a solution of (2E)-3-[4-azepan-1-yl-4'-(2-butoxyethoxy)-1,1'-biphenyl-3-yl]acrylic acid (450 mg) in THF (10 ml) were added thionyl chloride (0.11 ml) and DMF (one drop) at room temperature, and the mixture was stirred for 1 hour. After concentration under reduced pressure, a solution of the residue in THF (30 ml) was added dropwise to a suspension of (S)-4-[[(1-propyl-1H-imidazol-5-yl)methyl]sulfinyl]aniline and triethylamine (0.85 ml) in THF (20 ml) at room temperature. After stirring the resulting mixture at room temperature for 20 hours, water was added thereto and the mixture was extracted with ethyl acetate. The organic layer was washed with an aqueous 5% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate) to give (S)-(2E)-3-[4-azepan-1-yl-4'-(2-butoxyethoxy)-1,1'-biphenyl-3-yl]-N-[4-[[(1-propyl-1H-imidazol-5-yl)methyl]sulfinyl]phenyl]acrylamide (Compound 1) (67.7 mg) as a yellow amorphous material.

$^1$H-NMR (300 MHz, $CDCl_3$) δ 0.90 (3H, t, J=7.2 Hz), 0.91 (3H, t, J=7.5 Hz), 1.31-1.46 (2H, m), 1.51-1.88 (12H, m), 3.23-3.26 (4H, m), 3.56 (2H, t, J=6.8 Hz), 3.76-3.84 (4H, m), 4.02 (1H, d, J=14.1 Hz), 4.10-4.18 (3H, m), 6.52-6.58 (2H, m), 6.98 (2H, d, J=9.0 Hz), 7.15 (1H, d, J=8.4 Hz), 7.35 (2H, d, J=8.7 Hz), 7.45-7.50 (4H, m), 7.64 (1H, d, J=2.1 Hz), 7.79 (2H, d, J=8.7 Hz), 8.05 (1H, s), 8.22 (1H, d, J=15.3 Hz) .

Example 2 (Preparation of Compound 2)

To (S)-4-[[(1-propyl-1H-imidazol-5-yl)methyl]sulfinyl]aniline di-p-toluoyl-D-tartrate monohydrate (0.93 g) was added 1 N hydrochloric acid (5 ml), and the mixture was extracted with ethyl acetate. To the aqueous layer was added an aqueous 25% potassium carbonate solution (5 ml), followed by extraction with ethyl acetate-2-propanol (4 : 1) three times. The organic layer was washed with saturated brine and dried over magnesium sulfate, which was concentrated under reduced pressure to give (S)-4-[[(1-propyl-1H-imidazol-5-yl)methyl]sulfinyl]aniline as a colorless amorphous material.

To a solution of (2E)-3-[4-azepan-1-yl-4'-(2-butoxyethoxy)-1,1'-biphenyl-3-yl]-2-methylacrylic acid (0.48 g) in THF (10 ml) were added thionyl chloride (0.12 ml) and DMF (one drop) at room temperature, and the mixture was stirred for 1.5 hours. After concentration under reduced pressure, a solution of the residue in THF (30 ml) was added dropwise to a suspension of (S)-4-[[(1-propyl-1H-imidazol-5-yl)methyl]sulfinyl]aniline and triethylamine (0.89 ml) in THF (30 ml) at room temperature. After stirring the resulting mixture at room temperature for 2 days, water was added thereto and the mixture was extracted with ethyl acetate. The organic layer was washed with an aqueous 5% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate) to give (S)-(2E)-3-[4-azepan-1-yl-4'-(2-butoxyethoxy)-1,1'-biphenyl-3-yl]-2-methyl-N-[4-[[(1-propyl-1H-imidazol-5-yl)methyl]sulfinyl]phenyl]acrylamide (Compound 2) (77.2 mg) as a yellow amorphous material.

$^1$H-NMR (300 MHz, $CDCl_3$) δ 0.92 (3H, t, J=7.5 Hz), 0.93 (3H, t, J=7.4 Hz), 1.32-1.45 (2H, m), 1.48-1.84 (12H, m), 2.26 (3H, d, J=1.2 Hz), 3.22-3.26 (4H, m), 3.55 (2H, t, J=6.8 Hz), 3.77-3.83 (4H, m), 4.04 (1H, d, J=14.1 Hz), 4.11 (1H, d, J=14.1 Hz), 4.16 (2H, t, J=5.0 Hz), 6.60 (1H, s), 6.99 (2H, d, J=9.0 Hz), 7.13 (1H, d, J=8.1 Hz), 7.37-7.48 (7H, m), 7.60 (1H, s), 7.76-7.86 (3H, m).

IR (KBr) 3102, 1669, 1590, 1518, 1489, 1456, 1397, 1312, 1246, 1121, 1047, 822 $cm^{-1}$.

Example 3 (Preparation of Compound 3)

To (S)-4-[[(1-propyl-1H-imidazol-5-yl)methyl]sulfinyl]aniline di-p-toluoyl-D-tartrate monohydrate (0.95 g) was added 1 N hydrochloric acid (5 ml), and the mixture was extracted with ethyl acetate. To the aqueous layer was added an aqueous 25% potassium carbonate solution (5 ml), followed by extraction with ethyl acetate-2-propanol (4 : 1) three times. The organic layer was washed with saturated brine and dried over magnesium sulfate, which was concentrated under reduced pressure to give (S)-4-[[(1-propyl-1H-imidazol-5-yl)methyl]sulfinyl]aniline as a colorless amorphous material.

To a solution of (2E)-3-[4-azocan-1-yl-4'-(2-butoxyethoxy)-1,1'-biphenyl-3-yl]acrylic acid (0.50 g) in THF (10 ml) were added oxalic chloride (0.106 ml) and DMF (one drop) at room temperature, and the mixture was stirred for 1 hour. The reaction mixture was added dropwise to a suspension of (S)-4-[[(1-propyl-1H-imidazol-5-yl)methyl]sulfinyl]aniline and triethylamine (0.92 ml) in THF (20 ml) at room temperature. After stirring the resulting mixture at room temperature for 4 days, water was added thereto and the mixture was extracted with ethyl acetate. The organic layer was washed with an aqueous 5% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate) to give (S)-(2E)-3-[4-azocan-1-yl-4'-(2-butoxyethoxy)-1,1'-biphenyl-3-yl]-N-[4-[[(1-propyl-1H-imidazol-5-yl)methyl]sulfinyl]phenyl]acrylamide (Compound 3) (280 mg) as a yellow amorphous material.

$[\alpha]_D$ = -147.7° (c = 0.467%,ethanol solution)

$^1$H-NMR (300 MHz, $CDCl_3$) δ 0.90 (3H, t, J=7.5 Hz), 0.94 (3H, t, J=7.5 Hz), 1.33-1.44 (2H, m), 1.50-1.82 (14H, m), 3.20-3.29 (4H, m), 3.56 (2H, t, J=6.6 Hz), 3.76-3.83 (4H, m), 4.01 (1H, d, J=13.8 Hz), 4.10-4.18 (3H, m), 6.53 (1H, d, J=15.5 Hz), 6.57 (1H, s), 6.98 (2H, d, J=9.0 Hz), 7.21 (1H, d, J=8.7 Hz), 7.34 (2H, d, J=8.7 Hz), 7.45-7.51 (4H, m), 7.63 (1H, d, J=2.1 Hz), 7.79 (2H, d, J=8.7 Hz), 8.20 (1H, s), 8.29 (1H, d, J=15.5 Hz).

IR (KBr) 3102, 1686, 1590, 1534, 1491, 1453, 1397, 1343, 1248, 1167, 1121, 1047, 829 $cm^{-1}$

Elementary analysis $C_{41}H_{52}N_4O_4S \cdot 0.5H_2O$, Calcd. C, 69.76; H, 7.57; N, 7.94 : Found. C, 69.73; H, 7.45; N, 7.97.

Example 4 (Preparation of Compound 4)

To (S)-4-[((1-propyl-1H-imidazol-5-yl)methyl]sulfinyl]aniline di-p-toluoyl-D-tartrate monohydrate (0.93 g) was added 1 N hydrochloric acid (5 ml), and the mixture was extracted with ethyl acetate. To the aqueous layer was added an aqueous 25% potassium carbonate solution (5 ml), followed by extraction with ethyl acetate-2-propanol (4 : 1) three times. The organic layer was washed with saturated brine and dried over magnesium sulfate, which was concentrated under reduced pressure to give (S)-4-[[(1-propyl-1H-imidazol-5-yl)methyl]sulfinyl]aniline as a colorless amorphous material.

To a solution of (2E)-3-[4'-(2-butoxyethoxy)-4-(diisobutylamino)-1,1'-biphenyl-3-yl]acrylic acid (0.50 g) in THF (10 ml) were added oxalic chloride (0.10 ml) and DMF (one drop) at room temperature, and the mixture was stirred for 1 hour. The reaction mixture was added dropwise to a suspension of (S)-4-[[(1-propyl-1H-imidazol-5-yl)methyl]sulfinyl]aniline and triethylamine (0.89 ml) in THF (30 ml) at room temperature. After stirring the resulting mixture at room temperature for 20 hours, water was added thereto and the mixture was extracted with ethyl acetate. The organic layer was washed with an aqueous 5% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate) to give (S)-(2E)-3-[4'-(2-butoxyethoxy)-4-(diisobutylamino)-1,1'-biphenyl-3-yl]-N-[4-[[(1-propyl-1H-imidazol-5-yl)methyl]sulfinyl]phenyl]acrylamide (Compound 4) (179.3 mg) as a yellow amorphous material.

$[\alpha]_D$ = -151.2° (c = 0.491%,ethanol solution)

$^1$H-NMR (200 MHz, $CDCl_3$) δ 0.86-0.97 (18H, m), 1.31-1.47 (2H, m), 1.51-1.93 (6H, m), 2.86 (4H, d, J=7.4 Hz), 3.56 (2H, t, J=6.6 Hz), 3.74-3.84 (4H, m), 4.01 (1H, d, J=14.4 Hz), 4.09-4.19 (3H, m), 6.57 (1H, d, J=15.4 Hz), 6.59 (1H, s), 6.98 (2H, d, J=8.6 Hz), 7.19 (1H, d, J=8.8 Hz), 7.34 (2H, d, J=8.4 Hz), 7.46-7.52 (4H, m), 7.67 (1H, d, J=2.2 Hz), 7.80 (2H, d, J=8.8 Hz), 8.14 (1H, s), 8.28 (1H d, J=15.4 Hz).

IR (KBr) 3103, 1686 1624, 1591, 1489, 1466, 1399, 1343, 1250, 1167, 1121, 1088, 1047, 997, 831 $cm^{-1}$

Elementary analysis $C_{42}H_{56}N_4O_4S \cdot 0.5H_2O$, Calcd. C, 69.87; H, 7.96; N, 7.76 : Found. C, 69.77; H, 7.79; N, 7.57.

Example 5 (Preparation of Compound 5)

To (S)-4-[[(1-propyl-1H-imidazol-5-yl)methyl]sulfinyl]aniline di-p-toluoyl-D-tartrate monohydrate (0.95 g) was added 1 N hydrochloric acid (5 ml), and the mixture was extracted with ethyl acetate. To the aqueous layer was added an aqueous 25% potassium carbonate solution (5 ml), followed by extraction with ethyl acetate-2-propanol (4 : 1) three times. The organic layer was washed with saturated brine and dried over magnesium sulfate, which was concentrated under reduced pressure to give (S)-4-[[(1-propyl-1H-imidazol-5-yl)methyl]sulfinyl]aniline as a colorless amorphous material.

To a solution of (2E)-3-[4'-(2-butoxyethoxy)-4-[isobutyl(propyl)amino]-1,1'-biphenyl-3-yl]acrylic acid (0.50 g) in THF (10 ml) were added oxalic chloride (0.106 ml) and DMF (one drop) at room temperature, and the mixture was stirred for 1 hour. The reaction mixture was added dropwise to a suspension of (S)-4-[[(1-propyl-1H-imidazol-5-yl)methyl]sulfinyl]aniline and triethylamine (0.92 ml) in THF (30 ml) at room temperature. After stirring the resulting mixture at room temperature for 20 hours, water was added thereto and the mixture was extracted with ethyl acetate. The organic layer was washed with an aqueous 5% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate) to give (S)-(2E)-3-[4'-(2-butoxyethoxy)-4-[isobutyl(propyl)amino]-1,1'-biphenyl-3-yl]-N-[4-[[(1-propyl-1H-imidazol-5-yl)methyl]sulfinyl]phenyl]acrylamide (Compound 5) (175.0 mg) as a yellow amorphous material.

$[\alpha]_D$ = -145.3° (c = 0.487%, ethanol solution)

$^1$H-NMR (200 MHz, $CDCl_3$) δ 0.79-0.97 (15H, m), 1.30-1.86 (9H, m), 2.87-3.00 (4H, m), 3.56 (2H, t, J=6.8 Hz), 3.74-3.84 (4H, m), 4.01 (1H, d, J=13.8 Hz), 4.10-4.19 (3H, m), 6.58 (1H, s), 6.60 (1H, d, J=15.6 Hz), 6.98 (2H, d, J=8.8 Hz), 7.16 (1H, d, J=8.4 Hz), 7.34 (2H, d, J=8.8 Hz), 7.45-7.50 (4H, m), 7.67 (1H, d, J=2.2 Hz), 7.80 (2H, d, J=8.8 Hz), 8.26 (1H, d, J=15.6 Hz), 8.28 (1H, s).

IR (KBr) 3104, 1686, 1624, 1591, 1537, 1487, 1399, 1343, 1250, 1169, 1119, 1088, 1049, 824 $cm^{-1}$

Elementary analysis $C_{41}H_{54}N_4O_4S \cdot 0.5H_2O$, Calcd. C, 69.56; H, 7.83; N, 7.91 : Found. C, 69.47; H, 7.82; N, 7.93.

Example 6 (Preparation of Compound 6)

To (S)-4-[[(1-propyl-1H-imidazol-5-yl)methyl]sulfinyl]aniline di-p-toluoyl-D-tartrate monohydrate (1.88 g) was added 1 N hydrochloric acid (10 ml), and the mixture was extracted with ethyl acetate. To the aqueous layer was added an aqueous 25% potassium carbonate solution (10 ml), followed by extraction with ethyl acetate-2-propanol (4 : 1) three times. The organic layer was washed with saturated brine and dried over magnesium sulfate, which was concentrated under reduced pressure to give (S)-4-[[(1-propyl-1H-imidazol-5-yl)methyl]sulfinyl]aniline as a colorless amorphous material. To a solution of (2E)-3-[4'-(2-butoxyethoxy)-4-[isobutyl(methyl)amino]-1,1'-biphenyl-3-yl]acrylic acid (1.0 g) in THF (10 ml) were added oxalic chloride (0.23 ml) and DMF (one drop) at room temperature, and the mixture was stirred for 1 hour. The reaction mixture was added dropwise to a suspension of (S)-4-[[(1-propyl-1H-imidazol-5-yl)methyl]sulfinyl]aniline and triethylamine (1.84 ml) in THF (20 ml) at room temperature. After stirring the resulting mixture at room temperature for 3 days, water was added thereto and the mixture was extracted with ethyl acetate. The organic layer was washed with an aqueous 5% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate) to give (S)-(2E)-3-[4'-(2-butoxyethoxy)-4-[isobutyl(methyl)amino]-1,1'-biphenyl-3-yl]-N-[4-[[(1-propyl-1H-imidazol-5-yl)methyl]sulfinyl]phenyl]acrylamide (Compound 6) (362 mg) as a yellow amorphous material.

$[\alpha]_D$ = -158.1° (c = 0.473%,ethanol solution)

$^1$H-NMR (200 MHz, $CDCl_3$) 8 0.85-0.97 (12H, m), 1.30-1.47 (4H, m), 1.52-1.78 (2H, m), 1.84-2.00 (1H, m), 2.73 (3H, s), 2.79 (2H, d, J=7.4 Hz), 3.56 (2H, t, J=6.6 Hz), 3.74-3.84 (4H, m), 3.99 (1H, d, J=14.4 Hz), 4.11-4.19 (3H, m), 6.56 (1H, s) 6.65 (1H, d, J=15.8 Hz), 6.97 (2H, d, J=8.8 Hz), 7.13 (1H, d, J=8.4 Hz), 7.32 (2H, d, J=8.8 Hz), 7.43-7.52 (4H, m), 7.64 (1H, d, J=2.2 Hz), 7.80 (2H, d, J=8.8 Hz), 8.22 (1H, d, J=15.8 Hz), 8.63 (1H, s).

IR (KBr) 3098, 1684, 1591, 1534, 1491, 1343, 1250, 1169, 1123, 1047, 826 $cm^{-1}$

Elementary analysis $C_{39}H_{50}N_4O_4S \cdot 0.5H_2O$, Calcd. C, 68.89; H, 7.56; N, 8.24 : Found. C, 68.83; H, 7.40; N, 8.14.

Example 7 (Preparation of Compound 7)

To (S)-4-[[(1-propyl-1H-imidazol-5-yl)methyl]sulfinyl]aniline di-p-toluoyl-D-tartrate monohydrate (1.82 g) was added 1 N hydrochloric acid (10 ml), and the mixture was extracted with ethyl acetate. To the aqueous layer was added an aqueous 25% potassium carbonate solution (10 ml), followed by extraction with ethyl acetate-2-propanol (4 : 1) three times. The organic layer was washed with saturated brine and dried over magnesium sulfate, which was concentrated under reduced pressure to give (S)-4-[[(1-propyl-1H-imidazol-5-yl)methyl]sulfinyl]aniline as a colorless amorphous material. To a solution of (2E)-3-[4-azepan-1-yl-4'-(2-butoxyethoxy)-1,1'-biphenyl-3-yl]-2-ethylacrylic acid (1.0 g) in THF (10 ml) were added oxalic chloride (0.21 ml) and DMF (one drop) at room temperature, and the mixture was stirred for 1 hour. The reaction mixture was added dropwise to a suspension of (S)-4-[[(1-propyl-1H-imidazol-5-yl)methyl]sulfinyl]aniline and triethylamine (1.76 ml) in THF (20 ml) at room temperature. After stirring the resulting mixture at room temperature for 20 hours, water was added thereto and the mixture was extracted with ethyl acetate. The organic layer was washed with an aqueous 5% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate) to give (S)-(2E)-3-[4-azepan-1-yl-4'-(2-butoxyethoxy)-1,1'-biphenyl-3-yl]-2-ethyl-N-[4-[[(1-propyl-1H-imidazol-5-yl)methyl]sulfinyl]phenyl]acrylamide (Compound 7) (278 mg) as a yellow amorphous material.

$[\alpha]_D$ = -123.7° (c = 0.494%,ethanol solution)

$^1$H-NMR (200 MHz, $CDCl_3$) $\delta$ 0.92 (3H, t, J=7.5 Hz), 0.93 (3H, t, J=7.3 Hz), 1.23 (3H, t, J=7.4 Hz), 1.29-1.48 (2H, m), 1.52-1.85 (12H, m), 2.73 (2H, q, J=7.4 Hz), 3.20-3.25 (4H, m), 3.55 (2H, t, J=6.8 Hz), 3.76-3.83 (4H, m), 4.02 (1H, d, J=14.0 Hz), 4.07-4.19 (3H, m), 6.61 (1H, s), 6.99 (2H, d, J=8.6 Hz), 7.12 (1H, d, J=8.4 Hz), 7.36-7.49 (8H, m), 7.78 (2H, d, J=8.8 Hz), 7.90 (1H, s).

IR (KBr) 3085, 1671, 1590, 1518, 1489, 1399, 1316, 1246, 1123, 1047, 820 $cm^{-1}$

Elementary analysis $C_{42}H_{54}N_4O_4S \cdot 0.5H_2O$, Calcd. C, 70.07 ; H, 7.70 ; N, 7.78 : Found. C, 70.10 ; H, 7.74 ; N, 7.70.

Example 8 (Preparation of Compound 8)

To (S)-4-[[(1-propyl-1H-imidazol-5-yl)methyl]sulfinyl]aniline di-p-toluoyl-D-tartrate monohydrate (1.58 g) was added 1 N hydrochloric acid (10 ml), and the mixture was extracted with ethyl acetate. To the aqueous layer was added an aqueous 25% potassium carbonate solution (10 ml), followed by extraction with ethyl acetate-2-propanol (4 : 1) three times. The organic layer was washed with saturated brine and dried over magnesium sulfate, which was concentrated under reduced pressure to give (S)-4-[[(1-propyl-1H-imidazol-5-yl)methyl]sulfinyl]aniline as a colorless amorphous material. To a solution of (2E)-3-[4'-(2-butoxyethoxy)-4-[ethyl(isobutyl)amino]-1,1'-biphenyl-3-yl]acrylic acid (0.71 g) in THF (10 ml) were added oxalic chloride (0.14 ml) and DMF (one drop) at room temperature, and the mixture was stirred for 1 hour. The reaction mixture was added dropwise to a suspension of aforementioned aniline and triethylamine (1.5 ml) in THF (20 ml) at 0°C. After stirring the resulting mixture at room temperature for 20 hours, water was added thereto and the mixture was extracted with ethyl acetate. The organic layer was washed with an aqueous 5% acetic acid solution, an aqueous saturated sodium hydrogen carbonate solution and saturated brine, and dried

over magnesium sulfate. After concentration under reduced pressure, the residue was separated and purified by column chromatography (basic silica gel, ethyl acetate) to give (S)-(2E)-3-[4'-(2-butoxyethoxy)-4-[ethyl(isobutyl)amino]-1,1'-biphenyl-3-yl]-N-[4-[[(1-propyl-1H-imidazol-5-yl)methyl]sulfinyl]phenyl]acrylamide (Compound 8) (250.6 mg) as a yellow amorphous material.

$[\alpha]_D$ = -156.9° (c = 0.485%,ethanol solution)

$^1$H-NMR (200 MHz, $CDCl_3$) δ 0.85-0.97 (12H, m), 1.04 (3H, t, J=7.0 Hz), 1.29-1.48 (2H, m), 1.51-1.87 (5H, m), 2.87 (2H, d, J=7.4 Hz), 3.06 (2H, q, J=7.0 Hz), 3.56 (2H, t, J=6.6 Hz), 3.74-3.84 (4H, m), 4.00 (1H, d, J=14.2 Hz), 4.10-4.19 (3H, m), 6.58 (1H, s), 6.61 (1H, d, J=15.4 Hz), 6.98 (2H, d, J=8.8 Hz), 7.15 (1H, d, J=8.6 Hz), 7.33 (2H, d, J=8.6 Hz), 7.44-7.52 (4H, m), 7.67 (1H, d, J=2.2 Hz), 7.79 (2H, d, J=8.6 Hz), 8.25 (1H, d, J=15.4 Hz), 8.38 (1H, s).

IR (KBr) 3103, 1684, 1591, 1534, 1489, 1399, 1345, 1250, 1169, 1123, 1047, 826 $cm^{-1}$

Elementary analysis $C_{40}H_{52}N_4O_4S{\cdot}0.5H_2O$, Calcd. C, 69.23 ; H, 7.70 ; N, 8.07 : Found. C, 69.13 ; H, 7.65 ; N, 7.82.

Example 9 (Preparation of Compound 9)

To (S)-4-[[(1-propyl-1H-imidazol-5-yl)methyl]sulfinyl]aniline di-p-toluoyl-D-tartrate monohydrate (1.64 g) was added 1 N hydrochloric acid (10 ml), and the mixture was extracted with ethyl acetate. To the aqueous layer was added an aqueous 25% potassium carbonate solution (10 ml), followed by extraction with ethyl acetate-2-propanol (4 : 1) three times. The organic layer was washed with saturated brine and dried over magnesium sulfate, which was concentrated under reduced pressure to give (S)-4-[[(1-propyl-1H-imidazol-5-yl)methyl]sulfinyl]aniline as a colorless amorphous material. To a solution of (2E)-3-[4'-(2-butoxyethoxy)-4-piperidin-1-yl-1,1'-biphenyl-3-yl]acrylic acid (0.80 g) in THF (10 ml) were added oxalic chloride (0.17 ml) and DMF (one drop) at 0°C, and the mixture was stirred at 0°C for 0.5 hour and then at room temperature for 1 hour. The reaction mixture was added dropwise to a suspension of (S)-4-[[(1-propyl-1H-imidazol-5-yl)methyl]sulfinyl]aniline and triethylamine (1.6 ml) in THF (20 ml) at 0°C. After stirring the resulting mixture at room temperature for 18 hours, water was added thereto and the mixture was extracted with ethyl acetate. The organic layer was washed with an aqueous 5% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 99) to give (S)-(2E)-3-[4'-(2-butoxyethoxy)-4-piperidin-1-yl-1,1'-biphenyl-3-yl]-N-[4-[[(1-propyl-1H-imidazol-5-yl)methyl]sulfinyl]phenyl]acrylamide (Compound 9) (410.5 mg) as a yellow amorphous material.

$[\alpha]_D$ = -155.1° (c = 0.504%, ethanol solution)

$^1$H-NMR (200 MHz, $CDCl_3$) δ 0.89 (3H, t, J=7.4 Hz), 0.94 (3H, t, J=7.2 Hz), 1.30-1.86 (12H, m), 2.89-3.00 (4H, m), 3.56 (2H, t, J=6.6 Hz), 3.75-3.84 (4H, m), 4.00 (1H, d, J=13.8 Hz), 4.10-4.19 (3H, m), 6.57 (1H, s), 6.66 (1H, d, J=15.3 Hz), 6.97 (2H, d, J=8.6 Hz), 7.09 (1H, d, J=8.4 Hz), 7.33 (2H, d, J=8.4 Hz), 7.43-7.53 (4H, m), 7.66 (1H, d, J=2.6 Hz), 7.80 (2H, d, J=8.4 Hz), 8.17 (1H, d, J=15.3 Hz), 8.25-8.45 (1H, m).

IR (KBr) 3031, 1684, 1590, 1534, 1489, 1343, 1250, 1231, 1169, 1123, 1044, 820 $cm^{-1}$

Elementary analysis $C_{39}H_{48}N_4O_4S{\cdot}0.5H_2O$, Calcd. C, 69.10 ; H, 7.29 ; N, 8.26 : Found. C, 69.12 ; H, 7.25 ; N, 8.13.

Example 10 (Preparation of Compound 10)

To (S)-4-[[(1-propyl-1H-imidazol-5-yl)methyl]sulfinyl]aniline di-p-toluoyl-D-tartrate monohydrate (1.58 g) was added 1 N hydrochloric acid (10 ml), and the mixture was extracted with ethyl acetate. To the aqueous layer was added an aqueous 25% potassium carbonate solution (10 ml), followed by extraction with ethyl acetate-2-propanol (4 : 1) three times. The organic layer was washed with saturated brine and dried over magnesium sulfate, which was concentrated under reduced pressure to give (S)-4-[[(1-propyl-1H-imidazol-5-yl)methyl]sulfinyl]aniline as a colorless amorphous material. To a solution of (2E)-3-[4'-(2-butoxyethoxy)-4-piperidin-1-yl-1,1'-biphenyl-3-yl]-2-methylacrylic acid (0.80 g) in THF (10 ml) were added oxalic chloride (0.17 ml) and DMF (one drop) at 0°C, and the mixture was stirred at 0°C for 30 minutes and then at room temperature for 1.5 hours. The reaction mixture was added dropwise to a suspension of (S)-4-[[(1-propyl-1H-imidazol-5-yl)methyl]sulfinyl]aniline and triethylamine (1.53 ml) in THF (20 ml) at 0°C. After stirring the resulting mixture at room temperature for 20 hours, water was added thereto and the mixture was extracted with ethyl acetate. The organic layer was washed with an aqueous 5% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate) to give (S)-(2E)-3-[4'-(2-butoxyethoxy)-4-piperidin-1-yl-1,1'-biphenyl-3-yl]-2-methyl-N-[4-[[(1-propyl-1H-imidazol-5-yl)methyl]sulfinyl]phenyl]acrylamide (Compound 10) (343.7 mg) as a yellow amorphous material.

$[\alpha]_D$ = -134.0° (c = 0.487%, ethanol solution)

$^1$H-NMR (200 MHz, $CDCl_3$) δ 0.92 (3H, t, J=7.5 Hz), 0.93 (3H, t, J=7.2 Hz), 1.28-1.81 (12H, m), 2.29 (3H, d, J=1.0

Hz), 2.86-2.98 (4H, m), 3.55 (2H, t, J=6.6 Hz), 3.76-3.83 (4H, m), 4.00-4.19 (4H, m), 6.60 (1H, s), 6.99 (2H, d, J=8.8 Hz), 7.09 (1H, d, J=9.2 Hz), 7.31-7.49 (7H, m), 7.54-7.60 (1H, m), 7.77 (2H, d, J=8.8 Hz), 7.82 (1H, s).

IR (KBr) 3032, 1661, 1591, 1522, 1487, 1453, 1310, 1233, 1178, 1125, 1042, 820 $cm^{-1}$

Elementary analysis $C_{40}H_{50}N_4O_4S \cdot 1.25H_2O$, Calcd. C, 68.10 ; H, 7.50 ; N, 7.94 : Found. C, 68.13 ; H, 7.40 ; N, 7.87.

Example 11 (Preparation of Compound 11)

To (S)-4-[[(1-propyl-1H-imidazol-5-yl)methyl]sulfinyl]aniline di-p-toluoyl-D-tartrate monohydrate (1.27 g) was added 1 N hydrochloric acid (10 ml), and the mixture was extracted with ethyl acetate. To the aqueous layer was added an aqueous 25% potassium carbonate solution (10 ml), followed by extraction with ethyl acetate-2-propanol (4 : 1) three times. The organic layer was washed with saturated brine and dried over magnesium sulfate, which was concentrated under reduced pressure to give (S)-4-[[(1-propyl-1H-imidazol-5-yl)methyl]sulfinyl]aniline as a colorless amorphous material. To a solution of (2E)-3-[4'-(2-butoxyethoxy)-4-pyrrolidin-1-yl-1,1'-biphenyl-3-yl]acrylic acid (0.60 g) in THF (10 ml) were added oxalic chloride (0.14 ml) and DMF (one drop) at 0°C, and the mixture was stirred at 0°C for 1 hour. The reaction mixture was added dropwise to a suspension of (S)-4-[[(1-propyl-1H-imidazol-5-yl)methyl]sulfinyl] aniline and triethylamine (1.23 ml) in THF (30 ml) at 0°C. After stirring the resulting mixture at room temperature for 18 hours, water was added thereto and the mixture was extracted with ethyl acetate. The organic layer was washed with an aqueous 5% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 49) to give (S)-(2E)-3-[4'-(2-butoxyethoxy)-4-pyrrolidin-1-yl-1,1'-biphenyl-3-yl]-N-[4-[[(1-propyl-1H-imidazol-5-yl)methyl]sulfinyl]phenyl] acrylamide (Compound 11) (23 mg) as a yellow amorphous material.

$^1$H-NMR (200 MHz, $CDCl_3$) δ 0.91 (3H, t, J=7.4 Hz), 0.94 (3H, t, J=7.0 Hz), 1.28-1.49 (6H, m), 1.90-2.01 (4H, m), 3.29-3.41 (4H, m), 3.56 (2H, t, J=6.6 Hz), 3.74-3.84 (4H, m), 3.99-4.19 (4H, m), 6.71 (1H, d, J=15.4 Hz), 6.59 (1H, s), 6.93 (1H, d, J=8.4 Hz), 6.98 (2H, d, J=8.6 Hz), 7.33-7.49 (6H, m), 7.59 (1H, d, J=2.6 Hz), 7.79 (2H, d, J=8.6 Hz), 7.84 (1H, s), 8.20 (1H, d, J=15.4 Hz).

Example 12 (Preparation of Compound 12)

To (S)-4-[[(1-propyl-1H-imidazol-5-yl)methyl]sulfinyl]aniline di-p-toluoyl-D-tartrate monohydrate (1.64 g) was added 1 N hydrochloric acid (10 ml), and the mixture was extracted with ethyl acetate. To the aqueous layer was added an aqueous 25% potassium carbonate solution (10 ml), followed by extraction with ethyl acetate-2-propanol (4 : 1) three times. The organic layer was washed with saturated brine and dried over magnesium sulfate, which was concentrated under reduced pressure to give (S)-4-[[(1-propyl-1H-imidazol-5-yl)methyl]sulfinyl]aniline as a colorless amorphous material. To a solution of (2E)-3-[4'-(2-butoxyethoxy)-4-pyrrolidin-1-yl-1,1'-biphenyl-3-yl]-2-methylacrylic acid (0.80 g) in THF (10 ml) were added oxalic chloride (0.18 ml) and DMF (one drop) at 0°C, and the mixture was stirred at 0°C for 1 hour and then at room temperature for 0.5 hour. The reaction mixture was added dropwise to a suspension of (S)-4-[[(1-propyl-1H-imidazol-5-yl)methyl]sulfinyl]aniline and triethylamine (1.58 ml) in THF (30 ml) at 0°C. After stirring the resulting mixture at room temperature for 20 hours, water was added thereto and the mixture was extracted with ethyl acetate. The organic layer was washed with an aqueous 10% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate) to give (S)-(2E)-3-[4'-(2-butoxyethoxy)-4-pyrrolidin-1-yl-1,1'-biphenyl-3-yl]-2-methyl-N-[4-[[(1-propyl-1H-imidazol-5-yl) methyl]sulfinyl]phenyl]acrylamide (Compound 12) (488.3 mg) as a yellow amorphous material.

$[\alpha]_D$ = -123.0° (c = 0.538%, ethanol solution)

$^1$H-NMR (300 MHz, $CDCl_3$) δ 0.91 (3H, t, J=7.4 Hz), 0.93 (3H, t, J=7.1 Hz), 1.29-1.47 (2H, m), 1.51-1.78 (4H, m), 1.87-1.99 (4H, m), 2.20 (3H, d, J=1.2 Hz), 3.30-3.41 (4H, m), 3.55 (2H, t, J=6.6 Hz), 3.76-3.83 (4H, m), 4.02 (1H, d, J=14.4 Hz), 4.08-4.18 (3H, m), 6.56 (1H, s), 6.91 (1H, d, J=8.4 Hz), 6.98 (2H, d, J=9.0 Hz), 7.33-7.48 (7H, m), 7.63 (1H, s), 7.78 (2H, d, J=8.8 Hz), 7.85 (1H, s).

IR (KBr) 3029, 1667, 1603, 1590, 1520, 1497, 1489, 1397, 1314, 1246, 1121, 912, 743 $cm^{-1}$

Elementary analysis $C_{39}H_{48}N_4O_4S \cdot 0.5H_2O$, Calcd. C, 69.10 ; H, 7.29 ; N, 8.26 : Found. C, 68.97 ; H, 7.24 ; N, 8.07.

Example 13 (Preparation of Compound 13)

To (S)-4-[[(1-propyl-1H-imidazol-5-yl)methyl]sulfinyl]aniline di-p-toluoyl-D-tartrate monohydrate (1.59 g) was added 1 N hydrochloric acid (10 ml), and the mixture was extracted with ethyl acetate. To the aqueous layer was added

an aqueous 25% sodium hydroxide solution (10 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl]sulfinyl]aniline. To a solution of (2E)-3-[4'-(2-butoxyethoxy)-4-(4-methylpiperidin-1-yl)-1,1'-biphenyl-3-yl]acrylic acid (0.80 g) and DMF (one drop) in THF (10 ml) was added oxalic chloride (0.176 ml) at 0°C, and the mixture was stirred at room temperature for 1.5 hours. The solution was added dropwise to a mixture of (S)-4-[[(1-propyl-1H-imidazol-5-yl)methyl]sulfinyl]aniline and triethylamine (1.52 ml) in THF (20 ml) at 0°C. After stirring the resulting mixture at room temperature for 20 hours, water was added thereto and the mixture was extracted with ethyl acetate. The organic layer was washed with an aqueous 10% acetic acid solution, an aqueous sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 49) to give (S)-(2E)-3-[4'-(2-butoxyethoxy)-4-(4-methylpiperidin-1-yl)-1,1'-biphenyl-3-yl]-N-[4-[[(1-propyl-1H-imidazol-5-yl)methyl]sulfinyl]phenyl]acrylamide (Compound 13) (11 mg) as a yellow amorphous material.

$^1$H-NMR (200 MHz, $CDCl_3$) δ 0.87-1.03 (9H, m), 1.31-1.82 (11H, m), 2.62-2.81 (2H, m), 3.13-3.26 (2H, m), 3.56 (2H, t, J=6.8 Hz), 3.75-3.84 (4H, m), 3.97-4.19 (4H, m), 6.55-6.65 (2H, m), 6.99 (2H, d, J=9.0 Hz), 7.10 (1H, d, J=8.8 Hz), 7.35 (2H, d, J=8.8 Hz), 7.45-7.52 (4H, m), 7.65-7.69 (1H, m), 7.80 (2H, d, J=8.8 Hz), 8.17 (1H, d, J=15.4 Hz).

Example 14 (Preparation of Compound 14)

To (S)-4-[[(1-propyl-1H-imidazol-5-yl)methyl]sulfinyl]aniline di-p-toluoyl-D-tartrate monohydrate (1.19 g) was added 1 N hydrochloric acid (10 ml), and the mixture was extracted with ethyl acetate. To the aqueous layer was added an aqueous 25% sodium hydroxide solution (10 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl]sulfinyl]aniline. To a solution of (2E)-3-[4'-(2-butoxyethoxy)-4-(4-methylpiperidin-1-yl)-1,1'-biphenyl-3-yl]-2-methylacrylic acid (0.60 g) and DMF (one drop) in THF (10 ml) was added oxalic chloride (0.12 ml) at 0°C, and the mixture was stirred at room temperature for 10 minutes. To the reaction system were added THF (20 ml) and DMF (4 ml) and the mixture was further stirred for 1 hour. The solution was added dropwise to a mixture of (S)-4-[[(1-propyl-1H-imidazol-5-yl)methyl]sulfinyl]aniline and triethylamine (1.15 ml) in THF (20 ml) at 0°C. After stirring the resulting mixture at room temperature for 20 hours, water was added thereto and the mixture was extracted with ethyl acetate. The organic layer was washed with an aqueous 10% acetic acid solution, an aqueous sodium hydrogen carbonate solution and saturated brine, and dried over magnesium sulfate. After concentration under reduced pressure, the residue was separated and purified by column chromatography (basic silica gel, ethyl acetate) to give (S)-(2E)-3-[4'-(2-butoxyethoxy)-4-(4-methylpiperidin-1-yl)-1,1'-biphenyl-3-yl]-2-methyl-N-[4-[[(1-propyl-1H-imidazol-5-yl)methyl]sulfinyl]phenyl]acrylamide (Compound 14) (536.6 mg) as a yellow amorphous material.

$[\alpha]_D$ = -131.6° (c = 0.472%, ethanol solution)

$^1$H-NMR (200 MHz, $CDCl_3$) δ 0.92 (3H, t, J=7.3 Hz), 0.93 (3H, t, J=7.2 Hz), 0.99 (3H, d, J=6.2 Hz), 1.26-1.81 (11H, m), 2.29 (3H, s), 2.61-2.79 (2H, m), 3.12-3.25 (2H, m), 3.55 (2H, t, J=6.6 Hz), 3.75-3.83 (4H, m), 4.03 (1H, d, J=14.2 Hz), 4.08-4.19 (3H, m), 6.61 (1H, s), 7.00 (2H, d, J=8.4 Hz), 7.09 (1H, d, J=9.2 Hz), 7.37-7.56 (8H, m), 7.78 (2H, d, J=8.8 Hz), 7.82 (1H, s).

IR (KBr) 3185, 1669, 1607, 1590, 1518, 1487, 1312, 1248, 1225, 1121, 823 $cm^{-1}$

Elementary analysis $C_{41}H_{52}N_4O_4S \cdot 0.5H_2O$, Calcd. C, 69.76 ; H, 7.57 ; N, 7.94 : Found. C, 69.73 ; H, 7.62 ; N, 7.85.

Example 15 (Preparation of Compound 15)

To (S)-4-[[(1-propyl-1H-imidazol-5-yl)methyl]sulfinyl]aniline di-p-toluoyl-D-tartrate monohydrate (1.59 g) was added 1 N hydrochloric acid (10 ml), and the mixture was extracted with ethyl acetate. To the aqueous layer was added an aqueous 25% sodium hydroxide solution (10 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl]sulfinyl]aniline. To a solution of (2E)-3-[4'-(2-butoxyethoxy)-4-pyrrolidin-1-yl-1,1'-biphenyl-3-yl]-2-ethylacrylic acid (0.80 g) and DMF (0.1 ml) in THF (10 ml) was added oxalic chloride (0.175 ml) at 0°C, and the mixture was stirred at room temperature for 1.5 hours. The solution was added dropwise to a mixture of (S)-4-[[(1-propyl-1H-imidazol-5-yl)methyl]sulfinyl]aniline and pyridine (1.48 ml) in THF (30 ml) at 0°C. After stirring the resulting mixture at room temperature for 20 hours, water was added thereto and the mixture was extracted with ethyl acetate. The organic layer was washed with an aqueous 10% acetic acid solution, an aqueous sodium hydrogen carbonate solution and saturated brine, and dried over magnesium sulfate. After concentration under reduced pressure, the residue was separated and purified by column chromatography (basic silica gel,

ethyl acetate) to give (S)-(2E)-3-[4'-(2-butoxyethoxy)-4-pyrrolidin-1-yl-1,1'-biphenyl-3-yl]-2-ethyl-N-[4-[[(1-propyl-1H-imidazol-5-yl)methyl]sulfinyl]phenyl]acrylamide (Compound 15) (917.6 mg) as a yellow amorphous material.

$^1$H-NMR (200 MHz, $CDCl_3$) δ 0.91 (3H, t, J=7.5 Hz), 0.93 (3H, t, J=7.1 Hz), 1.22 (3H, t, J=7.5 Hz), 1.30-1.48 (2H, m), 1.55-1.79 (4H, m), 1.86-2.01 (4H, m), 2.70 (2H, q, J=7.5 Hz), 3.21-3.32 (4H, m), 3.55 (2H, t, J=6.6 Hz), 3.77-3.84 (4H, m), 4.01 (1H, d, J=14.2 Hz), 4.07-4.19 (3H, m), 6.56 (1H, s), 6.91 (1H, d, J=8.4 Hz), 6.99 (2H, d, J=8.8 Hz), 7.34-7.48 (8H, m), 7.79 (2H, d, J=8.8 Hz), 7.99 (1H, s).

Elementary analysis $C_{40}H_{50}N_4O_4S \cdot 0.5H_2O$, Calcd. C, 70.35 ; H, 7.38 ; N, 8.20 : Found. C, 70.31 ; H, 7.63 ; N, 8.20.

Example 16 (Preparation of Compound 16)

To (S)-4-[[(1-propyl-1H-imidazol-5-yl)methyl]sulfinyl]aniline di-p-toluoyl-D-tartrate monohydrate (1.64 g) was added 1 N hydrochloric acid (10 ml), and the mixture was extracted with ethyl acetate. To the aqueous layer was added an aqueous 25% sodium hydroxide solution (10 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl)sulfinyl]aniline. To a solution of (2E)-3-[4'-(2-butoxyethoxy)-4-(2-methylpyrrolidin-1-yl)-1,1'-biphenyl-3-yl]acrylic acid (0.80 g) and DMF (0.1 ml) in THF (20 ml) was added oxalic chloride (0.18 ml) at 0°C, and the mixture was stirred at room temperature for 1.5 hours. The solution was added dropwise to a mixture of (S)-4-[[(1-propyl-1H-imidazol-5-yl)methyl]sulfinyl]aniline and pyridine (1.52 ml) in THF (20 ml) at 0°C. After stirring the resulting mixture at room temperature for 3 days, water was added thereto and the mixture was extracted with ethyl acetate. The organic layer was washed with an aqueous 10% acetic acid solution, an aqueous sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 49) to give (Ss)-(2E)-3-[4'-(2-butoxyethoxy)-4-(2-methylpyrrolidin-1-yl)-1,1'-biphenyl-3-yl] -N-[4-[[(1-propyl-1H-imidazol-5-yl)methyl]sulfinyl]phenyl]acrylamide (Compound 16) (976 mg) as a yellow amorphous material.

$[\alpha]_D$ = -155.4° (c = 0.525%, ethanol solution)

$^1$H-NMR (200 MHz, $CDCl_3$) δ 0.89 (3H, t, J=7.3 Hz), 0.94 (3H, t, J=7.3 Hz), 1.11 (3H, d, J=6.0 Hz), 1.29-1.49 (2H, m), 1.53-2.01 (7H, m), 2.08-2.28 (1H, m), 2.93-3.08 (1H, m), 3.56 (2H, t, J=6.6 Hz), 3.64-3.84 (6H, m), 3.99 (1H, d, J=14.2 Hz), 4.07-4.19 (3H, m), 6.54 (1H, s), 6.56 (1H, d, J=15.4 Hz), 6.97 (2H, d, J=8.8 Hz), 6.99 (1H, d, J=8.4 Hz), 7.32 (2H, d, J=8.8 Hz), 7.42-7.49 (4H, m), 7.59 (1H, d, J=2.2 Hz), 7.80 (2H, d, J=8.8 Hz), 8.13 (1H, d, J=15.4 Hz), 8.74-8.78 (1H, m).

Elementary analysis $C_{39}H_{48}N_4O_4S \cdot 0.5H_2O$, Calcd. C, 69.10 ; H, 7.29 ; N, 8.26 : Found. C, 68.96 ; H, 7.43 ; N, 8.21.

Example 17 (Preparation of Compound 17)

To (S)-4-[[(1-propyl-1H-imidazol-5-yl)methyl]sulfinyl]aniline di-p-toluoyl-D-tartrate monohydrate (1.59 g) was added 1 N hydrochloric acid (10 ml), and the mixture was extracted with ethyl acetate. To the aqueous layer was added an aqueous 25% sodium hydroxide solution (10 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl]sulfinyl]aniline. To a solution of (2E)-3-[4'-(2-butoxyethoxy)-4-(2-methylpyrrolidin-1-yl)-1,1'-biphenyl-3-yl]-2-methylacrylic acid (0.80 g) and DMF (0.1 ml) in THF (20 ml) was added oxalic chloride (0.175 ml) at 0°C, and the mixture was stirred at room temperature for 1.5 hours. The solution was added dropwise to a mixture of (S)-4-[[(1-propyl-1H-imidazol-5-yl)methyl]sulfinyl]aniline and pyridine (1.48 ml) in THF (20 ml) at 0°C. After stirring the resulting mixture at room temperature for 64 hours, water was added thereto and the mixture was extracted with ethyl acetate. The organic layer was washed with an aqueous 10% acetic acid solution, water, an aqueous sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 49) to give (Ss)-(2E)-3-[4'-(2-butoxyethoxy)-4-(2-methylpyrrolidin-1-yl)-1,1'-biphenyl-3-yl]-2-methyl-N-[4-[[(1-propyl-1H-imidazol-5-yl)methyl]sulfinyl]phenyl]acrylamide (Compound 17) (427 mg) as a yellow amorphous material.

$[\alpha]_D$ = -137.9° (c = 0.501%, ethanol solution)

$^1$H-NMR (200 MHz, $CDCl_3$) δ 0.89 (3H, t, J=7.3 Hz), 0.93 (3H, t, J=7.4 Hz), 1.12 (3H, d, J=5.8 Hz), 1.26-1.46 (2H, m), 1.51-1.95 (7H, m), 2.10-2.27 (4H, m), 2.84-3.04 (1H, m), 3.45-3.63 (3H, m), 3.73-3.91 (5H, m), 4.03 (1H, d, J=13.2 Hz), 4.08-4.18 (3H, m), 6.56 (1H, s), 6.96 (1H, d, J=8.4 Hz), 6.98 (2H, d, J=8.4 Hz), 7.35-7.53 (8H, m), 7.78 (2H, d, J=8.4 Hz), 7.82 (1H, s).

IR (KBr) 3026, 1669, 1590, 1518, 1489, 1312, 1248, 1119, 820 $cm^{-1}$

Elementary analysis $C_{40}H_{50}N_4O_4S{\cdot}0.5H_2O$, Calcd. C, 69.43 ; H, 7.43 ; N, 8.10 : Found. C, 69.24 ; H, 7.73 ; N, 7.97.

Example 18 (Preparation of Compound 18)

To (S)-4-[[(1-propyl-1H-imidazol-5-yl)methyl]sulfinyl]aniline di-p-toluoyl-D-tartrate monohydrate (1.63 g) was added 1 N hydrochloric acid (10 ml), and the mixture was extracted with ethyl acetate. To the aqueous layer was added an aqueous 25% sodium hydroxide solution (10 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl]sulfinyl]aniline. To a solution of (2E)-3-[4'-(2-butoxyethoxy)-4-morpholin-4-yl-1,1'-biphenyl-3-yl]acrylic acid (0.80 g) and DMF (0.1 ml) in THF (20 ml) was added oxalic chloride (0.18 ml) at 0°C, and the mixture was stirred at room temperature for 1.5 hours. The solution was added dropwise to a mixture of (S)-4-[[(1-propyl-1H-imidazol-5-yl)methyl]sulfinyl]aniline and pyridine (1.52 ml) in THF (20 ml) at 0°C. After stirring the resulting mixture at room temperature for 20 hours, water was added thereto and the mixture was extracted with ethyl acetate. The organic layer was washed with an aqueous 10% acetic acid solution, water, an aqueous sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 49) to give (S)-(2E)-3-[4'-(2-butoxyethoxy)-4-morpholin-4-yl-1,1'-biphenyl-3-yl]-N-[4-[[(1-propyl-1H-imidazol-5-yl)methyl]sulfinyl]phenyl]acrylamide (Compound 18) (1.17 g) as a yellow amorphous material.

$[\alpha]_D$ = -158.3° (c = 0.551%, ethanol solution)

$^1$H-NMR (200 MHz, $CDCl_3$) δ 0.88 (3H, t, J=7.3 Hz), 0.94 (3H, t, J=7.3 Hz), 1.31-1.49 (2H, m), 1.56-1.80 (4H, m), 2.92-3.05 (4H, m), 3.57 (2H, t, J=6.6 Hz), 3.80-3.98 (8H, m), 4.11-4.23 (4H, m), 6.48 (1H, s), 6.73 (1H, d, J=13.7 Hz), 6.96 (2H, d, J=8.4 Hz), 7.10 (1H, d, J=8.4 Hz), 7.30 (2H, d, J=8.8 Hz), 7.43 (2H, d, J=8.8 Hz), 7.50-7.55 (2H, m), 7.63 (1H, d, J=1.8 Hz), 7.80 (2H, d, J=8.8 Hz), 8.19 (1H, d, J=15.7 Hz), 9.23-9.34 (1H, m).

Elementary analysis $C_{38}H_{46}N_4O_5S{\cdot}0.5H_2O$, Calcd. C, 67.13 ; H, 6.97 ; N, 8.24 : Found. C, 67.07 ; H, 7.10 ; N, 7.97.

Example 19 (Preparation of Compound 19)

To (S)-4-[[(1-propyl-1H-imidazol-5-yl)methyl]sulfinyl]aniline di-p-toluoyl-D-tartrate monohydrate (1.69 g) was added 1 N hydrochloric acid (10 ml), and the mixture was extracted with ethyl acetate. To the aqueous layer was added an aqueous 25% sodium hydroxide solution (10 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl]sulfinyl]aniline. To a solution of (2E)-3-[4'-(2-butoxyethoxy)-4-[methyl(propyl)amino]-1,1'-biphenyl-3-yl]acrylic acid (0.80 g) and DMF (0.1 ml) in THF (20 ml) was added oxalic chloride (0.18 ml) at 0°C, and the mixture was stirred at room temperature for 2 hours. The solution was added dropwise to a mixture of (S)-4-[[(1-propyl-1H-imidazol-5-yl)methyl]sulfinyl]aniline and pyridine (1.57 ml) in THF (20 ml) at 0°C. After stirring the resulting mixture at room temperature for 18 hours, water was added thereto and the mixture was extracted with ethyl acetate. The organic layer was washed with an aqueous 10% acetic acid solution, water, an aqueous sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 49) to give (S)-(2E)-3-[4'-(2-butoxyethoxy)-4-[methyl(propyl)amino]-1,1'-biphenyl-3-yl]-N-[4-[[(1-propyl-1H-imidazol-5-yl)methyl]sulfinyl]phenyl]acrylamide (Compound 19) (1.17 g) as a yellow amorphous material.

$[\alpha]_D$ = -161.9° (c = 0.486%, ethanol solution)

$^1$H-NMR (200 MHz, $CDCl_3$) δ 0.85-0.97 (9H, m), 1.32-1.47 (2H, m), 1.52-1.81 (6H, m), 2.77 (3H, s), 2.91-2.98 (2H, m), 3.57 (2H, t, J=6.6 Hz), 3.75-3.85 (4H, m), 3.99 (1H, d, J=14.6 Hz), 4.12-4.19 (3H, m), 6.55 (1H, s), 6.66 (1H, d, J=15.8 Hz), 6.97 (2H, d, J=8.8 Hz), 7.12 (1H, d, J=8.6 Hz), 7.32 (2H, d, J=8.8 Hz), 7.43-7.52 (4H, m), 7.63 (1H, d, J=2.2 Hz), 7.80 (2H, d, J=8.8 Hz), 8.18 (1H, d, J=15.8 Hz), 8.64-8.75 (1H, m).

Elementary analysis $C_{38}H_{48}N_4O_4S{\cdot}0.25H_2O$, Calcd. C, 69.01 ; H, 7.39 ; N, 8.47 : Found. C, 69.17 ; H, 7.70 ; N, 8.30.

Example 20 (Preparation of Compound 20)

To (S)-4-[[(1-propyl-1H-imidazol-5-yl)methyl]sulfinyl]aniline di-p-toluoyl-D-tartrate monohydrate (1.59 g) was added 1 N hydrochloric acid (10 ml), and the mixture was extracted with ethyl acetate. To the aqueous layer was added an aqueous 25% sodium hydroxide solution (10 ml), followed by extraction with ethyl acetate-2-propanol (4 : 1). The

organic layer was washed with saturated brine and dried over magnesium sulfate, which was concentrated under reduced pressure to give (S)-4-[[(1-propyl-1H-imidazol-5-yl)methyl]sulfinyl]aniline.

To a solution of (2E)-3-[4'-(2-butoxyethoxy)-4-(3-methylpiperidin-1-yl)-1,1'-biphenyl-3-yl]acrylic acid (0.80 g) and DMF (0.1 ml) in THF (20 ml) was added oxalic chloride (0.175 ml) at 0°C, and the mixture was stirred at room temperature for 1.5 hours. The solution was added dropwise to a mixture of (S)-4-[[(1-propyl-1H-imidazol-5-yl)methyl] sulfinyl]aniline and pyridine (1.48 ml) in THF (20 ml) at 0°C. After stirring the resulting mixture at room temperature for 64 hours, water was added thereto and the mixture was extracted with ethyl acetate. The organic layer was washed with an aqueous 10% acetic acid solution, water, an aqueous sodium hydrogen carbonate solution and saturated brine, and dried over magnesium sulfate. After concentration under reduced pressure, the residue was separated and purified by column chromatography (basic silica gel, ethyl acetate) to give (Ss)-(2E)-3-[4'-(2-butoxyethoxy)-4-(3-methylpiperidin-1-yl)-1,1'-biphenyl-3-yl]-N-[4-[[(1-propyl-1H-imidazol-5-yl)methyl]sulfinyl]phenyl]acrylamide (Compound 20) (1.03 g) as a yellow amorphous material.

$[\alpha]_D$ = -158.3° (c = 0.508%, ethanol solution)

$^1$H-NMR (200 MHz, $CDCl_3$) $\delta$ 0.85-0.97 (9H, m), 1.30-1.49 (2H, m), 1.55-2.04 (9H, m), 2.33-2.44 (1H, m), 2.52-2.73 (1H, m), 3.04-3.21 (2H, m), 3.56 (2H, t, J=6.6 Hz), 3.75-3.84 (4H, m), 3.99 (1H, d, J=14.4 Hz), 4.11-4.19 (3H, m), 6.55 (1H, s), 6.69 (1H, d, J=15.6 Hz), 6.97 (2H, d, J=8.8 Hz), 7.09 (1H, d, J=8.4 Hz), 7.33 (2H, d, J=8.8 Hz), 7.43-7.53 (4H, m), 7.65 (1H, d, J=2.2 Hz), 7.80 (2H, d, J=8.8 Hz), 8.16 (1H, d, J=15.6 Hz), 8.47-8.60 (1H, m).

Elementary analysis $C_{40}H_{50}N_4O_4S \cdot 0.5H_2O$, Calcd. C, 69.43 ; H, 7.43 ; N, 8.10 : Found. C, 69.58 ; H, 7.44 ; N, 7.92.

Example 21 (Preparation of Compound 21)

To (S)-4-[[(1-propyl-1H-imidazol-5-yl)methyl]sulfinyl]aniline di-p-toluoyl-D-tartrate monohydrate (1.59 g) was added 1 N hydrochloric acid (10 ml), and the mixture was extracted with ethyl acetate. To the aqueous layer was added an aqueous 25% sodium hydroxide solution (10 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl]sulfinyl]aniline. To a solution of (2E)-3-[4'-(2-butoxyethoxy)-4-(2-methylpiperidin-1-yl)-1,1'-biphenyl-3-yl]acrylic acid (0.80 g) and DMF (0.1 ml) in THF (20 ml) was added oxalic chloride (0.175 ml) at 0°C, and the mixture was stirred at room temperature for 1.5 hours. The solution was added dropwise to a mixture of (S)-4-[[(1-propyl-1H-imidazol-5-yl)methyl]sulfinyl]aniline and pyridine (1.48 ml) in THF (20 ml) at 0°C. After stirring the resulting mixture at room temperature for 2 days, water was added thereto and the mixture was extracted with ethyl acetate. The organic layer was washed with an aqueous 10% acetic acid solution, water, an aqueous sodium hydrogen carbonate solution and saturated brine, and dried over magnesium sulfate. After concentration under reduced pressure, the residue was separated and purified by column chromatography (basic silica gel, ethyl acetate) to give (Ss)-(2E)-3-[4'-(2-butoxyethoxy)-4-(2-methylpiperidin-1-yl)-1,1'-biphenyl-3-yl]-N-[4-[[(1-propyl-1H-imidazol-5-yl)methyl]sulfinyl]phenyl]acrylamide (Compound 21) (0.99 g) as a yellow amorphous material.

$[\alpha]_D$ = -156.9° (c = 0.495%, ethanol solution)

$^1$H-NMR (200 MHz, $CDCl_3$) $\delta$ 0.85-0.97 (9H, m), 1.28-1.97 (12H, m), 2.54-2.75 (1H, m), 2.93-3.24 (2H, m), 3.57 (2H, t, J=6.6 Hz), 3.74-3.85 (4H, m), 3.99 (1H, d, J=14.4 Hz), 4.11-4.19 (3H, m), 6.56 (1H, s), 6.69 (1H, d, J=15.8 Hz), 6.98 (2H, d, J=8.8 Hz), 7.18 (1H, d, J=8.0 Hz), 7.32 (2H, d, J=8.4 Hz), 7.45-7.54 (4H, m), 7.69 (1H, d, J=2.2 Hz), 7.81 (2H, d, J=8.4 Hz), 8.35 (1H, d, J=15.8 Hz), 8.63 (1H, br s).

Elementary analysis $C_{40}H_{50}N_4O_4S \cdot 0.5H_2O$, Calcd. C, 69.43 ; H, 7.43 ; N, 8.10 : Found. C, 69.17 ; H, 7.42 ; N, 7.90.

Example 22 (Preparation of Compound 22)

To (S)-4-[[(1-propyl-1H-imidazol-5-yl)methyl]sulfinyl]aniline di-p-toluoyl-D-tartrate monohydrate (1.02 g) was added 1 N hydrochloric acid (7.0 ml), and the mixture was extracted with ethyl acetate. To the aqueous layer was added an aqueous 25% sodium hydroxide solution (7.0 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl]sulfinyl]aniline. To a solution of (2E)-3-[4'-(2-butoxyethoxy)-4-(3-methylpyrrolidin-1-yl)-1,1'-biphenyl-3-yl]acrylic acid (0.50 g) and DMF (0.1 ml) in THF (10 ml) was added oxalic chloride (0.11 ml) at 0°C, and the mixture was stirred at 0°C for 40 minutes. The solution was added dropwise to a mixture of (S)-4-[[(1-propyl-1H-imidazol-5-yl)methyl]sulfinyl]aniline and pyridine (0.95 ml) in THF (20 ml) at 0°C. After stirring the resulting mixture at room temperature for 3 days, water was added thereto and the mixture was extracted with ethyl acetate. The organic layer was washed with an aqueous 10% acetic acid solution, water, an aqueous sodium hydrogen carbonate solution and saturated brine, and dried over magnesium sulfate. After concentration under reduced pressure, the residue was separated and purified by column chromatography (basic silica gel, ethyl

acetate → ethanol : ethyl acetate 1 : 49) to give (Ss)-(2E)-3-[4'-(2-butoxyethoxy)-4-(3-methylpyrrolidin-1-yl)-1,1'-biphenyl-3-yl]-N-[4-[[(1-propyl-1H-imidazol-5-yl)methyl]sulfinyl]phenyl]acrylamide (Compound 22) (511 mg) as a yellow amorphous material.

$[\alpha]_D$ = -164.8° (c = 0.484%, ethanol solution)

$^1$H-NMR (200 MHz, $CDCl_3$) δ 0.87 (3H, t, J=7.3 Hz), 0.93 (3H, t, J=7.3 Hz), 1.11 (3H, d, J=6.6 Hz), 1.30-1.49 (2H, m), 1.55-1.82 (5H, m), 1.98-2.44 (2H, m), 2.98-3.07 (1H, m), 3.28-3.51 (3H, m), 3.56 (2H, t, J=6.6 Hz), 3.74-3.84 (4H, m), 3.97 (1H, d, J=14.4 Hz), 4.13-4.19 (3H, m), 6.50 (1H, d, J=15.0 Hz), 6.52 (1H, s), 6.86 (1H, d, J=8.8 Hz), 6.95 (2H, d, J=8.8 Hz), 7.30 (2H, d, J=8.8 Hz), 7.40-7.53 (5H, m), 7.80 (2H, d, J8.8 Hz), 8.17 (1H, d, J=15.0 Hz), 9.09 (1H, s).

Example 23 (Preparation of Compound 23)

To (S)-4-[[(1-propyl-1H-imidazol-5-yl)methyl]sulfinyl]aniline di-p-toluoyl-D-tartrate monohydrate (1.59 g) was added 1 N hydrochloric acid (10.0 ml), and the mixture was extracted with ethyl acetate. To the aqueous layer was added an aqueous 25% sodium hydroxide solution (10.0 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl]sulfinyl]aniline. To a solution of (2E)-3-[4'-(2-butoxyethoxy)-4-(3-methylpyrrolidin-1-yl)-1,1'-biphenyl-3-yl]-2-methylacrylic acid (0.80 g) and DMF (0.1 ml) in THF (20 ml) was added oxalic chloride (0.17 ml) at 0°C, and the mixture was stirred at room temperature for 1.5 hours. The solution was added dropwise to a mixture of (S)-4-[[(1-propyl-1H-imidazol-5-yl)methyl]sulfinyl]aniline and pyridine (1.48 ml) in THF (20 ml) at 0°C. After stirring the resulting mixture at room temperature for 20 hours, water was added thereto and the mixture was extracted with ethyl acetate. The organic layer was washed with an aqueous 10% acetic acid solution, water, an aqueous sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 49) to give (Ss)-(2E)-3-[4'-(2-butoxyethoxy)-4-(3-methylpyrrolidin-1-yl)-1,1'-biphenyl-3-yl]-2-methyl-N-[4-[[(1-propyl-1H-imidazol-5-yl)methyl]sulfinyl]phenyl]acrylamide (Compound 23) (990 mg) as a yellow amorphous material.

$[\alpha]_D$ = -132.3° (c = 0.498%, ethanol solution)

$^1$H-NMR (200 MHz, $CDCl_3$) δ 0.91 (3H, t, J=7.4 Hz), 0.93 (3H, t, J=7.1 Hz), 1.11 (3H, d, J=6.6 Hz), 1.30-1.48 (2H, m), 1.55-1.84 (5H, m), 1.98-2.44 (5H, m), 2.88-2.96 (1H, m), 3.17-3.48 (3H, m), 3.55 (2H, t, J=6.6 Hz), 3.76-3.83 (4H, m), 4.02 (1H, d, J=14.2 Hz), 4.08-4.18 (3H, m), 6.56 (1H, s), 6.87 (1H, d, J=8.6 Hz), 6.98 (2H, d, J=8.8 Hz), 7.32-7.47 (7H, m), 7.63 (1H, s), 7.78 (2H, d, J=8.8 Hz), 7.86 (1H, s).

Example 24 (Preparation of Compound 24)

To (S)-4-[[(1-propyl-1H-imidazol-5-yl)methyl]sulfinyl]aniline di-p-toluoyl-D-tartrate monohydrate (1.53 g) was added 1 N hydrochloric acid (10.0 ml), and the mixture was extracted with ethyl acetate. To the aqueous layer was added an aqueous 25% sodium hydroxide solution (10.0 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl]sulfinyl]aniline. To a solution of (2E)-3-[4'-(2-butoxyethoxy)-4-(3-methylpyrrolidin-1-yl)-1,1'-biphenyl-3-yl]-2-methylacrylic acid (0.80 g) and DMF (0.1 ml) in THF (20 ml) was added oxalic chloride (0.17 ml) at 0°C, and the mixture was stirred at room temperature for 1.5 hours. The solution was added dropwise to a mixture of (S)-4-[[(1-propyl-1H-imidazol-5-yl)methyl]sulfinyl]aniline and pyridine (1.42 ml) in THF (20 ml) at 0°C. After stirring the resulting mixture at room temperature for 4 days, water was added thereto and the mixture was extracted with ethyl acetate. The organic layer was washed with an aqueous 10% acetic acid solution, water, an aqueous sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 49) to give (Ss)-(2E)-3-[4'-(2-butoxyethoxy)-4-(3-methylpyrrolidin-1-yl)-1,1'-biphenyl-3-yl]-2-methyl-N-[4-[[(1-propyl-1H-imidazol-5-yl)methyl]sulfinyl]phenyl]acrylamide (Compound 24) (0.76 g) as a yellow amorphous material.

$[\alpha]_D$ = -127.6° (c = 0.488%, ethanol solution)

$^1$H-NMR (200 MHz, $CDCl_3$) δ 0.91 (3H, t, J=7.5 Hz), 0.93 (3H, t, J=7.4 Hz), 1.28-1.48 (2H, m), 1.51-1.83 (4H, m), 2.03-2.23 (5H, m), 3.12-3.51 (7H, m), 3.55 (2H, t, J=6.6 Hz), 3.75-3.83 (4H, m), 3.95-4.19 (5H, m), 6.59 (1H, s), 6.93 (1H, d, J=8.8 Hz), 6.99 (2H, d, J=9.0 Hz), 7.35-7.48 (7H, m), 7.57 (1H, s), 7.78 (2H, d, J=8.8 Hz), 7.95 (1H, s).

Example 25 (Preparation of Compound 25)

To (S)-4-[[(1-propyl-1H-imidazol-5-yl)methyl]sulfinyl]aniline di-p-toluoyl-D-tartrate monohydrate (1.80 g) was added 1 N hydrochloric acid (10.0 ml), and the mixture was extracted with ethyl acetate. To the aqueous layer was

added an aqueous 25% sodium hydroxide solution (10.0 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl]sulfinyl]aniline. To a solution of (2E)-3-[4-[3-(acetoxy)pyrrolidin-1-yl-4'-(2-butoxyethoxy)]-1,1'-biphenyl-3-yl]acrylic acid (1.0 g) and DMF (0.1 ml) in THF (20 ml) was added oxalic chloride (0.20 ml) at 0°C, and the mixture was stirred at 0°C for 2 hours. The solution was added dropwise to a mixture of (S)-4-[[(1-propyl-1H-imidazol-5-yl)methyl]sulfinyl]aniline and pyridine (1.68 ml) in THF (20 ml) at 0°C. After stirring the resulting mixture at room temperature for 3 days, water was added thereto and the mixture was extracted with ethyl acetate. The organic layer was washed with an aqueous 10% acetic acid solution, water, an aqueous sodium hydrogen carbonate solution and saturated brine, and dried over magnesium sulfate. After concentration under reduced pressure, the residue was separated and purified by column chromatography (basic silica gel, ethyl acetate) to give (S)-(2E)-3-[4-[3-(acetoxy)pyrrolidin-1-yl-4'-(2-butoxyethoxy)]-1,1'-biphenyl-3-yl]-2-methyl-N-[4-[[(1-propyl-1H-imidazol-5-yl)methyl]sulfinyl]phenyl]acrylamide (Compound 25) (739.3 mg) as a yellow amorphous material.

$[\alpha]_D$ = -127.5° (c = 0.493%, ethanol solution)

$^1$H-NMR (200 MHz, $CDCl_3$) δ 0.91 (3H, t, J=7.6 Hz), 0.93 (3H, t, J=7.1 Hz), 1.25-1.46 (2H, m), 1.51-1.83 (4H, m), 2.00-2.35 (8H, m), 3.17-3.31 (2H, m), 3.42-3.67 (4H, m), 3.76-3.83 (4H, m), 4.02 (1H, d, J=14.2 Hz), 4.08-4.19 (3H, m), 5.26-5.36 (1H, m), 6.57 (1H, s), 6.92 (1H, d, J=8.6 Hz), 6.99 (2H, d, J=8.8 Hz), 7.35-7.48 (7H, m), 7.63 (1H, s), 7.78 (2H, d, J=8.8 Hz), 7.86 (1H, s).

Example 26 (Preparation of Compound 26)

To a solution of (S)-(2E)-3-[4-[3-(acetoxy)pyrrolidin-1-yl-4'-(2-butoxyethoxy)]-1,1'-biphenyl-3-yl]-2-methyl-N-[4-[[(1-propyl-1H-imidazol-5-yl)methyl]sulfinyl]phenyl]acrylamide (640 mg) in ethanol (10 ml) was added a 1 N aqueous sodium hydroxide solution (1.5 ml) at room temperature. After stirring the mixture at room temperature for 20 hours, ethanol was distilled off under reduced pressure, which was extracted with ethyl acetate. The organic layer was washed with water and saturated brine and dried over magnesium sulfate. After concentration under reduced pressure, the residue was separated and purified by column chromatography (basic silica gel, ethanol : ethyl acetate 1 : 49) to give (Ss)-(2E)-3-[4'-(2-butoxyethoxy)-4-[3-hydroxypyrrolidin-1-yl-1,1'-biphenyl-3-yl]-2-methyl-N-[4-[[(1-propyl-1H-imidazol-5-yl)methyl]sulfinyl]phenyl]acrylamide (Compound 26) (500 mg) as a yellow amorphous material.

$[\alpha]_D$ = -131.4° (c = 0.488%, ethanol solution)

$^1$H-NMR (200 MHz, $CDCl_3$) δ 0.89 (3H, t, J=7.8 Hz) 0.93 (3H, t, J=7.0 Hz), 1.29-1.49 (2H, m), 1.54-1.82 (4H, m), 1.91-2.32 (5H, m), 3.12-3.38 (3H, m), 3.42-3.59 (3H, m), 3.73-3.83 (4H, m), 3.99 (1H, d, J=14.4 Hz), 4.06-4.18 (3H, m), 4.46-4.56 (1H, m), 6.50 (1H, s), 6.94 (1H, d, J=8.0 Hz), 6.99 (2H, d, J=8.8 Hz), 7.30 (2H, d, J=8.4 Hz), 7.36-7.48 (5H, m), 7.55 (1H, s), 7.79 (2H, d, J=8.4 Hz), 8.32-8.39 (1H, m).

Example 27 (Preparation of Compound 27)

To (S)-4-[[(1-propyl-1H-imidazol-5-yl)methyl]sulfinyl]aniline di-p-toluoyl-D-tartrate monohydrate (1.03 g) was added 1 N hydrochloric acid (8.0 ml), and the mixture was extracted with ethyl acetate. To the aqueous layer was added an aqueous 25% sodium hydroxide solution (8.0 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl]sulfinyl]aniline. To a solution of (2E)-3-[4'-(2-butoxyethoxy)-4-(1H-pyrazol-1-yl)-1,1'-biphenyl-3-yl]-2-methylacrylic acid (0.50 g) and DMF (0.1 ml) in THF (10 ml) was added oxalic chloride (0.094 ml) at 0°C, and the mixture was stirred at room temperature for 1 hour. The solution was added dropwise to a mixture of (S)-4-[[(1-propyl-1H-imidazol-5-yl)methyl]sulfinyl]aniline and pyridine (0.96 ml) in THF (20 ml) at 0°C. After stirring the resulting mixture at room temperature for 18 hours, water was added thereto and the mixture was extracted with ethyl acetate. The organic layer was washed with an aqueous 5% acetic acid solution, water, an aqueous sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 49) to give (S)-(2E)-3-[4'-(2-butoxyethoxy)-4-(1H-pyrazol-1-yl)-1,1'-biphenyl-3-yl]-2-methyl-N-[4-[[(1-propyl-1H-imidazol-5-yl)methyl]sulfinyl]phenyl]acrylamide (Compound 27) (433 mg) as a colorless amorphous material.

$[\alpha]_D$ = -136.3° (c = 0.484%, ethanol solution)

$^1$H-NMR (200 MHz, $CDCl_3$) δ 0.93 (3H, t, J=7.4 Hz), 0.94 (3H, t, J=7.4 Hz), 1.29-1.47 (2H, m), 1.55-1.82 (4H, m), 2.11 (3H, d, J=1.4 Hz), 3.56 (2H, t, J=6.6 Hz), 3.74-3.85 (4H, m), 4.02 (1H, d, J=14.2 Hz), 4.07-4.21 (3H, m), 6.50 (1H, t, J=2.2 Hz), 6.58 (1H, s), 7.04 (2H, d, J=8.8 Hz), 7.22-7.29 (1H, m), 7.35 (2H, d, J=8.4 Hz), 7.47-7.80 (10H, m), 7.96 (1H, s).

Example 28 (Preparation of Compound 28)

To (S)-4-[[(1-propyl-1H-imidazol-5-yl)methyl]sulfinyl]aniline di-p-toluoyl-D-tartrate monohydrate (1.48 g) was added 1 N hydrochloric acid (10.0 ml), and the mixture was extracted with ethyl acetate. To the aqueous layer was added an aqueous 25% sodium hydroxide solution (10.0 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl]sulfinyl]aniline. To a solution of (2E)-3-[6-[4-(2-butoxyethoxy)phenyl]-1-methyl-1,2,3,4-tetrahydroquinolin-8-yl]acrylic acid (0.70 g) and DMF (0.1 ml) in THF (10 ml) was added oxalic chloride (0.16 ml) at 0°C, and the mixture was stirred at room temperature for 1 hour. DMF (5 ml) and THF (20 ml) were added thereto, and the solution was then added dropwise to a mixture of (S)-4-[[(1-propyl-1H-imidazol-5-yl)methyl]sulfinyl]aniline and pyridine (1.38 ml) in THF (20 ml) at 0°C. After stirring the resulting mixture at room temperature for 20 hours, water was added thereto and the mixture was extracted with ethyl acetate. The organic layer was washed with an aqueous 10% acetic acid solution, water, an aqueous sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 49) to give (S)-(2E)-3-[6-[4-(2-butoxyethoxy)phenyl]-1-methyl-1,2,3,4-tetrahydroquinolin-8-yl]-N-[4-[[(1-propyl-1H-imidazol-5-yl)methyl]sulfinyl]phenyl]-2-acrylic amide (Compound 28) (784.5 mg) as a yellow amorphous material.

$[\alpha]_D$ = -115.4° (c = 0.525%, ethanol solution)

$^1$H-NMR (200 MHz, $CDCl_3$) δ 0.88 (3H, t, J=7.3 Hz), 0.94 (3H, t, J=7.2 Hz), 1.29-1.49 (2H, m), 1.55-1.77 (4H, m), 1.81-1.96 (2H, m), 2.80-2.86 (5H, m), 3.12-3.22 (2H, m), 3.56 (2H, t, J=6.6 Hz), 3.74-3.84 (4H, m), 3.98 (1H, d, J=13.8 Hz), 4.11-4.18 (3H, m), 6.56 (1H, s), 6.63 (1H, d, J=16.5 Hz), 6.95 (2H, d, J=8.8 Hz), 7.21 (1H, d, J=2.2 Hz), 7.32 (2H, d, J=8.6 Hz), 7.40-7.48 (4H, m), 7.80 (2H, d, J=8.6 Hz), 8.05 (1H, d, J=16.5 Hz), 8.73 (1H, s).

Example 29 (Preparation of Compound 29)

To (S)-4-[[(1-propyl-1H-imidazol-5-yl)methyl]sulfinyl]aniline di-p-toluoyl-D-tartrate monohydrate (1.23 g) was added 1 N hydrochloric acid (8.0 ml), and the mixture was extracted with ethyl acetate. To the aqueous layer was added an aqueous 25% sodium hydroxide solution (8.0 ml), followed by extraction with ethyl acetate-2-propanol (4 : 1). The organic layer was washed with saturated brine and dried over magnesium sulfate, which was concentrated under reduced pressure to give (S)-4-[[(1-propyl-1H-imidazol-5-yl)methyl]sulfinyl]aniline. To a solution of (2E)-3-[6-[4-(2-butoxyethoxy)phenyl]-1-methyl-1,2,3,4-tetrahydroquinolin-8-yl]-2-methylacrylic acid (0.60 g) and DMF (0.1 ml) in THF (10 ml) was added oxalic chloride (0.124 ml) at 0°C, and the mixture was stirred at 0°C for 1 hour. The solution was added dropwise to a mixture of (S)-4-[[(1-propyl-1H-imidazol-5-yl)methyl]sulfinyl]aniline and pyridine (1.15 ml) in THF (20 ml) at 0°C. After stirring the resulting mixture at room temperature for 20 hours, water was added thereto and the mixture was extracted with ethyl acetate. The organic layer was washed with an aqueous 10% acetic acid solution, water, an aqueous sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 49) to give (S)-(2E)-3-[6-[4-(2-butoxyethoxy)phenyl]-1-methyl-1,2,3,4-tetrahydroquinolin-8-yl]-2-methyl-N-[4-[[(1-propyl-1H-imidazol-5-yl)methyl]sulfinyl]phenyl]-2-acrylic amide (Compound 29) (461 mg) as a yellow amorphous material.

$[\alpha]_D$ = -134.2° (c = 0.495%, ethanol solution)

$^1$H-NMR (200 MHz, $CDCl_3$) δ 0.91 (3H, t, J=7.4 Hz), 0.93 (3H, t, J=7.4 Hz), 1.31-1.45 (2H, m), 1.56-1.89 (4H, m), 1.84-1.95 (2H, m), 2.26 (3H, d, J=1.2 Hz), 2.78 (3H, s), 2.84-2.88 (2H, m), 3.16-3.19 (2H, m), 3.55 (2H, t, J=6.6 Hz), 3.75-3.82 (4H, m), 4.03 (1H, d, J=14.1 Hz), 4.07-4.17 (3H, m), 6.57 (1H, s), 6.97 (2H, d, J=9.2 Hz), 7.21 (1H, d, J=1.8 Hz), 7.27 (1H, d, J=1.8 Hz), 7.35 (2H, d, J=8.6 Hz), 7.42-7.45 (4H, m), 7.77 (2H, d, J=8.6 Hz), 7.96 (1H, s).

Example 30 (Preparation of Compounds 30 and 31)

(Ss)-(2E)-3-[4'-(2-butoxyethoxy)-4-(3-methylpyrrolidin-1-yl)-1,1'-biphenyl-3-yl]-2-methyl-N-[4-[[(1-propyl-1H-imidazol-5-yl)methyl]sulfinyl]phenyl]acrylamide (Compound 23) (570 mg) was optically resolved by using CHIRALPAK AD (50 mmID × 500 mmL) (elusion solvent, ethanol : 2-propanol = 7 : 3). The fraction was concentrated into dry solid, and the residue was dissolved in ethanol, which was filtered by a 0.45 μm filter. The filtrate was concentrated to give two diastereomers of Compound 23 [the former fraction: diastereomer 1 (Compound 30) (170 mg, 99.6%de) and the latter fraction: diastereomer 2 (Compound 31) (173 mg, 98.0%de)].

Compound 30 $[\alpha]_D$ = -64.6° (c = 0.502%, ethanol solution)

Compound 31 $[\alpha]_D$ = -197.1° (c = 0.520%, ethanol solution)

Example 31 (Preparation of Compound 32)

(S)-4-(((1-propylimidazol-5-yl)methyl)sulfinyl)aniline di-p-toluoyl-D-tartrate monohydrate (1.01 g) was dissolved in ethyl acetate (10 ml) and 1 N hydrochloric acid (5.17 ml), followed by separation. To the aqueous layer was added an aqueous 25% potassium carbonate solution (5.17 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added tetrahydrofuran, and then the solvent was again distilled off under reduced pressure to give (S)-4-(((1-propylimidazol-5-yl)methyl)sulfinyl)aniline. To a solution of (2E)-3-[4'-(2-butoxyethoxy)-4-[(2-methoxyethyl)(methyl)amino]-1,1'-biphenyl-3-yl]acrylic acid (500 mg) in tetrahydrofuran (10 ml) was added a drop of DMF, and then oxalyl chloride (0.133 ml) at 0°C. The mixture was returned to room temperature and stirred for 30 minutes under a nitrogen atmosphere. The solution was then added dropwise to a solution of (S)-4-(((1-propylimidazol-5-yl)methyl)sulfinyl)aniline and pyridine (2.46 ml) in tetrahydrofuran (10 ml) at 0°C under a nitrogen atmosphere. The mixture was returned to room temperature and stirred for 3 hours. Then, water was added thereto and the mixture was extracted with ethyl acetate. The organic layer was washed with an aqueous 10% acetic acid solution twice, an aqueous saturated sodium hydrogen carbonate solution twice and saturated brine once, and dried over magnesium sulfate. The solvent was distilled off under reduced pressure, and then the resulting residue was separated and purified by basic silica gel column chromatography (ethyl acetate → methanol : ethyl acetate = 3 : 100) to give (S)-(2E)-3-[4'-(2-butoxyethoxy)-4-[(2-methoxyethyl)(methyl)amino]-1,1'-biphenyl-3-yl]-N-[4-[[(1-propyl-1H-imidazol-5-yl)methyl]sulfinyl]phenyl]acrylamide (527 mg) (Compound 32) as a yellow amorphous material.

$^1$H-NMR (300 MHz, $CDCl_3$) δ 0.88-0.96 (6H, m), 1.34-1.46 (2H, m), 1.57-1.79 (4H, m), 2.87 (3H, s), 3.18 (2H, t, J=5.7 Hz), 3.43 (3H, s), 3.56 (2H, t, J=6.9 Hz), 3.69 (2H, t, J=5.7 Hz), 3.77 (2H, t, J=7.5 Hz), 3.82 (2H, t, J=4.5 Hz), 4.03 (1H, d, J=14.1 Hz), 4.11 (1H, d, J=14.1 Hz), 4.17 (2H, t, J=4.5 Hz), 6.61-6.67 (2H, m), 7.00 (2H, d, J=8.7 Hz), 7.18 (1H, d, J=8.4 Hz), 7.36 (2H, d, J=8.7 Hz), 7.46-7.54 (4H, m), 7.70 (1H, d, J=2.1 Hz), 7.79 (2H, d, J=8.7 Hz), 8.13 (1H, d, J=16.2 Hz), 8.56 (1H, s).

Elementary analysis $C_{38}H_{48}N_4O_5S \cdot 0.25H_2O$, Calcd. C, 67.38 ; H, 7.22 ; N, 8.27 ; Found. C, 67.08 ; H, 7.62 ; N, 8.16.

$[\alpha]_D$ = -158.1° (C = 0.322%, in ethanol)

Example 32 (Preparation of Compound 33)

(S)-4-(((1-propylimidazol-5-yl)methyl)sulfinyl)aniline di-p-toluoyl-D-tartrate monohydrate (636 mg) was dissolved in ethyl acetate (10 ml) and 1 N hydrochloric acid (3.24 ml), followed by separation. To the aqueous layer was added an aqueous 25% potassium carbonate solution (3.24 ml), followed by extraction with 2-propanol-ethyl acetate (1 : 4). The organic layer was washed with saturated brine, dried over magnesium sulfate, and then the solvent was distilled off under reduced pressure. To the resulting residue was added tetrahydrofuran, and then the solvent was again distilled off under reduced pressure to give (S)-4-(((1-propylimidazol-5-yl)methyl)sulfinyl)aniline. To a solution of (2E)-3-[4'-(2-butoxyethoxy)-4-[methyl[(1-methyl-1H-pyrazol-4-yl)methyl]amino]-1,1'-biphenyl-3-yl]acrylic acid (350 mg) in dichloromethane (20 ml) was added a drop of DMF, and then oxalyl chloride (0.083 ml) at 0°C. The mixture was returned to room temperature and stirred for 30 minutes under a nitrogen atmosphere. The solution was then added dropwise to a solution of (S)-4-(((1-propylimidazol-5-yl)methyl)sulfinyl)aniline and pyridine (1.54 ml) in tetrahydrofuran (20 ml) at 0°C under a nitrogen atmosphere. The mixture was returned to room temperature and stirred for 3 hours. Then, water was added thereto and the mixture was extracted with ethyl acetate. The organic layer was washed with an aqueous 10% acetic acid solution twice, an aqueous saturated sodium hydrogen carbonate solution twice and saturated brine once, and dried over magnesium sulfate. The solvent was distilled off under reduced pressure, and then the resulting residue was separated and purified by basic silica gel column chromatography (ethyl acetate → methanol : ethyl acetate = 1 : 12) to give (S)-(2E)-3-[4'-(2-butoxyethoxy)-4-[methyl[(1-methyl-1H-pyrazol-4-yl)methyl]amino]-1,1'-biphenyl-3-yl]-N-[4-[[(1-propyl-1H-imidazol-5-yl)methyl]sulfinyl]phenyl]acrylamide (328 mg) (Compound 33) as a yellow amorphous material.

$^1$H-NMR (300 MHz, $CDCl_3$) δ 0.86-0.96 (6H, m), 1.34-1.47 (2H, m), 1.58-1.78 (4H, m), 2.71 (3H, s), 3.57 (2H, t, J=6.6 Hz), 3.77-3.84 (4H, m), 3.88 (3H, s), 3.95 (2H, s), 3.99 (1H, d, J=13.8 Hz), 4.13-4.18 (3H, m), 6.54 (1H, s), 6.71 (1H, d, J=15.6 Hz), 6.98 (2H, d, J=8.4 Hz), 7.07 (1H, d, J=8.4 Hz), 7.32 (2H, d, J=8.7 Hz), 7.38 (1H, s), 7.44-7.51 (5H, m), 7.66 (1H, d, J=2.1 Hz), 7.80 (2H, d, J=8.7 Hz), 8.27 (1H, d, J=15.6 Hz), 8.87 (1H, s).

Elementary analysis $C_{40}H_{48}N_6O_4S \cdot 0.75H_2O$, Calcd. C, 66.50 ; H, 6.91 ; N, 11.63 ;Found. C, 66.59 ; H, 7.02 ; N, 11.52.

$[\alpha]_D$ = -147.3° (C = 0.398%, in ethanol)

Example 33 (Preparation of Compound 34)

(S)-4-(((1-propylimidazol-5-yl)methyl)sulfinyl)aniline di-p-toluoyl-D-tartrate monohydrate (424 mg) was dissolved in ethyl acetate (5 ml) and 1 N hydrochloric acid (2.13 ml), followed by separation. To the aqueous layer was added an aqueous 25% potassium carbonate solution (2.13 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added tetrahydrofuran, and then the solvent was again distilled off under reduced pressure to give (S)-4-(((1-propylimidazol-5-yl)methyl)sulfinyl) aniline. To a solution of (2E)-3-[4'-(2-butoxyethoxy)-4-[methyl[(1-methyl-1H-pyrazol-4-yl)methyl]amino]-1,1'-biphenyl-3-yl]-2-methylacrylic acid (230 mg) in dichloromethane (10 ml) was added a drop of DMF, and then oxalyl chloride (0.055 ml) at 0°C. The mixture was returned to room temperature and stirred for 30 minutes under a nitrogen atmosphere. The solution was then added dropwise to a solution of (S)-4-(((1-propylimidazol-5-yl)methyl)sulfinyl)aniline and pyridine (1.01 ml) in tetrahydrofuran (10 ml) at 0°C under a nitrogen atmosphere. The mixture was returned to room temperature and stirred for 3 hours. Then, water was added thereto and the mixture was extracted with ethyl acetate. The organic layer was washed with an aqueous 10% acetic acid solution twice, an aqueous saturated sodium hydrogen carbonate solution twice and saturated brine once, and dried over magnesium sulfate. The solvent was distilled off under reduced pressure, and then the resulting residue was separated and purified by basic silica gel column chromatography (ethyl acetate → methanol : ethyl acetate = 1 : 12) to give (S)-(2E)-3-[4'-(2-butoxyethoxy)-4-[methyl[(1-methyl-1H-pyrazol-4-yl)methyl]amino]-1,1'-biphenyl-3-yl]-2-methyl-N-[4-[[(1-propyl-1H-imidazol-5-yl)methyl]sulfinyl]phenyl]acrylamide (236 mg) (Compound 34) as a yellow amorphous material.

$^1$H-NMR (300 MHz, $CDCl_3$) δ 0.89-0.96 (6H, m), 1.34-1.46 (2H, m), 1.56-1.78 (4H, m), 2.29 (3H, d, J=0.9 Hz), 2.74 (3H, s), 3.56 (2H, t, J=6.9 Hz), 3.80-3.83 (7H, m), 3.96 (2H, s), 4.04 (1H, d, J=14.1 Hz), 4.10 (1H, d, J=14.1 Hz), 4.17 (2H, t, J=5.4 Hz), 6.59 (1H, s), 7.01 (2H, d, J=8.7 Hz), 7.06 (1H, d, J=9.3 Hz), 7.26-7.28 (1H, m), 7.36-7.39 (3H, m), 7.47-7.50 (5H, m), 7.62 (1H, s), 7.73 (2H, d, J=8.7 Hz), 7.84 (1H, s) .

Elementary analysis $C_{41}H_{50}N_6O_4S \cdot 0.5H_2O$, Calcd. C, 67.28 ; H, 7.02 ; N, 11.48 ; Found. C, 66.97 ; H, 6.97 ; N, 11.37.

$[\alpha]_D$ = -126.7° (C = 0.357%, in ethanol)

Example 34 (Preparation of Compound 35)

(S)-4-(((1-propylimidazol-5-yl)methyl)sulfinyl)aniline di-p-toluoyl-D-tartrate monohydrate (256 mg) was dissolved in ethyl acetate (5 ml) and 1 N hydrochloric acid (2.6 ml), followed by separation. To the aqueous layer was added an aqueous 25% potassium carbonate solution (2.6 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added tetrahydrofuran, and then the solvent was again distilled off under reduced pressure to give (S)-4-(((1-propylimidazol-5-yl)methyl)sulfinyl) aniline. To a solution of (2E)-3-[4'-(2-butoxyethoxy)-4-pyrrolidin-1-yl-1,1'-biphenyl-3-yl]but-2-enoic acid (125 mg) in tetrahydrofuran (10 ml) was added a drop of DMF, and then oxalyl chloride (0.034 ml) at 0°C. The mixture was returned to room temperature and stirred for 30 minutes under a nitrogen atmosphere. The solution was then added dropwise to a solution of (S)-4-(((1-propylimidazol-5-yl)methyl)sulfinyl)aniline and pyridine (0.62 ml) in tetrahydrofuran (10 ml) at 0°C under a nitrogen atmosphere. The mixture was returned to room temperature and stirred for 3 hours. Then, water was added thereto and the mixture was extracted with ethyl acetate. The organic layer was washed with an aqueous 10% acetic acid solution twice, an aqueous saturated sodium hydrogen carbonate solution twice and saturated brine once, and dried over magnesium sulfate. The solvent was distilled off under reduced pressure, and then the resulting residue was separated and purified by basic silica gel column chromatography (hexane : ethyl acetate = 1 : 1 → ethyl acetate) to give (S)-(2E)-3-[4'-(2-butoxyethoxy)-4-pyrrolidin-1-yl-1,1'-biphenyl-3-yl]-N-[4-[[(1-propyl-1H-imidazol-5-yl)methyl]sulfinyl]phenyl]but-2-enoic amide (41 mg) (Compound 35) as a yellow amorphous material.

$^1$H-NMR (300 MHz, $CDCl_3$) δ 0.87-0.95 (6H, m), 1.35-1.43 (2H, m), 1.55-1.75 (4H, m), 1.88-1.95 (4H, m), 2.60 (3H, s), 3.20-3.30 (4H, m), 3.55 (2H, t, J=6.3 Hz), 3.75-3.82 (4H, m), 3.99 (1H, d, J=13.8 Hz), 4.07-4.16 (3H, m), 6.02 (1H, s), 6.53 (1H, s), 6.87 (1H, d, J=8.4 Hz), 6.96 (2H, d, J=9.0 Hz), 7.24-7.47 (7H, m), 7.74 (2H, d, J=8.7 Hz), 7.86 (1H, s).

Elementary analysis $C_{39}H_{48}N_4O_4S \cdot 0.5H_2O$, Calcd. C, 69.10 ; H, 7.29 ; N, 8.26 ; Found. C, 69.20 ; H, 7.37 ; N, 8.33.

$[\alpha]_D$ = -144.7° (C = 0.301%, in ethanol)

Example 35 (Preparation of Compound 36)

(S)-4-(((1-propylimidazol-5-yl)methyl)sulfinyl)aniline di-p-toluoyl-D-tartrate monohydrate (371 mg) was dissolved in ethyl acetate (5 ml) and 1 N hydrochloric acid (2.8 ml), followed by separation. To the aqueous layer was added an aqueous 25% potassium carbonate solution (2.8 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added tetrahydrofuran, and then the solvent was again distilled off under reduced pressure to give (S)-4-(((1-propylimidazol-5-yl)methyl)sulfinyl) aniline. To a solution of (2E)-3-[4'-(2-butoxyethoxy)-4-(2,5-dihydro-1H-pyrrol-1-yl)-1,1'-biphenyl-3-yl]-2-methylacrylic acid (180 mg) in tetrahydrofuran (10 ml) was added a drop of DMF, and then oxalyl chloride (0.048 ml) at 0°C. The mixture was returned to room temperature and stirred for 30 minutes under a nitrogen atmosphere. The solution was then added dropwise to a solution of (S)-4-(((1-propylimidazol-5-yl)methyl)sulfinyl)aniline and pyridine (0.9 ml) in tetrahydrofuran (10 ml) at 0°C under a nitrogen atmosphere. The mixture was returned to room temperature and stirred for 3 hours. Then, water was added thereto and the mixture was extracted with ethyl acetate. The organic layer was washed with an aqueous 10% acetic acid solution twice, an aqueous saturated sodium hydrogen carbonate solution twice and saturated brine once, and dried over magnesium sulfate. The solvent was distilled off under reduced pressure, and then the resulting residue was separated and purified by basic silica gel column chromatography (ethyl acetate → methanol : ethyl acetate = 1 : 25) to give (S)-(2E)-3-[4'-(2-butoxyethoxy)-4-(1H-pyrrol-1-yl)-1,1'-biphenyl-3-yl]-2-methyl-N-[4-[[(1-propyl-1H-imidazol-5-yl)methyl]sulfinyl]phenyl]acrylamide (107 mg) (Compound 36) as a yellow amorphous material.

$^1$H-NMR (300 MHz, $CDCl_3$) δ 0.89-0.96 (6H, m), 1.35-1.50 (2H, m), 1.55-1.80 (4H, m), 2.16 (3H, s), 3.56 (2H, t, J=6.3 Hz), 3.76-3.84 (4H, m), 4.00-4.20 (4H, m), 6.36-6.40 (2H, m), 6.57 (1H, s), 6.85-6.91 (3H, m), 7.04 (2H, d, J=8.7 Hz), 7.35 (2H, d, J=9.0 Hz), 7.43-7.69 (9H, m).

Elementary analysis $C_{39}H_{44}N_4O_4S \cdot 0.5H_2O$, Calcd. C, 69.51 ; H, 6.73 ; N, 8.31 ; Found. C, 69.38 ; H, 6.72 ; N, 8.06.

$[\alpha]_D$ = -130.1° (C = 0.244%, in ethanol)

Example 36 (Preparation of Compound 37)

(S)-4-(((1-propylimidazol-5-yl)methyl)sulfinyl)aniline di-p-toluoyl-D-tartrate monohydrate (721 mg) was dissolved in ethyl acetate (10 ml) and 1 N hydrochloric acid (5.4 ml), followed by separation. To the aqueous layer was added an aqueous 25% potassium carbonate solution (5.4 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added tetrahydrofuran, and then the solvent was again distilled off under reduced pressure to give (S)-4-(((1-propylimidazol-5-yl)methyl)sulfinyl) aniline. To a solution of (2E)-3-[4'-(2-butoxyethoxy)-4-(1,4-dioxa-7-azaspiro[4.4]non-7-yl)-1,1'-biphenyl-3-yl]-2-methylacrylic acid (400 mg) in tetrahydrofuran (10 ml) was added a drop of DMF, and then oxalyl chloride (0.094 ml) at 0°C. The mixture was returned to room temperature and stirred for 30 minutes under a nitrogen atmosphere. The solution was then added dropwise to a solution of (S)-4-(((1-propylimidazol-5-yl)methyl)sulfinyl)aniline and pyridine (1.75 ml) in tetrahydrofuran (10 ml) at 0°C under a nitrogen atmosphere. The mixture was returned to room temperature and stirred for 3 hours. Then, water was added thereto and the mixture was extracted with ethyl acetate. The organic layer was washed with water twice and saturated brine once, and then dried over magnesium sulfate. The solvent was distilled off under reduced pressure, which was separated and purified by basic silica gel column chromatography (ethyl acetate → methanol : ethyl acetate = 1 : 50). The resulting residue was recrystallized from diisopropyl ether-ethyl acetate to give (S)-(2E)-3-[4'-(2-butoxyethoxy)-4-(1,4-dioxa-7-azaspiro[4.4]non-7-yl)-1,1'-biphenyl-3-yl]-2-methyl-N-[4-[[(1-propyl-1H-imidazol-5-yl)methyl]sulfinyl]phenyl]acrylamide (319 mg) (Compound 37) as yellow crystals.

m.p. 139.5-140.5°C

$^1$H-NMR (300 MHz, $CDCl_3$) δ 0.89-0.96 (6H, m), 1.33-1.46 (2H, m), 1.56-1.77 (4H, m), 2.18-2.23 (5H, m), 3.26 (2H, s), 3.38 (2H, t, J=6.6 Hz), 3.55 (2H, t, J=6.6 Hz), 3.75-3.82 (4H, m), 3.89-4.18 (8H, m), 6.62 (1H, s), 6.94-7.00 (3H, m), 7.37-7.51 (8H, m), 7.81 (2H, d, J=8.7 Hz), 8.00 (1H, s).

Elementary analysis $C_{41}H_{50}N_4O_6S$, Calcd. C, 67.74 ; H, 6.93 ; N, 7.71 ; Found. C, 67.48 ; H, 7.17 ; N, 7.57.

$[\alpha]_D$ = -128.4° (C = 0.465%, in ethanol)

Example 37 (Preparation of Compound 38)

(-)-4-(((1-Propylimidazol-5-yl)methyl)sulfinyl)aniline di-p-toluoyl-D-tartrate monohydrate (473 mg) was dissolved in ethyl acetate (10 ml) and 1 N hydrochloric acid (3.54 ml), followed by separation. To the aqueous layer was added an aqueous 25% potassium carbonate solution (3.54 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added tetrahydrofuran, and then the solvent was again distilled off under reduced pressure to give (-)-4-(((1-propylimidazol-5-yl)methyl)sulfinyl)

aniline. To a solution of (2E)-3-[4-[3-[(acetyloxy)methyl]pyrrolidin-1-yl]-4'-(2-butoxyethoxy)-1,1'-biphenyl-3-yl]-2-methylacrylic acid (270 mg) in tetrahydrofuran (10 ml) was added a drop of DMF, and then oxalyl chloride (0.062 ml) at 0°C. The mixture was returned to room temperature and stirred for 30 minutes under a nitrogen atmosphere. The solution was then added dropwise to a solution of (-)-4-(((1-propylimidazol-5-yl)methyl)sulfinyl)aniline and pyridine (1.15 ml) in tetrahydrofuran (10 ml) at 0°C under a nitrogen atmosphere. The mixture was returned to room temperature and stirred overnight. Then, water was added thereto and the mixture was extracted with ethyl acetate. The organic layer was washed with water twice and saturated brine once, and then dried over magnesium sulfate. The solvent was distilled off under reduced pressure, and then the resulting residue was separated and purified by basic silica gel column chromatography (ethyl acetate → methanol : ethyl acetate = 1 : 25) to give (Ss)-(2E)-3-[4-[3-(acetoxymethyl)pyrrolidin-1-yl]-4'-(2-butoxyethoxy)-1,1'-biphenyl-3-yl]-2-methyl-N-[4-[[(1-propyl-1H-imidazol-5-yl)methyl]sulfinyl]phenyl]acrylamide (222 mg) (Compound 38) as a yellow amorphous material.

$^1$H-NMR (300 MHz, $CDCl_3$) δ 0.90-0.96 (6H, m), 1.36-1.43 (2H, m), 1.50-1.78 (5H, m), 2.05-2.19 (7H, m), 2.55-2.70 (1H, m), 3.09-3.15 (1H, m), 3.29-3.35 (3H, m), 3.55 (2H, t, J=6.6 Hz), 3.78-3.83 (4H, m), 4.02-4.18 (6H, m), 6.58 (1H, s), 6.92 (1H, d, J=9.0 Hz), 6.99 (2H, d, J=9.0 Hz), 7.35-7.47 (7H, m), 7.59 (1H, s), 7.77-7.80 (3H, m).

Elementary analysis $C_{42}H_{52}N_4O_6S \cdot 0.5H_2O$, Calcd. C, 67.26 ; H, 7.12 ; N, 7.47 ; Found. C, 67.01 ; H, 7.06 ; N, 7.28.

$[\alpha]_D$ = -118.2° (C = 0.350%, in ethanol)

Example 38 (Preparation of Compound 39)

To a solution of (Ss)-(2E)-3-[4-[3-(acetoxymethyl)pyrrolidin-1-yl]-4'-(2-butoxyethoxy)-1,1'-biphenyl-3-yl]-2-methyl-N-[4-[[(1-propyl-1H-imidazol-5-yl)methyl]sulfinyl]phenyl]acrylamide (Compound 38) (150 mg) in tetrahydrofuran (3 ml) and methanol (3 ml) was added a 1 N aqueous sodium hydroxide solution (0.3 ml), and the mixture was stirred at room temperature for 3 hours. To the reaction solution was added water, and the mixture was extracted with ethyl acetate. The organic layer was washed with saturated brine and dried over magnesium sulfate. The solvent was distilled off under reduced pressure, and then the resulting residue was separated and purified by basic silica gel column chromatography (ethyl acetate → methanol : ethyl acetate = 1 : 19) to give (Ss)-(2E)-3-[4'-(2-butoxyethoxy)-4-[3-(hydroxymethyl)pyrrolidin-1-yl]-1,1'-biphenyl-3-yl]-2-methyl-N-[4-[[(1-propyl-1H-imidazol-5-yl)methyl]sulfinyl]phenyl]acrylamide (91.8 mg) (Compound 39) as a yellow amorphous material.

$^1$H-NMR (300 MHz, $CDCl_3$) δ 0.89-0.96 (6H, m), 1.36-1.46 (2H, m), 1.50-1.85 (5H, m), 2.10-2.25 (4H, m), 2.45-2.55 (1H, m), 2.83-2.90 (1H, m), 3.15-3.30 (2H, m), 3.48-3.57 (3H, m), 3.75-3.82 (6H, m), 3.98-4.17 (4H, m), 6.51 (1H, d, J=6.3 Hz), 6.97-7.03 (3H, m), 7.31-7.35 (2H, m), 7.41-7.47 (6H, m), 7.82 (2H, d, J=8.1 Hz), 8.78 (1H, d, J=3.9 Hz).

Elementary analysis $C_{40}H_{50}N_4O_5S \cdot 0.5H_2O$, Calcd. C, 67.87 ; H, 7.26 ; N, 7.91 ; Found. C, 67.58 ; H, 7.24 ; N, 7.88.

$[\alpha]_D$ = -125.9° (C = 0.370%, in ethanol)

Example 39 (Preparation of Compound 40)

(-)-4-(((1-Propylimidazol-5-yl)methyl)sulfinyl)aniline di-p-toluoyl-D-tartrate monohydrate (423 mg) was dissolved in ethyl acetate (10 ml) and 1 N hydrochloric acid (3.17 ml), followed by separation. To the aqueous layer was added an aqueous 25% potassium carbonate solution (3.17 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added tetrahydrofuran, and then the solvent was again distilled off under reduced pressure to give (-)-4-(((1-propylimidazol-5-yl)methyl)sulfinyl)aniline. To a solution of (2E)-3-[4'-(2-butoxyethoxy)-4-[3-(methoxycarbonyl)pyrrolidin-1-yl]-1,1'-biphenyl-3-yl]-2-methylacrylic acid (235 mg) in tetrahydrofuran (10 ml) was added a drop of DMF, and then oxalyl chloride (0.055 ml) at 0°C. The mixture was returned to room temperature and stirred for 30 minutes under a nitrogen atmosphere. The solution was then added dropwise to a solution of (-)-4-(((1-propylimidazol-5-yl)methyl)sulfinyl)aniline and pyridine (1.03 ml) in tetrahydrofuran (10 ml) at 0°C under a nitrogen atmosphere. The mixture was returned to room temperature and stirred overnight. Then, water was added thereto and the mixture was extracted with ethyl acetate. The organic layer was washed with water twice and saturated brine once, and then dried over magnesium sulfate. The solvent was distilled off under reduced pressure, and then the resulting residue was separated and purified by basic silica gel column chromatography (ethyl acetate → methanol : ethyl acetate = 1 : 20) to give (Ss)-(2E)-3-[4'-(2-butoxyethoxy)-4-[3-(methoxycarbonyl)pyrrolidin-1-yl]-1,1'-biphenyl-3-yl]-2-methyl-N-[4-[[(1-propyl-1H-imidazol-5-yl)methyl]sulfinyl]phenyl]acrylamide (38 mg) (Compound 40) as a yellow amorphous material.

$^1$H-NMR (300 MHz, $CDCl_3$) δ 0.88-0.96 (6H, m), 1.35-1.47 (2H, m), 1.50-1.80 (4H, m), 2010-2.25 (4H, m), 2.30-2.50 (1H, m), 2.77-2.83 (1H, m), 3.10-3.35 (3H, m), 3.56 (2H, t, J=6.6 Hz), 3.70-3.77 (5H, m), 3.81 (2H, t, J=4.8 Hz), 3.95-4.18 (5H, m), 6.66 (1H, s), 6.97-7.01 (3H, m), 7.37 (2H, d, J=7.8 Hz), 7.44-7.49 (6H, m), 7.98-8.02 (2H, m),

9.04 (1H, s).

Elementary analysis $C_{41}H_{50}N_4O_6S\cdot 0.5H_2O$, Calcd. C, 66.91 ; H, 6.98 ; N, 7.61 ; Found. C, 66.83 ; H, 6.99 ; N, 7.44.

$[\alpha]_D$ = -123.6° (C = 0.223%, in ethanol)

Example 40 (Preparation of Compound 41)

To a solution of (Ss)-(2E)-3-[4'-(2-butoxyethoxy)-4-[3-(methoxycarbonyl)pyrrolidin-1-yl]-1,1'-biphenyl-3-yl]-2-methyl-N-[4-[[(1-propyl-1H-imidazol-5-yl)methyl]sulfinyl]phenyl]acrylamide (Compound 40) (220 mg) in tetrahydrofuran (6 ml) and methanol (6 ml) was added a 1 N aqueous sodium hydroxide solution (0.12 ml), and the mixture was stirred at room temperature for 7 hours. To the reaction solution were added water and 1 N hydrochloric acid (0.9 ml), and the mixture was extracted with ethyl acetate. The organic layer was washed with saturated brine and dried over magnesium sulfate. The solvent was distilled off under reduced pressure, and then the resulting residue was separated and purified by preparative HPLC to give (Ss)-(2E)-3-[4'-(2-butoxyethoxy)-4-(3-carboxypyrrolidin-1-yl)-1,1'-biphenyl-3-yl]-2-methyl-N-[4-[[(1-propyl-1H-imidazol-5-yl)methyl]sulfinyl]phenyl]acrylamide (117 mg) (Compound 41) as a yellow amorphous material.

m.p. 184.0-186.0°C

Elementary analysis $C_{40}H_{48}N_4O_6S\cdot 0.5H_2O$, Calcd. C, 66.55 ; H, 6.84 ; N, 7.76 ; Found. C, 66.68 ; H, 6.76 ; N, 7.49.

$[\alpha]_D$ = -155.0° (C = 0.324%, in ethanol)

Example 41 (Preparation of Compound 42)

(-)-4-(((1-Propylimidazol-5-yl)methyl)sulfinyl)aniline di-p-toluoyl-D-tartrate monohydrate (768 mg) was dissolved in ethyl acetate (10 ml) and 1 N hydrochloric acid (5.75 ml), followed by separation. To the aqueous layer was added an aqueous 25% potassium carbonate solution (5.75 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added tetrahydrofuran, and then the solvent was again distilled off under reduced pressure to give (-)-4-(((1-propylimidazol-5-yl)methyl)sulfinyl)aniline. To a solution of (2E)-3-[4'-(2-butoxyethoxy)-4-(3,4-dimethylpyrrolidin-1-yl)-1,1'-biphenyl-3-yl]-2-methylacrylic acid (400 mg) in tetrahydrofuran (10 ml) was added a drop of DMF, and then oxalyl chloride (0.1 ml) at 0°C. The mixture was returned to room temperature and stirred for 30 minutes under a nitrogen atmosphere. The solution was then added dropwise to a solution of (-)-4-(((1-propylimidazol-5-yl)methyl)sulfinyl)aniline and pyridine (1.86 ml) in tetrahydrofuran (15 ml) at 0°C under a nitrogen atmosphere. The mixture was returned to room temperature and stirred overnight. Then, water was added thereto and the mixture was extracted with ethyl acetate. The organic layer was washed with water twice and saturated brine once, and then dried over magnesium sulfate. The solvent was distilled off under reduced pressure, and then the resulting residue was separated and purified by basic silica gel column chromatography (ethyl acetate → methanol : ethyl acetate = 1 : 20) to give (S)-(2E)-3-[4'-(2-butoxyethoxy)-4-(3,4-dimethylpyrrolidin-1-yl)-1,1'-biphenyl-3-yl]-2-methyl-N-[4-[[(1-propyl-1H-imidazol-5-yl)methyl]sulfinyl]phenyl]acrylamide (316 mg) (Compound 42) as a yellow amorphous material.

$^1$H-NMR (300 MHz, $CDCl_3$) δ 0.89-0.98 (12H, m), 1.35-1.45 (2H, m), 1.50-1.65 (2H, m), 1.70-1.78 (2H, m), 2.16 (3H, d, J=1.2 Hz), 2.22-2.37 (2H, m), 2.99-3.04 (2H, m), 3.41-3.46 (2H, m), 3.54 (2H, t, J=6.6 Hz), 3.77-3.81 (4H, m), 4.03 (1H, d, J=14.1 Hz), 4.07-4.16 (3H, m), 6.56 (1H, s), 6.83 (1H, d, J=8.7 Hz), 6.96 (2H, d, J=8.7 Hz), 7.30 (1H, d, J=1.8 Hz), 7.35-7.45 (6H, m), 7.64 (1H, s), 7.75-7.79 (3H, m).

Elementary analysis $C_{41}H_{52}N_4O_4S\cdot 0.5H_2O$, Calcd. C, 69.76 ; H, 7.57 ; N, 7.94 ; Found. C, 69.62 ; H, 7.47 ; N, 7.69.

$[\alpha]_D$ = -126.6° (C = 0.374%, in ethanol)

Example 42 (Preparation of Compound 43)

To a solution of (Ss)-(2E)-3-[4'-(2-butoxyethoxy)-4-(3-carboxypyrrolidin-1-yl)-1,1'-biphenyl-3-yl]-2-methyl-N-[4-[[(1-propyl-1H-imidazol-5-yl)methyl]sulfinyl]phenyl]acrylamide (Compound 41) (120 mg), ammonium chloride (11.2 mg) and 1-hydroxybenzotriazole monohydrate (33.4 mg) in DMF (5 ml) were added triethylamine (0.03 ml) and a catalytic amount of 4-(N,N-dimethylamino)pyridine, followed by adding 1-ethyl-3-(3-dimethylaminopropyl)-carbodiimide hydrochloride (41.8 mg), and the mixture was stirred overnight under a nitrogen atmosphere. Water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (ethyl acetate → methanol : ethyl acetate = 1 : 11) to give (Ss)-(2E)-3-[4'-(2-butoxyethoxy)-4-(3-carbamoylpyrrolidin-1-yl)-1,1'-biphenyl-3-yl]-2-methyl-N-[4-[[(1-propyl-1H-imidazol-5-yl) methyl]sulfinyl]phenyl]acrylamide (Compound 43) (32.4 mg) as a yellow amorphous material.

$^1$H-NMR (300 MHz, $CDCl_3$) δ 0.87-0.96 (6H, m), 1.33-1.46 (2H, m), 1.56-1.90 (4H, m), 2.10-2.30 (4H, m), 2.35-2.45 (1H, m), 2.71 (1H, t, J=8.1 Hz), 2.95-3.07 (1H, m), 3.11-3.20 (1H, m), 3.32-3.37 (1H, m), 3.55 (2H, t, J=6.9 Hz), 3.73-3.83 (4H, m), 3.93-4.18 (5H, m), 5.78 (1H, br), 5.97-6.09 (1H, m), 6.50 (1H, d, J=17.1 Hz), 6.96-7.01 (3H, m), 7.26-7.32 (2H, m), 7.41-7.48 (6H, m), 6.69 (2H, d, J=7.5 Hz), 9.45 (1H, s).

Elementary analysis $C_{40}H_{49}N_5O_5S \cdot 1.0H_2O$, Calcd. C, 65.82 ; H, 7.04 ; N, 9.59 ; Found. C, 65.82 ; H, 7.01 ; N, 9.28.

$[\alpha]_D$ = -122.8° (C = 0.247%, in ethanol)

Example 43 (Preparation of Compound 44)

To a solution of (Ss)-(2E)-3-[4'-(2-butoxyethoxy)-4-[3-carboxypyrrolidin-1-yl]-1,1'-biphenyl-3-yl]-2-methyl-N-[4-[[(1-propyl-1H-imidazol-5-yl)methyl]sulfinyl]phenyl]acrylamide (Compound 41) (120 mg), methylammonium chloride (14.2 mg) and 1-hydroxybenzotriazole monohydrate (33.4 mg) in DMF (5 ml) were added triethylamine (0.03 ml) and a catalytic amount of 4-(N,N-dimethylamino)pyridine, followed by adding 1-ethyl-3-(3-dimethylaminopropyl)-carbodiimide hydrochloride (41.8 mg), and the mixture was stirred overnight under a nitrogen atmosphere. Water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (ethyl acetate → methanol : ethyl acetate = 1 : 11) and recrystallized from ethyl acetate-diisopropyl ether to give (Ss)-(2E)-3-[4'-(2-butoxyethoxy)-4-[3-(methylaminocarbonyl)pyrrolidin-1-yl]-1,1'-biphenyl-3-yl]-2-methyl-N-[4-[[(1-propyl-1H-imidazol-5-yl)methyl]sulfinyl]phenyl]acrylemide (Compound 44) (65.2 mg) as yellow crystals.

m.p. 127.0-128.5°C

$^1$H-NMR (300 MHz, $CDCl_3$) δ 0.87-0.96 (6H, m), 1.36-1.43 (2H, m), 1.50-1.74 (4H, m), 2.05-2.25 (4H, m), 2.30-2.45 (1H, m), 2.60-2.70 (1H, m), 2.85-3.00 (4H, m), 3.05-3.20 (1H, m), 3.30-3.40 (1H, m), 3.55 (2H, t, J=6.6 Hz), 3.65-3.75 (2H, m), 3.81 (2H, t, J=4.2 Hz), 4.00-4.18 (5H, m), 5.60-5.70 (1H, m), 6.71 (1H, s), 6.98-7.01 (3H, m), 7.35-7.38 (2H, m), 7.46-7.49 (6H, m), 8.14-8.19 (2H, m), 9.70 (1H, s).

Elementary analysis $C_{41}H_{51}N_5O_5S \cdot 0.5H_2O$, Calcd. C, 67.00 ; H, 7.13 ; N, 9.53 ; Found. C, 66.78 ; H, 7.06 ; N, 9.25.

$[\alpha]_D$ = -124.7° (C = 0.322%, in ethanol)

Example 44 (Preparation of Compound 45)

To a solution of (Ss)-(2E)-3-[4'-(2-butoxyethoxy)-4-[3-carboxypyrrolidin-1-yl]-1,1'-biphenyl-3-yl]-2-methyl-N-[4-[[(1-propyl-1H-imidazol-5-yl)methyl]sulfinyl]phenyl]acrylamide (Compound 41) (120 mg), dimethylammonium chloride (17.1 mg) and 1-hydroxybenzotriazole monohydrate (33.4 mg) in DMF (5 ml) were added triethylamine (0.03 ml) and a catalytic amount of 4-(N,N-dimethylamino)pyridine, followed by adding 1-ethyl-3-(3-dimethylaminopropyl)-carbodiimide hydrochloride (41.8 mg), and the mixture was stirred overnight under a nitrogen atmosphere. Water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (ethyl acetate → methanol : ethyl acetate = 1 : 11) and recrystallized from ethyl acetate-diisopropyl ether to give (Ss)-(2E)-3-[4'-(2-butoxyethoxy)-4-[3-(dimethylaminocarbonyl)pyrrolidin-1-yl]-1,1'-biphenyl-3-yl]-2-methyl-N-[4-[[(1-propyl-1H-imidazol-5-yl)methyl]sulfinyl]phenyl]acrylamide (Compound 45) (76.5 mg) as yellow crystals.

m.p. 151.0-152.0°C

$^1$H-NMR (300 MHz, $CDCl_3$) δ 0.86-0.96 (6H, m), 1.36-1.73 (6H, m), 1.90-2.05 (1H, m), 2.25 (3H, s), 2.45-2.60 (2H, m), 2.95-3.20 (7H, m), 3.22-3.40 (2H, m), 3.55 (2H, t, J=6.6 Hz), 3.62-3.70 (2H, m), 3.81 (2H, t, J=4.5 Hz), 4.00-4.29 (5H, m), 6.74 (1H, d, J=4.8 Hz), 6.96-7.01 (3H, m), 7.33-7.37 (2H, m), 7.44-7.54 (6H, m), 8.13-8.19 (2H, m), 10.09 (1H, s).

Elementary analysis $C_{42}H_{53}N_5O_5S \cdot 0.75H_2O$, Calcd. C, 66.95 ; H, 7.29 ; N, 9.29 ; Found. C, 66.96 ; H, 7.13 ; N, 9.32.

$[\alpha]_D$ = -117.7° (C = 0.331%, in ethanol)

Example 45 (Preparation of Compound 46)

(-)-4-(((1-Propylimidazol-5-yl)methyl)sulfinyl)aniline di-p-toluoyl-D-tartrate monohydrate (360 mg) was dissolved in ethyl acetate (5 ml) and 1 N hydrochloric acid (2.7 ml), followed by separation. To the aqueous layer was

added an aqueous 25% potassium carbonate solution (2.7 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added tetrahydrofuran, and then the solvent was again distilled off under reduced pressure to give (-)-4-(((1-propylimidazol-5-yl)methyl)sulfinyl) aniline. To a solution of (2E)-3-[5-[4-(2-butoxyethoxy)phenyl]-2-pyrrolidin-1-ylpyridin-3-yl]acrylic acid (170 mg) in dichloromethane (10 ml) was added a drop of DMF, and then oxalyl chloride (0.047 ml) at 0°C. The mixture was returned to room temperature and stirred for 30 minutes under a nitrogen atmosphere. The solution was then added dropwise to a solution of (-)-4-(((1-propylimidazol-5-yl)methyl)sulfinyl)aniline and pyridine (0.87 ml) in tetrahydrofuran (10 ml) at 0°C under a nitrogen atmosphere. The mixture was returned to room temperature and stirred for 3 hours. Then, water was added thereto and the mixture was extracted with ethyl acetate. The organic layer was washed with water twice and saturated brine once, and then dried over magnesium sulfate. The solvent was distilled off under reduced pressure, and then the resulting residue was separated and purified by basic silica gel column chromatography (ethyl acetate → methanol : ethyl acetate = 3 : 100) to give (S)-(2E)-3-[5-[4-(2-butoxyethoxy)phenyl]-2-pyrrolidin-1-ylpyridin-3-yl] -N-[4-[[(1-propyl-1H-imidazol-5-yl)methyl]sulfinyl]phenyl]acrylamide (131 mg) (Compound 46) as a yellow amorphous material.

$^1$H-NMR (200 MHz, $CDCl_3$) δ 0.87-0.96 (6H, m), 1.34-1.44 (2H, m), 1.50-1.80 (4H, m), 1.90-1.96 (4H, m), 3.56 (2H, t, J=6.9 Hz), 3.60-3.68 (4H, m), 3.78-3.83 (4H, m), 3.98 (1H, d, J=14.1 Hz), 4.13-4.18 (3H, m), 6.40 (1H, d, J=15.0 Hz), 6.51 (1H, s), 6.98 (2H, d, J=8.7 Hz), 7.32 (2H, d, J=9.0 Hz), 7.40 (2H, d, J=8.7 Hz), 7.48 (1H, s), 7.71 (1H, d, J=2.4 Hz), 7.78 (2H, d, J=9.0 Hz), 8.11 (2H, d, J=15.0 Hz), 8.36 (1H, d, J=2.4 Hz), 8.59 (1H, s) .

Elementary analysis $C_{37}H_{45}N_5O_4S\cdot 0.5H_2O$, Calcd. C, 66.84 ; H, 6.97 ; N, 10.53 ; Found. C, 66.72 ; H, 6.96 ; N, 10.24.

$[\alpha]_D$ = -166.5° (C = 0.327%, in ethanol)

Example 46 (Preparation of Compound 47)

(-)-4-(((1-Propylimidazol-5-yl)methyl)sulfinyl)aniline di-p-toluoyl-D-tartrate monohydrate (327 mg) was dissolved in ethyl acetate (5 ml) and 1 N hydrochloric acid (2.45 ml), followed by separation. To the aqueous layer was added an aqueous 25% potassium carbonate solution (2.45 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added tetrahydrofuran, and then the solvent was again distilled off under reduced pressure to give (-)-4-(((1-propylimidazol-5-yl)methyl)sulfinyl) aniline. To a solution of (2E)-3-[5-[4-(2-butoxyethoxy)phenyl]-2-pyrrolidin-1-ylpyridin-3-yl]-2-methylacrylic acid (160 mg) in dichloromethane (10 ml) was added a drop of DMF, and then oxalyl chloride (0.043 ml) at 0°C. The mixture was returned to room temperature and stirred for 30 minutes under a nitrogen atmosphere. The solution was then added dropwise to a solution of (-)-4-(((1-propylimidazol-5-yl)methyl)sulfinyl)aniline and pyridine (0.79 ml) in tetrahydrofuran (10 ml) at 0°C under a nitrogen atmosphere. The mixture was returned to room temperature and stirred for 3 hours. Then, water was added thereto and the mixture was extracted with ethyl acetate. The organic layer was washed with water twice and saturated brine once, and then dried over magnesium sulfate. The solvent was distilled off under reduced pressure, and then the resulting residue was separated and purified by basic silica gel column chromatography (ethyl acetate → methanol : ethyl acetate = 1 : 100) to give (S)-(2E)-3-[5-[4-(2-butoxyethoxy)phenyl]-2-pyrrolidin-1-ylpyridin-3-yl]-2-methyl-N-[4-[[(1-propyl-1H-imidazol-5-yl)methyl]sulfinyl]phenyl]acrylamide (156 mg) (Compound 47) as a yellow amorphous material.

$^1$H-NMR (200 MHz, $CDCl_3$) δ 0.91-0.96 (6H, m), 1.36-1.43 (2H, m), 1.52-1.66 (2H, m), 1.72-1.79 (2H, m), 1.90-2.00 (4H, m), 2.13 (3H, s), 3.50-3.57 (6H, m), 3.79-3.85 (4H, m), 4.03 (1H, d, J=14.4 Hz), 4.09-4.18 (3H, m), 6.53 (1H, s), 7.00 (2H, d, J=8.7 Hz), 7.35-7.47 (5H, m), 7.50 (1H, d, J=2.4 Hz), 7.64 (1H, s), 7.76-7.79 (3H, m), 8.36 (1H, d, J=2.4 Hz).

Elementary analysis $C_{38}H_{47}N_5O_4S\cdot 0.5H_2O$, Calcd. C, 67.23 ; H, 7.13 ; N, 10.32 ; Found. C, 67.11 ; H, 7.05 ; N, 10.08.

$[\alpha]_D$ = -134.8° (C = 0.407%, in ethanol)

Example 47 (Preparation of Compound 48)

(-)-4-(((1-Propylimidazol-5-yl)methyl)sulfinyl)aniline di-p-toluoyl-D-tartrate monohydrate (988 mg) was dissolved in ethyl acetate (7 ml) and 1 N hydrochloric acid (5.03 ml), followed by separation. To the aqueous layer was added an aqueous 25% potassium carbonate solution (5.03 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added tetrahydrofuran, and then the solvent was again distilled off under reduced pressure to give (-)-4-(((1-propylimidazol-5-yl)methyl)sulfinyl)

aniline. To a solution of (2E)-3-[5-[4-(2-butoxyethoxy)phenyl]-2-(3-methylpyrrolidin-1-yl)pyridin-3-yl]-2-methylacrylic acid (500 mg) in dichloromethane (10 ml) was added a drop of DMF, and then oxalyl chloride (0.13 ml) at 0°C. The mixture was returned to room temperature and stirred for 30 minutes under a nitrogen atmosphere. The solution was then added dropwise to a solution of (-)-4-(((1-propylimidazol-5-yl)methyl)sulfinyl)aniline and pyridine (2.4 ml) in tetrahydrofuran (10 ml) at 0°C under a nitrogen atmosphere. The mixture was returned to room temperature and stirred for 3 hours. Then, water was added thereto and the mixture was extracted with ethyl acetate. The organic layer was washed with water twice and saturated brine once, and then dried over magnesium sulfate. The solvent was distilled off under reduced pressure, and then the resulting residue was separated and purified by basic silica gel column chromatography (ethyl acetate → methanol : ethyl acetate = 3 : 100) to give (Ss)-(2E)-3-[5-[4-(2-butoxyethoxy)phenyl]-2-(3-methylpyrrolidin-1-yl)pyridin-3-yl]-2-methyl-N-[4-[[(1-propyl-1H-imidazol-5-yl)methyl]sulfinyl]phenyl]acrylamide (660 mg) (Compound 48) as a yellow amorphous material.

$^1$H-NMR (200 MHz, $CDCl_3$) δ 0.90-0.95 (6H, m), 1.10 (3H, d, J=6.6 Hz), 1.36-1.43 (2H, m), 1.50-1.78 (5H, m), 2.00-2.11 (1H, m), 2.13 (3H, d, J=1.2 Hz), 2.20-2.40 (1H, m), 3.11-3.18 (1H, m), 3.48-3.66 (5H, m), 3.78-3.84 (4H, m), 4.02 (1H, d, J=14.1 Hz), 4.09-4.17 (3H, m), 6.52 (1H, s), 6.99 (2H, d, J=9.0 Hz), 7.34-7.48 (6H, m), 7.62 (1H, s), 7.75-7.80 (3H, m), 8.34 (1H, d, J=2.4 Hz).

Elementary analysis $C_{39}H_{49}N_5O_4S \cdot 0.5H_2O$, Calcd. C, 67.60 ; H, 7.27 ; N, 10.11 ; Found. C, 67.50 ; H, 7.18 ; N, 9.88.

$[\alpha]_D$ = -135.6° (C = 0.333%, in ethanol)

Example 48 (Preparation of Compound 49)

(-)-4-(((1-Propylimidazol-5-yl)methyl)sulfinyl)aniline di-p-toluoyl-D-tartrate monohydrate (701 mg) was dissolved in ethyl acetate (10 ml) and 1 N hydrochloric acid (3.6 ml), followed by separation. To the aqueous layer was added an aqueous 25% potassium carbonate solution (3.6 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added tetrahydrofuran, and then the solvent was again distilled off under reduced pressure to give (-)-4-(((1-propylimidazol-5-yl)methyl)sulfinyl) aniline. To a solution of (2E)-3-[2-(3-acetoxymethyl)pyrrolidin-1-yl]-5-[4-(2-butoxyethoxy)phenyl]pyridin-3-yl]-2-methylacrylic acid (400 mg) in dichloromethane (10 ml) was added a drop of DMF, and then oxalyl chloride (0.092 ml) at 0°C. The mixture was returned to room temperature and stirred for 30 minutes under a nitrogen atmosphere. The solution was then added dropwise to a solution of (-)-4-(((1-propylimidazol-5-yl)methyl)sulfinyl)aniline and pyridine (2.08 ml) in tetrahydrofuran (10 ml) at 0°C under a nitrogen atmosphere. The mixture was returned to room temperature and stirred for 3 hours. Then, water was added thereto and the mixture was extracted with ethyl acetate. The organic layer was washed with water twice and saturated brine once, and then dried over magnesium sulfate. The solvent was distilled off under reduced pressure, and then the resulting residue was separated and purified by basic silica gel column chromatography (ethyl acetate → methanol : ethyl acetate = 1 : 19) to give (Ss)-(2E)-3-[2-[3-(acetoxymethyl)pyrrolidin-1-yl]-5-[4-(2-butoxyethoxy)phenyl]pyridin-3-yl]-2-methyl-N-[4-[[(1-propyl-1H-imidazol-5-yl)methyl]sulfinyl]phenyl]acrylamide (458 mg) (Compound 49) as a yellow amorphous material.

$^1$H-NMR (200 MHz, $CDCl_3$) δ 0.90-0.96 (6H, m), 1.33-1.43 (2H, m), 1.55-1.79 (5H, m), 1.95-2.13 (7H, m), 2.55-2.65 (1H, m), 3.37-3.43 (1H, m), 3.53-3.59 (4H, m), 3.63-3.69 (1H, m), 3.79-3.85 (4H, m), 4.01-4.18 (6H, m), 6.53 (1H, s), 7.00 (2H, d, J=8.7 Hz), 7.36-7.47 (5H, m), 7.52 (1H, d, J=1.8 Hz), 7.60 (1H, s), 7.79 (2H, d, J=8.7 Hz), 7.85 (1H, s), 8.36 (1H, d, J=2.4 Hz).

Elementary analysis $C_{41}H_{51}N_5O_6S \cdot 0.5H_2O$, Calcd. C, 65.58 ; H, 6.98 ; N, 9.33 ; Found. C, 65.59 ; H, 7.01 ; N, 9.03.

$[\alpha]_D$ = -119.8° (C = 0.408%, in ethanol)

Example 49 (Preparation of Compound 50)

To a solution of (Ss)-(2E)-3-[2-[3-(acetoxymethyl)pyrrolidin-1-yl]-5-[4-(2-butoxyethoxy)phenyl]pyridin-3-yl]-2-methyl-N-[4-[[(1-propyl-1H-imidazol-5-yl)methyl]sulfinyl]phenyl]acrylamide (Compound 49) (220 mg) in tetrahydrofuran (4 ml) and methanol (4 ml) was added a 1 N aqueous sodium hydroxide solution (0.445 ml), and the mixture was stirred at room temperature for 3.5 hours. To the reaction solution were added water and 1 N hydrochloric acid (0.4 ml), and the mixture was extracted with ethyl acetate. The organic layer was washed with saturated brine and dried over magnesium sulfate. The solvent was distilled off under reduced pressure, and then the resulting residue was separated and purified by silica gel column chromatography (ethyl acetate → methanol : ethyl acetate = 1 : 9) to give (Ss)-(2E)-3-[5-[4-(2-butoxyethoxy)phenyl]-2-[3-(hydroxymethyl)pyrrolidin-1-yl]pyridin-3-yl]-2-methyl-N-[4-[[(1-propyl-1H-imidazol-5-yl)methyl]sulfinyl]phenyl]acrylamide (150 mg) (Compound 50) as a yellow amorphous material.

$^1$H-NMR (200 MHz, $CDCl_3$) δ 0.88-0.96 (6H, m), 1.33-1.46 (2H, m), 1.50-1.79 (5H, m), 2.05-2.16 (4H, m),

2.45-2.55 (1H, m), 3.35-3.50 (1H, m), 3.53-3.58 (4H, m), 3.67-3.71 (1H, m), 3.79-4.18 (10H, m), 6.41 (1H, d, J=4.8 Hz), 7.00 (2H, d, J=9.0 Hz), 7.28-7.46 (6H, m), 7.52 (1H, d, J=2.4 Hz), 7.80 (2H, dd, J=8.7, 1.8 Hz), 8.26 (1H, d, J=3.9 Hz), 8.36 (1H, d, J=2.7 Hz).

Elementary analysis $C_{39}H_{49}N_5O_5S \cdot 0.75H_2O$, Calcd. C, 65.66 ; H, 7.13 ; N, 9.82 ; Found. C, 65.60 ; H, 7.08 ; N, 9.54.

$[\alpha]_D$ = -128.6° (C = 0.436%, in ethanol)

Example 50 (Preparation of Compound 51)

(-)-4-(((1-Propylimidazol-5-yl)methyl)sulfinyl)aniline di-p-toluoyl-D-tartrate monohydrate (409 mg) was dissolved in ethyl acetate (5 ml) and 1 N hydrochloric acid (2.1 ml), followed by separation. To the aqueous layer was added an aqueous 25% potassium carbonate solution (2.1 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added tetrahydrofuran, and then the solvent was again distilled off under reduced pressure to give (-)-4-(((1-propylimidazol-5-yl)methyl)sulfinyl) aniline. To a solution of (2E)-3-[5-[4-(2-butoxyethoxy)phenyl]-2-(3,4-dimethylpyrrolidin-1-yl)pyridin-3-yl]-2-methylacrylic acid (220 mg) in dichloromethane (10 ml) was added a drop of DMF, and then oxalyl chloride (0.053 ml) at 0°C. The mixture was returned to room temperature and stirred for 30 minutes under a nitrogen atmosphere. The solution was then added dropwise to a solution of (-)-4-(((1-propylimidazol-5-yl)methyl)sulfinyl)aniline and pyridine (0.99 ml) in tetrahydrofuran (10 ml) at 0°C under a nitrogen atmosphere. The mixture was returned to room temperature and stirred for 3 hours. Then, water was added thereto and the mixture was extracted with ethyl acetate. The organic layer was washed with water twice and saturated brine once, and then dried over magnesium sulfate. The solvent was distilled off under reduced pressure, and then the resulting residue was separated and purified by basic silica gel column chromatography (hexane : ethyl acetate = 1 : 1 → ethyl acetate) to give (S)-(2E)-3-[5-[4-(2-butoxyethoxy)phenyl]-2-(3,4-dimethylpyrrolidin-1-yl)pyridin-3-yl]-2-methyl-N-[4-[[(1-propyl-1H-imidazol-5-yl)methyl]sulfinyl]phenyl]acrylamide (220 mg) (Compound 51) as a yellow amorphous material.

$^1$H-NMR (200 MHz, $CDCl_3$) δ 0.90-0.98 (12H, m), 1.36-1.46 (2H, m), 1.56-1.80 (4H, m), 2.12 (3H, s), 2.20-2.40 (2H, m), 3.22-3.27 (2H, m), 3.55 (2H, t, J=6.6 Hz), 3.58-3.65 (2H, m), 3.79-3.85 (4H, m), 4.03 (1H, d, J=14.1 Hz), 4.09-4.17 (3H, m), 6.53 (1H, s), 6.99 (2H, d, J=8.7 Hz), 7.35-7.52 (6H, m), 7.64 (1H, s), 7.77-7.81 (3H, m), 8.39 (1H, d, J=2.1 Hz).

Elementary analysis $C_{40}H_{51}N_5O_4S \cdot 0.5H_2O$, Calcd. C, 67.96 ; H, 7.41 ; N, 9.91 ; Found. C, 67.86 ; H, 7.23 ; N, 9.67.

$[\alpha]_D$ = -133.5° (C = 0.260%, in ethanol)

Example 51 (Preparation of Compound 52)

To a solution of (2E)-3-[5-[4-(2-butoxyethoxy)phenyl]-2-(3-methylpyrrolidin-1-yl)pyridin-3-yl]-2-methylacrylic acid (210 mg) in dichloromethane (10 ml) was added a drop of DMF, and then oxalyl chloride (0.054 ml) at 0°C. The mixture was returned to room temperature and stirred for 30 minutes under a nitrogen atmosphere. The solution was then added dropwise to a solution of 4-[[(4-propyl-4H-1,2,4-triazol-3-yl)methyl]sulfanyl]aniline (155 mg) in pyridine (10 ml) at 0°C under a nitrogen atmosphere. The mixture was returned to room temperature and stirred for 3 hours. Then, water was added thereto and the mixture was extracted with ethyl acetate. The organic layer was washed with water twice and saturated brine once, and then dried over magnesium sulfate. The solvent was distilled off under reduced pressure, and then the resulting residue was separated and purified by basic silica gel column chromatography (hexane : ethyl acetate = 3 : 2 → ethyl acetate) to give (2E)-3-[5-[4-(2-butoxyethoxy)phenyl]-2-(3-methylpyrrolidin-1-yl) pyridin-3-yl]-2-methyl-N-[4-[[(4-propyl-4H-1,2,4-triazol-3-yl)methyl]sulfanyl]phenyl]acrylamide (270 mg) (Compound 52) as a yellow amorphous material.

$^1$H-NMR (200 MHz, $CDCl_3$) δ 0.93 (3H, t, J=7.2 Hz), 1.00 (3H, t, J=7.5 Hz), 1.11 (3H, d, J=6.6 Hz), 1.36-1.45 (2H, m), 1.50-1.70 (3H, m), 1.83-1.93 (2H, m), 2.05-2.15 (4H, m), 2.25-2.35 (1H, m), 3.10-3.20 (1H, m), 3.52-3.70 (5H, m), 3.81 (2H, t, J=4.5 Hz), 3.98 (2H, t, J=7.5 Hz), 4.16 (2H, t, J=4.5 Hz), 4.21 (2H, s), 7.00 (2H, d, J=9.0 Hz), 7.33-7.65 (9H, m), 8.08 (1H, s), 8.35 (1H, d, J=2.1 Hz).

Elementary analysis $C_{38}H_{48}N_6O_3S \cdot 0.75H_2O$, Calcd. C, 66.88 ; H, 7.31 ; N, 12.32 ; Found. C, 66.79 ; H, 7.36 ; N, 12.04.

Example 52 (Preparation of Compound 53)

To a solution of (2E)-3-[5-[4-(2-butoxyethoxy)phenyl]-2-(3-methylpyrrolidin-1-yl)pyridin-3-yl]-2-methyl-N-[4-[[(4-propyl-4H-1,2,4-triazol-3-yl)methyl]sulfanyl]phenyl]acrylamide (200 mg) in dichloromethane (10 ml) was

added dropwise at -78°C a solution of 3-chloroperbenzoic acid (70%, 112 mg) in dichloromethane (10 ml). After stirring the mixture as such for 30 minutes, the dry ice-acetone bath was removed, and an aqueous sodium thiosulfate solution was added thereto while vigorously stirring. The resulting mixture was returned to room temperature and stirred for 30 minutes, which was then extracted with ethyl acetate. The organic layer was washed with an aqueous saturated sodium hydrogen carbonate solution and saturated brine, and dried over magnesium sulfate. The solvent was distilled off under reduced pressure, and then the resulting residue was separated and purified by basic silica gel column chromatography (ethyl acetate → methanol : ethyl acetate = 1 : 19) to give (2E)-3-[5-[4-(2-butoxyethoxy)phenyl]-2-(3-methylpyrrolidin-1-yl)pyridin-3-yl]-2-methyl-N-[4-[[(4-propyl-4H-1,2,4-triazol-3-yl)methyl]sulfinyl]phenyl]acrylamide (112 mg) (Compound 53) as a yellow amorphous material.

$^1$H-NMR (200 MHz, $CDCl_3$) δ 0.90-0.99 (6H, m), 1.12 (3H, d, J=6.6 Hz), 1.33-1.46 (2H, m), 1.50-1.85 (5H, m), 2.00-2.15 (4H, m), 2.20-2.40 (1H, m), 3.12-3.18 (1H, m), 3.45-3.66 (5H, m), 3.81 (2H, t, J=4.5 Hz), 4.00 (2H, t, J=7.8 Hz), 4.10-4.23 (3H, m), 4.33 (1H, d, J=13.8 Hz), 7.00 (2H, d, J=8.7 Hz), 7.38-7.50 (5H, m), 7.61 (1H, s), 7.79-7.82 (3H, m), 8.13 (1H, s), 8.35 (1H, d, J=2.4 Hz).

Elementary analysis $C_{38}H_{48}N_6O_4S \cdot 0.5H_2O$, Calcd. C, 65.77 ; H, 7.12 ; N, 12.11 ; Found. C, 65.62 ; H, 7.29 ; N, 11.82.

Example 53 (Preparation of Compound 54)

To a solution of (2E)-3-[2-[3-(acetoxymethyl)pyrrolidin-1-yl]-5-[4-(2-butoxyethoxy)phenyl]pyridin-3-yl]-2-methylacrylic acid (370 mg) in dichloromethane (10 ml) was added a drop of DMF, and then oxalyl chloride (0.085 ml) at 0°C. The mixture was returned to room temperature and stirred for 30 minutes under a nitrogen atmosphere. The solution was then added dropwise to a solution of 4-[[(4-propyl-4H-1,2,4-triazol-3-yl)methyl]sulfanyl]aniline (241 mg) in pyridine (10 ml) at 0°C under a nitrogen atmosphere. The mixture was returned to room temperature and stirred for 3 hours. Then, water was added thereto and the mixture was extracted with ethyl acetate. The organic layer was washed with water twice and saturated brine once, and then dried over magnesium sulfate. The solvent was distilled off under reduced pressure, and then the resulting residue was separated and purified by basic silica gel column chromatography (hexane : ethyl acetate = 3 : 2 → ethyl acetate) to give (2E)-3-[2-[3-(acetoxymethyl)pyrrolidin-1-yl]-5-[4-(2-butoxyethoxy)phenyl]pyridin-3-yl]-2-methyl-N-[4-[[(4-propyl-4H-1,2,4-triazol-3-yl)methyl]sulfanyl]phenyl]acrylamide (388 mg) (Compound 54) as a yellow amorphous material.

$^1$H-NMR (200 MHz, $CDCl_3$) δ 0.91-1.03 (6H, m), 1.33-1.46 (2H, m), 1.53-1.90 (5H, m), 2.03-2.10 (7H, m), 2.55-2.65 (1H, m), 3.36-3.41 (1H, m), 3.53-3.57 (4H, m), 3.63-3.69 (1H, m), 3.81 (2H, t, J=4.8 Hz), 3.98 (2H, t, J=7.5 Hz), 4.05-4.17 (4H, m), 4.21 (2H, s), 7.00 (2H, d, J=9.0 Hz), 7.35 (2H, d, J=9.0 Hz), 7.42 (2H, d, J=9.0 Hz), 7.51 (1H, d, J=2.4 Hz), 7.55-7.59 (3H, m), 7.69 (1H, s), 8.07 (1H, s), 8.35 (1H, d, J=2.4 Hz).

Elementary analysis $C_{40}H_{50}N_6O_5S$, Calcd. C, 66.09 ; H, 6.93 ; N, 11.56 ; Found. C, 65.92 ; H, 7.04 ; N, 11.59.

Example 54 (Preparation of Compound 55)

To a solution of (2E)-3-[2-[3-(acetoxymethyl)pyrrolidin-1-yl]-5-[4-(2-butoxyethoxy)phenyl]pyridin-3-yl]-2-methyl-N-[4-[[(4-propyl-4H-1,2,4-triazol-3-yl)methyl]sulfanyl]phenyl]acrylamide (Compound 54) (240 mg) in dichloromethane (10 ml) was added dropwise at -78°C a solution of 3-chloroperbenzoic acid (70%, 106 mg) in dichloromethane (10 ml). After stirring the mixture as such for 10 minutes, the dry ice-acetone bath was removed, and an aqueous sodium thiosulfate solution was added thereto while vigorously stirring. The resulting mixture was returned to room temperature and stirred for 30 minutes, which was extracted with ethyl acetate. The organic layer was washed with an aqueous saturated sodium hydrogen carbonate solution and saturated brine, and dried over magnesium sulfate. The solvent was distilled off under reduced pressure, and then the resulting residue was separated and purified by basic silica gel column chromatography (ethyl acetate → methanol : ethyl acetate = 1 : 19) to give (2E)-3-[2-[3-(acetoxymethyl)pyrrolidin-1-yl]-5-[4-(2-butoxyethoxy)phenyl]pyridin-3-yl]-2-methyl-N-[4-[[(4-propyl-4H-1,2,4-triazol-3-yl)methyl]sulfinyl]phenyl]acrylamide (147 mg) (Compound 55) as a yellow amorphous material.

$^1$H-NMR (200 MHz, $CDCl_3$) δ 0.89-0.99 (6H, m), 1.36-1.43 (2H, m), 1.55-1.83 (5H, m), 2.02-2.22 (7H, m), 2.55-2.65 (1H, m), 3.37-3.42 (1H, m), 3.50-3.57 (4H, m), 3.62-3.70 (1H, m), 3.80 (2H, t, J=4.8 Hz), 4.00 (2H, t, J=7.8 Hz), 4.09-4.23 (5H, m), 4.32 (1H, d, J=14.1 Hz), 6.99 (2H, d, J=9.0 Hz), 7.40-7.57 (6H, m), 7.79-7.82 (3H, m), 8.12 (1H, s), 8.35 (1H, d, J=2.1 Hz).

Elementary analysis $C_{40}H_{50}N_6O_6S \cdot 0.5H_2O$, Calcd. C, 63.89 ; H, 6.84 ; N, 11.18 ; Found. C, 63.61 ; H, 6.75 ; N, 10.88.

Example 55 (Preparation of Compound 56)

To a solution of (2E)-3-[2-[3-(acetoxymethyl)pyrrolidin-1-yl]-5-[4-(2-butoxyethoxy)phenyl]pyridin-3-yl]-2-methyl-N-[4-[[(4-propyl-4H-1,2,4-triazol-3-yl)methyl]sulfinyl]phenyl]acrylamide (Compound 55) (110 mg) in tetrahydrofuran (2 ml) and methanol (2 ml) was added a 1 N aqueous sodium hydroxide solution (0.222 ml), and the mixture was stirred at room temperature for 5.5 hours. To the reaction solution were added water and 1 N hydrochloric acid (0.25 ml), and the mixture was extracted with ethyl acetate. The organic layer was washed with saturated brine and dried over magnesium sulfate. The solvent was distilled off under reduced pressure, and then the resulting residue was separated and purified by basic silica gel column chromatography (ethyl acetate → methanol : ethyl acetate = 1 : 6) to give (2E)-3-[5-[4-(2-butoxyethoxy)phenyl]-2-[3-(hydroxymethyl)pyrrolidin-1-yl]pyridin-3-yl]-2-methyl-N-[4-[[(4-propyl-4H-1,2,4-triazol-3-yl)methyl]sulfinyl]phenyl]acrylamide (74 mg) (Compound 56) as a yellow amorphous material.

$^1$H-NMR (200 MHz, $CDCl_3$) δ 0.90-0.98 (6H, m), 1.36-1.43 (2H, m), 1.50-1.83 (5H, m), 2.00-2.55 (6H, m), 3.39-4.21 (15H, m), 4.31 (1H, d, J=12.6 Hz), 6.99 (2H, d, J=8.7 Hz), 7.32-7.60 (6H, m), 7.79-7.82 (2H, m), 8.11-8.14 (2H, m), 8.35 (1H, d, J=2.1 Hz).

Elementary analysis $C_{38}H_{48}N_6O_5S{\cdot}0.5H_2O$, Calcd. C, 64.29 ; H, 6.96 ; N, 11.84 ; Found. C, 64.19 ; H, 7.06 ; N, 11.61.

Example 56 (Preparation of Compounds 57 and 58)

(Ss)-(2E)-3-[4'-(2-butoxyethoxy)-4-[3-(methoxycarbonyl)pyrrolidin-1-yl]-1,1'-biphenyl-3-yl]-2-methyl-N-[4-[[(1-propyl-1H-imidazol-5-yl)methyl]sulfinyl]phenyl]acrylamide (300 mg) was resolved by using CHIRAKPAK AD (50 mmID × 500 mmL) (hexane : 2-propanol = 1 : 1) to give two diastereomers [the former fraction: diastereomer 1 (Compound 57) (147 mg, >99%de) and the latter fraction: diastereomer 2 (Compound 58) (146 mg, >99%de)].

Compound 57 $[\alpha]_D$ = -197.8° (C = 0.177%, in ethanol)

Compound 58 $[\alpha]_D$ = -44.2° (C = 0.175%, in ethanol)

Example 57 (Preparation of Compound 59)

The optically resolved (Ss)-(2E)-3-[4'-(2-butoxyethoxy)-4-[3-(methoxycarbonyl)pyrrolidin-1-yl]-1,1'-biphenyl-3-yl]-2-methyl-N-[4-[[(1-propyl-1H-imidazol-5-yl)methyl]sulfinyl]phenyl]acrylamide (75 mg) (the former fraction: diastereomer 1 (Compound 57)) in Example 56 was dissolved in tetrahydrofuran (2.5 ml) and methanol (2.5 ml), and a 1 N aqueous sodium hydroxide solution (0.31 ml) was added thereto. The mixture was stirred for 7 hours under a nitrogen atmosphere and light shielding. After adding 1 N hydrochloric acid (0.5 ml), water and saturated brine were added thereto, and the mixture was extracted with ethyl acetate. The organic layer was dried over magnesium sulfate, and then the solvent was distilled off under reduced pressure. The resulting residue was recrystallized from methanol-acetone to give (Ss)-(2E)-3-[4'-(2-butoxyethoxy)-4-(3-carboxypyrrolidin-1-yl)-1,1'-biphenyl-3-yl]-2-methyl-N-[4-[[(1-propyl-1H-imidazol-5-yl)methyl]sulfinyl]phenyl]acrylamide (diastereomer 1 (Compound 59)) (21.0 mg, 99.0%de) as colorless crystals.

$[\alpha]_D$ = -376.1° (C = 0.224%, in chloroform)

Example 58 (Preparation of Compound 60)

The optically resolved (Ss)-(2E)-3-[4'-(2-butoxyethoxy)-4-[3-(methoxycarbonyl)pyrrolidin-1-yl]-1,1'-biphenyl-3-yl]-2-methyl-N-[4-[[(1-propyl-1H-imidazol-5-yl)methyl]sulfinyl]phenyl]acrylamide (115 mg) (the latter fraction: diastereomer 2 (Compound 58)) in Example 56 was dissolved in tetrahydrofuran (4 ml) and methanol (4 ml), and a 1 N aqueous sodium hydroxide solution (0.47 ml) was added thereto. The mixture was stirred for 7 hours under a nitrogen atmosphere and light shielding. After adding 1 N hydrochloric acid (0.75 ml), water and saturated brine were added thereto, and the mixture was extracted with ethyl acetate. The organic layer was dried over magnesium sulfate, and then the solvent was distilled off under reduced pressure. The resulting residue was recrystallized from methanol-acetone to give (Ss)-(2E)-3-[4'-(2-butoxyethoxy)-4-(3-carboxypyrrolidin-1-yl)-1,1'-biphenyl-3-yl]-2-methyl-N-[4-[[(1-propyl-1H-imidazol-5-yl)methyl]sulfinyl]phenyl]acrylamide (diastereomer 2 (Compound 60)) (26.9 mg, 99.8%de) as colorless crystals.

$[\alpha]_D$ = +76.6° (C = 0.208%, in chloroform)

Example 59 (Preparation of Compound 61)

(S)-4-[[(1-propyl-1H-imidazol-5-yl)methyl]sulfinyl]aniline di-p-toluoyl-D-tartrate monohydrate (1.20 g) was dissolved in ethyl acetate (10 ml) and 1 N hydrochloric acid (6.09 ml), followed by separation. To the aqueous layer was added an aqueous 25% potassium carbonate solution (6.09 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added toluene, and then the solvent was again distilled off under reduced pressure to give (S)-4-[[(1-propyl-1H-imidazol-5-yl)methyl]sulfinyl]aniline.

To a solution of (2E)-3-[4'-(2-butoxyethoxy)-4-[3-(2-ethoxy-2-oxoethyl)pyrrolidin-1-yl]-1,1'-biphenyl-3-yl]-2-methylacrylic acid (700 mg) in tetrahydrofuran (10 ml) was added a drop of DMF, and then oxalyl chloride (0.156 ml) at 0°C. The mixture was returned to room temperature and stirred for 30 minutes under a nitrogen atmosphere. The solution was then added dropwise to a solution of (S)-4-[[(1-propyl-1H-imidazol-5-yl)methyl]sulfinyl]aniline and pyridine (2.89 ml) in tetrahydrofuran (10 ml) at 0°C under a nitrogen atmosphere. The mixture was returned to room temperature and stirred overnight. Then, water was added thereto and the mixture was extracted with ethyl acetate. The organic layer was washed with water twice and saturated brine once, and then dried over magnesium sulfate. The solvent was distilled off under reduced pressure, and then the resulting residue was separated and purified by basic silica gel column chromatography (ethyl acetate → methanol : ethyl acetate = 3 : 100) to give (Ss)-(2E)-3-[4'-(2-butoxyethoxy)-4-[3-(2-ethoxy-2-oxoethyl)pyrrolidin-1-yl]-1,1'-biphenyl-3-yl]-2-methyl-N-[4-[[(1-propyl-1H-imidazol-5-yl)methyl]sulfinyl]phenyl]acrylamide (710 mg) (Compound 61) as a yellow amorphous material.

$^1$H-NMR (300 MHz, $CDCl_3$) δ 0.89-0.96 (6H, m), 1.25 (3H, t, J=7.2 Hz), 1.30-1.43 (2H, m), 1.55-1.80 (5H, m), 2.10-2.25 (4H, m), 2.47-2.49 (2H, m), 2.60-2.75 (1H, m), 3.08-3.13 (2H, m), 3.25-3.45 (3H, m), 3.55 (2H, t, J=6.9 Hz), 3.77-3.82 (4H, m), 4.02-4.17 (6H, m), 6.59 (1H, s), 6.89 (1H, d, J=8.1 Hz), 6.98 (2H, d, J=8.7 Hz), 7.34-7.47 (7H, m), 7.57 (1H, s), 7.80 (2H, d, J=8.7 Hz), 7.95 (1H, s).

Elementary analysis $C_{43}H_{54}N_4O_6S \cdot 0.5H_2O$, Calcd. C, 67.60 ; H, 7.26 ; N, 7.33 ; Found. C, 67.41 ; H, 7.26 ; N, 7.25.

$[\alpha]_D$ = -108.3° (C = 0.492%, in ethanol)

Example 60 (Preparation of Compound 62)

To a solution of (Ss)-(2E)-3-[4'-(2-butoxyethoxy)-4-[3-(2-ethoxy-2-oxoethyl)pyrrolidin-1-yl]-1,1'-biphenyl-3-yl]-2-methyl-N-[4-[[(1-propyl-1H-imidazol-5-yl)methyl]sulfinyl]phenyl]acrylamide (360 mg) in tetrahydrofuran (10 ml) and methanol (10 ml) was added a 1 N aqueous sodium hydroxide solution (1.43 ml), and the mixture was stirred for 1 day at room temperature. To the reaction solution were added water, 1 N hydrochloric acid (2.0 ml) and saturated brine, and the mixture was then extracted with ethyl acetate. The organic layer was washed with saturated brine and dried over magnesium sulfate. The solvent was distilled off under reduced pressure, and then the resulting residue was separated and purified by preparative HPLC to give (Ss)-(2E)-3-[4'-(2-butoxyethoxy)-4-[3-(carboxymethyl)pyrrolidin-1-yl]-1,1'-biphenyl-3-yl]-2-methyl-N-[4-[[(1-propyl-1H-imidazol-5-yl)methyl]sulfinyl]phenyl]acrylamide (182 mg) (Compound 62) as a yellow amorphous material.

Elementary analysis $C_{41}H_{50}N_4O_6S \cdot 0.5H_2O$, Calcd. C, 66.91 ; H, 6.98 ; N, 7.61 ; Found. C, 66.71 ; H, 6.99 ; N, 7.47.

$[\alpha]_D$ = -118.5° (C = 0.501%, in ethanol)

Reference Example 1

A mixture of 5-bromo-2-fluorobenzaldehyde (5.0 g, 24.6 mmol), hexahydro-1H-azepine (3.33 ml, 29.5 mmol) and potassium carbonate (5.1 g, 36.9 mmol) in DMF (50 ml) was stirred at 80°C for 20 hours. To the reaction system was added water, and the mixture was extracted with ethyl acetate. The organic layer was washed with water and saturated brine, and dried over magnesium sulfate. After concentration under reduced pressure, the residue was separated and purified by column chromatography (ethyl acetate : hexane = 1 : 19) to give 2-azepan-1-yl-5-bromobenzaldehyde (5.2 g) as a yellow oily material.

$^1$H-NMR (200 MHz, $CDCl_3$) δ 1.51-1.86 (8H, m), 3.56-3.41 (4H, m), 6.96 (1H, d, J=9.0 Hz), 7.46 (1H, dd, J=9.0, 2.6 Hz), 7.82 (1H, d, J=2.6 Hz), 10.10 (1H, s).

IR (neat) 1680, 1588, 1481, 1404, 1267, 1175 $cm^{-1}$

Reference Example 2

To a solution of 2-azepan-1-yl-5-bromobenzaldehyde (1.0 g) and ethyl acetate (0.42 ml) in dimethyl carbonate (10 ml) was added sodium methoxide (28% solution in methanol, 2.2 g) at room temperature, and the mixture was stirred at 50°C for 20 hours. 1 N Hydrochloric acid was added to the reaction system until the pH reached 3 to 4, and the resulting mixture was extracted with ethyl acetate. The organic layer was washed with saturated brine, and dried over magnesium sulfate. After concentration under reduced pressure, the residue was separated and purified by column chromatography (ethyl acetate : hexane = 1 : 19) to give ethyl (2E)-3-(2-azepan-1-yl-5-bromophenyl)acrylate (0.96 g) as a yellow oily material.

$^1$H-NMR (200 MHz, $CDCl_3$) δ 1.34 (3H, t, J=7.1 Hz), 1.63-1.86 (8H, m), 3.05-3.19 (4H, m), 4.26 (2H, q, J=7.1 Hz), 6.30 (1H, d, J=16.2 Hz), 6.95 (1H, d, J=8.8 Hz), 7.35 (1H, dd, J=8.8, 2.6 Hz), 7.58 (1H, d, J=2.6 Hz), 7.99 (1H, d, J=16.2 Hz).

IR (neat) 1713, 1630, 1480, 1260, 1177, 912, 743 $cm^{-1}$

Reference Example 3

Under an argon atmosphere, a mixture of ethyl (2E)-3-(2-azepan-1-yl-5-bromophenyl)acrylate (0.96 g), 4-(2-butoxyethoxy)phenylboric acid (0.78 g) and potassium carbonate (0.75 g) in toluene (30 ml), ethanol (3 ml) and water (3 ml) was stirred for 1 hour at room temperature. Tetrakis(triphenylphosphine)palladium (0.16 g) was added to the reaction system, and the mixture was heated under reflux for 6 hours. After cooling to room temperature, the resulting mixture was extracted with ethyl acetate. The organic layer was washed with saturated brine, and dried over magnesium sulfate. After concentration under reduced pressure, the residue was separated and purified by column chromatography (ethyl acetate : hexane = 1 : 19 → 1 : 15) to give ethyl (2E)-3-[4-azepan-1-yl-4'-(2-butoxyethoxy)-1,1'-biphenyl-3-yl]acrylate (842 mg) as a yellow oily material.

$^1$H-NMR (200 MHz, $CDCl_3$) δ 0.93 (3H, t, J=7.3 Hz), 1.28-1.48 (5H, m), 1.53-1.67 (2H, m), 1.69-1.90 (8H, m), 3.12-3.26 (4H, m), 3.55 (2H, t, J=6.6 Hz), 3.81 (2H, t, J=4.8 Hz), 4.16 (2H, t, J=4.8 Hz), 4.28 (2H, q, J=6.9 Hz), 6.40 (1H , d, J=16.1 Hz), 6.98 (2H, d, J=8.8 Hz), 7.13 (1H , d, J=8.8 Hz), 7.44-7.51 (3H, m), 7.67 (1H, d, J=2.2 Hz), 8.51 (1H, d, J=16.1 Hz).

IR (neat) 1709, 1630, 1607, 1487, 1453, 1302, 1246, 1175, 1125, 820 $cm^{-1}$

Reference Example 4

To a solution of ethyl (2E)-3-[4-azepan-1-yl-4'-(2-butoxyethoxy)-1,1'-biphenyl-3-yl]acrylate (842 mg, 1.81 mmol) in THF (5 ml) and ethanol (10 ml) was added a 1 N aqueous sodium hydroxide solution (4.0 ml, 4.0 mmol) at room temperature, and the mixture was stirred at 60°C for 24 hours. After cooling to room temperature, 1 N hydrochloric acid (4.0 ml) was added thereto, and the resulting mixture was extracted with ethyl acetate. The organic layer was washed with saturated brine, and dried over magnesium sulfate. After concentration under reduced pressure, the precipitated crystals were collected by filtration. The crystals were washed with diisopropyl ether and hexane to give (2E)-3-[4-azepan-1-yl-4'-(2-butoxyethoxy)-1,1'-biphenyl-3-yl]acrylic acid (551 mg) as yellow crystals.

m.p. 135-138°C

$^1$H-NMR (300 MHz, $CDCl_3$) δ 0.93 (3H, t, J=7.2 Hz), 1.34-1.46 (2H, m), 1.57-1.66 (2H, m), 1.70-1.86 (8H, m), 3.21-3.25 (4H, m), 3.56 (2H, t, J=6.6 Hz), 3.81 (2H, t, J=5.0 Hz), 4.17 (2H, t, J=5.0 Hz), 6.41 (1H , d, J=15.9 Hz), 6.99 (2H, d, J=9.0 Hz), 7.15 (1H, d, J=8.7 Hz), 7.47-7.52 (3H, m), 7.68 (1H, d, J=2.4 Hz), 8.24 (1H, d, J=15.9 Hz).

IR (KBr) 1692, 1618, 1605, 1487, 1327, 1304, 1279, 1246, 1117, 816 $cm^{-1}$

Elementary analysis $C_{27}H_{35}NO_4$, Calcd. C, 74.11 ; H, 8.06 ; N, 3.20 : Found. C, 74.18 ; H, 8.07 ; N, 2.98.

Reference Example 5

To a solution of 2-azepan-1-yl-5-bromobenzaldehyde (2.0 g) and methyl propionate (0.75 ml) in dimethyl carbonate (20 ml) was added sodium methoxide (28% solution in methanol, 2.0 g), and the mixture was stirred at 60°C for 64 hours. After neutralization with 1 N hydrochloric acid, the resulting mixture was extracted with ethyl acetate. The organic layer was washed with saturated brine, and dried over magnesium sulfate. After concentration under reduced pressure, the residue was separated and purified by column chromatography (ethyl acetate : hexane = 1 : 99) to give methyl (2E)-3-(2-azepan-1-yl-5-bromophenyl)-2-methylacrylate (1.306 g) as a yellow oily material.

$^1$H-NMR (300 MHz, $CDCl_3$) δ 1.63-1.78 (8H, m), 2.06 (3H, d, J=1.5 Hz), 3.12-3.15 (4H, m), 3.82 (3H, s), 6.91 (1H, d, J=9.3 Hz), 7.29-7.33 (2H, m), 7.69 (1H, s).

IR (neat) 1713, 1481, 1449, 1275, 1248, 1192, 1119, 909, 737 $cm^{-1}$

Reference Example 6

Under an argon atmosphere, a mixture of methyl (2E)-3-(2-azepan-1-yl-5-bromophenyl)-2-methylacrylate (1.3 g), 4-(2-butoxyethoxy)phenylboric acid (1.05 g) and potassium carbonate (1.02 g) in toluene (40 ml), ethanol (4 ml) and water (4 ml) was stirred for 1 hour at room temperature. Tetrakis(triphenylphosphine)palladium (0.20 g) was added to the reaction system, and the mixture was heated under reflux for 5 hours. After cooling to room temperature, water was added thereto, and the resulting mixture was extracted with ethyl acetate. The organic layer was washed with saturated brine, and dried over magnesium sulfate. After concentration under reduced pressure, the residue was separated and purified by column chromatography (ethyl acetate : hexane = 1 : 19) to give methyl (2E)-3-[4-azepan-1-yl-4'-(2-butoxyethoxy)-1,1'-biphenyl-3-yl]-2-methylacrylate (1.38 g) as yellow crystals.

m.p. 86-89°C

$^1$H-NMR (300 MHz, $CDCl_3$) δ 0.93 (3H, t, J=7.5 Hz), 1.34-1.46 (2H, m), 1.55-1.66 (2H, m), 1.67-1.82 (8H, m),

2.13 (3H, d, J=1.8 Hz), 3.18-3.22 (4H, m), 3.55 (2H, t, J=6.6 Hz), 3.80 (2H, t, J=5.0 Hz), 3.83 (3H, s), 4.15 (2H, t, J=5.0 Hz), 6.98 (2H, d, J=8.7 Hz), 7.09 (1H, d, J=9.3 Hz), 7.41-7.48 (4H, m), 7.85 (1H, s).

IR (KBr) 1711, 1605, 1487, 1273, 1246, 1119, 909, 820, 737 $cm^{-1}$

Elementary analysis $C_{29}H_{39}NO_4$, Calcd. C, 74.81 ; H, 8.44 ; N, 3.01 : Found. C, 74.83 ; H, 8.38 ; N, 2.88.

Reference Example 7

To a solution of methyl (2E)-3-[4-azepan-1-yl-4'-(2-butoxyethoxy)-1,1'-biphenyl-3-yl]-2-methylacrylate (1.38 g) in THF (5 ml) and ethanol (10 ml) was added a 1 N aqueous sodium hydroxide solution (5.0 ml) at room temperature, and the mixture was stirred at 60°C for 5 days. 1 N Hydrochloric acid (5.0 ml) was added thereto at 0°C, and the resulting mixture was extracted with ethyl acetate. The organic layer was washed with saturated brine, and dried over magnesium sulfate. After concentration under reduced pressure, the precipitated crystals were collected by filtration. The crystals were washed with diisopropyl ether to give (2E)-3-[4-azepan-1-yl-4'-(2-butoxyethoxy)-1,1'-biphenyl-3-yl] 2-methylacrylic acid (877.9 mg) as yellow crystals.

m.p. 124-126°C

$^1$H-NMR (300 MHz, $CDCl_3$) $\delta$ 0.93 (3H, t, J=7.2 Hz), 1.32-1.45 (2H, m), 1.56-1.66 (2H, m), 1.67-1.84 (8H, m), 2.16 (3H, d, J=1.5 Hz), 3.20-3.23 (4H, m), 3.55 (2H, t, J=6.8 Hz), 3.81 (2H, t, J=5.0 Hz), 4.10 (2H, t, J=5.0 Hz), 6.98 (2H, d, J=8.7 Hz), 7.11 (1H, d, J=9.0 Hz), 7.42-7.48 (4H, m), 7.99 (1H, s).

IR (KBr) 1663, 1605, 1590, 1495, 1316, 1246, 1182, 1117, 1046, 831 $cm^{-1}$

Elementary analysis $C_{28}H_{37}NO_4$, Calcd. C, 74.47 ; H, 8.26 ; N, 3.10 : Found. C, 74.30 ; H, 8.19 ; N, 2.93.

Reference Example 8

A mixture of 5-bromo-2-fluorobenzaldehyde (2.5 g), hexamethyleneimine (1.7 ml) and potassium carbonate (2.5 g) in DMF (25 ml) was stirred at 80°C for 16 hours. To the reaction system was added water, and the mixture was extracted with ethyl acetate. The organic layer was washed with water and saturated brine, and dried over magnesium sulfate. After concentration under reduced pressure, the residue was separated and purified by column chromatography (ethyl acetate : hexane = 1 : 19) to give 2-azocan-1-yl-5-bromobenzaldehyde (2.91 g) as a yellow oily material.

$^1$H-NMR (300 MHz, $CDCl_3$) $\delta$ 1.55-1.80 (10H, m), 3.39-3.42 (4H, m), 7.00 (1H, d, J=9.0 Hz), 7.47 (1H, dd, J=9.0, 2.6 Hz), 7.82 (1H, d, J=2.6 Hz), 10.17 (1H, s).

Reference Example 9

To a solution of 2-azocan-1-yl-5-bromobenzaldehyde (2.9 g) and ethyl acetate (1.24 ml) in diethyl carbonate (30 ml) was added sodium ethoxide (20% solution in ethanol, 5.0 g), and the mixture was stirred at 50°C for 18 hours. After neutralization with 1 N hydrochloric acid, the resulting mixture was extracted with ethyl acetate. The organic layer was washed with saturated brine, and dried over magnesium sulfate. After concentration under reduced pressure, the residue was separated and purified by column chromatography (ethyl acetate : hexane = 1 : 19) to give ethyl (2E)-3-(2-azocan-1-yl-5-bromophenyl)acrylate (2.77 g) as a yellow oily material.

$^1$H-NMR (200 MHz, $CDCl_3$) $\delta$ 1.34 (3H, t, J=7.1 Hz), 1.61-1.80 (10H, m), 3.06-3.21 (4H, m), 4.26 (2H, q, J=7.1 Hz), 6.28 (1H, d, J=15.9 Hz), 7.02 (1H, d, J=8.6 Hz), 7.37 (1H, dd, J=8.6, 2.5 Hz), 7.59 (1H, d, J=2.5 Hz), 8.11 (1H, d, J=15.9 Hz).

IR (neat) 1713, 1480, 1312, 1264, 1177, 909, 737 $cm^{-1}$

Reference Example 10

Under an argon atmosphere, a mixture of ethyl (2E)-3-(2-azocan-1-yl-5-bromophenyl)acrylate (2.77 g), 4-(2-butoxyethoxy)phenylboric acid (2.16 g) and potassium carbonate (2.09 g) in toluene (80 ml), ethanol (8 ml) and water (8 ml) was stirred for 1 hour at room temperature. Tetrakis(triphenylphosphine)palladium (0.44 g) was added to the reaction system, and the mixture was heated under reflux for 6 hours. After cooling to room temperature, water was added thereto and the mixture was extracted with ethyl acetate. The organic layer was washed with saturated brine, and dried over magnesium sulfate. After concentration under reduced pressure, the residue was separated and purified by column chromatography (ethyl acetate : hexane = 1 : 19) to give ethyl (2E)-3-[4-azocan-1-yl-4'-(2-butoxyethoxy)-1,1'-biphenyl-3-yl]acrylate (2.97 g) as a yellow oily material.

$^1$H-NMR (200 MHz, $CDCl_3$) $\delta$ 0.93 (3H, t, J=7.3 Hz), 1.25-1.48 (5H, m), 1.53-1.84 (12H, m), 3.12-3.27 (4H, m), 3.55 (2H, t, J=6.6 Hz), 3.81 (2H, t, J=4.9 Hz), 4.16 (2H, t, J=4.9 Hz), 4.27 (2H, q, J=7.2 Hz), 6.38 (1H, d, J=16.3 Hz), 6.98 (2H, d, J=8.8 Hz), 7.21 (1H, d, J=8.8 Hz), 7.44-7.52 (3H, m), 7.68 (1H, d, J=2.6 Hz), 8.27 (1H, d, J=16.3 Hz).

IR (neat) 1709, 1630, 1609, 1487, 1453, 1366, 1248, 1175, 1128, 1044, 910, 826, 737 $cm^{-1}$

Reference Example 11

To a solution of ethyl (2E)-3-[4-azocan-1-yl-4'-(2-butoxyethoxy)-1,1'-biphenyl-3-yl]acrylate (2.97 g) in THF (30 ml) and ethanol (60 ml) was added a 1 N aqueous sodium hydroxide solution (12.0 ml) at room temperature, and the mixture was stirred at 60°C for 3 days. 1 N Hydrochloric acid (12.0 ml) was added thereto at 0°C, and the resulting mixture was extracted with ethyl acetate. The organic layer was washed with saturated brine, and dried over magnesium sulfate. After concentration under reduced pressure, the residue was separated and purified by column chromatography (ethyl acetate : hexane = 1 : 9 → 1 : 8 → 1 : 7 → 1 : 5) to give (2E)-3-[4-azocan-1-yl-4'-(2-butoxyethoxy)-1,1'-biphenyl-3-yl]acrylic acid (1.48 g) as yellow crystals.

$^1$H-NMR (300 MHz, $CDCl_3$) δ 0.94 (3H, t, J=7.2 Hz), 1.34-1.46 (2H, m), 1.57-1.66 (2H, m), 1.67-1.85 (10H, m), 3.17-3.27 (4H, m), 3.56 (2H, t, J=6.6 Hz), 3.81 (2H, t, J=5.0 Hz), 4.17 (2H, t, J=5.0 Hz), 6.39 (1H, d, J=16.0 Hz), 7.00 (2H, d, J=9.0 Hz), 7.21 (1H, d, J=8.7 Hz), 7.46-7.53 (3H, m), 7.69 (1H, d, J=2.1 Hz), 8.35 (1H, d, J=16.0 Hz).

IR (KBr) 1682, 1620, 1607, 1487, 1451, 1418, 1271, 1246, 1208, 1127, 1067, 831, 814 $cm^{-1}$

Elementary analysis $C_{28}H_{37}NO_4$, Calcd. C, 74.47 ; H, 8.26 ; N, 3.10 : Found. C, 74.47 ; H, 8.28 ; N, 2.93.

Reference Example 12

A mixture of 5-bromo-2-fluorobenzaldehyde (2.5 g), diisobutylamine (2.8 ml) and sodium carbonate (2.0 g) in DMSO (25 ml) and water (10 ml) was heated under reflux for 13 hours. After cooling to room temperature, the resulting mixture was extracted with ethyl acetate. The organic layer was washed with water and saturated brine, and dried over magnesium sulfate. After concentration under reduced pressure, the residue was separated and purified by column chromatography (ethyl acetate : hexane = 1 : 49) to give 5-bromo-2-(diisobutylamino)benzaldehyde (1.93 g) as a yellow oily material.

$^1$H-NMR (300 MHz, $CDCl_3$) δ 0.84 (12H, d, J=6.6 Hz), 1.82-1.98 (2H, m), 3.10 (4H, d, J=7.5 Hz), 7.02 (1H, d, J=8.7 Hz), 7.50 (1H, dd, J=8.7, 2.7 Hz), 7.85 (1H, d, J=2.7 Hz), 10.19 (1H, s).

IR (neat) 1684, 1586, 1480, 1468, 1389, 1254, 1177, 1152, 1113 $cm^{-1}$

Reference Example 13

To a suspension of sodium hydride (60%, 0.30 g) in toluene (30 ml) was added dropwise a solution of ethyl diethylphosphonoacetate (1.66 g) in toluene (10 ml) at 0°C under a nitrogen atmosphere. After stirring at 0°C for 30 minutes, a solution of aldehyde (1.93 g) in toluene (20 ml) was added dropwise thereto, and the mixture was heated under reflux for 2 hours. Then, water was added thereto, and the mixture was extracted with ethyl acetate. The organic layer was washed with saturated brine, and dried over magnesium sulfate. After concentration under reduced pressure, the residue was separated and purified by column °chromatography (ethyl acetate : hexane = 1 : 49) to give ethyl (2E)-3-[4'-(2-buthoxyethoxy)-4-(diisobutylamino)-1,1'-biphenyl-3-yl]acrylate (2.21 g) as a yellow oily material.

$^1$H-NMR (300 MHz, $CDCl_3$) δ 0.86 (12H, d, J=6.6 Hz), 1.33 (3H, t, J=7.2 Hz), 1.72-1.86 (2H, m), 2.75 (4H, d, J=7.2 Hz), 4.26 (2H, q, J=7.2 Hz), 6.32 (1H, d, J=16.2 Hz), 7.01 (1H, d, J=8.7 Hz), 7.38 (1H, dd, J=8.7, 2.4 Hz), 7.63 (1H, d, J=2.4 Hz), 8.08 (1H, d, J=16.2 Hz).

IR (neat) 1715, 1632, 1480, 1391, 1368, 1312, 1279, 1175, 909, 739 $cm^{-1}$

Reference Example 14

Under an argon atmosphere, a mixture of ethyl (2E)-3-[5-bromo-2-(diisobutylamino)phenyl]acrylate (2.21 g), 4-(2-butoxyethoxy)phenylboric acid (1.65 g) and potassium carbonate (1.60 g) in toluene (60 ml), ethanol (6 ml) and water (6 ml) was stirred for 1 hour at room temperature. Tetrakis(triphenylphosphine)palladium (0.33 g) was added to the reaction system, and the mixture was heated under reflux for 6 hours. After cooling to room temperature, water was added thereto and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 49 → 1 : 29 → 1 : 19) to give ethyl (2E)-3-[4'-(2-butoxyethoxy)-4-(diisobutylamino)-1,1'-biphenyl-3-yl]acrylate (2.12 g) as a yellow oily material.

$^1$H-NMR (300 MHz, $CDCl_3$) δ 0.90 (12H, d, J=6.6 Hz), 0.94 (3H, t, J=7.5 Hz), 1.32-1.45 (5H, m), 1.52-1.66 (2H, m), 1.78-1.92 (2H, m), 2.79 (4H, d, J=7.2 Hz), 3.56 (2H, t, J=6.8 Hz), 3.81 (2H, t, J=4.9 Hz), 4.17 (2H, t, J=4.9 Hz), 4.27 (2H, q, J=7.2 Hz), 6.41 (1H, d, J=16.2 Hz), 6.99 (2H, d, J=8.7 Hz), 7.19 (1H, d, J=8.7 Hz), 7.48-7.51 (3H, m), 7.71 (1H, d, J=2.4 Hz), 8.26 (1H, d, J=16.2 Hz).

IR (neat) 1711, 1630, 1487, 1466, 1277, 1248, 1177, 1127, 910, 737 $cm^{-1}$

Reference Example 15

To a solution of ethyl (2E)-3-[4'-(2-butoxyethoxy)-4-(diisobutylamino)-1,1'-biphenyl-3-yl]acrylate (2.12 g) in THF (10 ml) and ethanol (20 ml) was added a 1 N aqueous sodium hydroxide solution (10.0 ml) at room temperature, and the mixture was stirred at 60°C for 20 hours. 1 N Hydrochloric acid (10.0 ml) was added thereto at 0°C, and the resulting mixture was extracted with ethyl acetate. The organic layer was washed with saturated brine, and dried over magnesium sulfate. After concentration under reduced pressure, the precipitated crystals were collected by filtration. The crystals were washed with hexane to give (2E)-3-[4'-(2-butoxyethoxy)-4-(diisobutylamino)-1,1'-biphenyl-3-yl]acrylic acid (1.90 g) as yellow crystals.

$^1$H-NMR (200 MHz, $CDCl_3$) δ 0.89 (12H, d, J=6.6 Hz), 0.93 (3H, t, J=7.4 Hz), 1.30-1.48 (2H, m), 1.55-1.67 (2H, m), 1.75-1.96 (2H, m), 2.84 (4H, d, J=7.4 Hz), 3.56 (2H, t, J=6.6 Hz), 3.81 (2H, t, J=5.0 Hz), 4.17 (2H, t, J=5.0 Hz), 6.42 (1H, d, J=16.1 Hz), 6.99 (2H, d, J=8.8 Hz), 7.19 (1H, d, J=8.6 Hz), 7.47-7.54 (3H, m), 7.72 (1H, d, J=2.2 Hz), 8.32 (1H, d, J=16.1 Hz).

IR (KBr) 1707, 1674, 1624, 1485, 1275, 1244, 1128, 995, 814 $cm^{-1}$

Elementary analysis $C_{29}H_{41}NO_4$, Calcd. C, 74.48 ; H, 8.84 ; N, 3.00 : Found. C, 74.36 ; H, 8.84 ; N, 2.92.

Reference Example 16

A mixture of 5-bromo-2-fluorobenzaldehyde (2.5 g), isobutylpropylamine hydrochloride (2.80 g) and sodium carbonate (3.91 g) in DMSO (25 ml) and water (10 ml) was heated under reflux for 24 hours. After cooling to room temperature, the resulting mixture was extracted with ethyl acetate. The organic layer was washed with water and saturated brine, and dried over magnesium sulfate. After concentration under reduced pressure, the residue was separated and purified by column chromatography (ethyl acetate : hexane = 1 : 49) to give 5-bromo-2-[isobutyl(propyl)amino]benzaldehyde (2.18 g) as a yellow oily material.

To a suspension of sodium hydride (60%, 0.48 g) in toluene (10 ml) was added dropwise a solution of ethyl diethylphosphonoacetate (2.4 ml) in toluene (10 ml) at 0°C under a nitrogen atmosphere. After stirring at 0°C for 30 minutes, a solution of 5-bromo-2-[isobutyl(propyl)amino]benzaldehyde (2.18 g) in toluene (20 ml) was added dropwise thereto, and the mixture was heated under reflux for 2 hours. Then, water was added thereto, and the mixture was extracted with ethyl acetate. The organic layer was washed with saturated brine, and dried over magnesium sulfate. After concentration under reduced pressure, the residue was separated and purified by column chromatography (ethyl acetate : hexane = 1 : 49) to give ethyl (2E)-3-[5-bromo-2-[isobutyl(propyl)amino]phenyl]acrylate (1.96 g) as a yellow oily material.

$^1$H-NMR (300 MHz, $CDCl_3$) δ 0.81 (3H, t, J=7.5 Hz), 0.87 (6H, d, J=6.6 Hz), 1.34 (3H, t, J=7.1 Hz), 1.41-1.54 (2H, m), 1.68-1.81 (1H, m), 2.79 (2H, d, J=7.1 Hz), 2.85-2.90 (2H, m), 4.26 (2H, q, J=7.1 Hz), 6.33 (1H, d, J=16.2 Hz), 6.97 (1H, d, J=8.7 Hz), 7.38 (1H, dd, J=8.7, 2.4 Hz), 7.64 (1H, d, J=2.4 Hz), 8.04 (1H, d, J=16.2 Hz).

IR (neat) 1717, 1480, 1312, 1275, 1179, 1111, 1034, 909, 739 $cm^{-1}$

Reference Example 17

Under an argon atmosphere, a mixture of ethyl (2E)-3-[5-bromo-2-[isobutyl(propyl)amino]phenyl]acrylate (1.96 g), 4-(2-butoxyethoxy)phenylboric acid (1.51 g) and potassium carbonate (1.47 g) in toluene (50 ml), ethanol (5 ml) and water (5 ml) was stirred for 1 hour at room temperature. Tetrakis(triphenylphosphine)palladium (0.30 g) was added to the reaction system, and the mixture was heated under reflux for 6 hours. After cooling to room temperature, water was added thereto and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 29 → 1 : 19) to give ethyl (2E)-3-[4'-(2-butoxyethoxy)-4-[isobutyl(propyl)amino]-1,1'-biphenyl-3-yl]acrylate (1.56 g) as a yellow oily material.

$^1$H-NMR (300 MHz, $CDCl_3$) δ 0.83 (3H, t, J=7.4 Hz), 0.91 (6H, d, J=6.6 Hz), 0.93 (3H, t, J=7.4 Hz), 1.32-1.66 (9H, m), 1.74-1.85 (1H, m), 2.81-2.96 (4H, m), 3.56 (2H, t, J=6.6 Hz), 3.81 (2H, t, J=5.0 Hz), 4.17 (2H, t, J=5.0 Hz), 4.27 (2H, q, J=7.1 Hz), 6.43 (1H, d, J=16.4 Hz), 6.99 (2H, d, J=8.7 Hz), 7.16 (1H, d, J=8.7 Hz), 7.47-7.50 (3H, m), 7.71 (1H, d, J=2.4 Hz), 8.20 (1H, d, J=16.4 Hz).

IR (neat) 1711, 1487, 1279, 1248, 1175, 909, 737 $cm^{-1}$

Reference Example 18

To a solution of ethyl (2E)-3-[4'-(2-butoxyethoxy)-4-[isobutyl(propyl)amino]-1,1'-biphenyl-3-yl]acrylate (1.56 g) in THF (10 ml) and ethanol (20 ml) was added a 1 N aqueous sodium hydroxide solution (6.5 ml) at room temperature, and the mixture was stirred at 60°C for 20 hours. 1 N Hydrochloric acid (6.5 ml) was added thereto at 0°C, and the

resulting mixture was extracted with ethyl acetate. The organic layer was washed with saturated brine, and dried over magnesium sulfate. After concentration under reduced pressure, the residue was separated and purified by column chromatography (ethyl acetate : hexane 1 : 9 → 1 : 3 → 1 : 2) to give (2E)-3-[4'-(2-butoxyethoxy)-4-[isobutyl(propyl) amino]-1,1'-biphenyl-3-yl]acrylic acid (1.03 g) as yellow crystals.

$^1$H-NMR (300 MHz, $CDCl_3$) δ 0.83 (3H, t, J=7.4 Hz), 0.91 (6H, d, J=6.6 Hz), 0.94 (3H, t, J=7.4 Hz), 1.33-1.65 (6H, m), 1.74-1.87 (1H, m), 2.87 (2H, d, J=7.5 Hz), 2.92-2.97 (2H, m), 3.56 (2H, t, J=6.8 Hz), 3.82 (2H, t, J=5.0 Hz), 4.17 (2H, t, J=5.0 Hz), 6.45 (1H, d, J=16.2 Hz), 7.00 (2H, d, J=9.0 Hz), 7.16 (1H, d, J=8.7 Hz), 7.48-7.53 (3H, m), 7.73 (1H, d, J=2.4 Hz), 8.29 (1H, d, J=16.2 Hz).

IR (KBr) 1707, 1688, 1626, 1485, 1279, 1248, 1127, 1071, 995, 818 $cm^{-1}$

Elementary analysis $C_{28}H_{39}NO_4$, Calcd. C, 74.14 ; H, 8.67 ; N, 3.09 : Found. C, 73.90 ; H, 8.47 ; N, 3.08.

Reference Example 19

A mixture of 5-bromo-2-fluorobenzaldehyde (2.5 g), isobutylmethylamine (1.61 g) and sodium carbonate (2.60 g) in DMSO (40 ml) and water (25 ml) was heated under reflux for 20 hours. After cooling to room temperature, the resulting mixture was extracted with ethyl acetate. The organic layer was washed with water and saturated brine, and dried over magnesium sulfate. After concentration under reduced pressure, the residue was separated and purified by column chromatography (ethyl acetate : hexane = 1 : 29) to give 5-bromo-2-[isobutyl(methyl)amino]benzaldehyde (3.14 g) as a yellow oily material.

$^1$H-NMR (300 MHz, $CDCl_3$) δ 0.90 (6H, d, J=6.6 Hz), 1.89-2.04 (1H, m), 2.89 (3H, s), 2.93 (2H, d, J=7.2 Hz), 6.98 (1H, d, J=8.7 Hz), 7.52 (1H, dd, J=8.7, 2.6 Hz), 7.86 (1H, d, J=2.6 Hz), 10.19 (1H, s).

IR (neat) 1684, 1588, 1485, 1389, 1260, 1179, 1154, 1113, 912, 880, 741 $cm^{-1}$

Reference Example 20

To a suspension of sodium hydride (60%, 0.55 g) in toluene (50 ml) was added dropwise a solution of ethyl diethylphosphonoacetate (2.7 ml) in toluene (10 ml) at 0°C under a nitrogen atmosphere. After stirring at 0°C for 30 minutes, a solution of 5-bromo-2-[isobutyl(methyl)amino]benzaldehyde (3.14 g) in toluene (20 ml) was added dropwise thereto, and the mixture was heated under reflux for 2 hours. Then, water was added thereto, and the mixture was extracted with ethyl acetate. The organic layer was washed with saturated brine, and dried over magnesium sulfate. After concentration under reduced pressure, the residue was separated and purified by column chromatography (ethyl acetate : hexane = 1 : 29) to give ethyl (2E)-3-[5-bromo-2-[isobutyl(methyl)amino]phenyl]acrylate (3.72 g) as a yellow oily material.

$^1$H-NMR (300 MHz, $CDCl_3$) δ 0.92 (6H, d, J=6.6 Hz), 1.34 (3H, t, J=7.1 Hz), 1.81-1.96 (1H, m), 2.67 (3H, s), 2.69 (2H, d, J=7.2 Hz), 4.26 (2H, q, J=7.1 Hz), 6.35 (1H, d, J=16.2 Hz), 6.95 (1H, d, J=8.7 Hz), 7.39 (1H, dd, J=8.7, 2.4 Hz), 7.62 (1H, d, J=2.4 Hz), 8.00 (1H, d, J=16.2 Hz).

IR (neat) 1713, 1632, 1481, 1314, 1175, 912, 743 $cm^{-1}$

Reference Example 21

Under an argon atmosphere, a mixture of ethyl (2E)-3-[5-bromo-2-[isobutyl(methyl)amino]phenyl]acrylate (3.72 g), 4-(2-butoxyethoxy)phenylboric acid (3.11 g) and potassium carbonate (3.01 g) in toluene (100 ml), ethanol (10 ml) and water (10 ml) was stirred for 1 hour at room temperature. Tetrakis(triphenylphosphine)palladium (0.63 g) was added to the reaction system, and the mixture was heated under reflux for 6 hours. After cooling to room temperature, water was added thereto and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 29 → 1 : 19) to give ethyl (2E)-3-[4'-(2-butoxyethoxy)-4-[isobutyl(methyl)amino]-1,1'-biphenyl-3-yl]acrylate (3.29 g) as a yellow oily material.

$^1$H-NMR (300 MHz, $CDCl_3$) δ 0.93 (3H, t, J=7.4 Hz), 0.95 (6H, d, J=6.6 Hz), 1.32-1.44 (5H, m), 1.54-1.66 (2H, m), 1.85-2.01 (1H, m), 2.72 (3H, s), 2.74 (2H, d, J=7.2 Hz), 3.55 (2H, t, J=6.8 Hz), 3.80 (2H, t, J=5.0 Hz), 4.16 (2H, t, J=5.0 Hz), 4.27 (2H, q, J=7.1 Hz) 6.44 (1H, d, J=16.4 Hz), 6.98 (2H, d, J=9.0 Hz), 7.12 (1H, d, J=8.4 Hz), 7.44-7.51 (3H, m), 7.68 (1H, d, J=2.1 Hz), 8.15 (1H, d, J=16.4 Hz).

IR (neat) 1711, 1489, 1300, 1246, 1177, 1127, 912, 823 $cm^{-1}$

Reference Example 22

To a solution of ethyl (2E)-3-[4'-(2-butoxyethoxy)-4-[isobutyl(methyl)amino]-1,1'-biphenyl-3-yl]acrylate (3.49 g) in THF (10 ml) and ethanol (20 ml) was added a 1 N aqueous sodium hydroxide solution (15 ml) at room temperature,

and the mixture was stirred at 60°C for 2 days. 1 N Hydrochloric acid (15 ml) was added thereto at 0°C, and the resulting mixture was extracted with ethyl acetate. The organic layer was washed with saturated brine, and dried over magnesium sulfate. After concentration under reduced pressure, the precipitated crystals were collected by filtration. The crystals were washed with ethyl acetate and hexane to give (2E)-3-[4'-(2-butoxyethoxy)-4-[isobutyl(methyl)amino]-1,1'-biphenyl-3-yl]acrylic acid (2.61 g) as yellow crystals.

$^1$H-NMR (200 MHz, $CDCl_3$) δ 0.90-0.97 (9H, m), 1.28-1.47 (2H, m), 1.52-1.71 (2H, m), 1.82-2.05 (1H, m), 2.74 (3H, s), 2.78 (2H, d, J=7.4 Hz), 3.56 (2H, t, J=6.6 Hz), 3.81 (2H, t, J=5.0 Hz), 4.17 (2H, t, J=5.0 Hz), 6.47 (1H, d, J=16.1 Hz), 7.00 (2H, d, J=8.8 Hz) 7.14 (1H, d, J=8.6 Hz), 7.46-7.55 (3H, m), 7.71 (1H, d, J=2.2 Hz), 8.26 (1H, d, J=16.1 Hz).

IR (KBr) 1686, 1624, 1487, 1466, 1422, 1300, 1269, 1246, 1182, 1127, 1065, 974, 924, 826 $cm^{-1}$

Elementary analysis $C_{26}H_{35}NO_4$, Calcd. C, 73.38 ; H, 8.29 ; N, 3.29 : Found. C, 73.15 ; H, 8.35 ; N, 3.32.

Reference Example 23

To a suspension of sodium hydride (60%, 0.39 g) in toluene (10 ml) was added dropwise a solution of ethyl 2-(diethylphosphono)butyrate (2.47 g) in toluene (10 ml) at 0°C under a nitrogen atmosphere. After stirring at room temperature for 1 hour, a solution of 2-azepan-1-yl-5-bromobenzaldehyde (2.3 g) in toluene (20 ml) was added dropwise thereto. The reaction mixture was heated under reflux for 5 hours. Then, water was added thereto, and the mixture was extracted with ethyl acetate. The organic layer was washed with saturated brine, and dried over magnesium sulfate. After concentration under reduced pressure, the residue was separated and purified by column chromatography (ethyl acetate : hexane = 1 : 39) to give ethyl (2E)-3-(2-azepan-1-yl-5-bromophenyl)-2-ethylacrylate (2.95 g) as a yellow oily material.

$^1$H-NMR (200 MHz, $CDCl_3$) δ 1.14 (3H, t, J=7.5 Hz), 1.34 (3H, t, J=7.2 Hz), 1.62-1.82 (8H, m), 2.52 (2H, q, J=7.5 Hz), 3.04-3.18 (4H, m), 4.28 (2H, q, J=7.2 Hz), 6.90 (1H, d, J=9.2 Hz), 7.26-7.33 (2H, m), 7.65 (1H, s).

IR (neat) 1709, 1480, 1235, 1130, 912, 743 $cm^{-1}$

Reference Example 24

Under an argon atmosphere, a mixture of ethyl (2E)-3-(2-azepan-1-yl-5-bromophenyl)-2-ethylacrylate (2.95 g), 4-(2-butoxyethoxy)phenylboric acid (2.21 g) and potassium carbonate (2.14 g) in toluene (80 ml), ethanol (8 ml) and water (8 ml) was stirred for 1 hour at room temperature. Tetrakis(triphenylphosphine)palladium (0.45 g) was added to the reaction system, and the mixture was heated under reflux for 7 hours. After cooling to room temperature, water was added thereto and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 39 → 1 : 19 → 1 : 9) to give ethyl (2E)-3-[4-azepan-1-yl-4'-(2-butoxyethoxy)-1,1'-biphenyl-3-yl]-2-ethylacrylate (2.63 g) as pale yellow crystals.

$^1$H-NMR (200 MHz, $CDCl_3$) δ 0.93 (3H, t, J=7.1 Hz), 1.19 (3H, t, J=7.3 Hz), 1.30-1.46 (5H, m), 1.51-1.84 (10H, m), 2.61 (2H, q, J=7.3 Hz), 3.16-3.22 (4H, m), 3.55 (2H, t, J=6.6 Hz), 3.80 (2H, t, J=5.0 Hz), 4.16 (2H, t, J=5.0 Hz), 4.29 (2H, q, J=7.1 Hz), 6.98 (2H, d, J=8.8 Hz), 7.09 (1H, d, J=9.2 Hz), 7.40-7.48 (4H, m), 7.81 (1H, s).

IR (KBr) 1707, 1607, 1489, 1454, 1246, 1128, 818 $cm^{-1}$

Elementary analysis $C_{31}H_{43}NO_4$, Calcd. C, 75.42 ; H, 8.78 ; N, 2.84 : Found. C, 75.39 ; H, 8.61 ; N, 2.61.

Reference Example 25

To a solution of ethyl (2E)-3-[4-azepan-1-yl-4'-(2-butoxyethoxy)-1,1'-biphenyl-3-yl]-2-ethylacrylate (2.63 g) in THF (20 ml) and ethanol (40 ml) was added a 1 N aqueous sodium hydroxide solution (12 ml) at room temperature, and the mixture was stirred at 60°C for 20 hours. 1 N Hydrochloric acid (12 ml) was added thereto at 0°C, and the resulting mixture was extracted with ethyl acetate. The organic layer was washed with saturated brine, and dried over magnesium sulfate. After concentration under reduced pressure, the precipitated crystals were collected by filtration to give (2E)-3-[4-azepan-1-yl-4'-(2-butoxyethoxy)-1,1'-biphenyl-3-yl]-2-ethylacrylic acid (1.90 g) as yellow crystals.

$^1$H-NMR (200 MHz, $CDCl_3$) δ 0.93 (3H, t, J=7.1 Hz), 1.24 (3H, t, J=7.4 Hz), 1.31-1.48 (2H, m), 1.52-1.86 (10H, m), 2.63 (2H, q, J=7.4 Hz), 3.19-3.24 (4H, m), 3.55 (2H, t, J=6.6 Hz), 3.80 (2H, t, J=5.0 Hz), 4.16 (2H, t, J=5.0 Hz), 6.99 (2H, d, J=8.8 Hz), 7.10 (1H, d, J=9.2 Hz), 7.44-7.48 (4H, m), 7.96 (1H, s).

IR (KBr) 1672, 1603, 1487, 1472, 1453, 1296, 1244, 1123, 816 $cm^{-1}$

Elementary analysis $C_{29}H_{39}NO_4$, Calcd. C, 74.08 ; H, 8.47 ; N, 2.98 : Found. C, 73.98 ; H, 8.53 ; N, 2.73.

Reference Example 26 .

A mixture of 5-bromo-2-fluorobenzaldehyde (2.5 g), ethylisobutylamine hydrochloride (2.7 g) and sodium carbonate (4.16 g) in DMSO (25 ml) and water (10 ml) was heated under reflux for 5 hours. After cooling to room temperature, the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 49) to give 5-bromo-2-[ethyl(isobutyl)amino]benzaldehyde (1.90 g) as a yellow oily material. To a suspension of sodium hydride (60%, 0.35 g) in toluene (20 ml) was added dropwise a solution of ethyl diethylphosphonoacetate (1.6 ml) in toluene (5 ml) at 0°C under a nitrogen atmosphere. After stirring at 0°C for 30 minutes, a solution of 5-bromo-2-[ethyl(isobutyl)amino]benzaldehyde (1.90 g) in toluene (10 ml) was added dropwise thereto, and the mixture was heated under reflux for 2 hours. Then, water was added thereto, and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 49) to give ethyl (2E)-3-[5-bromo-2-[ethyl(isobutyl)amino]phenyl]acrylate (1.38 g) as a yellow oily material. A mixture of ethyl (2E)-3-[5-bromo-2-[ethyl(isobutyl)amino]phenyl]acrylate (1.38 g), 4-(2-butoxyethoxy)phenylboric acid (1.21 g) and potassium carbonate (1.08 g) in toluene (40 ml), ethanol (4 ml) and water (4 ml) was stirred for 1 hour at room temperature under an argon atmosphere. Tetrakis(triphenylphosphine) palladium (0.23 g) was added to the reaction system, and the mixture was heated under reflux for 4 hours. After cooling to room temperature, water was added thereto and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 29 → 1 : 19) to give ethyl (2E)-3-[4'-(2-butoxyethoxy)-4-[ethyl(isobutyl)amino]-1,1'-biphenyl-3-yl]acrylate (947 mg) as a yellow oily material.

$^1$H-NMR (200 MHz, $CDCl_3$) δ 0.90-1.07 (12H, m), 1.30-1.50 (5H, m), 1.52-1.67 (2H, m), 1.71-1.88 (1H, m), 2.85 (2H, d, J=7.2 Hz), 3.02 (2H, q, J=7.1 Hz), 3.56 (2H, t, J=6.4 Hz), 3.81 (2H, t, J=4.9 Hz), 4.17 (2H, t, J=4.9 Hz), 4.27 (2H, q, J=7.2 Hz), 6.44 (1H, d, J=16.0 Hz), 6.99 (2H, d, J=8.8 Hz), 7.14 (1H, d, J=8.8 Hz), 7.47-7.52 (3H, m), 7.72 (1H, d, J=2.2 Hz), 8.18 (1H, d, J=16.0 Hz).

IR (neat) 1711, 1488, 1277, 1248, 1175, 912, 743 $cm^{-1}$

Reference Example 27

To a solution of ethyl (2E)-3-[4'-(2-butoxyethoxy)-4-[ethyl(isobutyl)amino]-1,1'-biphenyl-3-yl]acrylate (947 mg) in THF (5 ml) and ethanol (10 ml) was added a 1 N aqueous sodium hydroxide solution (4.0 ml) at room temperature, and the mixture was stirred at 60°C for 20 hours. 1 N Hydrochloric acid (4.0 ml) was added thereto at 0°C, and the resulting mixture was extracted with ethyl acetate. The organic layer was washed with saturated brine, and dried over magnesium sulfate. After concentration under reduced pressure, the residue was separated and purified by column chromatography (ethyl acetate : hexane 1 : 2) to give (2E)-3-[4'-(2-butoxyethoxy)-4-[ethyl(isobutyl)amino]-1,1'-biphenyl-3-yl]acrylic acid (870.3 mg) as yellow crystals.

$^1$H-NMR (200 MHz, $CDCl_3$) δ 0.90-1.08 (12H, m), 1.30-1.47 (2H, m), 1.52-1.88 (3H, m), 2.87 (2H, d, J=7.4 Hz), 3.04 (2H, q, J=7.1 Hz), 3.56 (2H, t, J=6.6 Hz), 3.81 (2H, t, J=5.0 Hz), 4.17 (2H, t, J=5.0 Hz), 6.45 (1H, d, J=16.6 Hz), 7.00 (2H, d, J=8.8 Hz), 7.16 (1H, d, J=8.4 Hz), 7.47-7.55 (3H, m), 7.73 (1H, d, J=2.2 Hz), 8.28 (1H, d, J=16.6 Hz).

IR (KBr) 1684, 1628, 1603, 1485, 1279, 1248, 1127, 1071, 820 $cm^{-1}$

Elementary analysis $C_{27}H_{37}NO_4$, Calcd. C, 73.77 ; H, 7.48 ; N, 3.19 : Found. C, 73.51 ; H, 8.42 ; N, 2.91.

Reference Example 28

A mixture of 5-bromo-2-fluorobenzaldehyde (2.5 g), piperidine (1.46 ml) and potassium carbonate (2.70 g) in DMF (25 ml) was stirred at 80°C for 3 days. Water was added to the reaction system, and the resulting mixture was extracted with ethyl acetate. The organic layer was washed with water and saturated brine, and dried over magnesium sulfate. After concentration under reduced pressure, the residue was separated and purified by column chromatography (ethyl acetate : hexane = 1 : 49) to give 5-bromo-2-piperidin-1-ylbenzaldehyde (2.20 g) as a yellow oily material.

$^1$H-NMR (200 MHz, $CDCl_3$) δ 1.50-1.82 (6H, m), 3.00-3.05 (4H, m), 6.98 (1H, d, J=8.6 Hz), 7.57 (1H, dd, J=8.6, 2.6 Hz), 7.89 (1H, d, J=2.6 Hz), 10.20 (1H, s).

IR (neat) 1682, 1586, 1480, 1466, 1379, 1258, 1227, 1177, 912, 820, 747 $cm^{-1}$

Reference Example 29

To a suspension of sodium hydride (60%, 0.18 g) in toluene (10 ml) was added dropwise a solution of ethyl diethylphosphonoacetate (1.01 g) in toluene (5 ml) at 0°C under a nitrogen atmosphere. After stirring at 0°C for 30 minutes, a solution of 2-piperidin-5-bromobenzaldehyde (1.0 g) in toluene (20 ml) was added dropwise thereto. The reaction mixture was heated under reflux for 3 hours. Then, water was added thereto, and the mixture was extracted with ethyl acetate. The organic layer was washed with saturated brine, and dried over magnesium sulfate. After concentration under reduced pressure, the residue was separated and purified by column chromatography (ethyl acetate : hexane = 1 : 49) to give ethyl (2E)-3-(5-bromo-2-piperidin-1-ylphenyl)acrylate (1.22 g) as a yellow oily material.

$^1$H-NMR (200 MHz, $CDCl_3$) δ 1.31 (3H, t, J=7.2 Hz), 1.50-1.82 (6H, m), 2.84-2.89 (4H, m), 4.27 (2H, q, J=7.2 Hz), 6.37 (1H, d, J=16.1 Hz), 6.89 (1H, d, J=8.6 Hz), 7.40 (1H, dd, J=8.6, 2.2 Hz), 7.62 (1H, d, J=2.2 Hz), 7.95 (1H, d, J=16.1 Hz).

IR (neat) 1717, 1634, 1480, 1312, 1262, 1233, 1181, 1125, 1105, 1028, 912, 814, 743 $cm^{-1}$

Reference Example 30

Under an argon atmosphere, a mixture of ethyl (2E)-3-(5-bromo-2-piperidin-1-ylphenyl)acrylate (1.22 g), 4-(2-butoxyethoxy)phenylboric acid (1.03 g) and potassium carbonate (1.00 g) in toluene (36 ml), ethanol (3.6 ml) and water (3.6 ml) was stirred for 1 hour at room temperature. Tetrakis(triphenylphosphine)palladium (0.20 g) was added to the reaction system, and the mixture was heated under reflux for 7 hours. After cooling to room temperature, water was added thereto, and the resulting mixture was extracted with ethyl acetate. The organic layer was washed with saturated brine, and dried over magnesium sulfate. After concentration under reduced pressure, the residue was separated and purified by column chromatography (ethyl acetate : hexane 1 : 19 → 1 : 9) to give ethyl (2E)-3-[4'-(2-butoxyethoxy)-4-piperidin-1-yl-1,1'-biphenyl-3-yl]acrylate (1.53 g) as a yellow oily material.

$^1$H-NMR (200 MHz, $CDCl_3$) δ 0.93 (3H, t, J=7.1 Hz), 1.30-1.46 (5H, m), 1.51-1.67 (4H, m), 1.70-1.85 (4H, m), 2.91-2.96 (4H, m), 3.55 (2H, t, J=6.4 Hz), 3.80 (2H, t, J=4.8 Hz), 4.16 (2H, t, J=4.8 Hz), 4.26 (2H, q, J=7.2 Hz), 6.47 (1H, d, J=16.3 Hz), 6.98 (2H, d, J=8.6 Hz), 7.07 (1H, d, J=8.4 Hz), 7.44-7.54 (3H, m), 7.70 (1H, d, J=2.2 Hz), 8.10 (1H, d, J=16.3 Hz).

IR (neat) 1713, 1634, 1607, 1487, 1248, 1231, 1177, 1125, 1038, 820 $cm^{-1}$

Reference Example 31

To a solution of ethyl (2E)-3-[4'-(2-butoxyethoxy)-4-piperidin-1-yl-1,1'-biphenyl-3-yl]acrylate (1.53 g) in THF (5 ml) and ethanol (10 ml) was added a 1 N aqueous sodium hydroxide solution (7.0 ml) at room temperature, and the mixture was stirred at 65°C for 3 days. 1 N Hydrochloric acid (7.0 ml) was added thereto at 0°C, and the resulting mixture was extracted with ethyl acetate. The organic layer was washed with saturated brine, and dried over magnesium sulfate. After concentration under reduced pressure, the precipitated crystals were collected by filtration. The crystals were washed with diisopropyl ether to give (2E)-3-[4'-(2-butoxyethoxy)-4-piperidin-1-yl-1,1'-biphenyl-3-yl]acrylic acid (1.13 g) as yellow crystals.

$^1$H-NMR (200 MHz, $CDCl_3$) δ 0.93 (3H, t, J=7.3 Hz), 1.30-1.45 (2H, m), 1.52-1.69 (4H, m), 1.72-1.87 (4H, m), 2.92-2.97 (4H, m), 3.56 (2H, t, J=6.6 Hz), 3.81 (2H, t, J=5.0 Hz), 4.17 (2H, t, J=5.0 Hz), 6.49 (1H, d, J=16.2 Hz), 6.99 (2H, d, J=8.8 Hz), 7.09 (1H, d, J=8.4 Hz), 7.44-7.56 (3H, m), 7.72 (1H, d, J=2.2 Hz), 8.21 (1H, d, J=16.2 Hz).

IR (KBr) 1684, 1624, 1607, 1489, 1302, 1248, 1231, 1121, 820, 808 $cm^{-1}$

Elementary analysis $C_{26}H_{33}NO_4$, Calcd. C, 73.73 ; H, 7.85 ; N, 3.31 : Found. C, 73.55 ; H, 7.81 ; N, 3.16.

Reference Example 32

To a suspension of sodium hydride (60%, 0.21 g) in toluene (10 ml) was added dropwise a solution of ethyl 2-(diethylphosphono)propionate (1.28 g) in toluene (5 ml) at 0°C under a nitrogen atmosphere. After stirring at 0°C for 1 hour, a solution of 5-bromo-2-piperidin-1-ylbenzaldehyde (1.2 g) in toluene (20 ml) was added dropwise thereto. The reaction mixture was heated under reflux for 3 hours. Then, water was added thereto, and the mixture was extracted with ethyl acetate. The organic layer was washed with saturated brine, and dried over magnesium sulfate. After concentration under reduced pressure, the residue was separated and purified by column chromatography (ethyl acetate : hexane = 1 : 49) to give ethyl (2E)-3-(5-bromo-2-piperidin-1-ylphenyl)-2-methylacrylate (1.47 g) as a yellow oily material.

$^1$H-NMR (200 MHz, $CDCl_3$) δ 1.35 (3H, t, J=7.1 Hz), 1.46-1.80 (6H, m), 2.09-2.10 (3H, m), 2.82-2.87 (4H, m), 4.28 (2H, q, J=7.1 Hz), 6.85 (1H, d, J=8.4 Hz), 7.34-7.41 (2H, m), 7.72 (1H, s).

IR (neat) 1709, 1480, 1451, 1275, 1250, 1233, 1130, 1113, 814 $cm^{-1}$

Reference Example 33

Under an argon atmosphere, a mixture of ethyl (2E)-3-(5-bromo-2-piperidin-1-ylphenyl)-2-methylacrylate (1.47 g), 4-(2-butoxyethoxy)phenylboric acid (1.19 g) and potassium carbonate (1.15 g) in toluene (40 ml), ethanol (4 ml) and water (4 ml) was stirred for 1 hour at room temperature. Tetrakis(triphenylphosphine)palladium (0.24 g) was added to the reaction system, and the mixture was heated under reflux for 6 hours. After cooling to room temperature,

water was added thereto, and the resulting mixture was extracted with ethyl acetate. The organic layer was washed with saturated brine, and dried over magnesium sulfate. After concentration under reduced pressure, the residue was separated and purified by column chromatography (ethyl acetate : hexane 1 : 29 → 1 : 19) to give ethyl (2E)-3-[4'-(2-butoxyethoxy)-4-piperidin-1-yl-1,1'-biphenyl-3-yl]-2-methylacrylate (1.68 g) as pale yellow crystals.

$^1$H-NMR (200 MHz, $CDCl_3$) δ 0.93 (3H, t, J=7.2 Hz), 1.28-1.45 (5H, m), 1.49-1.79 (8H, m), 2.17 (3H, d, J=1.6 Hz), 2.89-2.94 (4H, m), 3.55 (2H, t, J=6.6 Hz), 3.80 (2H, t, J=5.0 Hz), 4.16 (2H, t, J=5.0 Hz), 4.29 (2H, q, J=7.2 Hz), 6.96-7.05 (3H, m), 7.44-7.48 (4H, m), 7.87 (1H, s).

IR (neat) 1703, 1605, 1485, 1271, 1240, 1128, 1111, 820 $cm^{-1}$

Reference Example 34

To a solution of ethyl (2E)-3-[4'-(2-butoxyethoxy)-4-piperidin-1-yl-1,1'-biphenyl-3-yl]-2-methylacrylate (1.58 g) in THF (5 ml) and ethanol (10 ml) was added a 1 N aqueous sodium hydroxide solution (7.0 ml) at room temperature, and the mixture was stirred at 65°C for 20 hours. 1 N Hydrochloric acid (7.0 ml) was added thereto at 0°C, and the resulting mixture was extracted with ethyl acetate. The organic layer was washed with saturated brine, and dried over magnesium sulfate. After concentration under reduced pressure, the precipitated crystals were collected by filtration. The crystals were washed with diisopropyl ether and hexane to give (2E)-3-[4'-(2-butoxyethoxy)-4-piperidin-1-yl-1,1'-biphenyl-3-yl]-2-methylacrylic acid (1.11 mg) as yellow crystals.

$^1$H-NMR (200 MHz, $CDCl_3$) δ 0.93 (3H, t, J=7.1 Hz), 1.28-1.84 (10H, m), 2.19 (3H, d, J=1.4 Hz), 2.86-2.96 (4H, m), 3.55 (2H, t, J=6.6 Hz), 3.80 (2H, t, J=5.0 Hz), 4.16 (2H, t, J=5.0 Hz), 6.99 (2H, d, J=8.8 Hz), 7.06 (1H, d, J=8.0 Hz), 7.44-7.51 (4H, m), 8.00 (1H, s).

IR (KBr) 1678, 1609, 1487, 1450, 1285, 1235, 1132, 826 $cm^{-1}$

Elementary analysis $C_{27}H_{35}NO_4$, Calcd. C, 74.11 ; H, 8.06 ; N, 3.20 : Found. C, 73.39 ; H, 7.98 ; N, 3.07.

Reference Example 35

A mixture of 5-bromo-2-fluorobenzaldehyde (2.5 g), pyrrolidine (1.33 ml) and potassium carbonate (2.55 g) in DMF (25 ml) was stirred at 80°C for 4 days. Water was added to the reaction system, and the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 9) to give 5-bromo-2-pyrrolidin-1-ylbenzaldehyde (2.50 g) as a yellow oily material.

$^1$H-NMR (300 MHz, $CDCl_3$) δ 1.98-2.02 (4H, m), 3.32-3.37 (4H, m), 6.71 (1H, d, J=9.0 Hz), 7.41 (1H, dd, J=9.0, 2.7 Hz), 7.78 (1H, d, J=2.7 Hz), 10.01 (1H, s).

IR (neat) 1667, 1593, 1480, 1462, 1406, 1167, 912, 743 $cm^{-1}$

Reference Example 36

To a suspension of sodium hydride (60%, 0.236 g) in toluene (10 ml) was added dropwise a solution of ethyl diethylphosphonoacetate (1.32 g) in toluene (10 ml) at 0°C under a nitrogen atmosphere. After stirring at 0°C for 1 hour, a solution of 5-bromo-2-pyrrolidin-1-ylbenzaldehyde (1.25 g) in toluene (10 ml) was added dropwise thereto. The reaction mixture was heated under reflux for 4 hours. Then, water was added thereto, and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 15 → 1 : 9) to give ethyl (2E)-3-(5-bromo-2-pyrrolidin-1-ylphenyl)acrylate (1.387 g) as a yellow oily material.

$^1$H-NMR (200 MHz, $CDCl_3$) δ 1.33 (3H, t, J=7.1 Hz), 1.90-1.97 (4H, m), 3.23-3.29 (4H, m), 4.25 (2H, q, J=7.1 Hz), 6.21 (1H, d, J=15.8 Hz), 6.71 (1H, d, J=9.0 Hz), 7.29 (1H, dd, J=9.0, 2.4 Hz), 7.49 (1H, d, J=2.4 Hz), 7.93 (1H, d, J=15.8 Hz).

IR (neat) 1711, 1628, 1480, 1314, 1175, 912, 743 $cm^{-1}$

Reference Example 37

Under an argon atmosphere, a mixture of ethyl (2E)-3-(5-bromo-2-pyrrolidin-1-ylphenyl)acrylate (1.387 g), 4-(2-butoxyethoxy)phenylboric acid (1.22 g) and potassium carbonate (1.18 g) in toluene (40 ml), ethanol (4.0 ml) and water (4.0 ml) was stirred for 1 hour at room temperature. Tetrakis(triphenylphosphine)palladium (0.24 g) was added to the reaction system, and the mixture was heated under reflux for 6 hours. After cooling to room temperature, water was added thereto, and the resulting mixture was extracted with ethyl acetate. The organic layer was washed with saturated brine, and dried over magnesium sulfate. After concentration under reduced pressure, the residue was separated and purified by column chromatography (ethyl acetate : hexane 1 : 19 → 1 : 9) to give ethyl (2E)-3-[4'-(2-butoxyethoxy)-4-pyrrolidin-1-yl-1,1'-biphenyl-3-yl]acrylate (0.835 g) as yellow crystals.

$^1$H-NMR (200 MHz, $CDCl_3$) δ 0.93 (3H, t, J=7.1 Hz), 1.30-1.45 (5H, m), 1.51-1.69 (2H, m), 1.92-1.99 (4H, m), 3.29-3.36 (4H, m), 3.55 (2H, t, J=6.6 Hz), 3.80 (2H, t, J=4.9 Hz), 4.16 (2H, t, J=4.9 Hz), 4.26 (2H, q, J=7.1 Hz), 6.31 (1H, d, J=16.0 Hz), 6.91 (1H, d, J=8.8 Hz), 6.97 (2H, d, J=8.6 Hz), 7.41-7.48 (3H, m), 7.60 (1H, d, J=2.2 Hz), 8.08 (1H, d, J=16.0 Hz).

Reference Example 38

To a solution of ethyl (2E)-3-[4'-(2-butoxyethoxy)-4-pyrrolidin-1-yl-1,1'-biphenyl-3-yl]acrylate (0.835 g) in THF (5 ml) and ethanol (10 ml) was added a 1 N aqueous sodium hydroxide solution (4.0 ml) at room temperature, and the mixture was stirred at 60°C for 2 days. 1 N Hydrochloric acid (4.0 ml) was added thereto at 0°C, and the resulting mixture was extracted with ethyl acetate. The organic layer was washed with saturated brine, and dried over magnesium sulfate. After concentration under reduced pressure, the precipitated crystals were collected by filtration. The crystals were washed with diisopropyl ether to give (2E)-3-[4'-(2-butoxyethoxy)-4-pyrrolidin-1-yl-1,1'-biphenyl-3-yl]acrylic acid (707 mg) as yellow crystals.

$^1$H-NMR (200 MHz, $CDCl_3$) δ 0.93 (3H, t, J=7.2 Hz), 1.29-1.48 (2H, m), 1.51-1.66 (2H, m), 1.94-2.00 (4H, m), 3.31-3.38 (4H, m), 3.55 (2H, t, J=6.6 Hz), 3.81 (2H, t, J=4.9 Hz), 4.16 (2H, t, J=4.9 Hz), 6.32 (1H, d, J=15.8 Hz), 6.92 (1H, d, J=8.8 Hz), 6.98 (2H, d, J=8.6 Hz), 7.44-7.49 (3H, m), 7.62 (1H, d, J=2.2 Hz), 8.19 (1H, d, J=15.8 Hz).

Elementary analysis $C_{25}H_{31}NO_4$, Calcd. C, 73.32 ; H, 7.63 ; N, 3.42 : Found. C, 73.11 ; H, 7.54 ; N, 3.24.

Reference Example 39

To a suspension of sodium hydride (60%, 0.235 g) in toluene (10 ml) was added dropwise a solution of ethyl 2-(diethylphosphono)propionate (1.40 g) in toluene (10 ml) at 0°C under a nitrogen atmosphere. After stirring at 0°C for 1 hour, a solution of aldehyde (1.25 g) in toluene (10 ml) was added dropwise thereto. The reaction mixture was heated under reflux for 6 hours. Then, water was added thereto, and the mixture was extracted with ethyl acetate. The organic layer was washed with water and saturated brine, and dried over magnesium sulfate. After concentration under reduced pressure, the residue was separated and purified by column chromatography (ethyl acetate : hexane = 1 : 19) to give ethyl (2E)-3-(5-bromo-2-pyrrolidin-1-ylphenyl)-2-methylacrylate (1.489 g) as a pale yellow oily material.

$^1$H-NMR (200 MHz, $CDCl_3$) δ 1.34 (3H, t, J=7.1 Hz), 1.87-1.94 (4H, m), 1.97 (3H, d, J=1.6 Hz), 3.15-3.21 (4H, m), 4.26 (2H, q, J=7.1 Hz), 6.66 (1H, d, J=8.4 Hz), 7.19-7.29 (2H, m), 7.67 (1H, s).

IR (neat) 1709, 1478, 1273, 1111, 912, 745 $cm^{-1}$

Reference Example 40

Under an argon atmosphere, a mixture of ethyl (2E)-3-(5-bromo-2-pyrrolidin-1-ylphenyl)-2-methylacrylate (1.489 g), 4-(2-butoxyethoxy)phenylboric acid (1.26 g) and potassium carbonate (1.22 g) in toluene (45 ml), ethanol (4.5 ml) and water (4.5 ml) was stirred for 1 hour at room temperature. Tetrakis(triphenylphosphine)palladium (0.25 g) was added to the reaction system, and the mixture was heated under reflux for 6 hours. After cooling to room temperature, water was added thereto, and the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 9) to give ethyl (2E)-3-[4'-(2-butoxyethoxy)-4-pyrrolidin-1-yl-1,1'-biphenyl-3-yl]-2-methylacrylate (1.51 g) as pale yellow crystals.

$^1$H-NMR (200 MHz, $CDCl_3$) δ 0.93 (3H, t, J=7.3 Hz), 1.29-1.48 (5H, m), 1.51-1.68 (2H, m), 1.89-1.95 (4H, m), 2.04 (3H, d, J=1.4 Hz), 3.22-3.28 (4H, m), 3.55 (2H, t, J=6.8 Hz), 3.80 (2H, t, J=4.9 Hz), 4.15 (2H, t, J=4.9 Hz), 4.27 (2H, q, J=7.1 Hz), 6.86 (1H, d, J=8.4 Hz), 6.96 (2H, d, J=8.8 Hz), 7.32-7.48 (4H, m), 7.83 (1H, s).

IR (KBr) 1705, 1605, 1495, 1483, 1271, 1246, 1113, 909, 737 $cm^{-1}$

Elementary analysis $C_{28}H_{37}NO_4$, Calcd. C, 74.47 ; H, 8.26 ; N, 3.10 : Found. C, 74.34 ; H, 8.32 ; N, 2.89.

Reference Example 41

To a solution of ethyl (2E)-3-[4'-(2-butoxyethoxy)-4-pyrrolidin-1-yl-1,1'-biphenyl-3-yl]-2-methylacrylate (1.46 g) in THF (5 ml) and ethanol (10 ml) was added a 1 N aqueous sodium hydroxide solution (7.0 ml) at room temperature, and the mixture was stirred at 60°C for 3 days. 1 N Hydrochloric acid (7.0 ml) was added thereto at 0°C, and the resulting mixture was extracted with ethyl acetate. The organic layer was washed with saturated brine, and dried over magnesium sulfate. After concentration under reduced pressure, the precipitated crystals were collected by filtration. The crystals were washed with diisopropyl ether and hexane to give (2E)-3-[4'-(2-butoxyethoxy)-4-pyrrolidin-1-yl-1,1'-biphenyl-3-yl]-2-methylacrylic acid (1.04 g) as yellow crystals.

$^1$H-NMR (300 MHz, $CDCl_3$) δ 0.93 (3H, t, J=7.2 Hz), 1.34-1.71 (4H, m), 1.91-1.95 (4H, m), 2.07 (3H, d, J=1.2 Hz), 3.24-3.29 (4H, m), 3.54 (2H, t, J=6.6 Hz), 3.80 (2H, t, J=5.0 Hz), 4.15 (2H, t, J=5.0 Hz), 6.87 (1H, d, J=8.7 Hz), 6.96 (2H, d, J=9.0 Hz), 7.34-7.46 (4H, m), 7.95 (1H, s).

IR (KBr) 1671, 1607, 1483, 1287, 1244, 1123, 816 $cm^{-1}$

Elementary analysis $C_{26}H_{33}NO_4$, Calcd. C, 73.73 ; H, 7.85 ; N, 3.31 : Found. C, 73.53 ; H, 7.71 ; N, 3.10.

Reference Example 42

A mixture of 5-bromo-2-fluorobenzaldehyde (2.50 g), 4-methylpiperidine (1.46 ml) and potassium carbonate (2.55 g) in DMF (25 ml) was stirred at 80°C for 3 days. Water was added to the reaction system, and the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 49) to give 5-bromo-(4-methylpiperidin-1-yl)benzaldehyde (2.95 g) as a yellow oily material.

$^1$H-NMR (200 MHz, $CDCl_3$) δ 1.06 (3H, d, J=5.8 Hz), 1.31-1.55 (3H, m), 1.71-1.83 (2H, m), 2.81-2.92 (2H, m), 3.16-3.29 (2H, m), 6.98 (1H, d, J=8.8 Hz), 7.56 (1H, dd, J=8.8, 2.6 Hz), 7.88 (1H, d, J=2.6 Hz), 10.19 (1H, s).

IR (neat) 1682, 1586, 1480, 1464, 1379, 1219, 910, 737 $cm^{-1}$

Reference Example 43

To a suspension of sodium hydride (60%, 0.24 g) in toluene (10 ml) was added dropwise a solution of ethyl diethylphosphonoacetate (1.18 ml) in toluene (10 ml) at 0°C under an argon atmosphere. After stirring at 0°C for 1 hour, a solution of 5-bromo-(4-methylpiperidin-1-yl)benzaldehyde (1.40 g) in toluene (10 ml) was added thereto, and the resulting mixture was heated under reflux for 4 hours. Water was added to the reaction system and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 49) to give ethyl (2E)-3-[5-bromo-2-(4-methylpiperidin-1-yl)phenyl]acrylate (1.59 g) as yellow crystals.

$^1$H-NMR (200 MHz, $CDCl_3$) δ 1.00 (3H, d, J=5.6 Hz), 1.31-1.56 (6H, m), 1.63-1.79 (2H, m), 2.56-2.72 (2H, m), 3.03-3.16 (2H, m), 4.27 (2H, q, J=7.1 Hz), 6.37 (1H, d, J=16.1 Hz), 6.89 (1H, d, J=8.4 Hz), 7.39 (1H, dd, J=8.4, 2.6 Hz), 7.62 (1H, d, J=2.6 Hz), 7.94 (1H, d, J=16.1 Hz).

Elementary analysis $C_{17}H_{22}NO_2Br$, Calcd. C, 57.96 ; H, 6.29 ; N, 3.98 : Found. C, 57.80 ; H, 6.12 ; N, 3.86.

Reference Example 44

Under an argon atmosphere, a mixture of ethyl (2E)-3-[5-bromo-2-(4-methylpiperidin-1-yl)phenyl]acrylate (1.47 g), 4-(2-butoxyethoxy)phenylboric acid (1.24 g) and potassium carbonate (1.11 g) in toluene (40 ml), ethanol (4 ml) and water (4 ml) was stirred for 1 hour at room temperature. Tetrakis(triphenylphosphine)palladium (0.23 g) was added to the reaction system, and the mixture was heated under reflux for 6 hours. After cooling to room temperature, water was added thereto, and the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 39 → 1 : 19 → 1 : 9) to give ethyl (2E)-3-[4'-(2-butoxyethoxy)-4-(4-methylpiperidin-1-yl)-1,1'-biphenyl-3-yl]acrylate (1.83 g) as a yellow oily material.

$^1$H-NMR (200 MHz, $CDCl_3$) δ 0.93 (3H, t, J=7.2 Hz), 1.02 (3H, d, J=5.2 Hz), 1.28-1.82 12H, m), 2.64-2.79 (2H, m), 3.11-3.24 (2H, m), 3.56 (2H, t, J=6.6 Hz), 3.81 (2H, t, J=4.9 Hz), 4.17 (2H, t, J=4.9 Hz), 4.28 (2H, q, J=7.1 Hz), 6.47 (1H, d, J=16.2 Hz), 6.99 (2H, d, J=8.8 Hz), 7.08 (1H, d, J=8.4 Hz), 7.44-7.54 (3H, m), 7.70 (1H, d, J=2.2 Hz), 8.09 (1H, d, J=16.2 Hz).

IR (neat) 1711, 1487, 1246, 1223, 1177, 822 $cm^{-1}$

Reference Example 45

To a solution of ethyl (2E)-3-[4'-(2-butoxyethoxy)-4-(4-methylpiperidin-1-yl)-1,1'-biphenyl-3-yl]acrylate (1.83 g) in ethanol (10 ml) and THF (5 ml) was added a 1 N aqueous sodium hydroxide solution (8.0 ml) at room temperature, and the mixture was stirred at 60°C for 20 hours. 1 N Hydrochloric acid (8.0 ml) was added thereto at 0°C, and the resulting mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether and hexane to give (2E)-3-[4'-(2-butoxyethoxy)- 4-(4-methylpiperidin-1-yl)-1,1'-biphenyl-3-yl]acrylic acid (1.556 g) as yellow crystals.

m.p. 159-160°C

$^1$H-NMR (200 MHz, $CDCl_3$) δ 0.94 (3H, t, J=7.2 Hz), 1.03 (3H, d, J=4.8 Hz), 1.31-1.82 (9H, m), 2.64-2.80 (2H, m), 3.11-3.25 (2H, m), 3.56 (2H, t, J=6.6 Hz), 3.82 (2H, t, J=5.0 Hz), 4.17 (2H, t, J=5.0 Hz), 6.49(1H, d, J=16.3 Hz), 7.00 (2H, d, J=8.8 Hz), 7.10 (1H, d, J=8.4 Hz), 7.47-7.57 (3H, m), 7.72 (1H, d, J=2.2 Hz), 8.19 (1H, d, J=16.3 Hz).

Elementary analysis $C_{27}H_{35}NO_4$, Calcd. C, 74.11 ; H, 8.06 ; N, 3.20 : Found. C, 73.92 ; H, 7.96 ; N, 2.98.

Reference Example 46

To a suspension of sodium hydride (60%, 0.26 g) in toluene (10 ml) was added dropwise a solution of ethyl 2-(diethylphosphono)propionate (1.57 ml) in toluene (5.0 ml) at 0°C under an argon atmosphere. After stirring at 0°C for 1 hour, a solution of 5-bromo-(4-methylpiperidin-1-yl)benzaldehyde (1.55 g) in toluene (20 ml) was added thereto, and the mixture was heated under reflux for 5 hours. Water was added to the reaction system, and the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 49) to give ethyl (2E)-3-[5-bromo-2-(4-methylpiperidin-1-yl)phenyl]-2-methylacrylate (2.0 g) as yellow crystals.

$^1$H-NMR (200 MHz, $CDCl_3$) δ 0.98 (3H, d, J=5.8 Hz), 1.23-1.49 (6H, m), 1.61-1.76 (2H, m), 2.10 (3H, d, J=1.0 Hz), 2.53-2.68 (2H, m), 3.04-3.16 (2H, m), 4.28 (2H, q, J=7.2 Hz), 6.86 (1H, d, J=8.4 Hz), 7.34-7.41 (2H, m), 7.71 (1H, s).

IR (neat) 1709, 1464, 1277, 1254, 1225, 1132, 1115, 909, 737 $cm^{-1}$

Reference Example 47

Under an argon atmosphere, a mixture of ethyl (2E)-3-[5-bromo-2-(4-methylpiperidin-1-yl)phenyl]-2-methylacrylate (2.00 g), 4-(2-butoxyethoxy)phenylboric acid (1.69 g) and potassium carbonate (1.51 g) in toluene (50 ml), ethanol (5 ml) and water (5 ml) was stirred for 1 hour at room temperature. Tetrakis(triphenylphosphine)palladium (0.19 g) was added to the reaction system, and the mixture was heated under reflux for 6 hours. After cooling to room temperature, the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 39 → 1 : 19 → 1 : 9) to give ethyl (2E)-3-[4'-(2-butoxyethoxy)-4-(4-methylpiperidin-1-yl)-1,1'-biphenyl-3-yl]-2-methylacrylate (2.30 g) as yellow crystals.

$^1$H-NMR (200 MHz, $CDCl_3$) δ 0.93 (3H, t, J=7.3 Hz), 1.00 (3H, d, J=5.4 Hz), 1.28-1.79 (12H, m), 2.17 (3H, d, J=1.6 Hz), 2.59-2.74 (2H, m), 3.11-3.25 (2H, m), 3.55 (2H, t, J=6.6 Hz), 3.81 (2H, t, J=4.9 Hz), 4.16 (2H, t, J=4.9 Hz), 4.30 (2H, q, J=7.1 Hz), 6.98 (2H, d, J=8.6 Hz), 7.04 (1H, d, J=8.0 Hz), 7.44-7.48 (4H, m), 7.86 (1H, s).

IR (neat) 1705, 1607, 1487, 1273, 1244, 1130, 1117, 824 $cm^{-1}$

Reference Example 48

To a solution of ethyl (2E)-3-[4'-(2-butoxyethoxy)-4-(4-methylpiperidin-1-yl)-1,1'-biphenyl-3-yl]-2-methylacrylate (2.30 g) in ethanol (20 ml) and THF (10 ml) was added a 1 N aqueous sodium hydroxide solution (10.0 ml) at room temperature, and the mixture was stirred at 60°C for 4 days. 1 N Hydrochloric acid (10.0 ml) was added thereto at 0°C, and the resulting mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether and hexane to give (2E)-3-[4'-(2-butoxyethoxy)-4-(4-methylpiperidin-1-yl)-1,1'-biphenyl-3-yl]-2-methylacrylic acid (1.60 g) as yellow crystals.

m.p. 154-155°C

$^1$H-NMR (200 MHz, $CDCl_3$) δ 0.93 (3H, t, J=7.3 Hz), 1.01 (3H, d, J=5.6 Hz), 1.28-1.80 (9H, m), 2.20 (3H, d, J=1.4 Hz), 2.61-2.75 (2H, m), 3.11-3.25 (2H, m), 3.56 (2H, t, J=6.6 Hz), 3.81 (2H, t, J=5.0 Hz), 4.17 (2H, t, J=5.0 Hz), 6.99 (2H, d, J=8.8 Hz), 7.07 (1H, d, J=8.0 Hz), 7.45-7.52 (4H, m), 8.00 (1H, s).

Elementary analysis $C_{28}H_{37}NO_4$, Calcd. C, 74.47 ; H, 8.26 ; N, 3.10 : Found. C, 74.59 ; H, 8.39 ; N, 3.02.

Reference Example 49

To a suspension of sodium hydride (60%, 0.238 g) in toluene (10 ml) was added dropwise a solution of ethyl 2-(diethylphosphono)butyrate (1.41 ml) in toluene (10 ml) at 0°C under an argon atmosphere. After stirring at 0°C for 1 hour, a solution of 5-bromo-2-pyrrolidin-1-ylbenzaldehyde (1.263 g) in toluene (30 ml) was added thereto, and the mixture was heated under reflux for 6 hours. Water was added to the reaction system, and the resulting mixture was extracted with ethyl acetate. The organic layer was washed with water and saturated brine, and dried over magnesium

sulfate. After concentration under reduced pressure, the residue was separated and purified by column chromatography (ethyl acetate : hexane 1 : 9) to give ethyl (2E)-3-(5-bromo-2-pyrrolidin-1-ylphenyl)-2-ethylacrylate (1.666 g) as a yellow oily material.

$^1$H-NMR (200 MHz, $CDCl_3$) δ 1.10 (3H, t, J=7.3 Hz), 1.34 (3H, t, J=7.1 Hz), 1.87-1.94 (4H, m), 2.48 (2H, q, J=7.3 Hz), 3.17-3.23 (4H, m), 4.27 (2H, q, J=7.1 Hz), 6.65 (1H, d, J=8.8 Hz), 7.20-7.29 (2H, m), 7.62 (1H, s).

IR (neat) 1709, 1480, 1236, 1128, 912, 741 $cm^{-1}$

Reference Example 50

Under an argon atmosphere, a mixture of ethyl (2E)-3-(5-bromo-2-pyrrolidin-1-ylphenyl)-2-ethylacrylate (1.666 g), 4-(2-butoxyethoxy)phenylboric acid (1.35 g) and potassium carbonate (1.31 g) in toluene (50 ml), ethanol (5 ml) and water (5 ml) was stirred for 1 hour at room temperature. Tetrakis(triphenylphosphine)palladium (0.16 g) was added to the reaction system, and the mixture was heated under reflux for 6 hours. After cooling to room temperature, the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19) to give ethyl (2E)-3-[4'-(2-butoxyethoxy)-4-pyrrolidin-1-yl-1,1'-biphenyl-3-yl]-2-ethylacrylate (1.46 g) as pale yellow crystals.

$^1$H-NMR (200 MHz, $CDCl_3$) δ 0.93 (3H, t, J=7.1 Hz), 1.15 (3H, t, J=7.3 Hz), 1.29-1.48 (5H, m), 1.54-1.68 (2H, m), 1.89-1.95 (4H, m), 2.57 (2H, q, J=7.3 Hz), 3.24-3.31 (4H, m), 3.55 (2H, t, J=6.6 Hz), 3.80 (2H, t, J=5.0 Hz), 4.16 (2H, t, J=5.0 Hz), 4.28 (2H, q, J=7.1 Hz), 6.85 (1H, d, J=8.4 Hz), 6.97 (2H, d, J=8.6 Hz), 7.34-7.49 (4H, m), 7.77 (1H, s).

IR (neat) 1709, 1480, 1236, 1128, 912, 741 $cm^{-1}$

Elementary analysis $C_{29}H_{39}NO_4$, Calcd. C, 74.81 ; H, 8.44 ; N, 3.01 : Found. C, 74.70 ; H, 8.53 ; N, 2.73.

Reference Example 51

To a solution of ethyl (2E)-3-[4'-(2-butoxyethoxy)-4-pyrrolidin-1-yl-1,1'-biphenyl-3-yl]-2-ethylacrylate (1.40 g) in ethanol (20 ml) and THF (10 ml) was added a 1 N aqueous sodium hydroxide solution (6.0 ml) at room temperature, and the mixture was stirred at 65°C for 24 hours. 1 N Hydrochloric acid (6.0 ml) was added thereto at 0°C, and the resulting mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether and hexane to give (2E)-3-[4'-(2-butoxyethoxy)-4-pyrrolidin-1-yl-1,1'-biphenyl-3-yl]-2-ethylacrylic acid (962 mg) as yellow crystals.

m.p. 155-156°C

$^1$H-NMR (200 MHz, $CDCl_3$) δ 0.93 (3H, t, J=7.1 Hz), 1.21 (3H, t, J=7.5 Hz), 1.30-1.48 (2H, m), 1.54-1.68 (2H, m), 1.90-1.99 (4H, m), 2.60 (2H, q, J=7.5 Hz), 3.26-3.33 (4H, m), 3.55 (2H, t, J=6.6 Hz), 3.80 (2H, t, J=5.0 Hz), 4.16 (2H, t, J=5.0 Hz), 6.87 (1H, d, J=8.8 Hz), 6.98 (2H, d, J=8.8 Hz), 7.37-7.48 (4H, m), 7.93 (1H, s).

Elementary analysis $C_{27}H_{35}NO_4$, Calcd. C, 74.11 ; H, 8.06 ; N, 3.20 : Found. C, 73.98 ; H, 8.15 ; N, 3.22.

Reference Example 52

A mixture of 5-bromo-2-fluorobenzaldehyde (2.50 g), 2-methylpyrrolidine (1.63 ml) and sodium carbonate (2.6 g) in DMSO (25 ml) and water (5 ml) was stirred at 100°C for 12 hours. Water was added to the reaction system, and the resulting mixture was extracted with ethyl acetate. The organic layer was washed with saturated brine, and dried over magnesium sulfate. After concentration under reduced pressure, the residue was separated and purified by column chromatography (ethyl acetate : hexane 1 : 19 → 1 : 9) to give 5-bromo-2-(2-methylpyrrolidin-1-yl)benzaldehyde (2.76 g) as a yellow oily material.

$^1$H-NMR (200 MHz, $CDCl_3$) δ 1.20 (3H, d, J=5.8 Hz), 1.63-1.82 (2H, m), 1.88-2.03 (1H, m), 2.08-2.32 (1H, m), 3.03-3.13 (1H, m), 3.69-3.96 (2H, m), 6.81 (1H, d, J=9.2 Hz), 7.44 (1H, dd, J=9.0, 2.6 Hz), 7.82 (1H, d, J=2.6 Hz), 10.04 (1H, s).

IR (neat) 1674, 1590, 1474, 1402, 1175, 912, 741 $cm^{-1}$ Reference Example 53

To a suspension of sodium hydride (60%, 0.25 g) in toluene (10 ml) was added dropwise a solution of ethyl diethylphosphonoacetate (1.23 ml) in toluene (10 ml) at 0°C under an argon atmosphere. After stirring at 0°C for 30 minutes, a solution of 5-bromo-2-(2-methylpyrrolidin-1-yl)benzaldehyde (1.38 g) in toluene (20 ml) was added thereto, and the resulting mixture was heated under reflux for 3 hours. Water was added to the reaction system, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19) to give ethyl (2E)-3-[5-bromo-2-(2-methylpyrrolidin-1-yl)phenyl]acrylate (1.64 g) as a yellow oily material.

$^1$H-NMR (200 MHz, $CDCl_3$) δ 1.06 (3H, d, J=6.0 Hz), 1.34 (3H, t, J=7.1 Hz), 1.50-1.96 (3H, m), 2.06-2.24 (1H, m), 2.88-3.00 (1H, m), 3.52-3.76 (2H, m), 4.26 (2H, q, J=7.1 Hz), 6.27 (1H, d, J=16.1 Hz), 6.80 (1H, d, J=8.6 Hz), 7.33 (1H, dd, J=8.6, 2.3 Hz), 7.55 (1H, d, J=2.3 Hz), 7.88 (1H, d, J=16.1 Hz).

IR (neat) 1713, 1632, 1476, 1314, 1175, 1038, 909, 742 $cm^{-1}$

Reference Example 54

Under an argon atmosphere, a mixture of ethyl (2E)-3-[5-bromo-2-(2-methylpyrrolidin-1-yl)phenyl]acrylate (1.64 g), 4-(2-butoxyethoxy)phenylboric acid (1.39 g) and potassium carbonate (1.24 g) in toluene (50 ml), ethanol (5 ml) and water (5 ml) was stirred for 1 hour at room temperature. Tetrakis(triphenylphosphine)palladium (0.17 g) was added to the reaction system, and the mixture was heated under reflux for 6 hours. After cooling to room temperature, the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 14 → 1 : 9) to give ethyl (2E)-3-[4'-(2-butoxyethoxy)-4-(2-methylpyrrolidin-1-yl)-1,1'-biphenyl-3-yl]acrylate (1.87 g) as a yellow oily material.

$^1$H-NMR (200 MHz, $CDCl_3$) δ 0.93 (3H, t, J=7.2 Hz), 1.11 (3H, d, J=6.2 Hz), 1.31-1.48 (6H, m), 1.61-1.98 (4H, m), 2.08-2.28 (1H, m), 2.91-3.06 (1H, m), 3.56 (2H, t, J=6.6 Hz), 3.62-3.84 (4H, m), 4.16 (2H, t, J=4.8 Hz), 4.27 (2H, q, J=7.1 Hz), 6.37 (1H, d, J=15.8 Hz), 6.98 (2H, d, J=8.8 Hz), 6.99 (1H, d, J=8.4 Hz), 7.44-7.50 (3H, m), 7.64 (1H, d, J=2.0 Hz), 8.04 (1H, d, J=15.8 Hz).

IR (neat) 1709, 1628, 1605, 1489, 1300, 1279, 1246, 1177, 1123, 910, 741 $cm^{-1}$

Reference Example 55

To a solution of ethyl (2E)-3-[4'-(2-butoxyethoxy)-4-(2-methylpyrrolidin-1-yl)-1,1'-biphenyl-3-yl]acrylate (1.87 g) in ethanol (20 ml) and THF (10 ml) was added a 1 N aqueous sodium hydroxide solution (12.0 ml) at room temperature, and the mixture was stirred at 65°C for 20 hours. 1 N Hydrochloric acid (12.0 ml) was added thereto at 0°C, and the resulting mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether to give (2E)-3-[4'-(2-butoxyethoxy)-4-(2-methylpyrrolidin-1-yl)-1,1'-biphenyl-3-yl]acrylic acid (1.438 g) as yellow crystals.

m.p. 102-103°C

$^1$H-NMR (200 MHz, $CDCl_3$) δ 0.93 (3H, t, J=7.3 Hz), 1.13 (3H, d, J=5.8 Hz), 1.29-1.49 (2H, m), 1.53-2.04 (5H, m), 2.08-2.29 (1H, m), 2.96-3.11 (1H, m), 3.56 (2H, t, J=6.6 Hz), 3.60-3.84 (4H, m), 4.17 (2H, t, J=5.0 Hz), 6.38 (1H, d, J=16.2 Hz), 6.97-7.02 (3H, m), 7.44-7.51 (3H, m), 7.66 (1H, d, J=2.2 Hz), 8.15 (1H, d, J=16.2 Hz).

Elementary analysis $C_{26}H_{33}NO_4$, Calcd. C, 73.73 ; H, 7.85 ; N, 3.31 : Found. C, 73.72 ; H, 7.69 ; N, 3.10.

Reference Example 56

To a suspension of sodium hydride (60%, 0.25 g) in toluene (10 ml) was added dropwise a solution of ethyl 2-(diethylphosphono)propionate (1.48 g) in toluene (10 ml) at 0°C under an argon atmosphere. After stirring at 0°C for 1 hour, a solution of 5-bromo-2-(2-methylpyrrolidin-1-yl)benzaldehyde (1.39 g) in toluene (20 ml) was added thereto, and the resulting mixture was heated under reflux for 7 hours. Water was added to the reaction system, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19) to give ethyl (2E)-3-[5-bromo-2-(2-methylpyrrolidin-1-yl)phenyl]-2-methylacrylate (1.74 g) as a yellow oily material.

$^1$H-NMR (200 MHz, $CDCl_3$) δ 1.04 (3H, d, J=5.8 Hz), 1.34 (3H, t, J=7.1 Hz), 1.49-1.92 (3H, m), 2.00 (3H, d, J=1.6 Hz), 2.07-2.26 (1H, m), 2.80-2.95 (1H, m), 3.38-3.49 (1H, m), 3.63-3.82 (1H, m), 4.27 (2H, q, J=7.1 Hz), 6.73 (1H, d, J=9.6 Hz), 7.21-7.31 (2H, m), 7.59 (1H, s).

IR (neat) 1709, 1476, 1273, 1250, 1111 $cm^{-1}$

Reference Example 57

Under an argon atmosphere, a mixture of ethyl (2E)-3-[5-bromo-2-(2-methylpyrrolidin-1-yl)phenyl]-2-methylacrylate (1.74 g), 4-(2-butoxyethoxy)phenylboric acid (1.41 g) and potassium carbonate (1.36 g) in toluene (50 ml), ethanol (5 ml) and water (5 ml) was stirred for 1 hour at room temperature. Tetrakis(triphenylphosphine)palladium (0.17 g) was added to the reaction system, and the mixture was heated under reflux for 6 hours. After cooling to room temperature, the resulting mixture was extracted with ethyl acetate. The organic layer was washed with water and

saturated brine, and dried over magnesium sulfate. After concentration under reduced pressure, the residue was separated and purified by column chromatography (ethyl acetate : hexane 1 : 19 → 1 : 9) to give ethyl (2E)-3-[4'-(2-butoxyethoxy)-4-(2-methylpyrrolidin-1-yl)-1,1'-biphenyl-3-yl]-2-methylacrylate (1.528 g) as a yellow oily material.

$^{1}$H-NMR (200 MHz, $CDCl_3$) δ 0.93 (3H, t, J=7.3 Hz), 1.11 (3H, d, J=6.2 Hz), 1.28-1.48 (5H, m), 1.51-1.94 (5H, m), 2.07 (3H, d, J=1.4 Hz), 2.08-2.25 (1H, m), 2.85-3.01 (1H, m), 3.45-3.58 (3H, m), 3.78-3.90 (3H, m), 4.16 (2H, t, J=4.8 Hz), 4.28 (2H, q, J=7.1 Hz), 6.90-6.99 (3H, m), 7.38-7.48 (4H, m), 7.76 (1H, s).

IR (neat) 1705, 1489, 1269, 1244, 1115, 912, 743 $cm^{-1}$

Reference Example 58

To a solution of ethyl (2E)-3-[4'-(2-butoxyethoxy)-4-(2-methylpyrrolidin-1-yl)-1,1'-biphenyl-3-yl]-2-methylacrylate (1.528 g) in ethanol (20 ml) and THF (10 ml) was added a 1 N aqueous sodium hydroxide solution (10.0 ml) at room temperature, and the mixture was stirred at 65°C for 2 days. 1 N Hydrochloric acid (10.0 ml) was added thereto at 0°C, and the resulting mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether and hexane to give (2E)-3-[4'-(2-butoxyethoxy)-4-(2-methylpyrrolidin-1-yl)-1,1'-biphenyl-3-yl]-2-methylacrylic acid (1.07 g) as yellow crystals.

m.p. 137-139°C

$^{1}$H-NMR (200 MHz, $CDCl_3$) δ 0.93 (3H, t, J=7.3 Hz), 1.12 (3H, d, J=6.0 Hz), 1.28-1.49 (2H, m), 1.52-1.96 (5H, m), 2.05-2.27 (4H, m), 2.89-3.02 (1H, m), 3.47-3.63 (3H, m), 3.75-3.89 (3H, m), 4.16 (2H, t, J=4.9 Hz), 6.92-7.00 (3H, m), 7.41-7.48 (4H, m), 7.91 (1H, s).

Elementary analysis $C_{27}H_{35}NO_4$, Calcd. C, 74.11 ; H, 8.06 ; N, 3.20 : Found. C, 74.08 ; H, 7.90 ; N, 3.10.

Reference Example 59

A mixture of 5-bromo-2-fluorobenzaldehyde (1.30 g), morpholine (0.67 g) and potassium carbonate (1.33 g) in DMF (10 ml) was stirred at 80°C for 3 days. Water was added to the reaction system, and the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 9 → 1 : 4) to give 5-bromo-2-morpholin-4-ylbenzaldehyde (720.5 mg) as pale yellow crystals.

$^{1}$H-NMR (200 MHz, $CDCl_3$) δ 3.04-3.09 (4H, m), 3.87-3.92 (4H, m), 7.02 (1H, d, J=8.4 Hz), 7.64 (1H, dd, J=8.4, 2.6 Hz), 7.92 (1H, d, J=2.6 Hz), 10.26 (1H, s).

Reference Example 60

To a suspension of sodium hydride (60%, 0.266 g) in toluene (10 ml) was added dropwise a solution of ethyl diethylphosphonoacetate (1.32 ml) in toluene (10 ml) at 0°C under an argon atmosphere. After stirring at 0°C for 30 minutes, a solution of 5-bromo-2-morpholin-4-ylbenzaldehyde (1.5 g) in toluene (40 ml) was added thereto, and the resulting mixture was heated under reflux for 3 hours. Water was added to the reaction system, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 9 → 1 : 4) to give ethyl (2E)-3-(5-bromo-2-morpholin-4-ylphenyl)acrylate (1.757 g) as pale yellow crystals.

$^{1}$H-NMR (200 MHz, $CDCl_3$) δ 1.34 (3H, t, J=7.1 Hz), 2.90-2.95 (4H, m), 3.86-3.90 (4H, m), 4.27 (2H, q, J=7.1 Hz), 6.39 (1H, d, J=16.1 Hz), 6.92 (1H, d, J=8.8 Hz), 7.45 (1H, dd, J=8.8, 2.6 Hz), 7.65 (1H, d, J=2.6 Hz), 7.97 (1H, d, J=16.1 Hz).

Elementary analysis $C_{15}H_{18}NO_3Br$, Calcd. C, 52.96 ; H, 5.33 ; N, 4.12 : Found. C, 52.97 ; H, 5.32 ; N, 4.25.

Reference Example 61

Under an argon atmosphere, a mixture of ethyl (2E)-3-(5-bromo-2-morpholin-4-ylphenyl)acrylate (1.697 g), 4-(2-butoxyethoxy)phenylboric acid (1.43 g) and potassium carbonate (1.38 g) in toluene (50 ml), ethanol (5 ml) and water (5 ml) was stirred for 30 minutes at room temperature. Tetrakis(triphenylphosphine)palladium (0.17 g) was added to the reaction system, and the mixture was heated under reflux for 6 hours. After cooling to room temperature, the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 15 → 1 : 9 → 1 : 4) to give ethyl (2E)-3-[4'-(2-butoxyethoxy)-4-morpholin-4-yl-1,1'-biphenyl-3-yl]acrylate (2.176 g) as yellow crystals.

$^1$H-NMR (200 MHz, $CDCl_3$) δ 0.93 (3H, t, J=7.2 Hz), 1.29-1.49 (5H, m), 1.55-1.69 (2H, m), 2.97-3.02 (4H, m), 3.56 (2H, t, J=6.8 Hz), 3.81 (2H, t, J=5.0 Hz), 3.89-3.94 (4H, m), 4.17 (2H, t, J=5.0 Hz), 4.28 (2H, q, J=7.1 Hz), 6.49 (1H, d, J=16.2 Hz), 7.00 (2H, d, J=8.8 Hz), 7.11 (1H, d, J=8.4 Hz), 7.49 (2H, d, J=8.8 Hz), 7.55 (1H, dd, J=8.4, 2.2 Hz), 7.72 (1H, d, J=2.2 Hz), 8.12 (1H, d, J=16.2 Hz).

Elementary analysis $C_{27}H_{35}NO_5$, Calcd. C, 71.50 ; H, 7.78 ; N, 3.09 : Found. C, 71.54 ; H, 7.95 ; N, 2.96.

Reference Example 62

To a solution of ethyl (2E)-3-[4'-(2-butoxyethoxy)-4-morpholin-4-yl-1,1'-biphenyl-3-yl]acrylate (2.07 g) in ethanol (20 ml) and THF (10 ml) was added a 1 N aqueous sodium hydroxide solution (10.0 ml) at room temperature, and the mixture was stirred at 65°C for 4 hours. 1 N Hydrochloric acid (10.0 ml) was added thereto at 0°C, and the resulting mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether and hexane to give (2E)-3-[4'-(2-butoxyethoxy)-4-morpholin-4-yl-1,1'-biphenyl-3-yl]acrylic acid (1.79 g) as yellow crystals.

m.p. 155-157°C

$^1$H-NMR (200 MHz, $CDCl_3$) δ 0.94 (3H, t, J=7.3 Hz), 1.27-1.48 (2H, m), 1.55-1.69 (2H, m), 2.98-3.03 (4H, m), 3.56 (2H, t, J=6.6 Hz), 3.82 (2H, t, J=5.0 Hz), 3.91-3.95 (4H, m), 4.18 (2H, t, J=5.0 Hz), 6.51 (1H, d, J=16.2 Hz), 7.01 (2H, d, J=8.4 Hz), 7.13 (1H, d, J=8.4 Hz), 7.50 (2H, d, J=8.4 Hz), 7.58 (1H, dd, J=8.4, 2.2 Hz), 7.74 (1H, d, J=2.2 Hz), 8.23 (1H, d, J=16.2 Hz).

Elementary analysis $C_{25}H_{31}NO_5$, Calcd. C, 70.57 ; H, 7.34 ; N, 3.29 : Found. C, 70.37 ; H, 7.53 ; N, 3.11.

Reference Example 63

A mixture of 5-bromo-2-fluorobenzaldehyde (1.35 g), methylpropylamine (0.54 g) and sodium carbonate (0.98 g) in DMSO (10 ml) and water (2.5 ml) was stirred at 125°C for 20 hours. The resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 49 → 1 : 19) to give 5-bromo-2-[methyl(propyl)amino]benzaldehyde (1.43 g) as a yellow oily material.

$^1$H-NMR (200 MHz, $CDCl_3$) δ 0.88 (3H, t, J=7.5 Hz), 1.52-1.71 (2H, m), 2.87 (3H, s), 3.04-3.11 (2H, m), 6.97 (1H, d, J=8.8 Hz), 7.53 (1H, dd, J=8.8, 2.6 Hz), 7.87 (1H, d, J=2.6 Hz), 10.17 (1H, s).

Reference Example 64

To a suspension of sodium hydride (60%, 0.27 g) in toluene (10 ml) was added dropwise a solution of ethyl diethylphosphonoacetate (1.33 ml) in toluene (10 ml) at 0°C under an argon atmosphere. After stirring at 0°C for 30 minutes, a solution of 5-bromo-2-[methyl(propyl)amino]benzaldehyde (1.43 g) in toluene (20 ml) was added thereto, and the resulting mixture was heated under reflux for 2 hours. Water was added to the reaction system, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19) to give ethyl (2E)-[5-bromo-2-[methyl(propyl)amino]phenyl]acrylate (1.86 g) as a yellow oily material.

$^1$H-NMR (200 MHz, $CDCl_3$) δ 0.89 (3H, t, J=7.4 Hz), 1.34 (3H, t, J=7.2 Hz), 1.48-1.66 (2H, m), 2.71 (3H, s), 2.85 (2H, t, J=7.3 Hz), 4.27 (2H, q, J=7.2 Hz), 6.35 (1H, d, J=16.1 Hz), 6.92 (1H, d, J=8.8 Hz), 7.39 (1H, dd, J=8.8, 2.4 Hz), 7.61 (1H, d, J=2.4 Hz), 7.95 (1H, d, J=16.1 Hz).

IR (neat) 1715, 1634, 1481, 1314, 1175, 912, 743 $cm^{-1}$

Reference Example 65

Under an argon atmosphere, a mixture of ethyl (2E)-[5-bromo-2-[methyl(propyl)amino]phenyl]acrylate (1.869 g), 4-(2-butoxyethoxy)phenylboric acid (1.73 g) and potassium carbonate (1.54 g) in toluene (60 ml), ethanol (6 ml) and water (6 ml) was stirred for 0.5 hour at room temperature. Tetrakis(triphenylphosphine)palladium (0.19 g) was added to the reaction system, and the mixture was heated under reflux for 6 hours. After cooling to room temperature, the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 9) to give ethyl (2E)-3-[4'-(2-butoxyethoxy)-4-[methyl(propyl)amino]-1,1'-biphenyl-3-yl]acrylate (2.22 g) as a yellow oily material.

$^1$H-NMR (200 MHz, $CDCl_3$) δ 0.90 (3H, t, J=7.3 Hz), 0.93 (3H, t, J=7.2 Hz), 1.28-1.46 (5H, m), 1.55-1.69 (4H, m), 2.76 (3H, s), 2.91 (2H, t, J=7.5 Hz), 3.56 (2H, t, J=6.8 Hz), 3.81 (2H, t, J=5.0 Hz), 4.17 (2H, t, J=5.0 Hz), 4.28 (2H, q, J=7.1 Hz), 6.45 (1H, d, J=16.3 Hz), 6.99 (2H, d, J=8.8 Hz), 7.11 (1H, d, J=8.4 Hz), 7.44-7.53 (3H, m), 7.69 (1H, d, J=2.2 Hz), 8.11 (1H, d, J=16.3 Hz).

IR (neat) 1709, 1632, 1607, 1489, 1302, 1273, 1246, 1177, 912, 821, 739 $cm^{-1}$

Reference Example 66

To a solution of ethyl (2E)-3-[4'-(2-butoxyethoxy)-4-[methyl(propyl)amino]-1,1'-biphenyl-3-yl]acrylate (2.22 g) in ethanol (20 ml) and THF (10 ml) was added a 1 N aqueous sodium hydroxide solution (10.0 ml) at room temperature, and the mixture was stirred at 65°C for 6 hours. 1 N Hydrochloric acid (10.0 ml) was added thereto at 0°C, and the resulting mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether and hexane to give (2E)-3-[4'-(2-butoxyethoxy)-4-[methyl (propyl)amino]-1,1'-biphenyl-3-yl]acrylic acid (1.52 g) as yellow crystals.

m.p. 95-97°C

$^1$H-NMR (200 MHz, $CDCl_3$) δ 0.90 (3H, t, J=7.3 Hz), 0.93 (3H, t, J=7.3 Hz), 1.29-1.49 (2H, m), 1.55-1.68 (4H, m), 2.78 (3H, s), 2.93 (2H, t, J=7.5 Hz), 3.56 (2H, t, J=6.6 Hz), 3.81 (2H, t, J=5.0 Hz), 4.17 (2H, t, J=5.0 Hz), 6.47 (1H, d, J=16.1 Hz), 7.00 (2H, d, J=8.6 Hz), 7.12 (1H, d, J=8.4 Hz), 7.47-7.55 (3H, m), 7.71 (1H, d, J=2.6 Hz), 8.22 (1H, d, J=16.1 Hz).

Elementary analysis $C_{25}H_{33}NO_4$, Calcd. C, 72.96 ; H, 8.08 ; N, 3.40 : Found. C, 72.70 ; H, 8.16 ; N, 3.37.

Reference Example 67

A mixture of 5-bromo-2-fluorobenzaldehyde (1.30 g), 3-methylpiperidine (1.13 ml) and potassium carbonate (1.77 g) in DMF (20 ml) was stirred at 80°C for 2 days. Water was added to the reaction system, and the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 49) to give 5-bromo-2-(3-methylpiperidin-1-yl)benzaldehyde (1.47 g) as a yellow oily material.

$^1$H-NMR (200 MHz, $CDCl_3$) δ 0.93 (3H, d, J=6.0 Hz), 0.96-1.15 (1H, m), 1.68-1.98 (4H, m), 2.49 (1H, dd, J=11.8, 10.2 Hz), 2.67-2.88 (1H, m), 3.09-3.25 (2H, m), 6.98 (1H, d, J=8.8 Hz), 7.57 (1H, dd, J=8.8, 2.6 Hz), 7.89 (1H, d, J=2.6 Hz), 10.19 (1H, s) .

IR (neat) 1682, 1586, 1480, 1383, 1231, 1177, 912, 743 $cm^{-1}$

Reference Example 68

To a suspension of sodium hydride (60%, 0.27 g) in toluene (10 ml) was added dropwise a solution of ethyl diethylphosphonoacetate (1.34 ml) in toluene (10 ml) at 0°C under an argon atmosphere. After stirring at 0°C for 30 minutes, a solution of 5-bromo-2-(3-methylpiperidin-1-yl)benzaldehyde (1.47 g) in toluene (20 ml) was added thereto, and the resulting mixture was heated under reflux for 3 hours. Water was added to the reaction system, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 49 → 1 : 19) to give ethyl (2E)-3-[5-bromo-2-(3-methylpiperidin-1-yl)phenyl] acrylate (1.78 g) as a yellow oily material.

$^1$H-NMR (200 MHz, $CDCl_3$) δ 0.93 (3H, d, J=6.6 Hz), 0.95-1.14 (1H, m), 1.34 (3H, t, J=7.2 Hz), 1.68-1.98 (4H, m), 2.27-2.37 (1H, m), 2.49-2.66 (1H, m), 2.97-3.12 (2H, m), 4.27 (2H, q, J=7.2 Hz), 6.37 (1H, d, J=16.1 Hz), 6.70 (1H, d, J=8.6 Hz), 7.40 (1H, dd, J=8.6, 2.4 Hz), 7.62 (1H, d, J=2.4 Hz), 7.94 (1H, d, J=16.1 Hz).

Reference Example 69

Under an argon atmosphere, a mixture of ethyl (2E)-3-[5-bromo-2-(3-methylpiperidin-1-yl)phenyl]acrylate (1.78 g), 4-(2-butoxyethoxy)phenylboric acid (1.56 g) and potassium carbonate (1.40 g) in toluene (50 ml), ethanol (5 ml) and water (5 ml) was stirred for 30 minutes at room temperature. Tetrakis(triphenylphosphine)palladium (0.17 g) was added to the reaction system, and the mixture was heated under reflux for 6 hours. After cooling to room temperature, the resulting mixture was extracted with ethyl acetate. The organic layer was washed with water and saturated brine, and dried over magnesium sulfate. After concentration under reduced pressure, the residue was separated and

purified by column chromatography (ethyl acetate : hexane 1 : 29 → 1 : 19 → 1 : 9) to give ethyl (2E)-3-[4'-(2-butoxyethoxy)-4-(3-methylpiperidin-1-yl)-1,1'-biphenyl-3-yl]acrylate (2.15 g) as a yellow oily material.

$^1$H-NMR (200 MHz, $CDCl_3$) δ 0.93 (3H, t, J=7.2 Hz), 0.95 (3H, d, J=6.6 Hz), 1.28-1.48 (5H, m), 1.53-2.04 (6H, m), 2.33-2.43 (2H, m), 2.52-2.73 (1H, m), 3.03-3.17 (2H, m), 3.56 (2H, t, J=6.6 Hz), 3.81 (2H, t, J=4.9 Hz), 4.17 (2H, t, J=4.9 Hz), 4.28 (2H, q, J=7.2 Hz), 6.47 (1H, d, J=16.3 Hz), 6.99 (2H, d, J=8.8 Hz), 7.08 (1H, d, J=8.4 Hz), 7.44-7.54 (3H, m), 7.70 (1H, d, J=2.2 Hz), 8.09 (1H, d, J=16.3 Hz).

IR (neat) 1711, 1632, 1607, 1489, 1248, 1235, 1177, 912, 821, 743 $cm^{-1}$

Reference Example 70

To a solution of ethyl (2E)-3-[4'-(2-butoxyethoxy)-4-(3-methylpiperidin-1-yl)-1,1'-biphenyl-3-yl]acrylate (2.17 g) in ethanol (20 ml) and THF (10 ml) was added a 1 N aqueous sodium hydroxide solution (10.0 ml) at room temperature, and the mixture was stirred at 65°C for 4 hours. 1 N Hydrochloric acid (10.0 ml) was added thereto at 0°C, and the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 4 → 1 : 2) to give (2E)-3-[4'-(2-butoxyethoxy)-4-(3-methylpiperidin-1-yl)-1,1'-biphenyl-3-yl]acrylic acid (1.48 g) as yellow crystals.

m.p. 125-127°C

$^1$H-NMR (200 MHz, $CDCl_3$) δ 0.93 (3H, t, J=7.4 Hz), 0.95 (3H, d, J=6.6 Hz), 1.30-2.02 (9H, m), 2.33-2.44 (1H, m), 2.52-2.78 (1H, m), 3.03-3.18 (2H, m), 3.56 (2H, t, J=6.6 Hz), 3.81 (2H, t, J=4.9 Hz), 4.17 (2H, t, J=4.9 Hz), 6.49 (1H, d, J=16.2 Hz), 6.99 (2H, d, J=8.6 Hz), 7.09 (1H, d, J=8.6 Hz), 7.46-7.56 (3H, m), 7.71 (1H, d, J=2.2 Hz), 8.19 (1H, d, J=16.2 Hz).

Elementary analysis $C_{27}H_{35}NO_4$, Calcd. C, 74.11 ; H, 8.06 ; N, 3.20 : Found. C, 74.41 ; H, 7.94 ; N, 2.89.

Reference Example 71

A mixture of 5-bromo-2-fluorobenzaldehyde (1.50 g), 2-methylpiperidine (1.74 ml) and sodium carbonate (1.57 g) in DMSO (20 ml) and water (5 ml) was stirred at 110°C for 2.5 days. Water was added to the reaction system, and the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 49) to give 5-bromo-2-(2-methylpiperidin-1-yl)benzaldehyde (1.365 g) as a yellow oily material.

$^1$H-NMR (200 MHz, $CDCl_3$) δ 0.91 (3H, d, J=6.2 Hz), 1.37-1.59 (2H, m), 1.63-1.97 (4H, m), 2.72-2.86 (1H, m), 2.99-3.25 (2H, m), 7.11 (1H, d, J=8.6 Hz), 7.62 (1H, dd, J=8.6, 2.6 Hz), 7.92 (1H, d, J=2.6 Hz), 10.40 (1H, s).

IR (neat) 1682, 1584, 1474, 1370, 1256, 1175, 876 $cm^{-1}$

Reference Example 72

To a suspension of sodium hydride (60%, 0.23 g) in toluene (10 ml) was added dropwise a solution of ethyl diethylphosphonoacetate (1.15 ml) in toluene (10 ml) at 0°C under an argon atmosphere. After stirring at 0°C for 30 minutes, a solution of 5-bromo-2-(2-methylpiperidin-1-yl)benzaldehyde (1.365 g) in toluene (20 ml) was added thereto, and the resulting mixture was heated under reflux for 2 hours. Water was added to the reaction system, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 49 → 1 : 19) to give ethyl (2E)-3-[5-bromo-2-(2-methylpiperidin-1-yl)phenyl] acrylate (1.666 g) as a yellow oily material.

$^1$H-NMR (300 MHz, $CDCl_3$) δ 0.85 (3H, d, J=6.3 Hz), 1.34 (3H, t, J=7.1 Hz), 1.41-1.54 (2H, m), 1.59-1.94 (4H, m), 2.54-2.62 (1H, m), 2.89-2.97 (1H, m), 3.02-3.12 (1H, m), 4.21-4.32 (2H, m), 6.37 (1H, d, J=16.2 Hz), 6.98 (1H, d, J=8.6 Hz), 7.41 (1H, dd, J=8.6, 2.4 Hz), 7.66 (1H, d, J=2.4 Hz), 8.11 (1H, d, J=16.2 Hz).

IR (neat) 1713, 1634, 1478, 1312, 1179, 912, 743 $cm^{-1}$

Reference Example 73

Under an argon atmosphere, a mixture of ethyl (2E)-3-[5-bromo-2-(2-methylpiperidin-1-yl)phenyl]acrylate (1.666 g), 4-(2-butoxyethoxy)phenylboric acid (1.35 g) and potassium carbonate (1.31 g) in toluene (50 ml), ethanol (5 ml) and water (5 ml) was stirred for 30 minutes at room temperature. Tetrakis(triphenylphosphine)palladium (0.16 g) was added to the reaction system, and the mixture was heated under reflux for 6 hours. After cooling to room temperature, the resulting mixture was extracted with ethyl acetate. The organic layer was washed with water and

saturated brine, and dried over magnesium sulfate. After concentration under reduced pressure, the residue was separated and purified by column chromatography (ethyl acetate : hexane 1 : 19 → 1 : 9) to give ethyl (2E)-3-[4'-(2-butoxyethoxy)-4-(2-methylpiperidin-1-yl)-1,1'-biphenyl-3-yl]acrylate (2.137 g) as yellow crystals.

$^1$H-NMR (200 MHz, $CDCl_3$) δ 0.90 (3H, d, J=6.2 Hz), 0.93 (3H, t, J=7.1 Hz), 1.28-1.98 (13H, m), 2.56-2.72 (1H, m), 2.91-3.21 (2H, m), 3.56 (2H, t, J=6.6 Hz), 3.81 (2H, t, J=4.9 Hz), 4.14-4.33 (4H, m), 6.49 (1H, d, J=16.2 Hz), 6.99 (2H, d, J=8.8 Hz), 7.16 (1H, d, J=8.0 Hz), 7.17-7.55 (3H, m), 7.74 (1H, d, J=2.2 Hz), 8.27 (1H, d, J=16.2 Hz).

IR (neat) 1711, 1632, 1609, 1485, 1283, 1246, 1175, 1125, 912, 742 $cm^{-1}$

Reference Example 74

To a solution of ethyl (2E)-3-[4'-(2-butoxyethoxy)-4-(2-methylpiperidin-1-yl)-1,1'-biphenyl-3-yl]acrylate (2.137 g) in ethanol (20 ml) and THF (10 ml) was added a 1 N aqueous sodium hydroxide solution (10.0 ml) at room temperature, and the mixture was stirred at 60°C for 4 hours. 1 N Hydrochloric acid (10.0 ml) was added thereto at 0°C, and the resulting mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether and hexane to give (2E)-3-[4'-(2-butoxyethoxy)-4-(2-methylpiperidin-1-yl)-1,1'-biphenyl-3-yl]acrylic acid (1.26 g) as yellow crystals.

m.p. 106-108°C

$^1$H-NMR (200 MHz, $CDCl_3$) δ 0.90-0.97 (6H, m), 1.29-2.01 (10H, m), 2.59-2.75 (1H, m), 2.93-3.24 (2H, m), 3.56 (2H, t, J=6.6 Hz), 3.82 (2H, t, J=5.0 Hz), 4.17 (2H, t, J=5.0 Hz), 6.51 (1H, d, J=16.3 Hz), 7.00 (2H, d, J=8.8 Hz), 7.19 (1H, d, J=8.4 Hz), 7.49-7.58 (3H, m), 7.76 (1H, d, J=2.4 Hz), 8.38 (1H, d, J=16.3 Hz).

Elementary analysis $C_{27}H_{35}NO_4$, Calcd. C, 74.11 ; H, 8.06 ; N, 3.20 : Found. C, 74.08 ; H, 8.08 ; N, 3.04.

Reference Example 75

A mixture of 5-bromo-2-fluorobenzaldehyde (2.50 g), 3-methylpiperidine (3.32 g) and potassium carbonate (5.1 g) in DMF (30 ml) was stirred at 80°C for 2 days. Water was added to the reaction system, and the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 49 → 1 : 19) to give 5-bromo-2-(3-methylpyrrolidin-1-yl)benzaldehyde (2.37 g) as a yellow oily material.

$^1$H-NMR (200 MHz, $CDCl_3$) δ 1.13 (3H, d, J=6.6 Hz), 1.53-1.72 (1H, m), 2.05-2.19 (1H, m), 2.23-2.44 (1H, m), 3.02-3.11 (1H, m), 3.25-3.57 (3H, m), 6.69 (1H, d, J=9.2 Hz), 7.41 (1H, dd, J=9.2, 2.6 Hz), 7.79 (1H, d, J=2.6 Hz), 10.02 (1H, s).

Reference Example 76

To a suspension of sodium hydride (60%, 0.21 g) in toluene (10 ml) was added dropwise a solution of ethyl diethylphosphonoacetate (1.04 ml) in toluene (10 ml) at 0°C under an argon atmosphere. After stirring at 0°C for 30 minutes, a solution of 5-bromo-2-(3-methylpyrrolidin-1-yl)benzaldehyde (1.17 g) in toluene (10 ml) was added thereto, and the resulting mixture was heated under reflux for 2 hours. Water was added to the reaction system, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19) to give ethyl (2E)-3-[5-bromo-2-(3-methylpyrrolidin-1-yl)phenyl]acrylate (1.32 g) as a yellow oily material.

$^1$H-NMR (200 MHz, $CDCl_3$) δ 1.10 (3H, d, J=6.6 Hz), 1.33 (3H, t, J=7.1 Hz), 1.42-1.66 (1H, m), 1.99-2.15 (1H, m), 2.23-2.43 (1H, m), 2.90-2.98 (1H, m), 3.21-3.47 (3H, m), 4.25 (2H, q, J=7.1 Hz), 6.20 (1H, d, J=16.0 Hz), 6.67 (1H, d, J=8.8 Hz), 7.28 (1H, dd, J=8.8, 2.6 Hz), 7.48 (1H, d, J=2.6 Hz), 7.93 (1H, d, J=16.0 Hz).

IR (neat) 1713, 1626, 1474, 1316, 1175, 912, 741 $cm^{-1}$

Reference Example 77

Under an argon atmosphere, a mixture of ethyl (2E)-3-[5-bromo-2-(3-methylpyrrolidin-1-yl)phenyl]acrylate (1.32 g), 4-(2-butoxyethoxy)phenylboric acid (1.11 g) and potassium carbonate (1.08 g) in toluene (40 ml), ethanol (4 ml) and water (4 ml) was stirred for 30 minutes at room temperature. Tetrakis(triphenylphosphine)palladium (0.13 g) was added to the reaction system, and the mixture was heated under reflux for 6 hours. After cooling to room temperature, the resulting mixture was extracted with ethyl acetate. The organic layer was washed with water and saturated brine, and dried over magnesium sulfate. After concentration under reduced pressure, the residue was separated and

purified by column chromatography (ethyl acetate : hexane 1 : 19 → 1 : 9) to give ethyl (2E)-3-[4'-(2-butoxyethoxy)-4-(3-methylpyrrolidin-1-yl)-1,1'-biphenyl-3-yl]acrylate (761 mg) as a yellow oily material.

$^1$H-NMR (200 MHz, $CDCl_3$) δ 0.93 (3H, t, J=7.3 Hz), 1.12 (3H, d, J=6.6 Hz), 1.28-1.48 (5H, m), 1.51-1.68 (3H, m), 1.99-2.20 (1H, m), 2.24-2.47 (1H, m), 2.97-3.05 (1H, m), 3.26-3.59 (5H, m), 3.81 (2H, t, J=4.9 Hz), 4.16 (2H, t, J=4.9 Hz), 4.26 (2H, q, J=7.2 Hz), 6.30 (1H, d, J=15.8 Hz), 6.87 (1H, d, J=8.4 Hz), 6.97 (2H, d, J=8.6 Hz), 7.41-7.48 (3H, m), 7.59 (1H, d, J=2.6 Hz), 8.08 (1H, d, J=15.8 Hz).

IR (neat) 1709, 1607, 1495, 1478, 1300, 1246, 1175, 912, 743 $cm^{-1}$

Reference Example 78

To a solution of ethyl (2E)-3-[4'-(2-butoxyethoxy)-4-(3-methylpyrrolidin-1-yl)-1,1'-biphenyl-3-yl]acrylate (761 mg) in ethanol (10 ml) and THF (5 ml) was added a 1 N aqueous sodium hydroxide solution (4.0 ml) at room temperature, and the mixture was stirred at 65°C for 6 hours. 1 N Hydrochloric acid (4.0 ml) was added thereto at 0°C, and the resulting mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether and hexane to give (2E)-3-[4'-(2-butoxyethoxy)-4-(3-methylpyrrolidin-1-yl)-1,1'-biphenyl-3-yl]acrylic acid (596 mg) as yellow crystals.

m.p. 136-139°C

$^1$H-NMR (200 MHz, $CDCl_3$) δ 0.93 (3H, t, J=7.3 Hz), 1.13 (3H, d, J=6.6 Hz), 1.31-1.49 (2H, m), 1.53-1.68 (3H, m), 2.02-2.19 (1H, m), 2.25-2.47 (1H, m), 2.99-3.07 (1H, m), 3.28-3.59 (5H, m), 3.81 (2H, t, J=4.9 Hz), 4.16 (2H, t, J=4.9 Hz), 6.30 (1H, d, J=15.8 Hz), 6.88 (1H, d, J=8.8 Hz), 6.98 (2H, d, J=8.6 Hz), 7.43-7.49 (3H, m), 7.61 (1H, d, J=2.2 Hz), 8.19 (1H, d, J=15.8 Hz).

Reference Example 79

To a suspension of sodium hydride (60%, 0.21 g) in toluene (10 ml) was added dropwise a solution of ethyl 2-(diethylphosphono)propionate (1.28 g) in toluene (10 ml) at 0°C under an argon atmosphere. After stirring at 0°C for 1 hour, a solution of 5-bromo-2-(3-methylpyrrolidin-1-yl)benzaldehyde (1.20 g) in toluene (20 ml) was added thereto, and the resulting mixture was heated under reflux for 6 hours. Water was added to the reaction system, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 9) to give ethyl (2E)-3-[5-bromo-2-(3-methylpyrrolidin-1-yl)phenyl]-2-methylacrylate (1.49 g) as yellow crystals.

$^1$H-NMR (200 MHz, $CDCl_3$) δ 1.08 (3H, d, J=7.0 Hz), 1.34 (3H, t, J=7.1 Hz), 1.44-1.62 (1H, m), 1.95-2.10 (4H, m), 2.19-2.39 (1H, m), 2.80-2.89 (1H, m), 3.10-3.38 (3H, m), 4.26 (2H, q, J=7.1 Hz), 6.64 (1H, d, J=8.8 Hz), 7.20-7.28 (2H, m), 7.67 (1H, s).

Reference Example 80

Under an argon atmosphere, a mixture of ethyl (2E)-3-[5-bromo-2-(3-methylpyrrolidin-1-yl)phenyl]-2-methylacrylate (1.49 g), 4-(2-butoxyethoxy)phenylboric acid (1.21 g) and potassium carbonate (1.17 g) in toluene (40 ml), ethanol (4 ml) and water (4 ml) was stirred for 30 minutes at room temperature. Tetrakis(triphenylphosphine)palladium (0.15 g) was added to the reaction system, and the mixture was heated under reflux for 6 hours. After cooling to room temperature, the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 9) to give ethyl (2E)-3-[4'-(2-butoxyethoxy)-4-(3-methylpyrrolidin-1-yl)-1,1'-biphenyl-3-yl]-2-methylacrylate (1.4467 g) as a yellow oily material.

$^1$H-NMR (200 MHz, $CDCl_3$) δ 0.93 (3H, t, J=7.1 Hz), 1.10 (3H, d, J=6.6 Hz), 1.27-1.48 (6H, m), 1.54-1.68 (2H, m), 1.96-2.15 (4H, m), 2.21-2.45 (1H, m), 2.88-2.96 (1H, m), 3.17-3.45 (3H, m), 3.55 (2H, t, J=6.6 Hz), 3.80 (2H, t, J=5.0 Hz), 4.15 (2H, t, J=5.0 Hz), 4.27 (2H, q, J=7.2 Hz), 6.83 (1H, d, J=8.8 Hz), 6.97 (2H, d, J=8.8 Hz), 7.32-7.49 (4H, m), 7.83 (1H, s) .

Reference Example 81

To a solution of ethyl (2E)-3-[4'-(2-butoxyethoxy)-4-(3-methylpyrrolidin-1-yl)-1,1'-biphenyl-3-yl]-2-methylacrylate (1.45 g) in ethanol (20 ml) and THF (10 ml) was added a 1 N aqueous sodium hydroxide solution (7.5 ml) at room temperature, and the mixture was stirred at 65°C for 4 hours. 1 N Hydrochloric acid (7.5 ml) was added thereto at 0°C, and the resulting mixture was extracted with ethyl acetate. The organic layer was washed with water and saturated

brine, and dried over magnesium sulfate. After concentration under reduced pressure, the precipitated crystals were collected by filtration. The crystals were washed with diisopropyl ether and hexane to give (2E)-3-[4'-(2-butoxyethoxy)-4-(3-methylpyrrolidin-1-yl)-1,1'-biphenyl-3-yl]-2-methylacrylic acid (1.057 g) as yellow crystals.

m.p. 139-141°C

$^1$H-NMR (200 MHz, $CDCl_3$) δ 0.93 (3H, t, J=7.3 Hz), 1.11 (3H, d, J=6.6 Hz), 1.30-1.69 (5H, m), 1.95-2.16 (4H, m), 2.21-2.45 (1H, m), 2.89-2.98 (1H, m), 3.21-3.48 (3H, m), 3.55 (2H, t, J=6.6 Hz), 3.81 (2H, t, J=4.8 Hz), 4.16 (2H, t, J=4.8 Hz), 6.85 (1H, d, J=8.8 Hz), 6.97 (2H, d, J=8.8 Hz), 7.35-7.47 (4H, m), 7.98 (1H, s).

Reference Example 82

A mixture of 5-bromo-2-fluorobenzaldehyde (3.0 g), 3-pyrrolidinol (2.57 g) and sodium carbonate (3.12 g) in DMSO (30 ml) and water (6 ml) was stirred at 100°C for 4 hours, which was extracted with ethyl acetate. Next, the organic layer was washed with water and saturated brine, and dried over magnesium sulfate. After concentration under reduced pressure, the residue was separated and purified by column chromatography (ethyl acetate : hexane 1 : 1 → 1 : 2) to give 5-bromo-2-(3-hydroxypyrrolidin-1-yl)benzaldehyde (3.53 g) as a yellow oily material.

$^1$H-NMR (200 MHz, $CDCl_3$) δ 1.70 (1H, d, J=4.0 Hz), 1.99-2.28 (2H, m), 2.99-3.08 (1H, m), 3.30-3.41 (1H, m), 3.64-3.77 (2H, m), 4.55-4.66 (1H, m), 6.75 (1H, d, J=9.1 Hz), 7.46 (1H, dd, J=9.1, 2.5 Hz), 7.79 (1H, d, J=2.5 Hz), 9.99 (1H, s).

IR (neat) 3347, 1661, 1593, 1489, 1472, 1408, 1179, 912, 741 $cm^{-1}$

Reference Example 83

To a solution of 5-bromo-2-(3-hydroxypyrrolidin-1-yl)benzaldehyde (2.17 g) in DMF (20 ml) was added sodium hydride (60%, 0.36 g) at 0°C, and the mixture was stirred at 0°C for 1 hour. To the reaction system was added iodomethane (0.75 ml), and the mixture was stirred at room temperature for 14 hours. Water was added to the reaction system, and the resulting mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 9 → 1 : 4) to give 5-bromo-2-(3-methoxypyrrolidin-1-yl)benzaldehyde (1.0275 g) as a yellow oily material.

$^1$H-NMR (200 MHz, $CDCl_3$) δ 1.97-2.25 (2H, m), 3.04-3.13 (1H, m), 3.26-3.43 (4H, m), 3.51-3.59 (1H, m), 3.68 (1H, dd, J=11.6, 4.6 Hz), 4.02-4.10 (1H, m), 6.73 (1H, d, J=8.8 Hz), 7.44 (1H, dd, J=8.8, 2.6 Hz), 7.79 (1H, d, J=2.6 Hz), 10.00 (1H, s).

IR (neat) 1676, 1593, 1489, 1470, 1406, 1177, 1103, 912, 743 $cm^{-1}$

Reference Example 84

Under an argon atmosphere, to a suspension of sodium hydride (60%, 0.17 g) in toluene (10 ml), was added dropwise a solution of ethyl 2-(diethylphosphono)propionate (1.03 g) in toluene (10 ml) at 0°C. After stirring at 0°C for 301 hours, a solution of 5-bromo-2-(3-methoxypyrrolidin-1-yl)benzaldehyde (1.0275 g) in toluene (20 ml) was added thereto, and the mixture was heated under reflux for 3.5 hours. To the reaction system was added water, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 4) to give ethyl (2E)-3-[5-bromo-2-(3-methoxypyrrolidin-1-yl)phenyl]-2-methylacrylate (1.199 g) as a yellow oily material.

$^1$H-NMR (200 MHz, $CDCl_3$) δ 1.34 (3H, t, J=7.1 Hz), 1.99 (3H, d, J=1.4 Hz), 2.01-2.09 (2H, m), 3.10-3.23 (2H, m), 3.32-3.46 (5H, m), 3.94-4.04 (1H, m), 4.26 (2H, q, J=7.1 Hz), 6.67 (1H, d, J=8.8 Hz), 7.22-7.30 (2H, m), 7.66 (1H, s).

IR (neat) 1705, 1474, 1273, 912, 743 $cm^{-1}$

Reference Example 85

Under an argon atmosphere, a mixture of ethyl (2E)-3-[5-bromo-2-(3-methoxypyrrolidin-1-yl)phenyl]-2-methylacrylate (1.199 g), 4-(2-butoxyethoxy)phenylboric acid (0.93 g) and potassium carbonate (0.90 g) in toluene (30 ml), ethanol (3 ml) and water (3 ml) was stirred for 30 minutes at room temperature. Tetrakis(triphenylphosphine)palladium (0.11 g) was added to the reaction system, and the mixture was heated under reflux for 6 hours. After cooling to room temperature, the reaction mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 9 → 1 : 4) to give ethyl (2E)-3-[4'-(2-butoxyethoxy)-4-(3-methoxypyrrolidin-1-yl)-1,1'-biphenyl-3-yl]-2-methylacrylate (1.177 g) as a yellow oily material.

$^1$H-NMR (300 MHz, $CDCl_3$) δ 0.93 (3H, t, J=7.4 Hz), 1.32-1.45 (5H, m), 1.51-1.65 (2H, m), 1.97-2.13 (5H, m), 3.19-3.28 (2H, m), 3.33 (3H, s), 3.36-3.57 (4H, m), 3.80 (2H, t, J=5.0 Hz), 3.96-4.05 (1H, m), 4.15 (2H, t, J=5.0 Hz), 4.27 (2H, q, J=7.1 Hz), 6.87 (1H, d, J=8.1 Hz), 6.97 (2H, d, J=8.7 Hz), 7.33 (1H, d, J=2.4 Hz), 7.39-7.46 (3H, m), 7.81 (1H, s).

IR (neat) 1705, 1605, 1495, 1271, 1244, 1113, 912, 743 $cm^{-1}$

Reference Example 86

To a solution of ethyl (2E)-3-[4'-(2-butoxyethoxy)-4-(3-methoxypyrrolidin-1-yl)-1,1'-biphenyl-3-yl]-2-methylacrylate (1.177 g) in ethanol (10 ml) and THF (5 ml), was added a 1 N aqueous sodium hydroxide solution (5.0 ml) at room temperature. After stirring at 65°C for 6 hours, 1 N hydrochloric acid (5.0 ml) was added thereto at 0°C, and the mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether and hexane to give (2E)-3-[4'-(2-butoxyethoxy)-4-(3-methoxypyrrolidin-1-yl)-1,1'-biphenyl-3-yl]-2-methylacrylic acid (967 mg) as yellow crystals.

m.p. 144-145°C

$^1$H-NMR (300 MHz, $CDCl_3$) δ 0.93 (3H, t, J=7.2 Hz), 1.33-1.45 (2H, m), 1.56-1.65 (2H, m), 2.02-2.12 (5H, m), 3.18-3.29 (2H, m), 3.34 (3H, s), 3.40-3.57 (4H, m), 3.80 (2H, t, J=5.0 Hz), 3.99-4.06 (1H, m), 4.15 (2H, t, J=5.0 Hz), 6.88 (1H, d, J=8.7 Hz), 6.96 (2H, d, J=8.7 Hz), 7.35 (1H, d, J=1.8 Hz), 7.39-7.45 (3H, m), 7.94 (1H, s).

Elementary analysis $C_{27}H_{35}NO_5$, Calcd. C, 71.50 ; H, 7.78 ; N, 3.09 : Found. C, 71.63 ; H, 7.78 ; N, 3.03.

Reference Example 87

To a solution of 5-bromo-2-(3-hydroxypyrrolidin-1-yl)benzaldehyde (3.53 g) in pyridine (20 ml), was added acetic anhydride (2.5 ml) at 0°C. The mixture was stirred for 4 days at room temperature and concentrated under reduced pressure. Water was added to the residue, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 9 → 1 : 4 → 1 : 2) to give 5-bromo-2-(3-acetoxypyrrolidin-1-yl)benzaldehyde (4.15 g) as a yellow oily material.

$^1$H-NMR (200 MHz, $CDCl_3$) δ 2.04 (3H, s), 2.13-2.31 (2H, m), 3.06-3.13 (1H, m), 3.30-3.40 (1H, m), 3.59-3.72 (1H, m), 3.82 (1H, dd, J=12.1, 4.7 Hz), 5.31-5.44 (1H, m), 6.74 (1H, d, J=8.8 Hz), 7.48 (1H, dd, J=8.8, 2.6 Hz), 7.80 (1H, d, J=2.6 Hz), 9.98 (1H, s).

Reference Example 88

Under an argon atmosphere, a mixture of 5-bromo-2-(3-acetoxypyrrolidin-1-yl)benzaldehyde (1.0 g) and tert-butyl 2-(triphenylphosphoranylidene)propionate (1.88 g) in toluene (10 ml) was heated under reflux for 2 hours. To the reaction system was added water, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 9) to give tert-butyl (2E)-3-[2-[3-(acetoxy)pyrrolidin-1-yl]-5-bromophenyl]-2-methylacrylate (1.21 g) as a pale yellow oily material.

$^1$H-NMR (200 MHz, $CDCl_3$) δ 1.53 (9H, s), 1.95 (3H, d, J=1.2 Hz), 1.98-2.31 (5H, m), 3.09-3.28 (2H, m), 3.36-3.58 (2H, m), 5.25-5.36 (1H, m), 6.68 (1H, d, J=8.6 Hz), 7.26-7.31 (2H, m), 7.54 (1H, s).

Reference Example 89

Under an argon atmosphere, a mixture of tert-butyl (2E)-3-[2-[3-(acetoxy)pyrrolidin-1-yl]-5-bromophenyl]-2-methylacrylate (5.37 g), 4-(2-butoxyethoxy)phenylboric acid (3.92 g) and potassium carbonate (3.50 g) in toluene (130 ml), ethanol (13 ml) and water (13 ml) was stirred for 30 minutes at room temperature. Tetrakis(triphenylphosphine) palladium (0.44 g) was added to the reaction system, and the mixture was heated under reflux for 6 hours. After cooling to room temperature, the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 9 → 1 : 4) to give tert-butyl (2E)-3-[4-[3-(acetoxy)pyrrolidin-1-yl-4'-(2-butoxyethoxy)]-1,1'-biphenyl-3-yl]acrylate (5.66 g) as a yellow oily material.

$^1$H-NMR (200 MHz, $CDCl_3$) δ 0.93 (3H, t, J=7.2 Hz), 1.30-1.41 (2H, m), 1.55-1.66 (11H, m), 2.01-2.38 (8H, m), 3.18-3.65 (6H, m), 3.80 (2H, t, J=4.9 Hz), 4.15 (2H, t, J=4.9 Hz), 5.26-5.38 (1H, m), 6.87 (1H, d, J=8.4 Hz), 6.97 (2H, d, J=8.8 Hz), 7.35-7.47 (4H, m), 7.69 (1H, s).

IR (neat) 1740, 1703, 1493, 1478, 1277, 1246, 1123 $cm^{-1}$

Reference Example 90

To a solution of tert-butyl (2E)-3-[4-[3-(acetoxy)pyrrolidin-1-yl-4'-(2-butoxyethoxy)]-1,1'-biphenyl-3-yl]acrylate (2.0 g) in ethyl acetate (20 ml) was added 4 N hydrochloric acid (in ethyl acetate, 5.0 ml) at 0°C. After stirring for 14 hours at room temperature, 4 N hydrochloric acid (in ethyl acetate, 10.0 ml) was further added thereto, and the mixture was stirred for 1 hour. To the reaction system was added water, and the mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether to give (2E)-3-[4-[3-(acetoxy)pyrrolidin-1-yl-4'-(2-butoxyethoxy)]-1,1'-biphenyl-3-yl]acrylic acid (1.41 g) as yellow crystals.

m.p. 135-137°C

$^1$H-NMR (200 MHz, $CDCl_3$) δ 0.93 (3H, t, J=7.2 Hz), 1.28-1.47 (2H, m), 1.55-1.69 (2H, m), 2.07-2.33 (8H, m), 3.18-3.34 (2H, m), 3.42-3.64 (4H, m), 3.80 (2H, t, J=5.0 Hz), 4.16 (2H, t, J=5.0 Hz), 5.27-5.38 (1H, m), 6.90 (1H, d, J=8.4 Hz), 6.98 (2H, d, J=8.8 Hz), 7.38-7.47 (4H, m), 7.94 (1H, s).

Reference Example 91

A mixture of 5-bromo-2-fluorobenzonitrile (1.0 g), pyrazole (0.34 g) and potassium carbonate (0.76 g) in DMSO (10 ml) was stirred for 6 hours at 100°C. To the reaction system was added water, and the mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether and hexane to give 5-bromo-2-(1H-pyrazol-1-yl)benzonitrile (1.048 g) as colorless crystals.

$^1$H-NMR (200 MHz, $CDCl_3$) δ 6.56 (1H, dd, J=2.4, 2.1 Hz), 7.70 (1H, d, J=8.7 Hz), 7.79-7.83 (2H, m), 7.89 (1H, d, J=2.1 Hz), 8.13 (1H, d, J=2.4 Hz).

IR (KBr) 2232, 1526, 1491, 1399, 934, 750 $cm^{-1}$

Reference Example 92

Under an argon atmosphere, a mixture of 5-bromo-2-(1H-pyrazol-1-yl)benzonitrile (2.50 g), 4-(2-butoxyethoxy)phenylboric acid (2.88 g) and potassium carbonate (2.79 g) in toluene (100 ml), ethanol (10 ml) and water (10 ml) was stirred for 30 minutes at room temperature. Tetrakis(triphenylphosphine)palladium (0.35 g) was added to the reaction system, and the mixture was heated under reflux for 7 hours. After cooling to room temperature, the reaction mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 4) to give 4'-(2-butoxyethoxy)-4-(1H-pyrazol-1-yl)-1,1'-biphenyl-3-carbonitrile (3.25 g) as a pale yellow oily material.

$^1$H-NMR (200 MHz, $CDCl_3$) δ 0.94 (3H, t, J=7.2 Hz), 1.30-1.50 (2H, m), 1.54-1.69 (2H, m), 3.56 (2H, t, J=6.6 Hz), 3.82 (2H, t, J=5.0 Hz), 4.19 (2H, t, J=5.0 Hz), 6.56 (1H, t, J=2.6 Hz), 7.05 (2H, d, J=9.0 Hz), 7.53 (2H, d, J=9.0 Hz), 7.80-7.94 (4H, m), 8.17 (1H, d, J=2.6 Hz).

IR (neat) 2230, 1609, 1518, 1499, 1397, 1252, 1125, 936, 910, 826, 737 $cm^{-1}$

Reference Example 93

Under an argon atmosphere, to a solution of 4'-(2-butoxyethoxy)-4-(1H-pyrazol-1-yl)-1,1'-biphenyl-3-carbonitrile (3.25 g) in toluene (100 ml) was added dropwise diisobutylaluminum hydride (in 1.0 M toluene, 14.0 ml) at 0°C. After stirring for 2 hours at room temperature, diisobutylaluminum hydride (in 1.0 M toluene, 9.0 ml) was added dropwise at room temperature. After stirring the mixture for 30 minutes at room temperature, an aqueous ammonium chloride solution was added thereto. The precipitates were removed by filtration, and the filtrate was extracted with ethyl acetate. The organic layer was washed with saturated brine, and dried over magnesium sulfate. After concentration under reduced pressure, the residue was separated and purified by column chromatography (ethyl acetate : hexane 1 : 4 → 1 : 3 → 1 : 2) to give 4'-(2-butoxyethoxy)-4-(1H-pyrazol-1-yl)-1,1'-biphenyl-3-carbaldehyde (788 mg) as a pale yellow oily material.

$^1$H-NMR (200 MHz, $CDCl_3$) δ 0.94 (3H, t, J=7.1 Hz), 1.30-1.48 (2H, m), 1.51-1.68 (2H, m), 3.56 (2H, t, J=6.6 Hz), 3.82 (2H, t, J=5.0 Hz), 4.19 (2H, t, J=5.0 Hz), 6.57 (1H, t, J=2.2 Hz), 7.04 (2H, d, J=8.8 Hz), 7.53-7.61 (3H, m), 7.82-7.89 (3H, m), 8.20 (1H, d, J=2.2 Hz), 10.08 (1H, s).

IR (neat) 1688, 1609, 1512, 1397, 1252, 1184, 1128, 937, 828 $cm^{-1}$

Reference Example 94

Under an argon atmosphere, to a suspension of sodium hydride (60%, 0.11 g) in toluene (10 ml) was added dropwise a solution of ethyl 2-(diethylphosphono)propionate (0.67 g) in toluene (10 ml) at 0°C. After stirring for 1 hour at 0°C, a solution of 4'-(2-butoxyethoxy)-4-(1H-pyrazol-1-yl)-1,1'-biphenyl-3-carbaldehyde (788 mg) in toluene (10 ml) was added thereto, and the mixture was heated under reflux for 4 hours. To the reaction system was added water, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 4) to give ethyl (2E)-3-[4'-(2-butoxyethoxy)-4-(1H-pyrazol-1-yl)-1,1'-biphenyl-3-yl]-2-methylacrylate (842 g) as pale yellow crystals.

$^1$H-NMR (200 MHz, $CDCl_3$) δ 0.94 (3H, t, J=7.4 Hz), 1.26-1.50 (5H, m), 1.55-1.69 (2H, m), 2.03 (3H, d, J=1.6 Hz), 3.56 (2H, t, J=6.6 Hz), 3.82 (2H, t, J=4.9 Hz), 4.16-4.29 (4H, m), 6.45 (1H, t, J=1.8 Hz), 7.03 (2H, d, J=8.8 Hz), 7.52-7.58 (4H, m), 7.63-7.65 (3H, m), 7.74 (1H, d, J=1.8 Hz).

IR (neat) 1709, 1609, 1514, 1493, 1399, 1271, 1246, 1115, 912, 745 $cm^{-1}$

Reference Example 95

To a solution of ethyl (2E)-3-[4'-(2-butoxyethoxy)-4-(1H-pyrazol-1-yl)-1,1'-biphenyl-3-yl]-2-methylacrylate (790 mg) in ethanol (20 ml) and THF (10 ml) was added a 1 N aqueous sodium hydroxide solution (3.5 ml) at room temperature. After stirring for 4 hours at 50°C, 1 N hydrochloric acid (3.5 ml) was added thereto at 0°C, and the mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether to give (2E)-3-[4'-(2-butoxyethoxy)-4-(1H-pyrazol-1-yl)-1,1'-biphenyl-3-yl]-2-methylacrylic acid (700 mg) as yellow crystals.

m.p. 132-134°C

$^1$H-NMR (300 MHz, $CDCl_3$) δ 0.94 (3H, t, J=7.4 Hz), 1.34-1.46 (2H, m), 1.57-1.66 (2H, m), 2.05 (3H, d, J=1.5 Hz), 3.56 (2H, t, J=6.6 Hz), 3.82 (2H, t, J=4.9 Hz), 4.18 (2H, q, J=4.9 Hz), 6.45 (1H, t, J=1.8 Hz), 7.02 (2H, d, J=8.7 Hz), 7.52 (2H, d, J=8.7 Hz), 7.58 (1H, s), 7.62-7.66 (4H, m), 7.74 (1H, d, J=1.8 Hz).

Reference Example 96

Under an argon atmosphere, to a suspension of sodium hydride (1.44 g) in DMF (150 ml) was added dropwise a solution of 1,2,3,4-tetrahydroquinoline (4.0 g) in DMF (20 ml) at 0°C. After stirring for 1 hour at 0°C, iodomethane (2.06 ml) was added to the reaction system at 0°C, and the mixture was stirred for 12 hours at room temperature. To the reaction system was added water, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19) to give 1-methyl-1,2,3,4-tetrahydroquinoline (3.64 g) as a colorless oily material.

$^1$H-NMR (300 MHz, $CDCl_3$) δ 1.94-2.02 (2H, m), 2.76 (2H, t, J=6.6 Hz), 2.88 (3H, s), 3.21 (2H, t, J=5.7 Hz), 6.57-6.62 (2H, m), 6.93-6.96 (1H, m), 7.04-7.25 (1H, m).

Reference Example 97

To a solution of 1-methyl-1,2,3,4-tetrahydroquinoline (3.64 g) in dichloromethane (50 ml) was added tetrabutylammonium tribromide (11.92 g) at 0°C. The mixture was stirred for 30 minutes at 0°C and for 18 hours at room temperature. Water was added to the reaction system, and the mixture was extracted with dichloromethane. The organic layer was washed with an aqueous sodium thiosulfate solution and saturated brine, and dried over magnesium sulfate. After concentration under reduced pressure, the residue was separated and purified by column chromatography (ethyl acetate : hexane 1 : 19) to give 6-bromo-1-methyl-1,2,3,4-tetrahydroquinoline (4.65 g) as a pale yellow oily material.

$^1$H-NMR (200 MHz, $CDCl_3$) δ 1.89-2.01 (2H, m), 2.73 (2H, t, J=6.4 Hz), 2.85 (3H, s), 3.20 (2H, t, J=5.7 Hz), 6.43 (1H, d, J=8.6 Hz), 7.02-7.05 (1H, m), 7.10-7.16 (1H, m).

IR (neat) 1501, 1323, 1208, 912, 740 $cm^{-1}$

Reference Example 98

To a solution of 6-bromo-1-methyl-1,2,3,4-tetrahydroquinoline (4.65 g) in DMF (40 ml) was added chloromethylenedimethylammonium chloride (3.95 g) at room temperature, and the mixture was stirred for 1 hour at 65°C. The reaction mixture was added to ice water. After neutralization using potassium carbonate, the mixture was extracted

with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 9) to give 6-bromo-1-methyl-1,2,3,4-tetrahydroquinoline-8-carbaldehyde (2.82 g) as a yellow oily material.

$^1$H-NMR (200 MHz, $CDCl_3$) δ 1.87-1.99 (2H, m), 2.75 (2H, t, J=6.2 Hz), 3.04 (3H, s), 3.27 (2H, t, J=5.6 Hz), 7.20 (1H, d, J=2.6 Hz), 7.63 (1H, d, J=2.6 Hz), 9.94 (1H, s).

Reference Example 99

Under an argon atmosphere, to a suspension of sodium hydride (60%, 0.25 g) in toluene (10 ml) was added dropwise a solution of ethyl diethylphosphonoacetate (1.22 ml) in toluene (10 ml) at 0°C. After stirring for 30 minutes at 0°C, 6-bromo-1-methyl-1,2,3,4-tetrahydroquinoline-8-carbaldehyde (1.30 g) in toluene (20 ml) was added dropwise thereto, and the mixture was heated under reflux for 2 hours. To the reaction system was added water, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 9) to give ethyl (2E)-3-(6-bromo-1-methyl-1,2,3,4-tetrahydroquinolin-8-yl)acrylate (1.49 g) as a pale yellow oily material.

$^1$H-NMR (200 MHz, $CDCl_3$) δ 1.33 (3H, t, J=7.1 Hz), 1.76-1.90 (2H, m), 2.71-2.78 (5H, m), 3.10-3.15 (2H, m), 4.26 (2H, q, J=7.1 Hz), 6.32 (1H, d, J=16.1 Hz), 7.13 (1H, d, J=2.2 Hz), 7.41 (1H, d, J=2.2 Hz), 7.84 (1H, d, J=16.1 Hz).

IR (neat) 1711, 1632, 1451, 1470, 1416, 1310, 1265, 1233, 1175, 1040, 912, 743 $cm^{-1}$

Reference Example 100

Under an argon atmosphere, a mixture of ethyl (2E)-3-(6-bromo-1-methyl-1,2,3,4-tetrahydroquinolin-8-yl)acrylate (1.49 g), 4-(2-butoxyethoxy)phenylboric acid (1.31 g) and potassium carbonate (1.27 g) in toluene (50 ml), ethanol (5 ml) and water (5 ml) was stirred for 30 minutes at room temperature. Tetrakis(triphenylphosphine)palladium (0.26 g) was added to the reaction system, and the mixture was heated under reflux for 6 hours. After cooling to room temperature, the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 7) to give ethyl (2E)-3-[6-[4-(2-butoxyethoxy)phenyl]-1-methyl-1,2,3,4-tetrahydroquinolin-8-yl]acrylate (1.56 g) as a yellow oily material.

$^1$H-NMR (200 MHz, $CDCl_3$) δ 0.93 (3H, t, J=7.1 Hz), 1.26-1.48 (5H, m), 1.50-1.70 (2H, m), 1.79-1.97 (2H, m), 2.76-2.89 (5H, m), 3.11-3.24 (2H, m), 3.55 (2H, t, J=6.6 Hz), 3.81 (2H, t, J=5.0 Hz), 4.16 (2H, t, J=5.0 Hz), 4.20 (2H, q, J=7.2 Hz), 6.42 (1H, d, J=16.2 Hz), 6.97 (2H, d, J=8.4 Hz), 7.21-7.23 (1H, m), 7.46 (2H, d, J=8.4 Hz), 7.47-7.50 (1H, m), 7.99 (1H, d, J=16.2 Hz).

IR (neat) 1709, 1630, 1609, 1516, 1454, 1275, 1248, 1177, 1125, 1040, 910, 741 $cm^{-1}$

Reference Example 101

To a solution of ethyl (2E)-3-[6-[4-(2-butoxyethoxy)phenyl]-1-methyl-1,2,3,4-tetrahydroquinolin-8-yl]acrylate (1.56 g) in ethanol (20 ml) and THF (10 ml) was added a 1 N aqueous sodium hydroxide solution (8.0 ml) at room temperature. After stirring for 20 hours at 65°C, 1 N hydrochloric acid (8.0 ml) was added thereto at 0°C, and the mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether and hexane to give (2E)-3-[6-[4-(2-butoxyethoxy)phenyl]-1-methyl-1,2,3,4-tetrahydroquinolin-8-yl]acrylic acid (1.37 g) as yellow crystals.

m.p. 128-129°C

$^1$H-NMR (200 MHz, $CDCl_3$) δ 0.93 (3H, t, J=7.3 Hz), 1.29-1.48 (2H, m), 1.55-1.68 (2H, m), 1.82-1.97 (2H, m), 2.82-2.86 (5H, m), 3.16-3.21 (2H, m), 3.56 (2H, t, J=6.6 Hz), 3.81 (2H, t, J=4.9 Hz), 4.16 (2H, t, J=4.9 Hz), 6.44 (1H, d, J=16.1 Hz), 6.98 (2H, d, J=8.8 Hz), 7.25 (1H, d, J=2.0 Hz), 7.46 (2H, d, J=8.8 Hz), 7.52 (1H, d, J=2.0 Hz), 8.10 (1H, d, J=16.1 Hz).

Reference Example 102

Under an argon atmosphere, to a suspension of sodium hydride (60%, 0.27 g) in toluene (10 ml) was added dropwise a solution of ethyl 2-(diethylphosphono)propionate (1.58 g) in toluene (10 ml) at 0°C. After stirring for 1 hour at 0°C, 6-bromo-1-methyl-1,2,3,4-tetrahydroquinoline-8-carbaldehyde (1.40 g) in toluene (20 ml) was added dropwise thereto, and the mixture was heated under reflux for 4 hours. To the reaction system was added water, and the mixture

was extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 15) to give ethyl (2E)-3-(6-bromo-1-methyl-1,2,3,4-tetrahydroquinolin-8-yl)-2-methylacrylate (1.677 g) as a yellow oily material.

$^1$H-NMR (200 MHz, $CDCl_3$) δ 1.34 (3H, t, J=7.1 Hz), 1.75-1.89 (2H, m), 2.06 (3H, d, J=1.4 Hz), 2.71-2.78 (5H, m), 3.10-3.15 (2H, m), 4.27 (2H, q, J=7.1 Hz), 7.10 (1H, d, J=2.2 Hz), 7.16 (1H, d, J=2.2 Hz), 7.59 (1H, s).

IR (neat) 1707, 1449, 1412, 1264, 1240, 1223, 1111, 1044, 912, 748 $cm^{-1}$

Reference Example 103

Under an argon atmosphere, a mixture of ethyl (2E)-3-(6-bromo-1-methyl-1,2,3,4-tetrahydroquinolin-8-yl)-2-methylacrylate (1.677 g), 4-(2-butoxyethoxy)phenylboric acid (1.42 g) and potassium carbonate (1.37 g) in toluene (50 ml), ethanol (5 ml) and water (5 ml) was stirred for 30 minutes at room temperature. Tetrakis(triphenylphosphine) palladium (0.17 g) was added to the reaction system, and the mixture was heated under reflux for 6 hours. After cooling to room temperature, the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 9) to give ethyl (2E)-3-[6-[4-(2-butoxyethoxy)phenyl]-1-methyl-1,2,3,4-tetrahydroquinolin-8-yl]-2-methylacrylate (1.55 g) as a yellow oily material.

$^1$H-NMR (200 MHz, $CDCl_3$) δ 0.93 (3H, t, J=7.3 Hz), 1.27-1.47 (5H, m), 1.51-1.68 (2H, m), 1.82-1.96 (2H, m), 2.13 (3H, d, J=1.2 Hz), 2.77 (3H, s), 2.80-2.87 (2H, m), 3.15-3.20 (2H, m), 3.55 (2H, t, J=6.6 Hz), 3.80 (2H, t, J=4.9 Hz), 4.15 (2H, t, J=4.9 Hz), 4.28 (2H, q, J=7.1 Hz), 6.96 (2H, d, J=8.8 Hz), 7.19-7.29 (2H, m), 7.44 (2H, d, J=8.8 Hz), 7.74 (1H, s).

IR (neat) 1705, 1516, 1456, 1248, 1123, 912, 829 $cm^{-1}$

Reference Example 104

To a solution of ethyl (2E)-3-[6-[4-(2-butoxyethoxy)phenyl]-1-methyl-1,2,3,4-tetrahydroquinolin-8-yl]-2-methylacrylate (1.55 g) in ethanol (20 ml) and THF (10 ml) was added a 1 N aqueous sodium hydroxide solution (7.0 ml) at room temperature. After stirring for 4 hours at 65°C, 1 N hydrochloric acid (7.0 ml) was added thereto at 0°C, and the mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether and hexane to give (2E)-3-[6-[4-(2-butoxyethoxy)phenyl]-1-methyl-1,2,3,4-tetrahydroquinolin-8-yl]-2-methylacrylic acid (1.27 g) as yellow crystals.

m.p. 133-134°C

$^1$H-NMR (200 MHz, $CDCl_3$) δ 0.93 (3H, t, J=7.4 Hz), 1.28-1.47 (2H, m), 1.54-1.68 (2H, m), 1.82-1.97 (2H, m), 2.17 (3H, d, J=1.6 Hz), 2.79-2.87 (5H, m), 3.16-3.22 (2H, m), 3.55 (2H, t, J=6.6 Hz), 3.80 (2H, t, J=5.0 Hz), 4.16 (2H, t, J=5.0 Hz), 6.97 (2H, d, J=8.8 Hz), 7.21 (1H, d, J=2.2 Hz), 7.30 (1H, d, J=2.2 Hz), 7.44 (2H, d, J=8.8 Hz), 7.90 (1H, s).

Reference Example 105

5-Bromo-2-fluorobenzaldehyde (2.5 g), N-(2-methoxyethyl)methylamine (1.43 g), sodium carbonate (3.91 g) were added to dimethyl sulfoxide (40 ml) and water (20 ml), and the mixture was stirred for 6 hours at 90°C under a nitrogen atmosphere. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 6 : 1 → hexane : ethyl acetate = 1 : 1) to give 5-bromo-2-[(2-methoxyethyl)(methyl)amino]benzaldehyde (2.47 g) as a yellow oily material.

$^1$H-NMR (300 MHz, $CDCl_3$) δ 2.93 (3H, s), 3.29-3.33 (5H, m), 3.56 (2H, t, J=5.4 Hz), 7.02 (1H, d, J=9.0 Hz), 7.54 (1H, dd, J=9.0, 2.4 Hz), 7.86 (1H, d, J=2.4 Hz), 10.21 (1H, s).

Reference Example 106

To a suspension of sodium hydride (194 mg) in toluene (10 ml) was added dropwise a solution of ethyl diethylphosphonoacetate (987 mg) in toluene (10 ml) at 0°C under a nitrogen atmosphere, and then the mixture was stirred as such for 1 hour. Next, a solution of 5-bromo-2-[(2-methoxyethyl)(methyl)amino]benzaldehyde (1.0 g) in toluene (10 ml) was added dropwise thereto at 0°C under a nitrogen atmosphere, and then the resulting mixture was refluxed for 3 hours. After removing from the oil bath, water was added thereto and the mixture was extracted with ethyl acetate. The organic layer was washed with saturated brine and dried over magnesium sulfate. The solvent was

distilled off under reduced pressure, and then the resulting residue was purified by silica gel column chromatography (hexane : ethyl acetate = 6 : 1) to give ethyl (2E)-3-[5-bromo-2-[(2-methoxyethyl)(methyl)amino]phenyl]acrylate (1.03 g) as a yellow oily material.

$^1$H-NMR (300 MHz, $CDCl_3$) δ 1.34 (3H, t, J=7.2 Hz), 2.80 (3H, s), 3.12 (2H, t, J=6.0 Hz), 3.32 (3H, s), 3.54 (2H, t, J=6.0 Hz), 4.26 (2H, q, J=7.2 Hz), 6.35 (1H, d, J=16.2 Hz), 6.96 (1H, d, J=8.4 Hz), 7.39 (1H, dd, J=8.4, 2.4 Hz), 7.59 (1H, d, J=2.4 Hz), 7.94 (1H, d, J=16.2 Hz).

Reference Example 107

A suspension of ethyl (2E)-3-[5-bromo-2-[(2-methoxyethyl)(methyl)amino]phenyl]acrylate (900 mg), 4- (2-butoxyethoxy)phenylboric acid (814 mg) and potassium carbonate (945 mg) in toluene (15 ml), ethanol (1.5 ml) and water (1.5 ml) was stirred for 1 hour under an argon atmosphere. Then, tetrakis(triphenylphosphine)palladium (152 mg) was added thereto, and the resulting mixture was refluxed for 6 hours. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 19 : 1 → hexane : ethyl acetate = 5 : 1) to give ethyl (2E)-3-[4'-(2-butoxyethoxy)-4-[(2-methoxyethyl)(methyl)amino]-1,1'-biphenyl-3-yl]acrylate (1.19 g) as a yellow oily material. Ethyl (2E)-3-[4'-(2-butoxyethoxy)-4-[(2-methoxyethyl)(methyl)amino]-1,1'-biphenyl-3-yl]acrylate (980 mg) was dissolved in THF (25 ml) and methanol (25 ml). Then, a 1 N aqueous sodium hydroxide solution (8.6 ml) was added thereto, and the mixture was stirred for 3 hours at 90°C. After adding water at 0°C, the resulting mixture was neutralized with 1 N hydrochloric acid and extracted with ethyl acetate. The organic layer was washed with saturated brine and dried over magnesium sulfate. The solvent was distilled off under reduced pressure, and then the resulting residue was washed with hexane to give (2E)-3-[4'-(2-butoxyethoxy)-4-[(2-methoxyethyl)(methyl)amino]-1,1'-biphenyl-3-yl]acrylic acid (717 mg) as yellow crystals.

m.p. 86.4-87.4°C.

$^1$H-NMR (300 MHz, $CDCl_3$) δ 0.94 (3H, t, J=7.5 Hz), 1.34-1.46 (2H, m), 1.56-1.66 (2H, m), 2.87 (3H, s), 3.19 (2H, t, J=6.6 Hz), 3.37 (3H, s), 3.56 (2H, t, J=6.6 Hz), 3.61 (2H, t, J=6.0 Hz), 3.82 (2H, t, J=5.1 Hz), 4.17 (2H, t, J=5.1 Hz), 6.47 (1H, d, J=16.2 Hz), 7.01 (2H, d, J=7.8 Hz), 7.18 (1H, d, J=8.7 Hz), 7.49 (2H, d, J=7.8 Hz), 7.54 (1H, dd, J=8.7, 2.1 Hz), 7.71 (1H, d, J=2.1 Hz), 8.23 (1H, d, J=16.2 Hz).

Elementary analysis $C_{25}H_{33}NO_5$, Calcd. C, 70.23 ; H, 7.78 ; N, 3.28 : Found C, 70.08 ; H, 7.84 ; N, 3.26.

Reference Example 108

1-Methylpyrazole-4-carboxyaldehyde (1.2 g), methylamine hydrochloride (736 mg) and triethylamine (3.04 ml) were dissolved in methanol (15 ml), and then palladium carbon (10%, 0.2 g) was added thereto, and the mixture was stirred overnight under a hydrogen atmosphere. The insolubles were removed by filtration, and then the solvent was distilled off under reduced pressure. To the resulting residue were added DMSO (20 ml), water (15 ml) and sodium carbonate (3.47 g), and then a solution of 5-bromo-2-fluorobenzaldehyde (2.21 g) in DMSO (10 ml) was added dropwise thereto at 115°C under a nitrogen atmosphere. After stirring as such for 5 hours at 115°C, the reaction mixture was returned to room temperature. Water was added thereto and the mixture was extracted with ethyl acetate. The organic layer was washed with saturated brine and dried over magnesium sulfate. The solvent was distilled off under reduced pressure, and then the resulting residue was separated and purified by silica gel column chromatography (hexane : ethyl acetate = 3 : 1 → hexane : ethyl acetate = 1 : 1), which was recrystallized from hexane-ethyl acetate to give 5-bromo-2-[methyl[(1-methyl-1H-pyrazol-4-yl)methyl]amino]benzaldehyde (784 mg) as brown crystals.

m.p. 80.0-81.0°C.

$^1$H-NMR (300 MHz, $CDCl_3$) δ 2.78 (3H, s), 3.86 (3H, s), 4.14 (2H, s), 6.92 (1H, d, J=9.0 Hz), 7.18 (1H, s), 7.30 (1H, s), 7.54 (1H, dd, J=9.0, 2.7 Hz), 7.89 (1H, d, J=2.7 Hz), 10.25 (1H, s).

Elementary analysis $C_{13}H_{14}N_3OBr$, Calcd. C, 50.67 ; H, 4.58 ; N, 13.64 : Found C, 50.68 ; H, 4.48 ; N, 13.44.

Reference Example 109

To a suspension of sodium hydride (72 mg) in toluene (10 ml) was added dropwise a solution of ethyl diethylphosphonoacetate (365 mg) in toluene (10 ml) at 0°C under a nitrogen atmosphere, and then the mixture was stirred as such for 1 hour. Next, a solution of 5-bromo-2-[methyl[(1-methyl-1H-pyrazol-4-yl)methyl]amino]benzaldehyde (420 mg) in toluene (10 ml) was added dropwise thereto at 0°C under a nitrogen atmosphere, and then the resulting mixture was refluxed for 3 hours. After removing from the oil bath, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography

(hexane : ethyl acetate = 7 : 3 → ethyl acetate) and recrystallized from hexane-ethyl acetate to give ethyl (2E)-3-[5-bromo-2-[methyl[(1-methyl-1H-pyrazol-4-yl)methyl]amino]phenyl]acrylate (310 mg) as yellow crystals.

m.p. 67.0-68.0°C.

$^1$H-NMR (300 MHz, $CDCl_3$) δ 1.35 (3H, t, J=7.5 Hz), 2.65 (3H, s), 3.87 (5H, s), 4.27 (2H, q, J=7.5 Hz), 6.40 (1H, d, J=16.2 Hz), 6.86 (1H, d, J=8.7 Hz), 7.31 (1H, s), 7.34 (1H, s), 7.38 (1H, dd, J=8.7, 2.4 Hz), 7.64 (1H, d, J=2.4 Hz), 8.06 (1H, d, J=16.2 Hz).

Elementary analysis $C_{17}H_{20}N_3O_2Br$, Calcd. C, 53.98 ; H, 5.33 ; N, 11.11 : Found C, 53.94 ; H, 5.25 ; N, 10.96.

Reference Example 110

A suspension of ethyl (2E)-3-[5-bromo-2-[methyl[(1-methyl-1H-pyrazol-4-yl)methyl]amino]phenyl]acrylate (450 mg), 4-(2-butoxyethoxy)phenylboric acid (369 mg) and potassium carbonate (427 mg) in toluene (15 ml), ethanol (1.5 ml) and water (1.5 ml) was stirred for 1 hour under an argon atmosphere. Then, tetrakis(triphenylphosphine) palladium (69 mg) was added thereto, and the resulting mixture was refluxed for 6 hours. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 3 : 1 → ethyl acetate) to give ethyl (2E)-3-[4'-(2-butoxyethoxy)-4-[methyl[(1-methyl-1H-pyrazol-4-yl)methyl]amino]-1,1'-biphenyl-3-yl]acrylate (522 mg) as a yellow oily material.

$^1$H-NMR (300 MHz, $CDCl_3$) δ 0.94 (3H, t, J=7.2 Hz), 1.34-1.46 (5H, m), 1.55-1.67 (2H, m), 2.70 (3H, s), 3.56 (2H, t, J=6.6 Hz), 3.82 (2H, t, J=4.8 Hz), 3.89 (3H, s), 3.93 (2H, s), 4.17 (2H, t, J=4.8 Hz), 4.29 (2H, q, J=7.2 Hz), 6.51 (1H, d, J=13.2 Hz), 7.00 (2H, d, J=8.7 Hz), 7.07 (1H, d, J=8.4 Hz), 7.38 (1H, s), 7.40 (1H, s), 7.48-7.53 (3H, m), 7.73 (1H, d, J=2.1 Hz), 8.23 (1H, d, J=13.2 Hz).

Reference Example 111

Ethyl (2E)-3-[4'-(2-butoxyethoxy)-4-[methyl[(1-methyl-1H-pyrazol-4-yl)methyl]amino]-1,1'-biphenyl-3-yl]acrylate (495 mg) was dissolved in THF (12 ml) and methanol (12 ml). Then, a 1 N aqueous sodium hydroxide solution (4 ml) was added thereto, and the mixture was stirred for 5 hours at 90°C. After adding water at 0°C, the resulting mixture was neutralized with 1 N hydrochloric acid and extracted with ethyl acetate. The organic layer was washed with saturated brine and dried over magnesium sulfate. The solvent was distilled off under reduced pressure, and then the resulting residue was washed with hexane-diisopropyl ether to give (2E)-3-[4'-(2-butoxyethoxy)-4-[methyl[(1-methyl-1H-pyrazol-4-yl)methyl]amino]-1,1'-biphenyl-3-yl]acrylic acid (425 mg) as yellow crystals.

m.p. 125.0-127.0°C.

$^1$H-NMR (300 MHz, $CDCl_3$) δ 0.94 (3H, t, J=7.5 Hz), 1.36-1.44 (2H, m), 1.50-1.70 (2H, m), 2.72 (3H, s), 3.56 (2H, t, J=6.6 Hz), 3.82 (2H, t, J=4.5 Hz), 3.92 (5H, s), 4.17 (2H, t, J=4.5 Hz), 6.52 (1H, d, J=15.9 Hz), 7.01 (2H, d, J=8.7 Hz), 7.10 (1H, d, J=8.1 Hz), 7.37 (1H, s), 7.49-7.55 (4H, m), 7.76 (1H, d, J=2.1 Hz), 8.34 (1H, d, J=15.9 Hz).

Elementary analysis $C_{28}H_{33}N_3O_4\cdot 0.5H_2O$, Calcd. C, 69.40 ; H, 7.07 ; N, 8.67 : Found C, 69.69 ; H, 7.24 ; N, 8.87.

Reference Example 112

To a suspension of sodium hydride (52 mg) in toluene (10 ml) was added dropwise a solution of triethyl 2-phosphonopropionate (278 mg) in toluene (10 ml) at 0°C under a nitrogen atmosphere, and then the mixture was stirred as such for 1 hour. Next, a solution of 5-bromo-2-[methyl[(1-methyl-1H-pyrazol-4-yl)methyl]amino]benzaldehyde (300 mg) in toluene (10 ml) was added dropwise thereto at 0°C under a nitrogen atmosphere, and then the resulting mixture was refluxed for 3 hours. After removing from the oil bath, water was added thereto and the mixture was extracted with ethyl acetate. The organic layer was washed with saturated brine and dried over magnesium sulfate. The solvent was distilled off under reduced pressure, and then the resulting residue was purified by basic silica gel column chromatography (ethyl acetate) to give ethyl (2E)-3-[5-bromo-2-[methyl[(1-methyl-1H-pyrazol-4-yl)methyl]amino]phenyl]-2-methylacrylate (381 mg) as a brown oily material.

$^1$H-NMR (300 MHz, $CDCl_3$) δ 1.36 (3H, t, J=6.9 Hz), 2.10 (3H, d, J=1.5 Hz), 2.62 (3H, s), 3.82 (2H, s), 3.86 (3H, s), 4.28 (2H, q, J=6.9 Hz), 6.84 (1H, d, J=8.7 Hz), 7.25-7.40 (4H, m), 7.79 (1H, s) .

Reference Example 113

A suspension of ethyl (2E)-3-[5-bromo-2-[methyl[(1-methyl-1H-pyrazol-4-yl)methyl]amino]phenyl]-2-methylacrylate (360 mg), 4-(2-butoxyethoxy)phenylboric acid (283 mg) and potassium carbonate (330 mg) in toluene (10 ml), ethanol (1 ml) and water (1 ml) was stirred for 1 hour under an argon atmosphere. Then, tetrakis(triphenylphosphine)palladium (53 mg) was added thereto, and the resulting mixture was refluxed for 6 hours. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 2 : 1 → hexane : ethyl acetate = 1 : 4) to give ethyl (2E)-3-[4'-(2-butoxyethoxy)-4-[methyl[(1-methyl-1H-pyrazol-4-yl)methyl]amino]-1,1'-biphenyl-3-yl]-2-methylacrylate (344 mg) as a yellow oily material.

$^1$H-NMR (300 MHz, $CDCl_3$) δ 0.93 (3H, t, J=7.2 Hz), 1.33-1.46 (5H, m), 1.50-1.66 (2H, m), 2.17 (3H, d, J=1.2 Hz), 2.68 (3H, s), 3.55 (2H, t, J=6.9 Hz), 3.81 (2H, t, J=4.8 Hz), 3.85-3.87 (5H, m), 4.16 (2H, t, J=4.8 Hz), 4.30 (2H, q, J=7.2 Hz), 6.99 (2H, d, J=9.0 Hz), 7.05 (1H, d, J=8.7 Hz), 7.32 (1H, s), 7.40 (1H, s), 7.45-7.49 (4H, m), 7.96 (1H, s).

Reference Example 114

Ethyl (2E)-3-[4'-(2-butoxyethoxy)-4-[methyl[(1-methyl-1H-pyrazol-4-yl)methyl]amino]-1,1'-biphenyl-3-yl]-2-methylacrylate (330 mg) was dissolved in THF (8 ml) and methanol (8 ml). Then, a 1 N aqueous sodium hydroxide solution (2.6 ml) was added thereto, and the mixture was stirred for 5 hours at 90°C. After adding water at 0°C, the resulting mixture was neutralized with 1 N hydrochloric acid and extracted with ethyl acetate. The organic layer was washed with saturated brine and dried over magnesium sulfate. The solvent was distilled off under reduced pressure, and then the resulting residue was washed with hexane to give (2E)-3-[4'-(2-butoxyethoxy)-4-[methyl[(1-methyl-1H-pyrazol-4-yl)methyl]amino]-1,1'-biphenyl-3-yl]-2-methylacrylic acid (260 mg) as yellow crystals.

m.p. 139.5-140.5°C.

$^1$H-NMR (300 MHz, $CDCl_3$) δ 0.94 (3H, t, J=7.5 Hz), 1.34-1.46 (2H, m), 1.57-1.66 (2H, m), 2.21 (3H, d, J=1.5 Hz), 2.69 (3H, s), 3.56 (2H, t, J=6.6 Hz), 3.82 (2H, t, J=4.8 Hz), 3.85 (2H, s), 3.94 (3H, s), 4.17 (2H, t, J=4.8 Hz), 7.01 (2H, d, J=9.0 Hz), 7.10 (1H, d, J=7.8 Hz), 7.34 (1H, s), 7.46-7.56 (4H, m), 7.65 (1H, s), 8.12 (1H, s).

Elementary analysis $C_{28}H_{35}N_3O_4 \cdot 0.25H_2O$, Calcd. C, 69.76 ; H, 7.42 ; N, 8.72 : Found C, 69.98 ; H, 7.37 ; N, 8.42.

Reference Example 115

To a solution of 4-bromofluorobenzene (15.0 g) in dry tetrahydrofuran (150 ml) was added dropwise LDA (2.0 M, 55.7 ml) at -78°C under an argon atmosphere. After stirring as such for 2 hours, a solution of N-methoxy-N-methylacetamide (10.6 g) in dry tetrahydrofuran (20 ml) was added dropwise thereto. The reaction mixture was returned to room temperature and stirred for 2 hours, which was acidified with 1 N hydrochloric acid and extracted with ether. The organic layer was washed with saturated brine and dried over magnesium sulfate. The solvent was distilled off under reduced pressure, and then the resulting residue was separated and purified by silica gel column chromatography (hexane : ethyl acetate = 50 : 1 → hexane : ethyl acetate = 25 : 1) to give 1-(5-bromo-2-fluorophenyl)ethanone (13.1 g) as a colorless oily material.

$^1$H-NMR (300 MHz, $CDCl_3$) δ 2.64 (3H, d, J=5.1 Hz), 7.02-7.09 (1H, m), 7.59-7.65 (1H, m), 7.98-8.01 (1H, m).

Reference Example 116

To a solution of pyrrolidine (3.85 ml) and potassium carbonate (12.7 g) in DMF (60 ml) was added dropwise a solution of 1-(5-bromo-2-fluorophenyl)ethanone (5.0 g) in DMF (20 ml) at 75°C under a nitrogen atmosphere, and the mixture was heated while stirring for 6 hours as such. After returning to room temperature, water was added thereto and the mixture was extracted with ethyl acetate. The organic layer was washed with saturated brine and dried over magnesium sulfate. The solvent was distilled off under reduced pressure, and then the resulting residue was separated and purified by silica gel column chromatography (hexane : ethyl acetate = 19 : 1) to give 1-(5-bromo-2-pyrrolidin-1-ylphenyl)ethanone (2.28 g) as a brown oily material.

$^1$H-NMR (300 MHz, $CDCl_3$) δ 1.93-1.98 (4H, m), 2.57 (3H, s), 3.07-3.11 (4H, m), 6.70 (1H, d, J=9.0 Hz), 7.37 (1H, dd, J=9.0, 2.4 Hz), 7.58 (1H, d, J=2.4 Hz).

Reference Example 117

A suspension of 1-(5-bromo-2-pyrrolidin-1-ylphenyl)ethanone (750 mg), 4-(2-butoxyethoxy)phenylboric acid (867 mg) and potassium carbonate (1.0 g) in toluene (15 ml), ethanol (1.5 ml) and water (1.5 ml) was stirred for 1 hour under an argon atmosphere. Then, tetrakis(triphenylphosphine)palladium (162 mg) was added thereto, and the resulting mixture was refluxed for 6 hours. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 19 : 1 → hexane : ethyl acetate = 6 : 1). The resulting solids were washed

with hexane to give 1-[4'-(2-butoxyethoxy)-4-pyrrolidin-1-yl-1,1'-biphenyl-3-yl]ethanone (495 mg) as yellow crystals.

m.p. 87.0-88.0°C.

$^1$H-NMR (300 MHz, $CDCl_3$) δ 0.93 (3H, t, J=7.2 Hz), 1.36-1.46 (2H, m), 1.55-1.66 (2H, m), 1.94-1.99 (4H, m), 2.64 (3H, s), 3.15-3.20 (4H, m), 3.55 (2H, t, J=6.9 Hz), 3.81 (2H, t, J=4.8 Hz), 4.16 (2H, t, J=4.8 Hz), 6.88 (1H, d, J=9.0 Hz), 6.98 (2H, d, J=9.0 Hz), 7.46 (2H, d, J=9.0 Hz), 7.53 (1H, dd, J=9.0, 2.1 Hz), 7.66 (1H, d, J=2.1 Hz).

Elementary analysis $C_{24}H_{31}NO_3$, Calcd. C, 75.56 ; H, 8.19 ; N, 3.67 : Found C, 75.52 ; H, 8.19 ; N, 3.41.

Reference Example 118

To a suspension of sodium hydride (296 mg) in toluene (10 ml) was added dropwise a solution of ethyl diethylphosphonoacetate (1.24 ml) in toluene (20 ml) at 0°C under a nitrogen atmosphere, and the mixture was stirred as such for 1 hour. Next, a solution of 1-[4'-(2-butoxyethoxy)-4-pyrrolidin-1-yl-1,1'-biphenyl-3-yl]ethanone (470 mg) in toluene (10 ml) was added dropwise thereto at 0°C under a nitrogen atmosphere, and then the resulting mixture was refluxed for 3 hours. After removing from the oil bath, water was added thereto and the mixture was extracted with ethyl acetate. The organic layer was washed with saturated brine and dried over magnesium sulfate. The solvent was distilled off under reduced pressure, and then the resulting residue was purified by basic silica gel column chromatography(ethyl acetate). The resulting solids were washed with hexane to give ethyl (2E)-3-[4'-(2-butoxyethoxy)-4-pyrrolidin-1-yl-1,1'-biphenyl-3-yl]but-2-enoate (250 mg) as yellow crystals.

m.p. 81.0-82.0°C.

$^1$H-NMR (300 MHz, $CDCl_3$) δ 0.93 (3H, t, J=7.5 Hz), 1.24-1.43 (5H, m), 1.50-1.65 (2H, m), 1.85-1.95 (4H, m), 2.50 (3H, s), 3.15-3.25 (4H, m), 3.55 (2H, t, J=6.6 Hz), 3.80 (2H, t, J=4.8 Hz), 4.11-4.25 (4H, m), 5.98 (1H, s), 6.85 (1H, d, J=9.0 Hz), 6.96 (2H, d, J=8.4 Hz), 7.25 (1H, d, J=2.7 Hz), 7.40 (1H, dd, J=9.0, 2.7 Hz), 7.46 (2H, d, J=8.4 Hz).

Elementary analysis $C_{28}H_{37}NO_4 \cdot 0.5H_2O$, Calcd. C, 73.01 ; H, 8.32 ; N, 3.04 : Found C, 73.06 ; H, 8.15 ; N, 2.87.

Reference Example 119

Ethyl (2E)-3-[4'-(2-butoxyethoxy)-4-pyrrolidin-1-yl-1,1'-biphenyl-3-yl]but-2-enoate (260 mg) was dissolved in THF (7 ml) and methanol (7 ml). Then, a 1 N aqueous sodium hydroxide solution (2.3 ml) was added thereto, and the mixture was stirred for 3 hours at 90°C. After adding water at 0°C, the resulting mixture was neutralized with 1 N hydrochloric acid and extracted with ethyl acetate. The organic layer was washed with saturated brine and dried over magnesium sulfate. The solvent was distilled off under reduced pressure, and then the resulting residue was washed with hexane to give (2E)-3-[4'-(2-butoxyethoxy)-4-pyrrolidin-1-yl-1,1'-biphenyl-3-yl]but-2-enoic acid (158 mg) as yellow crystals.

m.p. 127.5-128.5°C.

$^1$H-NMR (300 MHz, $CDCl_3$) δ 0.93 (3H, t, J=7.2 Hz), 1.35-1.43 (2H, m), 1.56-1.63 (2H, m), 1.90-1.94 (4H, m), 2.52 (3H, d, J=1.2 Hz), 3.19-3.23 (4H, m), 3.54 (2H, t, J=6.6 Hz), 3.80 (2H, t, J=4.5 Hz), 4.15 (2H, t, J=4.5 Hz), 6.01 (1H, d, J=1.5 Hz), 6.86 (1H, d, J=8.7 Hz), 6.96 (2H, d, J=8.4 Hz), 7.26 (1H, d, J=2.4 Hz), 7.40 (1H, dd, J=8.7, 2.4 Hz), 7.45 (2H, d, J=8.4 Hz).

Elementary analysis $C_{26}H_{33}NO_4 \cdot 0.5H_2O$, Calcd. C, 72.19 ; H, 7.92 ; N, 3.24 : Found C, 72.20 ; H, 7.74 ; N, 2.97.

Reference Example 120

A suspension of 5-bromo-2-fluorobenzaldehyde (2.0 g), 3-pyrroline (0.98 ml) and potassium carbonate (1.77 g) in DMF (30 ml) was heated for 5 hours at 75°C under a nitrogen atmosphere. After returning to room temperature, water was added thereto and the mixture was extracted with ethyl acetate. The organic layer was washed with saturated brine and dried over magnesium sulfate. The solvent was distilled off under reduced pressure, and then the resulting residue was separated and purified by silica gel column chromatography (hexane : ethyl acetate = 19 : 1 → hexane : ethyl acetate = 6 : 1). The resulting solids were washed with hexane to give 5-bromo-2-(2,5-dihydro-1H-pyrrol-1-yl) benzaldehyde (592 mg) as yellow crystals.

m.p. 88.2-89.2°C.

$^1$H-NMR (300 MHz, $CDCl_3$) δ 4.21 (4H, s), 5.95 (2H, s), 6.68 (1H, d, J=9.0 Hz), 7.46 (1H, dd, J=9.0, 2.4 Hz), 7.82 (1H, d, J=2.4 Hz), 10.08 (1H, s).

Elementary analysis $C_{11}H_{10}NOBr$, Calcd. C, 52.41 ; H, 4.00 ; N, 5.56 : Found C, 52.24 ; H, 3.94 ; N, 5.33.

Reference Example 121

A suspension of 5-bromo-2-(2,5-dihydro-1H-pyrrol-1-yl)benzaldehyde (550 mg), 4-(2-butoxyethoxy)phenylboric acid (676 mg) and potassium carbonate (784 mg) in toluene (15 ml), ethanol(1.5 ml) and water (1.5 ml) was

stirred for 1 hour under an argon atmosphere. Then, tetrakis(triphenylphosphine)palladium (126 mg) was added thereto, and the resulting mixture was refluxed for 5 hours. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 19 : 1 → hexane : ethyl acetate = 5 : 1) and recrystallized from hexane-ethyl acetate to give 4'-(2-butoxyethoxy)-4-(2,5-dihydro-1H-pyrrol-1-yl)-1,1'-biphenyl-3-carbaldehyde (431 mg) as yellow crystals.

m.p. 79.0-81.0°C.

$^1$H-NMR (300 MHz, $CDCl_3$) δ 0.93 (3H, t, J=8.7 Hz), 1.34-1.45 (2H, m), 1.48-1.68 (2H, m), 3.55 (2H, t, J=6.6 Hz), 3.81 (2H, t, J=4.8 Hz), 4.16 (2H, t, J=4.8 Hz), 4.28 (4H, s), 5.96 (2H, s), 6.87 (1H, d, J=8.7 Hz), 6.99 (2H, d, J=9.0 Hz), 7.49 (2H, d, J=9.0 Hz), 7.64 (1H, dd, J=8.7, 2.4 Hz), 7.92 (1H, d, J=2.4 Hz), 10.21 (1H, s).

Elementary analysis $C_{23}H_{27}NO_3$, Calcd. C, 75.59 ; H, 7.45 ; N, 3.83 : Found C, 75.48 ; H, 7.46 ; N, 3.66.

Reference Example 122

To a suspension of sodium hydride (61 mg) in toluene (10 ml) was added dropwise a solution of triethyl 2-phosphonopropionate (0.3 ml) in toluene (10 ml) at 0°C under a nitrogen atmosphere, and the mixture was stirred as such for 1 hour. Next, a solution of 4'-(2-butoxyethoxy)-4-(2,5-dihydro-1H-pyrrol-1-yl)-1,1'-biphenyl-3-carbaldehyde (390 mg) in toluene (10 ml) was added dropwise thereto at 0°C under a nitrogen atmosphere, and then the resulting mixture was refluxed for 3 hours. After removing from the oil bath, water was added thereto and the mixture was extracted with ethyl acetate. The organic layer was washed with saturated brine and dried over magnesium sulfate. The solvent was distilled off under reduced pressure, and then the resulting residue was purified by basic silica gel column chromatography (hexane : ethyl acetate 10 : 1 → hexane : ethyl acetate = 4 : 1) to give ethyl (2E)-3-[4'-(2-butoxyethoxy)-4-(2,5-dihydro-1H-pyrrol-1-yl)-1,1'-biphenyl-3-yl]-2-methylacrylate (389 mg) as a yellow oily material.

$^1$H-NMR (300 MHz, $CDCl_3$) δ 0.93 (3H, t, J=7.5 Hz), 1.30-1.70 (7H, m), 2.01 (3H, s), 3.52-3.58 (2H, m), 3.79 (2H, t, J=4.8 Hz), 4.13-4.31 (8H, m), 5.87 (2H, t, J=4.2 Hz), 6.81 (1H, d, J=9.0 Hz), 6.95 (2H, d, J=9.0 Hz), 7.38-7.46 (4H, m), 7.91 (1H, s).

Reference Example 123

Ethyl (2E)-3-[4'-(2-butoxyethoxy)-4-(2,5-dihydro-1H-pyrrol-1-yl)-1,1'-biphenyl-3-yl]-2-methylacrylate (360 mg) was dissolved in THF (10 ml) and methanol (10 ml). Then, a 1 N aqueous sodium hydroxide solution (3.2 ml) was added thereto, and the mixture was stirred for 3 hours at 90°C. After adding water at 0°C, the resulting mixture was neutralized with 1 N hydrochloric acid and extracted with ethyl acetate. The organic layer was washed with saturated brine and dried over magnesium sulfate. The solvent was distilled off under reduced pressure, and then the resulting residue was washed with hexane to give (2E)-3-[4'-(2-butoxyethoxy)-4-(2,5-dihydro-1H-pyrrol-1-yl)-1,1'-biphenyl-3-yl]-2-methylacrylic acid (265 mg) as yellow crystals.

m.p. 138.5-139.5°C.

$^1$H-NMR (300 MHz, $CDCl_3$) δ 0.93 (3H, t, J=7.2 Hz), 1.33-1.45 (2H, m), 1.56-1.65 (2H, m), 2.04 (3H, s), 3.55 (2H, t, J=6.9 Hz), 3.80 (2H, t, J=5.1 Hz), 4.15 (2H, t, J=5.1 Hz), 4.20 (4H, s), 5.88 (2H, s), 6.82 (1H, d, J=9.0 Hz), 6.96 (2H, d, J=8.7 Hz), 7.28 (1H, d, J=2.1 Hz), 7.40-7.45 (3H, m), 8.06 (1H, s).

Elementary analysis $C_{26}H_{31}NO_4$, Calcd. C, 74.08 ; H, 7.41 ; N, 3.32 : Found C, 74.21 ; H, 7.29 ; N, 3.17.

Reference Example 124

To a solution of 5-bromo-2-(3-hydroxypyrrolidin-1-yl)benzaldehyde (6.4 g) and 3,4-dihydro-2H-pyran (4.33 ml) in dichloromethane (70 ml) was added pyridinium p-toluenesulfonate (1.19 g), and the mixture was stirred overnight under a nitrogen atmosphere. Water was added thereto and the mixture was extracted with ethyl acetate. The organic layer was washed with an aqueous saturated sodium hydrogen carbonate solution and saturated brine, and dried over magnesium sulfate. The solvent was distilled off under reduced pressure to give 5-bromo-2-[3-(tetrahydro-2H-pyran-2-yloxy)pyrrolidin-1-yl]benzaldehyde (8.15 g) as a brown oily material. To a suspension of sodium hydride (1.41 g) in toluene (50 ml) was added dropwise a solution of triethyl 2-phosphonopropionate (6.88 ml) in toluene (50 ml) at 0°C under a nitrogen atmosphere, and the mixture was stirred as such for 1 hour. Next, a solution of 5-bromo-2-[3-(tetrahydro-2H-pyran-2-yloxy)pyrrolidin-1-yl]benzaldehyde (8.5 g) in toluene (50 ml) was added dropwise thereto at 0°C under a nitrogen atmosphere, and then the resulting mixture was refluxed for 3 hours. After removing from the oil bath, water was added thereto and the mixture was extracted with ethyl acetate. The organic layer was washed with saturated brine, and then dried over magnesium sulfate. The solvent was distilled off under reduced pressure, and then the resulting residue was purified by basic silica gel column chromatography (hexane : ethyl acetate 6 : 1) to give ethyl

(2E)-3-[5-bromo-2-[3-(tetrahydro-2H-pyran-2-yloxy)pyrrolidin-1-yl]phenyl]-2-methylacrylate (9.69 g) as a yellow oily material. To a solution of ethyl (2E)-3-[5-bromo-2-[3-(tetrahydro-2H-pyran-2-yloxy)pyrrolidin-1-yl]phenyl]-2-methylacrylate (9.5 g) in methanol (270 ml) was added 1 N hydrochloric acid (86.8 ml), and the mixture was stirred for 2 hours. After distilling off the solvent under reduced pressure, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 1 : 1 → ethyl acetate) to give ethyl (2E)-3-[5-bromo-2-(3-hydroxypyrrolidin-1-yl)phenyl]-2-methylacrylate (7.38 g) as a yellow oily material.

$^1$H-NMR (300 MHz, $CDCl_3$) δ 1.34 (3H, t, J=7.2 Hz), 1.69 (1H, d, J=5.1 Hz), 1.90-2.05 (4H, m), 2.07-2.25 (1H, m), 3.08-3.18 (2H, m), 3.41-3.53 (2H, m), 4.26 (2H, q, J=7.2 Hz), 4.43-4.53 (1H, m), 6.69 (1H, d, J=8.7 Hz), 7.20-7.35 (2H, m), 7.66 (1H, s).

Reference Example 125

To a solution of oxalyl chloride (1.2 ml) in dichloromethane (20 ml) was added dropwise a solution of DMSO (2.1 ml) in dichloromethane (20 ml) at -78°C under an argon atmosphere. After stirring as such for 15 minutes, a solution of ethyl (2E)-3-[5-bromo-2-(3-hydroxypyrrolidin-1-yl)phenyl]-2-methylacrylate (3.5 g) in dichloromethane (20 ml) was added dropwise thereto. After stirring as such for 15 minutes, triethylamine (8.26 ml) was added dropwise thereto. The reaction mixture was stirred as such for 2 hours, and then returned to room temperature. Water was added thereto and extracted with ethyl acetate. The organic layer was washed with saturated brine. The solvent was distilled off under reduced pressure, and then the resulting residue was purified by silica gel column chromatography (hexane : ethyl acetate = 1 : 1) to give ethyl (2E)-3-[5-bromo-2-(3-oxopyrrolidin-1-yl)phenyl]-2-methylacrylate (2.67 g) as a yellow oily material.

$^1$H-NMR (300 MHz, $CDCl_3$) δ 1.34 (3H, t, J=6.9 Hz), 2.04 (3H, s), 2.62 (2H, t, J=6.9 Hz), 3.47-3.52 (4H, m), 4.27 (2H, q, J=6.9 Hz), 6.81 (1H, d, J=8.7 Hz), 7.33 (1H, d, J=2.4 Hz), 7.37 (1H, dd, J=8.7, 2.4 Hz), 7.59 (1H, s).

Reference Example 126

A suspension of ethyl (2E)-3-[5-bromo-2-(3-oxopyrrolidin-1-yl)phenyl]-2-methylacrylate (2.6 g), 4-(2-butoxyethoxy)phenylboric acid (2.29 g) and potassium carbonate (2.65 g) in toluene (50 ml), ethanol (5 ml) and water (5 ml) was stirred for 1 hour under an argon atmosphere. Then, tetrakis(triphenylphosphine)palladium (597 mg) was added thereto, and the resulting mixture was refluxed for 5 hours. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 19 : 1 → hexane : ethyl acetate = 5 : 1). The resulting solids were washed with hexane to give ethyl (2E)-3-[4'-(2-butoxyethoxy)-4-(3-oxopyrrolidin-1-yl)-1,1'-biphenyl-3-yl]-2-methylacrylate (1.51 g) as yellow crystals.

m.p. 98.5-99.0°C.

$^1$H-NMR (300 MHz, $CDCl_3$) δ 0.93 (3H, t, J=7.2 Hz), 1.32-1.46 (5H, m), 1.50-1.66 (2H, m), 2.11 (3H, d, J=1.5 Hz), 2.65 (2H, t, J=7.2 Hz), 3.53-3.58 (6H, m), 3.81 (2H, t, J=4.8 Hz), 4.16 (2H, t, J=4.8 Hz), 4.28 (2H, q, J=7.2 Hz), 6.98-7.02 (3H, m), 7.42-7.51 (4H, m), 7.55 (1H, s).

Elementary analysis $C_{28}H_{35}NO_5$, Calcd. C, 72.23 ; H, 7.58 ; N, 3.01 : Found C, 72.09 ; H, 7.37 ; N, 2.78.

Reference Example 127

To a solution of ethyl (2E)-3-[4'-(2-butoxyethoxy)-4-(3-oxopyrrolidin-1-yl)-1,1'-biphenyl-3-yl]-2-methylacrylate (1.25 g) and ethylene glycol (1.5 ml) in toluene (20 ml) was added p-toluenesulfonic acid monohydrate (26 mg), and the mixture was refluxed overnight while dehydrating under a nitrogen atmosphere. The reaction mixture was returned to room temperature. Water and an aqueous saturated sodium hydrogen carbonate solution were sequentially added thereto, which was extracted with ethyl acetate. The organic layer was washed with saturated brine, and the solvent was distilled off under reduced pressure. The resulting residue was subjected to a silica gel column chromatography (hexane : ethyl acetate = 4 : 1 → hexane : ethyl acetate = 1 : 1) to give ethyl (2E)-3-[4'-(2-butoxyethoxy)-4-(1,4-dioxa-7-azaspiro[4.4]non-7-yl)-1,1'-biphenyl-3-yl]-2-methylacrylate (1.14 g) as a brown oily material.

$^1$H-NMR (300 MHz, $CDCl_3$) δ 0.93 (3H, t, J=7.2 Hz), 1.33-1.45 (5H, m), 1.50-1.65 (2H, m), 2.05 (3H, d, J=1.2 Hz), 2.15 (2H, t, J=6.9 Hz), 3.31 (2H, s), 3.38 (2H, t, J=6.9 Hz), 3.55 (2H, t, J=6.9 Hz), 3.80 (2H, t, J=4.5 Hz), 3.95-3.99 (4H, m), 4.15 (2H, t, J=4.5 Hz), 4.28 (2H, q, J=6.9 Hz), 6.86 (1H, d, J=8.1 Hz), 6.96 (2H, d, J=8.7 Hz), 7.34 (1H, d, J=2.4 Hz), 7.39-7.46 (3H, m), 7.79 (1H, s).

Reference Example 128

Ethyl (2E)-3-[4'-(2-butoxyethoxy)-4-(1,4-dioxa-7-azaspiro[4.4]non-7-yl)-1,1'-biphenyl-3-yl]-2-methylacrylate (1.1 g) was dissolved in THF (12 ml) and methanol (12 ml). Then, a 1 N aqueous sodium hydroxide solution(4.3 ml) was added thereto, and the mixture was stirred overnight at 50°C. After adding water at 0°C, the resulting mixture was neutralized with 1 N hydrochloric acid and extracted with ethyl acetate. The organic layer was washed with saturated brine and dried over magnesium sulfate. The solvent was distilled off under reduced pressure, and then the resulting residue was washed with hexane-diisopropyl ether to give (2E)-3-[4'-(2-butoxyethoxy)-4-(1,4-dioxa-7-azaspiro[4.4]non-7-yl)-1,1'-biphenyl-3-yl]-2-methylacrylic acid (1.02 g) as yellow crystals.

m.p. 129.5-131.5°C.

$^1$H-NMR (300 MHz, $CDCl_3$) δ 0.93 (3H, t, J=7.2 Hz), 1.33-1.45 (2H, m), 1.56-1.65 (2H, m), 2.08 (3H, d, J=1.5 Hz), 2.16 (2H, t, J=7.2 Hz), 3.33 (2H, s), 3.40 (2H, t, J=6.9 Hz), 3.55 (2H, t, J=6.9 Hz), 3.80 (2H, t, J=4.8 Hz), 3.92-4.02 (4H, m), 4.15 (2H, t, J=4.8 Hz), 6.87 (1H, d, J=8.7 Hz), 6.97 (2H, d, J=8.7 Hz), 7.36 (1H, d, J=2.1 Hz), 7.40-7.46 (3H, m), 7.93 (1H, s).

Elementary analysis $C_{28}H_{35}NO_6$, Calcd. C, 69.83 ; H, 7.33 ; N, 2.91 : Found C, 69.67 ; H, 7.45 ; N, 2.65.

Reference Example 129

A suspension of 5-bromo-2-fluorobenzaldehyde (2.5 g), 3-hydroxymethylpyrrolidine hydrochloride (3.39 g) and sodium carbonate (3.26 g) in DMSO (75 ml) and water (37.5 ml) was heated for 5 hours at 75°C under a nitrogen atmosphere. After returning to room temperature, water was added thereto and the mixture was extracted with ethyl acetate. The organic layer was washed with saturated brine and dried over magnesium sulfate. The solvent was distilled off under reduced pressure, and then the resulting residue was separated and purified by silica gel column chromatography (hexane : ethyl acetate = 9 : 1 → hexane : ethyl acetate = 1 : 4) to give 5-bromo-2-[3-(hydroxymethyl)pyrrolidin-1-yl]benzaldehyde (2.9 g) as a yellow oily material.

$^1$H-NMR (300 MHz, $CDCl_3$) δ 1.77-1.89 (1H, m), 2.09-2.19 (1H, m), 2.51-2.60 (1H, m), 3.26-3.50 (4H, m), 3.65-3.78 (2H, m), 6.73 (1H, d, J=9.0 Hz), 7.45 (1H, dd, J=9.0, 2.7 Hz), 7.79 (1H, d, J=2.7 Hz), 10.01 (1H, s).

Reference Example 130

To a solution of 5-bromo-2-[3-(hydroxymethyl)pyrrolidin-1-yl]benzaldehyde (2.85 g) in pyridine (11 ml) was added dropwise acetic anhydride (3.77 ml) at 0°C under a nitrogen atmosphere. The mixture was returned to room temperature and stirred for 5 hours, and then the solvent was distilled off under reduced pressure. To the resulting residue was added water at 0°C and further added sodium carbonate, which was neutralized and extracted with ethyl acetate. The organic layer was then washed with saturated brine, and the solvent was distilled off under reduced pressure. The resulting residue was purified by silica gel column chromatography (hexane : ethyl acetate = 3 : 1 → hexane : ethyl acetate = 2 : 1) to give [1-(4-bromo-2-formylphenyl)pyrrolidin-3-yl]methyl acetate (2.91 g) as a yellow oily material.

$^1$H-NMR (300 MHz, $CDCl_3$) δ 1.80-1.90 (1H, m), 2.07 (3H, s), 2.10-2.33 (1H, m), 2.61-2.71 (1H, m), 3.20-3.53 (4H, m), 4.02-4.22 (2H, m), 6.72 (1H, d, J=9.3 Hz), 7.45 (1H, dd, J=9.3, 2.4 Hz), 7.79 (1H, d, J=2.4 Hz), 10.01 (1H, s).

Reference Example 131

A solution of [1-(4-bromo-2-formylphenyl)pyrrolidin-3-yl]methyl acetate (2.47 g) and tert-butyl 2-(triphenylphosphoranylidene)propanoate (3.4 g) in toluene (100 ml) was refluxed overnight under a nitrogen atmosphere. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by basic silica gel column chromatography (hexane : ethyl acetate = 4 : 1) to give tert-butyl (2E)-3-[2-[3-[(acetyloxy)methyl]pyrrolidin-1-yl]-5-bromophenyl]-2-methylacrylate (2.29 g) as a yellow oily material.

$^1$H-NMR (300 MHz, $CDCl_3$) δ 1.53 (9H, s), 1.60-1.75 (1H, m), 1.95 (3H, d, J=1.5 Hz), 2.00-2.15 (4H, m), 2.53-2.63 (1H, m), 2.98-3.08 (1H, m), 3.18-3.28 (3H, m), 3.95-4.16 (2H, m), 6.66 (1H, d, J=8.4 Hz), 7.20-7.33 (2H, m), 7.52 (1H, s).

Reference Example 132

A suspension of tert-butyl (2E)-3-[2-[3-(acetoxymethyl)pyrrolidin-1-yl]-5-bromophenyl]-2-methylacrylate (1.2 g), 4-(2-butoxyethoxy)phenylboric acid (848 mg) and potassium carbonate (984 mg) in toluene (20 ml), ethanol (2 ml) and water (2 ml) was stirred for 1 hour under an argon atmosphere. Then, tetrakis(triphenylphosphine)palladium (158

mg) was added thereto, and the resulting mixture was refluxed for 5 hours. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 9 : 1 → hexane : ethyl acetate = 3 : 1) to give tert-butyl (2E)-3-[4-[3-(acetoxymethyl)pyrrolidin-1-yl]-4'-(2-butoxyethoxy)-1,1'-biphenyl-3-yl]-2-methylacrylate (770 mg) as a yellow oily material.

$^1$H-NMR (300 MHz, $CDCl_3$) δ 0.93 (3H, t, J=7.2 Hz), 1.30-1.80 (14H, m), 1.95-2.15 (7H, m), 2.55-2.65 (1H, m), 3.07-3.13 (1H, m), 3.27-3.35 (3H, m), 3.55 (2H, t, J=6.6 Hz), 3.80 (2H, t, J=4.5 Hz), 4.02-4.18 (4H, m), 6.87 (1H, d, J=8.1 Hz), 6.97 (2H, d, J=9.0 Hz), 7.36-7.46 (4H, m), 7.69 (1H, s).

Reference Example 133

tert-Butyl (2E)-3-[4-[3-(acetoxymethyl)pyrrolidin-1-yl]-4'-(2-butoxyethoxy)-1,1'-biphenyl-3-yl]-2-methylacrylate (750 mg) was dissolved in ethyl acetate (7.5 ml). Then, a 4 N hydrochloric acid-ethyl acetate solution (11 ml) was added thereto, and the mixture was stirred overnight under a nitrogen atmosphere. Water was added thereto at 0°C and the mixture was extracted with ethyl acetate. The organic layer was washed with saturated brine and dried over magnesium sulfate. The solvent was distilled off under reduced pressure, and then the resulting residue was washed with hexane-diisopropyl ether to give (2E)-3-[4-[3-(acetoxymethyl)pyrrolidin-1-yl]-4'-(2-butoxyethoxy)-1,1'-biphenyl-3-yl]-2-methylacrylic acid (542 mg) as yellow crystals.

m.p. 109.5-111.0°C.

$^1$H-NMR (300 MHz, $CDCl_3$) δ 0.93 (3H, t, J=6.9 Hz), 1.33-1.46 (2H, m), 1.56-1.80 (3H, m), 2.00-2.20 (7H, m), 2.55-2.70 (1H, m), 3.10-3.15 (1H, m), 3.29-3.35 (3H, m), 3.55 (2H, t, J=6.6 Hz), 3.81 (2H, t, J=5.1 Hz), 4.04-4.17 (4H, m), 6.90 (1H, d, J=8.7 Hz), 6.98 (2H, d, J=9.0 Hz), 7.38 (1H, d, J=2.1 Hz), 7.41-7.47 (3H, m), 7.93 (1H, s).

Elementary analysis $C_{29}H_{37}NO_6$, Calcd. C, 70.28 ; H, 7.52 ; N, 2.83 : Found C, 70.03 ; H, 7.51 ; N, 2.72.

Reference Example 134

To a solution of methyl 1-benzylpyrrolidine-3-carboxylate in methanol (50 ml) and 1 N hydrochloric acid (16.9 ml) was added palladium carbon (10%, 1.8 g), and the mixture was stirred overnight under a hydrogen atmosphere. The insolubles were removed by filtration, and then the solvent was distilled off under reduced pressure. Toluene was added thereto, and then the solvent was again distilled off under reduced pressure to give methyl pyrrolidine-3-carboxylate hydrochloride (2.71 g) as a yellow oily material.

$^1$H-NMR (300 MHz, DMSO-$d_6$) δ 1.95-2.25 (2H, m), 3.11-3.45 (5H, m), 3.66 (3H, s), 9.23 (2H, br).

Reference Example 135

A suspension of 5-bromo-2-fluorobenzaldehyde (2.12 g), methyl pyrrolidine-3-carboxylate hydrochloride (2.6 g) and potassium carbonate (3.63 g) in DMF (40 ml) was stirred overnight at 80°C under a nitrogen atmosphere. After returning to room temperature, water was added thereto and the mixture was extracted with ethyl acetate. The organic layer was washed with saturated brine and dried over magnesium sulfate. The solvent was distilled off under reduced pressure, and then the resulting residue was separated and purified by silica gel column chromatography (hexane : ethyl acetate = 16 : 1 → hexane : ethyl acetate = 4 : 1) to give methyl 1-(4-bromo-2-formylphenyl)pyrrolidine-3-carboxylate (816 mg) as a yellow oily material.

$^1$H-NMR (300 MHz, $CDCl_3$) δ 2.26-2.33 (2H, m), 3.15-3.24 (1H, m), 3.33-3.41 (1H, m), 3.47-3.58 (3H, m), 3.73 (3H, s), 6.75 (1H, d, J=9.0 Hz), 7.46 (1H, dd, J=9.0, 2.4 Hz), 7.80 (1H, d, J=2.4 Hz), 10.01 (1H, s).

Reference Example 136

A solution of methyl 1-(4-bromo-2-formylphenyl)pyrrolidine-3-carboxylate (800 mg) and tert-butyl 2-(triphenylphosphoranylidene)propanoate (1.34 g) in toluene (20 ml) was refluxed for 6 hours under a nitrogen atmosphere. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 10 : 1 → hexane : ethyl acetate = 1 : 4) to give methyl 1-[4-bromo-2-[(1E)-3-tert-butoxy-2-methyl-3-oxoprop-1-enyl]phenyl]pyrrolidine-3-carboxylate (580 mg) as a yellow oily material.

$^1$H-NMR (300 MHz, $CDCl_3$) δ 1.53 (9H, s), 1.95 (3H, d, J=1.5 Hz), 2.17-2.24 (2H, m), 3.10-3.15 (1H, m), 3.21-3.29 (2H, m), 3.41 (2H, d, J=7.5 Hz), 3.71 (3H, s), 6.69 (1H, d, J=8.7 Hz), 7.24-7.30 (2H, m), 7.53 (1H, s).

Reference Example 137

A suspension of methyl 1-[4-bromo-2-[(1E)-3-tert-butoxy-2-methyl-3-oxoprop-1-enyl]phenyl]pyrrolidine-3-carboxylate (550 mg), 4-(2-butoxyethoxy)phenylboric acid (402 mg) and potassium carbonate (466 mg) in toluene (15 ml), ethanol (1.5 ml) and water (1.5 ml) was stirred for 1 hour under an argon atmosphere. Then, tetrakis(triphenylphosphine)palladium (75 mg) was added thereto, and the resulting mixture was refluxed for 5 hours. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 18 : 1 → hexane : ethyl acetate = 4 : 1) to give methyl 1-[4'-(2-butoxyethoxy)-3-[(1E)-3-tert-butoxy-2-methyl-3-oxoprop-1-enyl]-1'1-biphenyl-4-yl]pyrrolidine-3-carboxylate (409 mg) as a yellow oily material.

$^1$H-NMR (300 MHz, $CDCl_3$) δ 0.93 (3H, t, J=7.2 Hz), 1.33-1.45 (2H, m), 1.51-1.65 (11H, m), 2.02 (3H, d, J=1.2 Hz), 2.19-2.26 (2H, m), 3.10-3.20 (1H, m), 3.25-3.35 (2H, m), 3.48 (2H, d, J=7.5 Hz), 3.55 (2H, t, J=6.6 Hz), 3.72 (3H, s), 3.80 (2H, t, J=4.5 Hz), 4.15 (2H, t, J=4.5 Hz), 6.89 (1H, d, J=8.4 Hz), 6.97 (2H, d, J=8.7 Hz), 7.35-7.46 (4H, m), 7.68 (1H, s).

Reference Example 138

Methyl 1-[4'-(2-butoxyethoxy)-3-[(1E)-3-tert-butoxy-2-methyl-3-oxoprop-1-enyl]-1'1-biphenyl-4-yl]pyrrolidine-3-carboxylate (400 mg) was dissolved in ethyl acetate (4 ml). Then, a 4 N hydrochloric acid-ethyl acetate solution (7 ml) was added thereto, and the mixture was stirred for 4 hours under a nitrogen atmosphere. Water was added thereto at 0°C and the mixture was extracted with ethyl acetate. The organic layer was washed with saturated brine and dried over magnesium sulfate. After distilling off the solvent under reduced pressure, the resulting solids were washed with hexane to give (2E)-3-[4'-(2-butoxyethoxy)-4-[3-(methoxycarbonyl)pyrrolidin-1-yl]-1,1'-biphenyl-3-yl]-2-methylacrylic acid (273 mg) as yellow crystals.

m.p. 140.0-141.0°C.

$^1$H-NMR (300 MHz, $CDCl_3$) δ 0.93 (3H, t, J=7.2 Hz), 1.33-1.45 (2H, m), 1.50-1.70 (2H, m), 2.10 (3H, d, J=1.2 Hz), 2.20-2.28 (2H, m), 3.10-3.25 (1H, m), 3.28-3.40 (2H, m), 3.46-3.50 (2H, m), 3.55 (2H, t, J=6.6 Hz), 3.73 (3H, s), 3.80 (2H, t, J=4.5 Hz), 4.16 (2H, t, J=4.5 Hz), 6.92 (1H, d, J=8.7 Hz), 6.98 (2H, d, J=8.7 Hz), 7.38-7.47 (4H, m), 7.91 (1H, s).

Elementary analysis $C_{28}H_{35}NO_6$, Calcd. C, 69.83 ; H, 7.33 ; N, 2.91 : Found C, 69.76 ; H, 7.45 ; N, 2.64.

Reference Example 139

To a solution of 1-benzyl-3,4dimethylpyrrolidine (9.0 g) in methanol (100 ml) and 1 N hydrochloric acid (48.9 ml) was added palladium carbon (10%, 4.5 g), and the mixture was stirred overnight under a hydrogen atmosphere. The insolubles were removed by filtration, and then the solvent was distilled off under reduced pressure. Toluene was added thereto, and then the solvent was again distilled off under reduced pressure. The resulting residue was washed with hexane to give 3,4-dimethylpyrrolidine hydrochloride (5.93 g) as pale red crystals.

$^1$H-NMR (300 MHz, DMSO-$d_6$) δ 0.90 (6H, d, J=3.4 Hz), 2.22-2.31 (2H, m), 2.73-2.82 (2H, m), 3.17-3.27 (2H, m), 9.32 (2H, br).

Reference Example 140

A suspension of 5-bromo-2-fluorobenzaldehyde (2.5 g), 3,4-dimethylpyrrolidine hydrochloride (2.51 g) and sodium carbonate (3.59 g) in DMSO (50 ml) and water (25 ml) was stirred for 4 hours at 80°C under a nitrogen atmosphere. After returning to room temperature, water was added thereto and the mixture was extracted with ethyl acetate. The organic layer was washed with saturated brine and dried over magnesium sulfate. The solvent was distilled off under reduced pressure, and then the resulting residue was separated and purified by silica gel column chromatography (hexane : ethyl acetate = 20 : 1 → hexane : ethyl acetate = 6 : 1) to give 5-bromo-2-(3,4-dimethylpyrrolidin-1-yl)benzaldehyde (2.84 g) as a brown oily material.

$^1$H-NMR (300 MHz, $CDCl_3$) δ 0.99 (6H, d, J=6.9 Hz), 2.35-2.40 (2H, m), 3.06-3.11 (2H, m), 3.44-3.49 (2H, m), 6.66 (1H, d, J=9.0 Hz), 7.39 (1H, dd, J=9.0, 2.7 Hz), 7.78 (1H, d, J=2.7 Hz), 10.00 (1H, s).

Reference Example 141

To a suspension of sodium hydride (550 mg) in toluene (30 ml) was added dropwise a solution of triethyl 2-phosphonopropionate (2.66 ml) in toluene (20 ml) at 0°C under a nitrogen atmosphere, and the mixture was stirred

as such for 1 hour. Next, a solution of 5-bromo-2-(3,4-dimethylpyrrolidin-1-yl)benzaldehyde (2.7 g) in toluene (30 ml) was added dropwise thereto at 0°C under a nitrogen atmosphere, and then the resulting mixture was refluxed for 3 hours. After removing from the oil bath, water was added thereto and the mixture was extracted with ethyl acetate. The organic layer was washed with saturated brine and dried over magnesium sulfate. The solvent was distilled off under reduced pressure, and then the resulting residue was purified by basic silica gel column chromatography (hexane : ethyl acetate = 19 : 1 → hexane : ethyl acetate = 6 : 1) to give ethyl (2E)-3-[5-bromo-2-(3,4-dimethylpyrrolidin-1-yl)phenyl]-2-methylacrylate (3.48 g) as a yellow oily material.

$^1$H-NMR (300 MHz, $CDCl_3$) δ 0.95 (6H, d, J=6.6 Hz), 1.34 (3H, t, J=6.9 Hz), 1.95 (3H, d, J=1.5 Hz), 2.23-2.32 (2H, m), 2.90-2.95 (2H, m), 3.30-3.35 (2H, m), 4.26 (2H, q, J=6.9 Hz), 6.61 (1H, d, J=8.7 Hz), 7.19-7.27 (2H, m), 7.67 (1H, s).

Reference Example 142

A suspension of ethyl (2E)-3-[5-bromo-2-(3,4-dimethylpyrrolidin-1-yl)phenyl]-2-methylacrylate (3.35 g), 4-(2-butoxyethoxy)phenylboric acid (2.83 g) and potassium carbonate (3.29 g) in toluene (50 ml), ethanol(5 ml) and water (5 ml) was stirred for 1 hour under an argon atmosphere. Then, tetrakis(triphenylphosphine)palladium (530 mg) was added thereto, and the resulting mixture was refluxed for 5 hours. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 19 : 1 → hexane : ethyl acetate = 6 : 1). The resulting residue was washed with hexane to give ethyl (2E)-3-[4'-(2-butoxyethoxy)-4-(3,4-dimethylpyrrolidin-1-yl)-1,1'-biphenyl-3-yl]-2-methylacrylate (1.77 g) as yellow crystals.

m.p. 67.0-69.0°C.

$^1$H-NMR (300 MHz, $CDCl_3$) δ 0.88-0.98 (9H, m), 1.32-1.45 (5H, m), 1.55-1.65 (2H, m), 2.02 (3H, d, J=1.2 Hz), 2.22-2.35 (2H, m), 2.98-3.03 (2H, m), 3.37-3.42 (2H, m), 3.54 (2H, t, J=6.9 Hz), 3.80 (2H, t, J=4.8 Hz), 4.14 (2H, t, J=4.8 Hz), 4.27 (2H, q, J=6.9 Hz), 6.79 (1H, d, J=8.4 Hz), 6.95 (2H, d, J=8.7 Hz), 7.30 (1H, d, J=2.1 Hz), 7.38 (1H, dd, J=8.4, 2.1 Hz), 7.43 (2H, d, J=8.7 Hz), 7.81 (1H, s).

Elementary analysis $C_{30}H_{41}NO_4$, Calcd. C, 75.12 ; H, 8.62 ; N, 2.92 : Found C, 74.83 ; H, 8.33 ; N, 2.77.

Reference Example 143

Ethyl (2E)-3-[4'-(2-butoxyethoxy)-4-(3,4-dimethylpyrrolidin-1-yl)-1,1'-biphenyl-3-yl]-2-methylacrylate (1.04 g) was dissolved in THF (35 ml) and methanol (35 ml). Then, a 1 N aqueous sodium hydroxide solution (8.7 ml) was added thereto, and the mixture was stirred for 3 hours at 90°C. After adding water at 0°C, the resulting mixture was neutralized with 1 N hydrochloric acid and extracted with ethyl acetate. The organic layer was washed with saturated brine and dried over magnesium sulfate. The solvent was distilled off under reduced pressure, and then the resulting residue was washed with hexane to give (2E)-3-[4'-(2-butoxyethoxy)-4-(3,4-dimethylpyrrolidin-1-yl)-1,1'-biphenyl-3-yl]-2-methylacrylic acid (0.97 g) as yellow crystals.

m.p. 126.3-128.3°C.

$^1$H-NMR (300 MHz, $CDCl_3$) δ 0.87-0.99 (9H, m), 1.33-1.45 (2H, m), 1.56-1.65 (2H, m), 2.05 (3H, d, J=1.2 Hz), 2.27-2.37 (2H, m), 3.00-3.05 (2H, m), 3.40-3.45 (2H, m), 3.55 (2H, t, J=6.9 Hz), 3.80 (2H, t, J=4.8 Hz), 4.15 (2H, t, J=4.8 Hz), 6.83 (1H, d, J=7.2 Hz), 6.97 (2H, d, J=8.7 Hz), 7.34 (1H, d, J=1.8 Hz), 7.40 (1H, dd, J=7.2, 1.8 Hz), 7.44 (2H, d, J=8.7 Hz), 7.97 (1H, s).

Elementary analysis $C_{28}H_{37}NO_4$, Calcd. C, 74.47 ; H, 8.26 ; N, 3.10 : Found C, 74.32 ; H, 8.44 ; N, 2.87.

Reference Example 144

To 5-bromo-2-hydroxynicotinic acid (40 g) was added dropwise thionyl chloride (200 ml) at 0°C. Next, DMF (12.5 ml) was added dropwise thereto at 0°C, and the resulting mixture was refluxed for 2 hours. After returning to room temperature, excess thionyl chloride was distilled off under reduced pressure. To the resulting residue was added dropwise methanol (450 ml) at 0°C, and the solvent was distilled off under reduced pressure. To the resulting residue was added an aqueous saturated sodium hydrogen carbonate solution at 0°C, which was neutralized and then extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 9 : 1) to give methyl 5-bromo-2-chloronicotinate (39.5 g) as colorless crystals.

$^1$H-NMR (300 MHz, $CDCl_3$) δ 3.97 (3H, s), 8.28 (1H, d, J=2.4 Hz), 8.56 (1H, d, J=2.4 Hz).

Elementary analysis $C_7H_5NO_2ClBr$, Calcd. C, 33.57 ; H, 2.01 ; N, 5.59 : Found C, 33.53 ; H, 2.21 ; N, 5.66.

Reference Example 145

To a suspension of sodium hydride (60% oily material, 48 mg) in DMF (5 ml) was added pyrrolidine (0.1 ml) at 0°C, and the mixture was stirred for 1 hour at room temperature under a nitrogen atmosphere. Next, a solution of methyl 5-bromo-2-chloronicotinate (100 mg) in DMF (5 ml) was added dropwise at thereto 0°C under a nitrogen atmosphere, and the mixture was stirred overnight at 75°C. After returning to room temperature, 0.1 N hydrochloric acid was added thereto, which was acidified and then extracted with ethyl acetate. The organic layer was washed with saturated brine and dried over magnesium sulfate. The solvent was distilled off under reduced pressure, and then the resulting residue was washed with hexane to give 5-bromo-2-pyrrolidin-1-ylnicotinic acid (67 mg) as colorless crystals.

$^1$H-NMR (300 MHz, $CDCl_3$) δ 2.01-2.11 (4H, m), 3.38-3.43 (4H, m), 8.35 (1H, d, J=2.4 Hz), 8.43 (1H, d, J=2.4 Hz).

Elementary analysis $C_{10}H_{11}N_2O_2Br$, Calcd. C, 44.30 ; H, 4.09 ; N, 10.33 : Found C, 44.34 ; H, 4.06 ; N, 10.29.

Reference Example 146

To a solution of 5-bromo-2-pyrrolidin-1-ylnicotinic acid (800 mg), N,O-dimethylhydroxylamine hydrochloride (375 mg) and 1-hydroxybenzotriazole monohydrate (588 mg) in DMF (20 ml) was added triethylamine (0.54 ml) and a catalytic amount of 4-(N,N-dimethylamino)pyridine, followed by adding 1-ethyl-3-(3-dimethylaminopropyl)-carbodiimide hydrochloride (736 mg), and the mixture was stirred overnight under a nitrogen atmosphere. Water was added thereto and the mixture was extracted with ethyl acetate. The organic layer was washed with an aqueous saturated sodium hydrogen carbonate solution, water and saturated brine, and then dried over magnesium sulfate. The solvent was distilled off under reduced pressure, and then the resulting residue was purified by silica gel column chromatography (hexane : ethyl acetate = 8 : 1 → hexane : ethyl acetate = 1 : 1) and recrystallized from hexane-ethyl acetate to give 5-bromo-N-methoxy-N-methyl-2-pyrrolidin-1-ylnicotinamide (812 mg) as colorless crystals.

m.p. 94.0-96.0°C.

$^1$H-NMR (300 MHz, $CDCl_3$) δ 1.91-1.96 (4H, m), 3.29 (3H, s), 3.37-3.41 (4H, m), 3.57 (3H, br), 7.49 (1H, d, J=2.4 Hz), 8.17 (1H, d, J=2.4 Hz).

Elementary analysis $C_{12}H_{16}N_3O_2Br$, Calcd. C, 45.87 ; H, 5.13 ; N, 13.37 : Found C, 45.83 ; H, 5.07 ; N, 13.22.

Reference Example 147

To a solution of lithium aluminum hydride (74.2 mg) in tetrahydrofuran (10 ml) was added dropwise a solution of 5-bromo-N-methoxy-N-methyl-2-pyrrolidin-1-ylnicotinamide (615 mg) in tetrahydrofuran (10 ml) at 0°C under a nitrogen atmosphere, and then the mixture was returned to room temperature and stirred for 30 minutes. Water (0.08 ml), an aqueous 15% sodium hydroxide solution (0.08 ml) and water (0.24 ml) were sequentially added thereto at 0°C, and then the mixture was returned to room temperature and stirred overnight. After adding magnesium sulfate, the insolubles were removed by filtration. The solvent was distilled off under reduced pressure to give 5-bromo-2-pyrrolidin-1-ylnicotinaldehyde (470 mg) as a yellow oily material.

$^1$H-NMR (300 MHz, $CDCl_3$) δ 1.96-2.01 (4H, m), 3.50-3.54 (4H, m), 8.00 (1H, d, J=2.4 Hz), 8.30 (1H, d, J=2.4 Hz), 9.95 (1H, s).

Reference Example 148

A suspension of 5-bromo-2-pyrrolidin-1ylnicotinaldehyde (450 mg), 4-(2-butoxyethoxy)phenylboric acid (545 mg) and potassium carbonate (634 mg) in toluene (20 ml), ethanol (2 ml) and water (2 ml) was stirred for 1 hour under an argon atmosphere. Then, tetrakis(triphenylphosphine)palladium (102 mg) was added thereto, and the resulting mixture was refluxed for 7 hours. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 9 : 1 → hexane : ethyl acetate = 2 : 1) and recrystallized from hexane-ethyl acetate to give 5-[4-(2-butoxyethoxy)phenyl]-2-pyrrolidin-1-ylnicotinaldehyde (558 mg) as yellow crystals.

$^1$H-NMR (300 MHz, $CDCl_3$) δ 0.93 (3H, t, J=7.5 Hz), 1.33-1.47 (2H, m), 1.55-1.66 (2H, m), 1.99-2.05 (4H, m), 3.51-3.62 (6H, m), 3.81 (2H, t, J=5.1 Hz), 4.15 (2H, t, J=5.1 Hz), 7.01 (2H, d, J=9.0 Hz), 7.46 (2H, d, J=9.0 Hz), 8.11 (1H, d, J=2.4 Hz), 8.56 (1H, d, J=2.4 Hz), 10.11 (1H, s).

Elementary analysis $C_{22}H_{28}N_2O_3$, Calcd. C, 71.71 ; H, 7.66 ; N, 7.60 : Found C, 71.63 ; H, 7.71 ; N, 7.42.

Reference Example 149

To a suspension of sodium hydride (42 mg) in toluene (10 ml) was added dropwise a solution of ethyl diethylphosphonoacetate (0.189 ml) in toluene (10 ml) at 0°C under a nitrogen atmosphere, and the mixture was stirred as such for 1 hour. Next, a solution of 5-[4-(2-butoxyethoxy)phenyl]-2-pyrrolidin-1-ylnicotinaldehyde (270 mg) in toluene (10 ml) was added dropwise thereto at 0°C under a nitrogen atmosphere, and then the resulting mixture was refluxed for 3 hours. After removing from the oil bath, water was added thereto and the mixture was extracted with ethyl acetate. The organic layer was washed with saturated brine and dried over magnesium sulfate. The solvent was distilled off under reduced pressure, and then the resulting residue was purified by basic silica gel column chromatography (hexane : ethyl acetate = 13 : 1 → hexane : ethyl acetate = 1 : 1) to give ethyl (2E)-3-[5-[4-(2-butoxyethoxy)phenyl]-2-pyrrolidin-1-ylpyridin-3-yl]acrylate (229 mg) as a yellow oily material.

$^1$H-NMR (300 MHz, $CDCl_3$) δ 0.93 (3H, t, J=7.2 Hz), 0.96-1.43 (5H, m), 1.51-1.64 (2H, m), 1.92-1.97 (4H, m), 3.53-3.63 (6H, m), 3.81 (2H, t, J=5.1 Hz), 4.16 (2H, t, J=5.1 Hz), 4.26 (2H, q, J=7.2 Hz), 6.23 (1H, d, J=15.6 Hz), 6.99 (2H, d, J=8.7 Hz), 7.43 (2H, d, J=8.7 Hz), 7.76 (1H, d, J=1.8 Hz), 8.01 (1H, d, J=15.6 Hz), 8.37 (1H, d, J=1.8 Hz).

Reference Example 150

Ethyl (2E)-3-[5-[4-(2-butoxyethoxy)phenyl]-2-pyrrolidin-1-ylpyridin-3-yl]acrylate (215 mg) was dissolved in THF (6 ml) and methanol (6 ml). Then, a 1 N aqueous sodium hydroxide solution (2.0 ml) was added thereto, and the mixture was stirred for 3 hours at 90°C. After adding water at 0°C, the resulting mixture was neutralized with 1 N hydrochloric acid and extracted with ethyl acetate. The organic layer was washed with saturated brine and dried over magnesium sulfate. The solvent was distilled off under reduced pressure, and then the resulting residue was washed with hexane to give (2E)-3-[5-[4-(2-butoxyethoxy)phenyl]-2-pyrrolidin-1-ylpyridin-3-yl]acrylic acid (200 mg) as yellow crystals.

m.p. 162.5-164.5°C.

$^1$H-NMR (300 MHz, $CDCl_3$) δ 0.93 (3H, t, J=7.2 Hz), 1.35-1.47 (2H, m), 1.52-1.70 (2H, m), 1.93-1.98 (4H, m), 3.55 (2H, t, J=6.6 Hz), 3.58-3.63 (4H, m), 3.81 (2H, t, J=4.8 Hz), 4.16 (2H, t, J=4.8 Hz), 6.24 (1H, d, J=15.6 Hz), 7.00 (2H, d, J=8.7 Hz), 7.44 (2H, d, J=8.7 Hz), 7.79 (1H, d, J=2.4 Hz), 8.10 (1H, d, J=15.6 Hz), 8.39 (1H, d, J=2.4 Hz).

Elementary analysis $C_{24}H_{30}N_2O_4$, Calcd. C, 70.22 ; H, 7.37 ; N, 6.82 : Found C, 69.97 ; H, 7.22 ; N, 6.61.

Reference Example 151

To a suspension of sodium hydride (42 mg) in toluene (10 ml) was added dropwise a solution of triethyl 2-phosphonopropionate (0.204 ml) in toluene (10 ml) at 0°C under a nitrogen atmosphere, and the mixture was stirred as such for 1 hour. Next, a solution of 5-[4-(2-butoxyethoxy)phenyl]-2-pyrrolidin-1-ylnicotinaldehyde (270 mg) in toluene (10 ml) was added dropwise thereto at 0°C under a nitrogen atmosphere, and then the resulting mixture was refluxed for 3 hours. After removing from the oil bath, water was added thereto and the mixture was extracted with ethyl acetate. The organic layer was washed with saturated brine and dried over magnesium sulfate. The solvent was distilled off under reduced pressure, and then the resulting residue was purified by basic silica gel column chromatography (hexane : ethyl acetate = 9 : 1 → hexane : ethyl acetate = 6 : 1) to give ethyl (2E)-3-[5-[4-(2-butoxyethoxy)phenyl]-2-pyrrolidin-1-ylpyridin-3-yl]-2-methylacrylate (275 mg) as a green oily material.

$^1$H-NMR (300 MHz, $CDCl_3$) δ 0.93 (3H, t, J=8.1 Hz), 1.33-1.50 (5H, m), 1.55-1.65 (2H, m), 1.84-1.94 (4H, m), 1.99 (3H, d, J=1.5 Hz), 3.47-3.57 (6H, m), 3.80 (2H, t, J=4.5 Hz), 4.15 (2H, t, J=4.5 Hz), 4.28 (2H, q, J=6.9 Hz), 6.99 (2H, d, J=9.0 Hz), 7.41 (2H, d, J=9.0 Hz), 7.50 (1H, d, J=2.4 Hz), 7.77 (1H, s), 8.33 (1H, d, J=2.4 Hz).

Reference Example 152

Ethyl (2E)-3-[5-[4-(2-butoxyethoxy)phenyl]-2-pyrrolidin-1-ylpyridin-3-yl]-2-methylacrylate (265 mg) was dissolved in THF (7 ml) and methanol (7 ml). Then, a 1 N aqueous sodium hydroxide solution (2.34 ml) was added thereto, and the mixture was stirred for 3 hours at 90°C. After adding water at 0°C, the resulting mixture was neutralized with 1 N hydrochloric acid and extracted with ethyl acetate. The organic layer was washed with saturated brine and dried over magnesium sulfate. The solvent was distilled off under reduced pressure, and then the resulting residue was washed with hexane to give (2E)-3-[5-[4-(2-butoxyethoxy)phenyl]-2-pyrrolidin-1-ylpyridin-3-yl]-2-methylacrylic acid (184 mg) as yellow crystals.

m.p. 106.0-107.0°C.

$^1$H-NMR (300 MHz, $CDCl_3$) δ 0.93 (3H, t, J=7.5 Hz), 1.33-1.46 (2H, m), 1.56-1.65 (2H, m), 1.91-1.95 (4H, m), 2.02 (3H, d, J=1.2 Hz), 3.50-3.57 (6H, m), 3.80 (2H, t, J=4.8 Hz), 4.16 (2H, t, J=4.8 Hz), 6.99 (2H, d, J=8.7 Hz), 7.42 (2H, d, J=8.7 Hz), 7.53 (1H, d, J=1.8 Hz), 7.90 (1H, s), 8.35 (1H, d, J=1.8 Hz).

Elementary analysis $C_{25}H_{32}N_2O_4$, Calcd. C, 70.73 ; H, 7.60 ; N, 6.60 : Found C, 70.65 ; H, 7.86 ; N, 6.42.

Reference Example 153

To a suspension of calcium chloride (33.6 g) in ethanol (250 ml) and tetrahydrofuran (250 ml) was added sodium borohydride (23.0 g) at 0°C portionwise. After stirring for 1 hour at 0°C with a calcium chloride tube equipped, methyl 5-bromo-2-chloronicotinate (19.0 g) was added thereto, and the mixture was stirred as such overnight at 0°C. The reaction mixture was acidified with 2.5 N hydrochloric acid at 0°C, which was returned to room temperature and stirred for 1 hour. After neutralization with an aqueous sodium hydrogen carbonate solution, the insolubles were removed by filtration. The solvent was distilled off under reduced pressure, and then the resulting residue was purified by silica gel column chromatography (hexane : ethyl acetate = 9 : 1 → hexane : ethyl acetate = 4 : 1) to give (5-bromo-2-chloropyridin-3-yl)methanol (15.0 g) as colorless crystals.

m.p. 86.7-87.5°C.

$^{1}$R-NMR (300 MHz, $CDCl_3$) δ 4.77 (2H, s), 8.04 (1H, d, J=2.4 Hz), 8.36 (1H, d, J=2.4 Hz).

Elementary analysis $C_6H_5NOClBr$, Calcd. C, 32.39 ; H, 2.27 ; N, 6.30 : Found C, 32.38 ; H, 2.24 ; N, 6.28.

Reference Example 154

To a solution of oxalyl chloride (1.98 ml) in dichloromethane (20 ml) was added dropwise a solution of DMSO (3.45 ml) in dichloromethane (30 ml) at -78°C under a nitrogen atmosphere. The mixture was stirred as such for 10 minutes, and then a solution of 5-bromo-2-chloropyridin-3-yl)methanol (3.6 g) in dichloromethane (35 ml) was added dropwise thereto. The mixture was stirred as such for 10 minutes, and then triethylamine (13.5 ml) was added dropwise thereto. After stirring as such for 10 minutes, the resulting mixture was returned to room temperature and stirred for 1 hour. To the reaction solution was added water, followed by separation. The organic layer was washed with water and saturated brine, and dried over magnesium sulfate. The solvent was distilled off under reduced pressure, and then the resulting residue was purified by silica gel column chromatography (hexane : ethyl acetate = 6 : 1) to give 5-bromo-2-chloronicotinaldehyde (3.4 g) as colorless crystals.

m.p. 88.0-89.0°C.

$^{1}$H-NMR (300 MHz, $CDCl_3$) δ 8.33 (1H, d, J=2.7 Hz), 8.67 (1H, d, J=2.7 Hz), 10.38 (1H, s).

Elementary analysis $C_6H_3NOClBr$, Calcd. C, 32.69 ; H, 1.37 ; N, 6.35 : Found C, 32.51 ; H, 1.33 ; N, 6.18.

Reference Example 155

A suspension of 5-bromo-2-chloronicotinaldehyde (1.2 g), 3-methylpyrrolidine (928 mg) and sodium carbonate (1.16 g) in DMSO (40 ml) and water (20 ml) was stirred for 2 hours at 75°C under a nitrogen atmosphere. After returning to room temperature, water was added thereto and the mixture was extracted with ethyl acetate. The organic layer was washed with water and saturated brine, and dried over magnesium sulfate. The solvent was distilled off under reduced pressure, and then the resulting residue was separated and purified by silica gel column chromatography (hexane : ethyl acetate = 20 : 1 → hexane : ethyl acetate = 4 : 1). The resulting solids were washed with hexane to give 5-bromo-2-(3-methylpyrrolidin-1-yl)nicotinaldehyde (761 mg) as yellow crystals.

m.p. 72.0-73.0°C.

$^{1}$H-NMR (300 MHz, $CDCl_3$) δ 1.13 (3H, d, J=6.6 Hz), 1.53-1.67 (1H, m), 2.07-2.16 (1H, m), 2.28-2.41 (1H, m), 3.17-3.24 (1H, m), 3.46-3.56 (2H, m), 3.61-3.70 (1H, m), 8.01 (1H, d, J=2.4 Hz), 8.31 (1H, d, J=2.4 Hz), 9.96 (1H, s).

Elementary analysis $C_{11}H_{13}N_2OBr$, Calcd. C, 49.09 ; H, 4.87 ; N, 10.41 : Found C, 49.07 ; H, 4.88 ; N, 10.29.

Reference Example 156

To a suspension of sodium hydride (155 mg) in toluene (10 ml) was added dropwise a solution of triethyl 2-phosphonopropionate (0.76 ml) in toluene (10 ml) at 0°C under a nitrogen atmosphere, and the mixture was stirred as such for 1 hour. Next, a solution of 5-bromo-2-(3-methylpyrrolidin-1-yl)nicotinaldehyde (730 mg) in toluene (10 ml) was added dropwise thereto at 0°C under a nitrogen atmosphere, and the resulting mixture was refluxed for 3 hours. After removing from the oil bath, water was added thereto and the mixture was extracted with ethyl acetate. The organic layer was washed with saturated brine and dried over magnesium sulfate. The solvent was distilled off under reduced pressure, and then the resulting residue was purified by basic silica gel column chromatography (hexane : ethyl acetate = 19 : 1 → hexane : ethyl acetate = 4 : 1) to give ethyl (2E)-3-[5-bromo-2-(3-methylpyrrolidin-1-yl)pyridin-3-yl]-2-methylacrylate (914 mg) as a yellow oily material.

$^{1}$H-NMR (300 MHz, $CDCl_3$) δ 1.07 (3H, d, J=6.6 Hz), 1.34 (3H, t, J=6.9 Hz), 1.45-1.62 (1H, m), 1.94 (3H, d, J=1.5 Hz), 1.96-2.08 (1H, m), 2.20-2.35 (1H, m), 3.01-3.07 (1H, m), 3.42-3.55 (3H, m), 4.26 (2H, q, J=6.9 Hz), 7.37 (1H, d, J=2.4 Hz), 7.60 (1H, s), 8.10 (1H, d, J=2.4 Hz).

Reference Example 157

A suspension of ethyl (2E)-3-[5-bromo-2-(3-methylpyrrolidin-1-yl)pyridin-3-yl]-2-methylacrylate (880 mg), 4-(2-butoxyethoxy)phenylboric acid (774 mg) and potassium carbonate (898 mg) in toluene (15 ml), ethanol (1.5 ml) and water (1.5 ml) was stirred for 1 hour under an argon atmosphere. Then, tetrakis(triphenylphosphine)palladium (144 mg) was added thereto, and the resulting mixture was refluxed for 5 hours. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 19 : 1 → hexane : ethyl acetate = 4 : 1) to give ethyl (2E)-3-[5-[4-(2-butoxyethoxy)phenyl]-2-(3-methylpyrrolidin-1-yl)pyridin-3-yl]-2-methylacrylate (970 mg) as a green oily material.

$^1$H-NMR (300 MHz, $CDCl_3$) δ 0.93 (3H, t, J=7.5 Hz), 1.10 (3H, d, J=6.6 Hz), 1.32-1.65 (8H, m), 1.98-2.10 (4H, m), 2.20-2.40 (1H, m), 3.09-3.16 (1H, m), 3.47-3.63 (5H, m), 3.79 (2H, t, J=4.8 Hz), 4.16 (2H, t, J=4.8 Hz), 4.27 (2H, q, J=7.5 Hz), 6.97 (2H, d, J=8.7 Hz), 7.40 (2H, d, J=8.7 Hz), 7.48 (1H, d, J=2.4 Hz), 7.75 (1H, s), 8.31 (1H, d, J=2.4 Hz).

Reference Example 158

Ethyl (2E)-3-[5-[4-(2-butoxyethoxy)phenyl]-2-(3-methylpyrrolidin-1-yl)pyridin-3-yl]-2-methylacrylate (950 mg) was dissolved in THF (32 ml) and methanol (32 ml). Then, a 1 N aqueous sodium hydroxide solution (8.14 ml) was added thereto, and the mixture was stirred for 3 hours at 90°C. After adding water at 0°C, the resulting mixture was neutralized with 1 N hydrochloric acid and extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 1 : 1 → ethyl acetate). The resulting solids were washed with hexane to give (2E)-3-[5-[4-(2-butoxyethoxy)phenyl]-2-(3-methylpyrrolidin-1-yl)pyridin-3-yl]-2-methylacrylic acid (758 mg) as yellow crystals.

m.p. 106.5-108.5°C.

$^1$H-NMR (300 MHz, $CDCl_3$) δ 0.93 (3H, t, J=7.5 Hz), 1.11 (3H, d, J=6.6 Hz), 1.33-1.46 (2H, m), 1.51-1.66 (3H, m), 1.95-2.10 (4H, m), 2.22-2.40 (1H, m), 3.12-3.18 (1H, m), 3.50-3.64 (5H, m), 3.81 (2H, t, J=4.8 Hz), 4.16 (2H, t, J=4.8 Hz), 6.99 (2H, d, J=8.7 Hz), 7.42 (2H, d, J=8.7 Hz), 7.53 (1H, d, J=2.4 Hz), 7.90 (1H, s), 8.35 (1H, d, J=2.4 Hz).

Elementary analysis $C_{26}H_{34}N_2O_4$, Calcd. C, 71.21 ; H, 7.81 ; N, 6.39 : Found C, 71.07 ; H, 7.74 ; N, 6.13.

Reference Example 159

A suspension of 5-bromo-2-chloronicotinaldehyde (1.5 g), 3-hydroxymethylpyrrolidine hydrochloride (1.87 g) and sodium carbonate (1.8 g) in DMSO (45 ml) and water (22.5 ml) was heated for 2 hours at 75°C under a nitrogen atmosphere. After returning to room temperature, water was added thereto and the mixture was extracted with ethyl acetate. The organic layer was washed with water and saturated brine, and dried over magnesium sulfate. The solvent was distilled off under reduced pressure, and then the resulting residue was separated and purified by silica gel column chromatography (hexane : ethyl acetate = 2 : 1 → ethyl acetate). The resulting solids were washed with hexane to give 5-bromo-2-[3-(hydroxymethyl)pyrrolidin-1-yl]nicotinaldehyde (1.67 g) as yellow crystals.

m.p. 84.0-85.5°C.

$^1$H-NMR (300 MHz, $CDCl_3$) δ 1.47-1.60 (1H, m), 1.78-1.90 (1H, m), 2.08-2.20 (1H, m), 2.49-2.59 (1H, m), 3.42-3.74 (6H, m), 8.02 (1H, d, J=2.4 Hz), 8.32 (1H, d, J=2.4 Hz), 9.96 (1H, s) .

Elementary analysis $C_{11H13}N_2O_2Br$, Calcd. C, 46.33 ; H, 4.60 ; N, 9.82 : Found C, 46.50 ; H, 4.57 ; N, 9.74.

Reference Example 160

To a solution of 5-bromo-2-[3-(hydroxymethyl)pyrrolidin-1-yl]nicotinaldehyde (1.75 g) in pyridine (10 ml) was added dropwise acetic anhydride (2.32 ml) at 0°C under a nitrogen atmosphere. The mixture was returned to room temperature and stirred for 3 hours. Then, water was added thereto at 0°C, and further added sodium carbonate, which was neutralized. After extraction with ethyl acetate, the organic layer was washed with water and saturated brine, and the solvent was distilled off under reduced pressure. The resulting residue was purified by silica gel column chromatography (hexane : ethyl acetate= 3 : 1 → hexane : ethyl acetate = 1 : 1) to give [1-(5-bromo-3-formylpyridin-2-yl)pyrrolidin-3-yl]methyl acetate (1.76 g) as a yellow oily material.

$^1$H-NMR (300 MHz, $CDCl_3$) δ 1.74-1.86 (1H, m), 2.07 (3H, s), 2.12-2.17 (1H, m), 2.60-2.70 (1H, m), 3.38-3.45 (1H, m), 3.50-3.70 (3H, m), 4.02-4.21 (2H, m), 8.02 (1H, d, J=1.8 Hz), 8.32 (1H, d, J=1.8 Hz), 9.95 (1H, s).

Reference Example 161

A solution of [1-(5-bromo-3-formylpyridin-2-yl)pyrrolidin-3-yl]methyl acetate (1.6 g), tert-butyl 2-(triphenylphosphoranylidene)propanoate (2.48 g) in toluene (50 ml) was refluxed for 3 hours under a nitrogen atmosphere. After returning to room temperature, water was added thereto and the mixture was extracted with ethyl acetate. The organic layer was washed with saturated brine, and dried over magnesium sulfate. The solvent was distilled off under reduced pressure, and then the resulting residue was purified by basic silica gel column chromatography (hexane : ethyl acetate = 15 : 1 → hexane : ethyl acetate = 4 : 1) to give tert-butyl (2E)-3-[2-[3-(acetoxymethyl)pyrrolidin-1-yl]-5-bromopyridin-3-yl]-2-methylacrylate (1.44 g) as a yellow oily material.

$^1$H-NMR (300 MHz, $CDCl_3$) δ 1.53 (9H, s), 1.67-1.76 (1H, m), 1.90 (3H, d, J=1.5 Hz), 2.00-2.10 (4H, m), 2.52-2.61 (1H, m), 3.25-3.31 (1H, m), 3.45-3.50 (2H, m), 3.55-3.61 (1H, m), 4.00-4.15 (2H, m), 7.38 (1H, d, J=3.0 Hz), 7.48 (1H, s), 8.10 (1H, d, J=3.0 Hz).

Reference Example 162

A suspension of tert-butyl (2E)-3-[2-[3-(acetoxymethyl)pyrrolidin-1-yl]-5-bromopyridin-3-yl]-2-methylacrylate (1.4 g), 4-(2-butoxyethoxy)phenylboric acid (986 mg) and potassium carbonate (1.15 g) in toluene (25 ml), ethanol(2.5 ml) and water (2.5 ml) was stirred for 1 hour under an argon atmosphere. Then, tetrakis(triphenylphosphine)palladium (184 mg) was added thereto, and the resulting mixture was refluxed for 6 hours. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 9 : 1 → hexane : ethyl acetate = 3 : 1) to give tert-butyl (2E)-3-[2-[3-(acetoxymethyl)pyrrolidin-1-yl)-5-[4-(2-butoxyethoxy)phenyl]pyridin-3-yl]-2-methylacrylate (1.41 g) as a yellow oily material.

$^1$H-NMR (300 MHz, $CDCl_3$) δ 0.93 (3H, t, J=7.5 Hz), 1.30-1.80 (14H, m), 1.95 (3H, d, J=1.5 Hz), 2.00-2.20 (4H, m), 2.50-2.65 (1H, m), 3.34-3.40 (1H, m), 3.53-3.57 (4H, m), 3.63-3.69 (1H, m), 3.80 (2H, t, J=4.5 Hz), 4.03-4.18 (4H, m), 6.99 (2H, d, J=9.0 Hz), 7.41 (2H, d, J=9.0 Hz), 7.51 (1H, d, J=2.1 Hz), 7.64 (1H, s), 8.32 (1H, d, J=2.1 Hz).

Reference Example 163

tert-Butyl (2E)-3-[2-[3-(acetoxymethyl)pyrrolidin-1-yl]-5-[4-(2-butoxyethoxy)phenyl]pyridin-3-yl]-2-methylacrylate (1.25 g) was dissolved in ethyl acetate (17 ml). Then, a 4 N hydrochloric acid-ethyl acetate solution (17 ml) was added thereto, and the mixture was stirred overnight under a nitrogen atmosphere. After adding water at 0°C, potassium carbonate (4.7 g) was added thereto, and the mixture was extracted with ethyl acetate. The organic layer was washed with water and saturated brine, and dried over magnesium sulfate. The solvent was distilled off under reduced pressure, and then the resulting residue was purified by silica gel column chromatography (hexane : ethyl acetate = 1 : 1 → ethyl acetate). The resulting solids were washed with hexane-diisopropyl ether to give (2E)-3-[2-[3-(acetoxymethyl)pyrrolidin-1-yl]-5-[4-(2-butoxyethoxy)phenyl]pyridin-3-yl]-2-methylacrylic acid (823 mg) as yellow crystals.

m.p. 82.2-84.2°C.

$^1$H-NMR (300 MHz, $CDCl_3$) δ 0.93 (3H, t, J=7.2 Hz), 1.33-1.45 (2H, m), 1.50-1.80 (3H, m), 2.02-2.17 (7H, m), 2.50-2.67 (1H, m), 3.35-3.41 (1H, m), 3.49-3.59 (4H, m), 3.63-3.69 (1H, m), 3.81 (2H, t, J=4.8 Hz), 4.04-4.17 (4H, m), 7.00 (2H, d, J=8.7 Hz), 7.42 (2H, d, J=8.7 Hz), 7.54 (1H, d, J=2.1 Hz), 7.87 (1H, s), 8.35 (1H, d, J=1.5 Hz).

Elementary analysis $C_{28}H_{36}N_2O_6$, Calcd. C, 67.72 ; H, 7.31 ; N, 5.64 : Found C, 67.64 ; H, 7.26 ; N, 5.48.

Reference Example 164

A suspension of 5-bromo-2-chloronicotinaldehyde (1.5 g), 3,4-dimethylpyrrolidine hydrochloride (1.85 g) and sodium carbonate (1.8 g) in DMSO (45 ml) and water (22.5 ml) was stirred for 3 hours at 80°C under a nitrogen atmosphere. After returning to room temperature, water was added thereto and the mixture was extracted with ethyl acetate. The organic layer was washed with saturated brine, and dried over magnesium sulfate. The solvent was distilled off under reduced pressure, which was separated and purified by silica gel column chromatography (hexane : ethyl acetate = 19 : 1 → hexane : ethyl acetate = 4 : 1). The resulting residue recrystallized from hexane-ethyl acetate to give 5-bromo-2-(3,4-dimethylpyrrolidin-1-yl)nicotinaldehyde (1.56 g) as yellow crystals.

m.p. 98.5-99.5°C.

$^1$H-NMR (300 MHz, $CDCl_3$) δ 0.98 (6H, d, J=6.6 Hz), 2.31-2.41 (2H, m), 3.22-3.28 (2H, m), 3.59-3.65 (2H, m), 8.00 (1H, d, J=2.4 Hz), 8.29 (1H, d, J=2.4 Hz), 9.95 (1H, s).

Elementary analysis $C_{12}H_{15}N_2OBr$, Calcd. C, 50.90 ; H, 5.34 ; N, 9.89 : Found C, 50.93 ; H, 5.35 ; N, 9.82.

Reference Example 165

To a suspension of sodium hydride (303 mg) in toluene (20 ml) was added dropwise a solution of triethyl 2-phosphonopropionate (1.48 ml) in toluene (20 ml) at 0°C under a nitrogen atmosphere, and the mixture was stirred as such for 1 hour. Next, a solution of 5-bromo-2-(3,4-dimethylpyrrolidin-1-yl)nicotinaldehyde (1.5 g) in toluene (20 ml) was added dropwise thereto at 0°C under a nitrogen atmosphere, and then the resulting mixture was refluxed for 3 hours. After removing from the oil bath, water was added thereto and the mixture was extracted with ethyl acetate. The organic layer was washed with saturated brine, and dried over magnesium sulfate. The solvent was distilled off under reduced pressure, and then the resulting residue was purified by basic silica gel column chromatography (hexane : ethyl acetate = 10 : 1 → hexane : ethyl acetate = 1 : 1) to give ethyl (2E)-3-[5-bromo-2-(3,4-dimethylpyrrolidin-1-yl)pyridin-3-yl]-2-methylacrylate (1.94 g) as a yellow oily material.

$^1$H-NMR (300 MHz, $CDCl_3$) δ 0.94 (6H, d, J=6.6 Hz), 1.34 (3H, t, J=7.2 Hz), 1.93 (3H, d, J=1.5 Hz), 2.20-2.30 (2H, m), 3.11-3.17 (2H, m), 3.48-3.54 (2H, m), 4.26 (2H, q, J=7.2 Hz), 7.37 (1H, d, J=2.1 Hz), 7.60 (1H, s), 8.09 (1H, d, J=2.1 Hz).

Reference Example 166

A suspension of ethyl (2E)-3-[5-bromo-2-(3,4-dimethylpyrrolidin-1-yl)pyridin-3-yl]-2-methylacrylate (1.9 g), 4-(2-butoxyethoxy)phenylboric acid (1.6 g) and potassium carbonate (1.87 g) in toluene (30 ml), ethanol (3 ml) and water (3 ml) was stirred for 1 hour under an argon atmosphere. Then, tetrakis(triphenylphosphine)palladium (300 mg) was added thereto, and the resulting mixture was refluxed for 5 hours. After returning to room temperature, water was added thereto and the mixture was extracted with ethyl acetate. The organic layer was washed with saturated brine and dried over magnesium sulfate. The solvent was distilled off under reduced pressure, and then the resulting residue was purified by silica gel column chromatography (hexane : ethyl acetate = 19 : 1 → hexane : ethyl acetate = 4 : 1) to give ethyl (2E)-3-[5-[4-(2-butoxyethoxy)phenyl]-2-(3,4-dimethylpyrrolidin-1-yl)pyridin-3-yl]-2-methylacrylate (1.92 g) as a yellow oily material.

$^1$H-NMR (300 MHz, $CDCl_3$) δ 0.90-0.98 (9H, m), 1.33-1.45 (5H, m), 1.55-1.65 (2H, m), 1.98 (3H, d, J=1.2 Hz), 2.22-2.37 (2H, m), 3.20-3.25 (2H, m), 3.53-3.62 (4H, m), 3.80 (2H, t, J=4.8 Hz), 4.15 (2H, t, J=4.8 Hz), 4.28 (2H, q, J=7.2 Hz), 6.98 (2H, d, J=8.7 Hz), 7.41 (2H, d, J=8.7 Hz), 7.49 (1H, d, J=2.4 Hz), 7.76 (1H, s), 8.32 (1H, d, J=2.4 Hz).

Reference Example 167

Ethyl (2E)-3-[5-[4-(2-butoxyethoxy)phenyl]-2-(3,4-dimethylpyrrolidin-1-yl)pyridin-3-yl]-2-methylacrylate (1.9 g) was dissolved in THF (60 ml) and methanol (60 ml). Then, a 1 N aqueous sodium hydroxide solution (15.8 ml) was added thereto, and the mixture was stirred for 4 hours at 90°C. After adding water at 0°C, the resulting mixture was neutralized with 1 N hydrochloric acid and extracted with ethyl acetate. The organic layer was washed with saturated brine, and dried over magnesium sulfate. The solvent was distilled off under reduced pressure, and then the resulting residue was washed with hexane to give (2E)-3-[5-[4-(2-butoxyethoxy)phenyl]-2-(3,4-dimethylpyrrolidin-1-yl)pyridin-3-yl]-2-methylacrylic acid (1.51 g) as yellow crystals.

m.p. 90.0-92.0°C.

$^1$H-NMR (300 MHz, $CDCl_3$) δ 0.93 (3H, t, J=7.5Hz), 1.03 (6H, d, J=6.3 Hz), 1.33-1.45 (2H, m), 1.56-1.66 (2H, m), 2.03 (3H, d, J=1.2 Hz), 2.38-2.50 (2H, m), 3.52-3.60 (4H, m), 3.80 (2H, t, J=4.8 Hz), 3.85-4.00 (2H, m), 4.15 (2H, t, J=4.8 Hz), 7.00 (2H, d, J=8.7 Hz), 7.40 (2H, d, J=8.7 Hz), 7.75 (1H, s), 7.80 (1H, s), 8.44 (1H, s).

Elementary analysis $C_{27}H_{36}N_2O_4 \cdot 0.25H_2O$, Calcd. C, 70.95 ; H, 8.05 ; N, 6.13 : Found C, 71.17 ; H, 7.67 ; N, 6.08.

Reference Example 168

To a solution of hydrazine monohydrate (9.66 g) in ethanol (100 ml) was slowly added dropwise ethyl glycolate (20.09 g) at room temperature while the temperature of the reaction system was maintained at 10°C or lower. After stirring the mixture for 4 hours at room temperature, propyl isothiocyanate (20 ml) was slowly added dropwise thereto while the temperature of the reaction system was maintained at 10°C or lower. After stirring for 64 hours at 40°C, the resulting mixture was cooled to room temperature, and ice water (50 ml) was added thereto. The mixture was stirred for 15 minutes and a 5 N aqueous sodium hydroxide solution (40 ml) was then added thereto, which was stirred for 4 hours at 60°C. Concentrated hydrochloric acid was added dropwise thereto at 0°C until the pH reached 6, and the precipitated crystals were removed by filtration. After concentration under reduced pressure, the precipitated crystals were collected by filtration. The crystals were washed with water to give 3-hydroxymethyl-5-mercapto-4-propyl-4H-1,2,4-triazole (23.45 g) as colorless crystals. To a mixture of 90% nitric acid (18 ml) and water (26 ml) was added sodium nitrite (0.07 g), followed by slowly adding 3-hydroxymethyl-5-mercapto-4-propyl-4H-1,2,4-triazole (10 g) at

45°C over 0.5 hour. After cooling to room temperature, sodium carbonate was slowly added thereto at 0°C until the pH reached 7. After concentration under reduced pressure, methanol was added thereto, and the precipitates were removed by filtration. After concentration under reduced pressure, dichloromethane was added thereto, and the precipitates were removed by filtration. The filtrate was concentrated to give 3-hydroxymethyl-4-propyl-4H-1,2,4-triazole (5.95 g) as a crude product. To 3-hydroxymethyl-4-propyl-4H-1,2,4-triazole (5.95 g) was slowly added thionyl chloride (40 ml) at 0°C. The mixture was heated under reflux for 1 hour, and then concentrated under reduced pressure. To the residue was added ethanol, and further concentrated. To the residue was added ethyl acetate and a small amount of ethanol, and the precipitated crystals were collected by filtration. The crystals were washed with ethyl acetate to give 3-chloromethyl-4-propyl-4H-1,2,4-triazole hydrochloride (5.43 g) as pale yellow crystals.

m.p. 91-94°C.

$^1$H-NMR (200 MHz, $CDCl_3$) δ 0.80 (3H, t, J=7.3 Hz), 1.73-1.94 (2H, m), 4.11 (2H, t, J=7.4 Hz), 5.10 (2H, s), 9.26 (1H, s) .

IR (KBr) 3353, 1574, 1537, 1470, 1331, 1204, 1177, 957 $cm^{-1}$

Elementary analysis $C_6H_{11}N_3Cl_2 \cdot 0.25H_2O$, Calcd. C, 35.93 ; H, 5.78 ; N, 20.95 : Found. C, 36.13 ; H, 5.77 ; N, 21.23.

Reference Example 169

To a solution of aminothiophenol (2.9 g) and triethylamine (14.2 ml) in tetrahydrofuran (70 ml) was added dropwise a solution of 3-(chloromethyl)-4-propyl-4H-1,2,4-triazole hydrochloride (5.0 g) in methanol (30 ml) at 0°C under a nitrogen atmosphere. The mixture was returned to room temperature and stirred for 4 hours, and then the solvent was distilled off under reduced pressure. To the resulting residue was added an aqueous sodium hydrogen carbonate solution, and the mixture was extracted with ethyl acetate three times. The organic layers were combined and dried over magnesium sulfate, and the solvent was distilled off under reduced pressure. The resulting residue was purified by basic silica gel column chromatography (ethyl acetate → methanol : ethyl acetate = 1 : 10) to give 4-[[(4-propyl-4H-1,2,4-triazole-3-yl)methyl]sulfanyl]aniline (5.39 g) as a brown oily material.

$^1$H-NMR (300 MHz, $CDCl_3$) δ 0.98 (3H, t, J=7.2 Hz), 1.77-1.90 (2H, m), 3.74 (2H, br), 3.91 (2H, t, J=7.5 Hz), 4.08 (2H, s), 6.56 (2H, d, J=8.7 Hz), 7.12 (2H, d, J=8.7 Hz), 8.06 (1H, s).

Reference Example 170

A mixture of potassium thiocyanate (119.2 g), dihydroxyacetone dimer (73.9 g) and propylamine hydrochloride (100 g) was portionwise added to a mixed solution of acetic acid (89 ml) and 1-butanol (590 ml). The mixture was stirred at room temperature for 1 day, and then water (118 ml) was added thereto, followed by stirring for 30 minutes. The precipitated solid was collected by filtration, and further washed with water (180 ml) twice and hexane once. The resulting solid was dried under reduced pressure to give 5-hydroxymethyl-2-mercapto-1-propylimidazole (71.2 g) as colorless crystals.

$^1$H-NMR (200 MHz, $CDCl_3$) δ 0.87 (3H, t, J=7.4 Hz), 1.61-1.79 (2H, m), 3.91 (2H, t, J=7.4 Hz), 4.32 (2H, s), 5.26 (1H, br), 6.79 (1H, s), 11.95 (1H, s).

Elementary analysis $C_7H_{12}N_2OS \cdot 0.25H_2O$, Calcd. C, 47.57 ; H, 7.13 ; N, 15.85 ; Found. C, 47.22 ; H, 6.94 ; N, 15.99.

Reference Example 171

To 5.0 M nitric acid (370 ml) was added sodium nitrite (1.14 g), and then 5-hydroxymethyl-2-mercapto-1-propylimidazole (71.0 g) was added at 0°C portionwise. The mixture was returned to room temperature and stirred for 2 hours, followed by adding water (200 ml). Thereto was added potassium carbonate at 0°C to neutralize the mixture. Then, the solvent was distilled off under reduced pressure. Ethanol was added thereto, insolubles were filtered off, and the solvent was then distilled off under reduced pressure. To the resulting residue was added methanol-ethyl acetate, and then basic silica gel was added thereto. The resulting mixture was purified by basic silica gel column chromatography (methanol-ethyl acetate = 1 : 8). The resulting solid was recrystallized from diisopropyl ether-ethyl acetate to give 5-hydroxymethyl-1-propylimidazole (33.6 g) as brown crystals.

$^1$H-NMR (200 MHz, $CDCl_3$) δ 0.96 (3H, t, J=7.4 Hz), 1.76-1.94 (2H, m), 3.97 (2H, t, J=7.2 Hz), 4.63 (2H, s), 6.97 (1H, s), 7.48 (1H, s).

Reference Example 172

To 5-hydroxymethyl-1-propylimidazole (33.0 g) was added thionyl chloride (80 ml) at 0°C portionwise, and

the mixture was heated at 90°C for 30 minutes under a nitrogen atmosphere. The mixture was returned to room temperature, and then the solvent was distilled off under reduced pressure. The resulting residue was dissolved in methanol, and the solvent was again distilled off under reduced pressure. The resulting solid was recrystallized from ethyl acetate to give 5-chloromethyl-1-propylimidazole hydrochloride (43.8 g) as colorless crystals.

$^1$H-NMR (200 MHz, DMSO-$d_6$) δ 0.92 (3H, t, J=7.4 Hz), 1.84-1.95 (2H, m), 4.18 (2H, t, J=7.2 Hz), 5.04 (2H, s), 7. 82 (1H, s), 9.24 (1H, s).

Reference Example 173

4-Aminothiophenol (2.5 g) was dissolved in water (2.5 ml) and isopropanol (10 ml). Triethylamine (5.5 ml) was added thereto, and then the mixture was cooled to -15 to - 10°C. A solution of 5-(chloromethyl)-1-propyl-1H-imidazole hydrochloride (3.9 g) in water (2.5 ml) was added dropwise thereto at -15 to -10°C, and the mixture was stirred at the same temperature for 1 hour. After isopropanol was distilled off under reduced pressure, methyl isobutyl ketone (25 ml) was then added thereto, and the organic layer was washed with water. To the organic layer was added activated carbon (0.1 g), and the mixture was stirred at room temperature for 10 minutes. The organic layer was concentrated and dissolved in methyl isobutyl ketone (30 ml). Separately, di-p-toluoyl-(D)-tartaric acid (7.7 g) was dissolved in a mixed solution of toluene (90 ml) and methyl isobutyl ketone (60 ml), and to the solution was added water (3.6 ml). Then, the above methyl isobutyl ketone solution was slowly added dropwise thereto over 2 hours. After stirring the resulting mixture for 1 hour, aqueous 30% hydrogen peroxide (6.8 g) was added thereto, and the mixture was stirred at room temperature for 24 hours. Methanol (30 ml) was added thereto, and the mixture was stirred at 50°C for 8 hours. Water (30 ml) was added thereto, and the mixture was stirred at room temperature for 5 hours. The precipitated crystals were collected by filtration and washed with water (30 ml) to give (-)-4-{[(1-propyl-1H-imidazol-5-yl)methyl] sulfinyl}phenylamine di-p-toluoyl-D-tartrate monohydrate (7.1 g).

m.p. 134-136°C.

Reference Example 174

A suspension of 5-bromo-2-fluorobenzaldehyde (300 mg), ethyl pyrrolidin-3-yl acetate hydrochloride (401 mg) and sodium carbonate (330 mg) in DMSO (10 ml) and water (5 ml) was stirred for 4 hours at 90°C under a nitrogen atmosphere. After returning to room temperature, water was added thereto and the mixture was extracted with ethyl acetate. The organic layer was washed with water and saturated brine, and dried over magnesium sulfate. The solvent was distilled off under reduced pressure, and then the resulting residue was separated and purified by silica gel column chromatography (hexane : ethyl acetate = 10 : 1 → hexane : ethyl acetate = 3 : 1) to give 5-bromo-2-[3-(2-ethoxy-2-oxoethyl)pyrrolidin-1-yl]benzaldehyde (455 mg) as a yellow oily material.

$^1$H-NMR (300 MHz, $CDCl_3$) δ 1.27 (3H, t, J=6.9 Hz), 1.69-1.79 (1H, m), 2.15-2.30 (1H, m), 2.46-2.49 (2H, m), 2.65-2.80 (1H, m), 3.15-3.21 (1H, m), 3.30-3.60 (3H, m), 4.12-4.19 (2H, m), 6.71 (1H, d, J=9.0 Hz), 7.43 (1H, dd, J=9.0, 2.4 Hz), 7.79 (1H, d, J=2.4 Hz), 9.99 (1H, s).

Reference Example 175

A solution of 5-bromo-2-[3-(2-ethoxy-2-oxoethyl)pyrrolidin-1-yl]benzaldehyde (4.3 g) and tert-butyl 2-(triphenylphosphoranylidene)propanoate (7.4 g) in toluene (200 ml) was refluxed for 8 hours under a nitrogen atmosphere. After returning to room temperature, water was added thereto and the mixture was extracted with ethyl acetate. The organic layer was washed with saturated brine and dried over magnesium sulfate. After distilling off the solvent under reduced pressure, the resulting residue was purified by silica gel column chromatography (hexane : ethyl acetate = 9 : 1 → hexane : ethyl acetate = 7 : 3) to give tert-butyl (2E)-3-[5-bromo-2-[3-(2-ethoxy-2-oxoethyl)pyrrolidin-1-yl]phenyl]-2-methylacrylate (2.64 g) as a brown oily material.

$^1$H-NMR (300 MHz, $CDCl_3$) δ 1.26 (3H, t, J=7.2 Hz), 1.53-1.65 (10H, m), 1.94 (3H, d, J=1.5 Hz), 2.10-2.20 (1H, m), 2.43-2.46 (2H, m), 2.55-2.70 (1H, m), 2.93-2.96 (1H, m), 3.20-3.36 (3H, m), 4.11-4.18 (2H, m), 6.65 (1H, d, J=8.4 Hz), 7.20-7.28 (2H, m), 7.54 (1H, s).

Reference Example 176

A suspension of tert-butyl (2E)-3-[5-bromo-2-[3-(2-ethoxy-2-oxoethyl)pyrrolidin-1-yl]phenyl]-2-methylacrylate (2.5 g), 4-(2-butoxyethoxy)phenylboric acid (1.71 g) and potassium carbonate (1.99 g) in toluene (50 ml), ethanol (5 ml) and water (5 ml) was stirred for 1 hour under an argon atmosphere. Then, tetrakis(triphenylphosphine)palladium (324 mg) was added thereto, and the resulting mixture was refluxed for 5 hours. After returning to room temperature, water was added thereto and the mixture was extracted with ethyl acetate. The organic layer was washed with saturated

brine and dried over magnesium sulfate. The solvent was distilled off under reduced pressure, and then the resulting residue was purified by silica gel column chromatography (hexane : ethyl acetate = 18 : 1 → hexane : ethyl acetate = 4 : 1) to give tert-butyl (2E)-3-[4'-(2-butoxyethoxy)-4-[3-(2-ethoxy-2-oxoethyl)pyrrolidin-1-yl]-1,1'-biphenyl-3-yl]-2-methylacrylate (1.87 g) as a yellow oily material.

$^1$H-NMR (300 MHz, $CDCl_3$) δ 0.93 (3H, t, J=7.2 Hz), 1.27 (3H, t, J=6.3 Hz), 1.33-1.70 (14H, m), 2.01 (3H, d, J=1.2 Hz), 2.10-2.20 (1H, m), 2.46-2.48 (2H, m), 2.60-2.75 (1H, m), 3.00-3.06 (1H, m), 3.20-3.45 (3H, m), 3.55 (2H, t, J=6.6 Hz), 3.80 (2H, t, J=4.5 Hz), 4.11-4.19 (4H, m), 6.84 (1H, d, J=8.4 Hz), 6.96 (2H, d, J=8.7 Hz), 7.33-7.46 (4H, m), 7.69 (1H, s).

Reference Example 177

tert-Butyl (2E)-3-[4'-(2-butoxyethoxy)-4-[3-(2-ethoxy-2-oxoethyl)pyrrolidin-1-yl]-1,1'-biphenyl-3-yl]-2-methylacrylate (1.8 g) was dissolved in ethyl acetate (20 ml). Then, a 4 N hydrochloric acid-ethyl acetate solution (23.9 ml) was added thereto, and the mixture was stirred for 1 day under a nitrogen atmosphere. Water was added thereto at 0°C, and the mixture was extracted with ethyl acetate. The organic layer was washed with saturated brine and dried over magnesium sulfate. After distilling off the solvent under reduced pressure, the resulting solids were washed with hexane to give (2E)-3-[4'-(2-butoxyethoxy)-4-[3-(2-ethoxy-2-oxoethyl)pyrrolidin-1-yl]-1,1'-biphenyl-3-yl]-2-methylacrylic acid (1.42 g) as yellow crystals.

m.p. 115.5-116.5°C.

$^1$H-NMR (300 MHz, $CDCl_3$) δ 0.93 (3H, t, J=7.5 Hz), 1.27 (3H, t, J=7.2 Hz), 1.33-1.80 (5H, m), 2.08 (3H, d, J=1.2 Hz), 2.10-2.21 (1H, m), 2.46-2.49 (2H, m), 2.65-2.80 (1H, m), 3.02-3.07 (1H, m), 3.20-3.45 (3H, m), 3.55 (2H, t, J=6.6 Hz), 3.80 (2H, t, J=5.1 Hz), 4.12-4.19 (4H, m), 6.87 (1H, d, J=8.4 Hz), 6.98 (2H, d, J=8.7 Hz), 7.36-7.47 (4H, m), 7.94 (1H, s).

Elementary analysis $C_{30}H_{39}NO_6$, Calcd. C, 70.70 ; H, 7.71 ; N, 2.75 : Found C, 70.33 ; H, 7.67 ; N, 2.71.

Experimental Example

(1) Cloning of human CCR5 chemokine receptor

Cloning of a CCR5 gene was conducted from human spleen cDNA by a PCR method. Using 0.5 ng of spleen cDNA (Toyobo Co., Ltd., QUICK-Clone cDNA) as a template, the PCR reaction was carried out in a DNA Thermal Cycler 480 (Perkin Elmer) using a TaKaRa EX Taq (Takara Shuzo Co., Ltd.) (reaction conditions: 30 cycles of treatments at 95°C for 1 minute, at 60°C for 1 minute, and at 75°C for 5 minutes) by adding 25 pmol of primers, SEQ ID NO. 1 (sequence length: 34; sequence type: nucleic acid; number of chains: a single chain; topology: linear; sequence kind: other nucleic acid, synthetic DNA) described in Experimental Example (1) of WO 99/32100, and SEQ ID NO. 2 (sequence length: 34; sequence type: nucleic acid; number of chains: a single chain; topology: linear; sequence kind: other nucleic acid, synthetic DNA) described in Experimental Example (1) of WO 99/32100, respectively, which were prepared by referring to the base sequence of the CCR5 gene described by Samson et al. (Biochemistry 35 (11), 3362-3367 (1996)). The PCR products were subjected to agarose gel electrophoresis to collect DNA fragments of about 1.0 kb. Then, the CCR5 gene was cloned using an Original TA Cloning Kit (Funakoshi Co., Ltd.).

(2) Preparation of Plasmid for Expression of human CCR5

The plasmids obtained above were digested with restriction enzymes XbaI (Takara Shuzo Co., Ltd.) and BamHI (Takara Shuzo Co., Ltd.), and subjected to agarose gel electrophoresis to collect DNA fragments of about 1.0 kb. The DNA fragments and a plasmid pcDNA3.1 (Funakoshi Co., Ltd.) for expression in animal cells, which was previously digested with XbaI and BamHI, were mixed and ligated by DNA Ligation Kit Ver.2 (Takara Shuzo Co., Ltd.). Transformation of E. coli JM109 competent cells (Takara Shuzo Co., Ltd.) gave plasmid pCKR5.

(3) Introduction of the Plasmid for Expression of human CCR5 into CHO-K1 cells and Expression thereof

CHO-K1 cells grown in a 750 ml tissue culture flask (Becton Dickinson) using Ham's F12 medium (Nihon Pharmaceutical Co., Ltd.) containing 10% fetal bovine serum (Lifetech Oriental) were collected from the flask by using 0.5 g/L trypsin-0.2 g/L EDTA (Lifetech Oriental). The cells were then washed with PBS (Lifetech Oriental), centrifuged (1000 rpm, 5 minutes), and suspended in PBS. Next, DNA was introduced into the cells using Gene Pulser (Bio-Rad Laboratories Inc.) under the following conditions. Namely, $8 \times 10^6$ cells and 10 μg of plasmid pCKR5 for expression of human CCR5 were added into a cuvette of a 0.4 cm-gap, and electroporation was carried out at an electric voltage of 0.25 kV and a capacitance of 960 μF. Subsequently, the cells were transferred into Ham's F12 medium containing

10% fetal bovine serum, and incubated for 24 hours. The cells were again collected, centrifuged, and then suspended in Ham's F12 medium containing 10% fetal bovine serum and Geneticin (Lifetech Oriental) at a concentration of 500 μg/ml. The suspension of cells was diluted to a concentration of $10^4$ cells/ml, and inoculated on a 96-well plate (Becton Dickinson) to give Geneticin-resistant strains.

Subsequently, the Geneticin-resistant strains were cultured in the 96-well plate (Becton Dickinson), and then CCR5-expressing cells were selected from the resistant strains. Namely, an assay buffer (Ham's F12 medium containing 0.5% BSA, and 20 mM HEPES (Wako Pure Chemical Industries, Ltd., pH 7.2)) containing 200 pM [$^{125}$I]-RANTES (Amersham) as a ligand was added to each well and the binding reaction was carried out at room temperature for 40 minutes. Each well plate containing the cells was washed with ice-cooled PBS, and then to each well was added 1 M NaOH in an amount of 50 μl/well, which was stirred. The cells to which the ligand bound specifically, i.e., CCR5/CHO strains, were selected by measurement of radioactivity by γ-counter.

(4) Evaluation of Compound based on CCR5 antagonist activity

The CCR5/CHO strains were inoculated on a 96-well microplate at a concentration of $5 \times 10^4$ cells/well, respectively and were cultured for 24 hours. After the medium was removed by suction, to each well was added an assay buffer containing a test compound (1 μM), and [$^{125}$I]-RANTES (Amersham) used as a ligand at a concentration of 100 pM. The reaction was carried out at room temperature for 40 minutes. After the assay buffer was removed by suction, each well plate containing the cells were washed with ice-cooled PBS twice. Then, to each well was added 200 μl of MicroScint-20 (Packard Industry Company, Inc.), and the radioactivity was measured with TopCount (Packard Industry Company, Inc.).

According to the method above, inhibitory ratios to CCR5 binding of the test compounds were determined. The results are shown in Table 1.

[Table 1]

| Compound No. | Binding Inhibitory Ratio (%) |
|---|---|
| 17 | 100 |
| 23 | 100 |
| 26 | 90 |
| 29 | 100 |
| 35 | 100 |
| 38 | 93 |
| 39 | 99 |
| 40 | 100 |
| 41 | 100 |
| 42 | 100 |
| 43 | 86 |
| 47 | 96 |
| 48 | 98 |
| 49 | 100 |
| 50 | 100 |
| 57 | 100 |
| 58 | 95 |
| 59 | 89 |
| 60 | 91 |
| 61 | 94 |
| 62 | 100 |

Formulation Example 1 (capsules)

| | | |
|---|---|---|
| (1) | (Ss)-(2E)-3-[4'-(2-butoxyethoxy)-4-(3-carboxypyrrolidin1-yl)-1,1'-biphenyl-3-yl]-2-methyl-N-[4-[[(1-propyl-1H-imidazole-5-yl)methyl]sulfinyl]phenyl]acrylamide | 40 mg |

(continued)

| | | |
|---|---|---|
| (2) | lactose | 61 mg |
| (3) | microcrystalline cellulose | 18 mg |
| (4) | magnesium stearate | 1 mg |
| | contents of 1 capsule | 120 mg |

After mixing (1), (2), (3) and (4), the mixture is filled in gelatin capsules.

Formulation Example 2 (capsules)

| | | |
|---|---|---|
| (1) | (Ss)-(2E)-3-[4'-(2-butoxyethoxy)-4-(3-methylpyrrolidin1-yl)-1,1'-biphenyl-3-yl]-2-methyl-N-[4-[[(1-propyl-1H-imidazole-5-yl)methyl]sulfinyl]phenyl]acrylamide | 40 mg |
| (2) | lactose | 61 mg |
| (3) | microcrystalline cellulose | 18 mg |
| (4) | magnesium stearate | 1 mg |
| | contents of 1 capsule | 120 mg |

After mixing (1), (2), (3) and (4), the mixture is filled in gelatin capsules.

Formulation Example 3 (capsules)

| | | |
|---|---|---|
| (1) | (S)-(2E)-3-[5-[4-(2-butoxyethoxy)phenyl]-2-pyrrolidin-1-ylpyridin-3-yl]-2-methyl-N-[4-[[(1-propyl-1H-imidazole-5-yl)methyl]sulfinyl]phenyl]acrylamide | 40 mg |
| (2) | lactose | 61 mg |
| (3) | microcrystalline cellulose | 18 mg |
| (4) | magnesium stearate | 1 mg |
| | contents of 1 capsule | 120 mg |

After mixing (1), (2), (3) and (4), the mixture is filled in gelatin capsules.

Formulation Example 4 (tablets)

| | | |
|---|---|---|
| (1) | (Ss)-(2E)-3-[4'-(2-butoxyethoxy)-4-(3-methylpyrrolidin-1-yl)-1,1'-biphenyl-3-yl]-2-methyl-N-[4-[[(1-propyl-1H-imidazole-5-yl)methyl]sulfinyl]phenyl]acrylamide | 40 mg |
| (2) | mannitol | 51.2 mg |
| (3) | microcrystalline cellulose | 18 mg |
| (4) | hydroxypropyl cellulose | 3.6 mg |
| (5) | croscarmellose sodium | 6 mg |
| (6) | magnesium stearate | 1.2 mg |
| | 1 tablet | 120 mg |

(1), (2), (3) and (4) are mixed and granulated. To the granules are added (5) and (6), and the mixture is compressed into tablets.

Formulation Example 5 (tablets)

| | | |
|---|---|---|
| (1) | (Ss)-(2E)-3-[4'-(2-butoxyethoxy)-4-(3-carboxypyrrolidin-1-yl)-1,1'-biphenyl-3-yl]-2-methyl-N-[4-[[(1-propyl-1H-imidazole-5-yl)methyl]sulfinyl]phenyl]acrylamide | 40 mg |
| (2) | mannitol | 51.2 mg |
| (3) | microcrystalline cellulose | 18 mg |
| (4) | hydroxypropyl cellulose | 3.6 mg |
| (5) | croscarmellose sodium | 6 mg |
| (6) | magnesium stearate | 1.2 mg |
| | 1 tablet | 120 mg |

(1), (2), (3) and (4) are mixed and granulated. To the granules are added (5) and (6), and the mixture is compressed into tablets.

Formulation Example 6 (tablets)

| | | |
|---|---|---|
| (1) | (S)-(2E)-3-[5-[4-(2-butoxyethoxy)phenyl]-2-pyrrolidin-1-ylpyridin-3-yl]-2-methyl-N-[4-[[(1-propyl-1H-imidazole-5-yl)methyl]sulfinyl]phenyl]acrylamide | 40 mg |
| (2) | mannitol | 51.2 mg |
| (3) | microcrystalline cellulose | 18 mg |
| (4) | hydroxypropyl cellulose | 3.6 mg |
| (5) | croscarmellose sodium | 6 mg |
| (6) | magnesium stearate | 1.2 mg |
| | 1 tablet | 120 mg |

(1), (2), (3) and (4) are mixed and granulated. To the granules are added (5) and (6), and the mixture is compressed into tablets.

Industrial Applicability

The compound represented by formula (I) of the present invention or a salt thereof has strong CCR5 antagonistic activity and improved water solubility, and thus can be used advantageously in prevention and treatment of a variety of HIV infection, for example, AIDS, in humans.

## Claims

**1.** A compound represented by the formula:

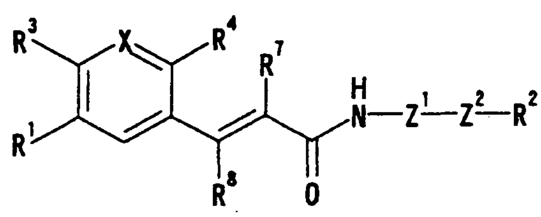

wherein $R^1$ is a 5- or 6-membered ring which may be substituted;

$R^3$ is a hydrogen atom, a lower alkyl group which may be substituted or a lower alkoxy group which may be substituted;

$R^7$ and $R^8$ are each a hydrogen atom or a lower alkyl group which may be substituted;

$Z^1$ is a 5- or 6-membered aromatic ring which may be further substituted;

$Z^2$ is a group represented by $-Z^{2a}-W^1-Z^{2b}-$, wherein $Z^{2a}$ and $Z^{2b}$ are each O, $S(O)_m$ (wherein m is 0, 1 or 2), an imino group which may be substituted, or a bond, and $W^1$ is an alkylene chain which may be substituted, an alkenylene chain which may be substituted, or a bond;

X is N or CR, wherein R represents a hydrogen atom, a lower alkyl group which may be substituted, a lower alkoxy group which may be substituted or an acyl group which may be substituted, or R and the adjacent $R^4$ may form a 5- or 6-membered alicyclic heterocyclic group;

$R^4$ is $NR^5R^6$, wherein $R^5$ and $R^6$ each represent a hydrogen atom, a hydrocarbon group which may be substituted, a heterocyclic group which may be substituted or an acyl group which may be substituted, or $R^5$ and $R^6$ are bonded to each other to form a heterocyclic group which may be substituted represented by $NR^5R^6$; and

$R^2$ is (1) an amino group which may be substituted, in which the nitrogen atom may be converted to a quaternary ammonium or an oxide, (2) a nitrogen-containing heterocyclic group which may be substituted and may contain a sulfur atom or an oxygen atom as the ring-constituting atom, in which the nitrogen atom may be converted to a quaternary ammonium or an oxide, (3) a group represented by the formula:

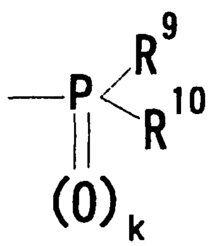

wherein k represents 0 or 1, and when k is 0, the phosphorus atom may form a phosphonium salt; $R^9$ and $R^{10}$ are each a hydrocarbon group which may be substituted, a hydroxy group which may be substituted or an amino group which may be substituted; or $R^9$ and $R^{10}$ may be bonded to each other to form a cyclic group with the adjacent phosphorus atom, (4) an amidino group which may be substituted, or (5) a guanidino group which may be substituted; or a salt thereof.

2. A prodrug of the compound according to claim 1.

3. The compound according to claim 1, wherein $R^1$ is a benzene, a furan, a thiophene, a pyridine, a cyclopentane, a cyclohexane, a pyrrolidine, a piperidine, a piperazine, a morpholine, a thiomorpholine or a tetrahydropyran, each of which may be substituted.

4. The compound according to claim 1, wherein $R^1$ is a benzene which may be substituted.

5. The compound according to claim 1, wherein $NR^5R^6$ is a heterocyclic group which may be substituted.

6. The compound according to claim 1, wherein $Z^1$ is a benzene which may be substituted with a substituent selected from (1) a halogen atom, (2) a $C_{1-4}$ alkyl group which may be substituted with a halogen atom, and (3) a $C_{1-4}$ alkoxy group which may be substituted with a halogen atom.

7. The compound according to claim 1, wherein $Z^1$ is a benzene which may be substituted with a methyl group or a trifluoromethyl group.

8. The compound according to claim 1, wherein $Z^2$ is a group represented by $-Z^{2a}-W^2-Z^{2b}-$, wherein $Z^{2a}$ and $Z^{2b}$ are each O, $S(O)_m$ (wherein m is 0, 1 or 2), an imino group which may be substituted, or a bond, and $W^2$ is an alkylene chain which may be substituted.

9. The compound according to claim 1, wherein $Z^2$ is a group represented by $-CH_2-$, $-CH(OH)-$ or $-S(O)_m-CH_2-$ (wherein m is 0, 1 or 2).

10. The compound according to claim 1, wherein $Z^2$ is a group represented by $-S(O)_m-CH_2-$ (wherein m is 0, 1 or 2).

**11.** The compound according to claim 1, wherein $R^2$ is (1) an amino group which may be substituted, in which the nitrogen atom may be converted to a quaternary ammonium or an oxide, (2) a nitrogen-containing heterocyclic group which may be substituted and may contain a sulfur atom or an oxygen atom as the ring-constituting atom, in which the nitrogen atom may be converted to a quaternary ammonium or an oxide, (3) an amidino group which may be substituted, or (4) a guanidino group which may be substituted.

**12.** The compound according to claim 1, wherein $R^2$ is an amino group which may be substituted, or a nitrogen-containing heterocyclic group which may be substituted and may contain a sulfur atom or an oxygen atom as the ring-constituting atom.

**13.** The compound according to claim 1, wherein $R^2$ is - NRR', wherein R and R' are each an aliphatic hydrocarbon group which may be substituted or an alicyclic heterocyclic group which may be substituted.

**14.** The compound according to claim 1, wherein $R^2$ is a nitrogen-containing aromatic heterocyclic group which may be substituted.

**15.** The compound according to claim 1, wherein $R^2$ is an imidazolyl group which may be substituted or a triazolyl group which may be substituted.

**16.** The compound according to claim 1, wherein $R^1$ is a benzene, a furan, a thiophene, a pyridine, a cyclopentane, a cyclohexane, a pyrrolidine, a piperidine, a piperazine, a morpholine, a thiomorpholine or a tetrahydropyran, each of which may be substituted with a halogen, a nitro, a cyano, a $C_{1-6}$ alkyl, a $C_{1-6}$ alkoxy, a $C_{1-6}$ alkoxy-$C_{1-6}$ alkyl or a $C_{1-6}$ alkoxy-$C_{1-6}$ alkoxy;

$Z^1$ is benzene which may be substituted with a substituent selected from (1) a halogen atom, (2) a $C_{1-4}$ alkyl group which may be substituted with a halogen atom, and (3) a $C_{1-4}$ alkoxy group which may be substituted with a halogen atom;

$Z^2$ is $-Z^{2a}-W^1-Z^{2b}-$, wherein $Z^{2a}$ and $Z^{2b}$ are each O, $S(O)_m$ (wherein m is 0, 1 or 2), an imino group which may be substituted with a $C_{1-4}$ alkyl group, or a bond, and $W^1$ is a bond, or a $C_{1-4}$ alkylene chain or a $C_{2-4}$ alkenylene chain, each of which may be substituted with a $C_{1-6}$ alkyl, a hydroxy group, a hydroxyimino or a $C_{1-6}$ alkoxyimino; and

$R^2$ is an amino group which may be substituted with a $C_{1-4}$ alkyl group, or a nitrogen-containing heterocyclic group which may contain a sulfur atom or an oxygen atom as the ring-constituting atom and may be substituted with a $C_{1-4}$ alkyl group.

**17.** A compound represented by the formula:

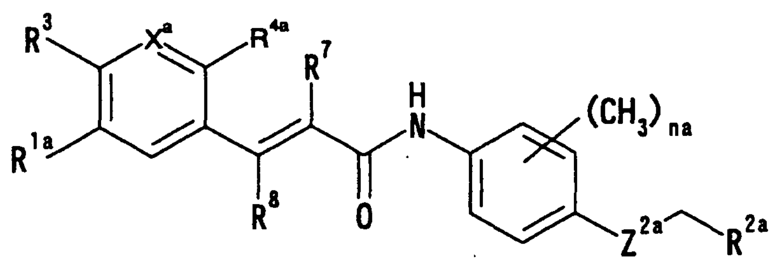

wherein $R^{1a}$ is a ($C_{1-6}$ alkoxy-$C_{1-6}$ alkoxy)phenyl;

$R^{2a}$ is (1) an N-$C_{1-6}$ alkyl-N-tetrahydropyranylamino, (2) an imidazolyl which may be substituted with $C_{1-6}$ alkyl which may be substituted, or (3) a triazolyl which may be substituted with a $C_{1-6}$ alkyl which may be substituted;

$R^3$ is a hydrogen atom, a lower alkyl group which may be substituted or a lower alkoxy group which may be substituted;

$R^{4a}$ is $NR^{5a}R^{6a}$, wherein $R^{5a}$ and $R^{6a}$ are bonded to each other to form a heterocyclic group which may be substituted represented by $NR^{5a}R^{6a}$;

$X^a$ is CH or N;

na is 0 or 1; and

$Z^{2a}$ is a bond, S, SO or $SO_2$;

or a salt thereof.

**18.** The compound according to claim 17, wherein $Z^{2a}$ is SO.

**19.** The compound according to claim 18, wherein $Z^{2a}$ is SO having a configuration of (S).

**20.** The compound according to claim 17, wherein $R^{4a}$ is a 1-pyrrolidinyl group which may be substituted.

**21.** (Ss)-(2E)-3-[4'-(2-butoxyethoxy)-4-(3-methylpyrrolidin-1-yl)-1,1'-biphenyl-3-yl]-2-methyl-N-[4-[[(1-propyl-1H-imidazol-5-yl)methyl]sulfinyl]phenyl]acrylamide, (Ss)-(2E)-3-[4'-(2-butoxyethoxy)-4-[3-(hydroxymethyl)pyrrolidin-1-yl]-1,1'-biphenyl-3-yl]-2-methyl-N-[4-[[(1-propyl-1H-imidazol-5-yl)methyl]sulfinyl]phenyl]acrylamide, (Ss)-(2E)-3-[4'-(2-butoxyethoxy)-4-(3-carboxypyrrolidin-1-yl)-1,1'-biphenyl-3-yl]-2-methyl-N-[4-[[(1-propyl-1H-imidazol)-5-yl]methyl]sulfinyl]phenyl]acrylamide and diastereomers thereof.

**22.** (Ss)-(2E)-3-[5-[4-(2-butoxyethoxy)phenyl]-2-[3-(hydroxymethyl)pyrrolidin-1-yl]pyridin-3-yl]-2-methyl-N-[4-[[(1-propyl-1H-imidazol-5-yl)methyl]sulfinyl]phenyl]acrylamide and a diastereomer thereof, and (S)-(2E)-3-[5-[4-(2-butoxyethoxy)phenyl]-2-pyrrolidin-1-ylpyridin-3-yl]-2-methyl-N-[4-[[(1-propyl-1H-imidzol-5-yl)methyl]sulfinyl]phenyl] acrylamide.

**23.** A process for producing a compound represented by the formula:

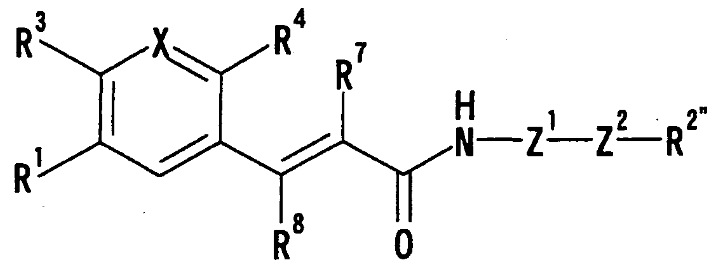

wherein $R^{2"}$ is (1) an amino group which may be substituted, in which the nitrogen atom may be converted to a quaternary ammonium, (2) a nitrogen-containing heterocyclic group which may be substituted and may contain a sulfur atom or an oxygen atom as the ring-constituting atom, in which the nitrogen atom may be converted to a quaternary ammonium, or (3) a group represented by formula:

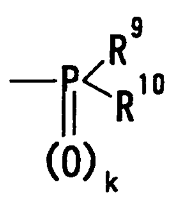

wherein k represents 0 or 1, and when k is 0, the phosphorus atom may form a phosphonium salt; $R^9$ and $R^{10}$ are each a hydrocarbon group which may be substituted, a hydroxy group which may be substituted or an amino group which may be substituted; or $R^5$ and $R^6$ may be bonded to each other to form a cyclic group with the adjacent phosphorus atom; and the other symbols have the same meanings as defined in claim 1; or a salt thereof, which comprises subjecting a compound represented by the formula:

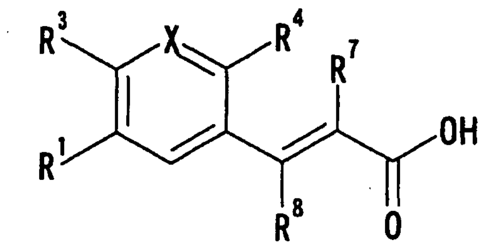

wherein each symbol has the same meaning as defined in claim 1;
or a salt thereof or a reactive derivative thereof, and a compound represented by the formula:

$$H_2N-Z^1-Z^2-R^{2''}$$

wherein $Z^1$ and $Z^2$ have the same meaning as defined in claim 1, and $R^{2''}$ has the same meaning as defined above; or a salt thereof to a condensation reaction, and then optionally to deprotection, oxidation-reduction and/or quaternization reaction.

**24.** A pharmaceutical composition comprising the compound represented by the formula:

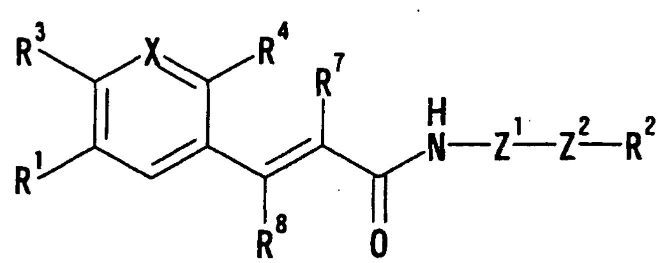

wherein $R^1$ is a 5- or 6-membered ring which may be substituted;

$R^3$ is a hydrogen atom, a lower alkyl group which may be substituted or a lower alkoxy group which may be substituted;

$R^7$ and $R^8$ are each a hydrogen atom or a lower alkyl group which may be substituted;

$Z^1$ is a 5- or 6-membered aromatic ring which may be further substituted;

$Z^2$ is a group represented by $-Z^{2a}-W^1-Z^{2b}-$, wherein $Z^{2a}$ and $Z^{2b}$ are each O, $S(O)_m$ (wherein m is 0, 1 or 2), an imino group which may be substituted, or a bond, and $W^1$ is an alkylene chain which may be substituted, an alkenylene chain which may be substituted, or a bond;

X is N or CR, wherein R represents a hydrogen atom, a lower alkyl group which may be substituted, a lower alkoxy group which may be substituted or an acyl group which may be substituted, or R and the adjacent $R^4$ may form a 5- or 6-membered alicyclic heterocyclic group;

$R^4$ is $NR^5R^6$, wherein $R^5$ and $R^6$ each represent a hydrogen atom, a hydrocarbon group which may be substituted, a heterocyclic group which may be substituted or an acyl group which may be substituted, or $R^5$ and $R^6$ are bonded to each other to form a heterocyclic group which may be substituted represented by $NR^5R^6$; and

$R^2$ is (1) an amino group which may be substituted, in which the nitrogen atom may be converted to a quaternary ammonium or an oxide, (2) a nitrogen-containing heterocyclic group which may be substituted and may contain a sulfur atom or an oxygen atom as the ring-constituting atom, in which the nitrogen atom may be converted to a quaternary ammonium or an oxide, (3) a group represented by the formula:

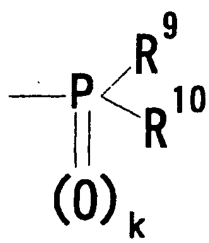

wherein k represents 0 or 1, and when k is 0, the phosphorus atom may form a phosphonium salt; $R^9$ and $R^{10}$ are each a hydrocarbon group which may be substituted, a hydroxy group which may be substituted or an amino group which may be substituted; or $R^9$ and $R^{10}$ may be bonded to each other to form a cyclic group with the adjacent phosphorus atom, (4) an amidino group which may be substituted, or (5) a guanidino group which may be substituted; or a salt thereof or a prodrug thereof.

**25.** The pharmaceutical composition according to claim 24, which is a CCR antagonist.

**26.** The pharmaceutical composition according to claim 25, wherein CCR is CCR5 and/or CCR2.

**27.** The pharmaceutical composition according to claim 25, wherein CCR is CCR5.

**28.** The pharmaceutical composition according to claim 24, which is a prophylactic and/or therapeutic agent for HIV infection, chronic rheumatoid arthritis, autoimmune diseases, allergic diseases, ischemic brain cell disorder, cardiac infarction, nephritis/nephropathy, arteriosclerosis or graft-versus-host diseases.

**29.** The pharmaceutical composition according to claim 24, which is a prophylactic and/or therapeutic agent for HIV infection.

**30.** The pharmaceutical composition according to claim 24, which is a prophylactic and/or therapeutic agent for AIDS.

**31.** The pharmaceutical composition according to claim 24, which is a suppressive agent for disease progression of AIDS.

**32.** A method for preventing or treating HIV infection, chronic rheumatoid arthritis, autoimmune diseases, allergic diseases, ischemic brain cell disorder, cardiac infarction, nephritis/nephropathy, arteriosclerosis or graft-versus-host diseases, which comprises administering an effective amount of the compound according to claim 1, a salt or prodrug thereof to a subject in need thereof.

**33.** Use of the compound according to claim 1, a salt or prodrug thereof, for the manufacture of a prophylactic and/or therapeutic agent for HIV infection, chronic rheumatoid arthritis, autoimmune diseases, allergic diseases, ischemic brain cell disorder, cardiac infarction, nephritis/nephropathy, arteriosclerosis or graft-versus-host diseases.

**INTERNATIONAL SEARCH REPORT**

International application No.
PCT/JP2004/001181

A. CLASSIFICATION OF SUBJECT MATTER
Int.Cl[7] C07D233/64, 401/12, 401/14, 403/12, 491/20, A61K31/4164, 31/4178, 31/4439, 41/454, 31/4709, 31/5377, 31/55, A61P9/00, 9/10, 13/12, 29/00, 31/18, 37/02, 37/04, 37/06, 37/08, 43/00

According to International Patent Classification (IPC) or to both national classification and IPC

B. FIELDS SEARCHED

Minimum documentation searched (classification system followed by classification symbols)
Int.Cl[7] C07D233/64, 401/12, 401/14, 403/12, 491/20, A61K31/4164, 31/4178, 31/4439, 41/454, 31/4709, 31/5377, 31/55, A61P9/00, 9/10, 13/12, 29/00, 31/18, 37/02, 37/04, 37/06, 37/08, 43/00

Documentation searched other than minimum documentation to the extent that such documents are included in the fields searched

Electronic data base consulted during the international search (name of data base and, where practicable, search terms used)
STN/CAS

C. DOCUMENTS CONSIDERED TO BE RELEVANT

| Category* | Citation of document, with indication, where appropriate, of the relevant passages | Relevant to claim No. |
|---|---|---|
| X | WO 99/32100 A2 (TAKEDA CHEMICAL INDUSTRIES, LTD.), 01 July, 1999 (01.07.99), & JP 2000-128782 A & EP 1039899 A & US 6268354 B1 & US 6376536 B1 | 1-31,33 |
| X | WO 00/68203 A1 (TAKEDA CHEMICAL INDUSTRIES, LTD.), 16 November, 2000 (16.11.00), & JP 2001-26586 A & EP 1182195 A | 1-31,33 |
| X | WO 01/41808 A1 (TAKEDA CHEMICAL INDUSTRIES, LTD.), 14 June, 2001 (14.06.01), & JP 2001-302544 A & EP 1236476 A & US 2003/78189 A1 | 1-31,33 |

[X] Further documents are listed in the continuation of Box C.  [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 April, 2004 (08.04.04) | 27 April, 2004 (27.04.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

| International application No. |
|---|
| PCT/JP2004/001181 |

C (Continuation). DOCUMENTS CONSIDERED TO BE RELEVANT

| Category* | Citation of document, with indication, where appropriate, of the relevant passages | Relevant to claim No. |
|---|---|---|
| P,X | JP 2004-2402 A (TAKEDA CHEMICAL INDUSTRIES, LTD.), 08 January, 2004 (08.01.04), (Family: none) | 1-31,33 |
| P,X | JP 2003-335776 A (TAKEDA CHEMICAL INDUSTRIES, LTD.), 28 November, 2003 (28.11.03), (Family: none) | 1-31,33 |

Form PCT/ISA/210 (continuation of second sheet) (January 2004)

**INTERNATIONAL SEARCH REPORT**

International application No.
PCT/JP2004/001181

**Box No. II Observations where certain claims were found unsearchable (Continuation of item 2 of first sheet)**

This international search report has not been established in respect of certain claims under Article 17(2)(a) for the following reasons:

1. [X] Claims Nos.: 32
because they relate to subject matter not required to be searched by this Authority, namely:
The invention as set forth in claim 32 pertains to method for treatment of the human body by therapy

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)